# EUROPEAN PATENT APPLICATION

(11) **EP 4 427 815 A2**
(43) Date of publication of application: **11.09.2024**
(21) Application number: 24176589.0
(22) Date of filing: 16.09.2019
(51) Int. Cl.: A61P 43/00

(54) **DEUTERIUM-ENRICHED PIRFENIDONE AND METHODS OF USE THEREOF**

(30) Priority: 14.09.2018 US 201862731570 P; 25.10.2018 US 201862750377 P; 26.04.2019 US 201962839256 P; 09.08.2019 US 201962884984 P
(62) Divisional of application: 19779319.3
(71) Applicant: PURETECH LYT 100, INC., Boston, Massachusetts 02210 (US)
(72) Inventor: Elenko, Eric, Boston, MA 02110 (US); Chen, Michael C., Norwood, MA 02062 (US); Sabounjian, LuAnn, Holliston, MA 01746 (US)
(74) Representative: Murgitroyd & Company

(57) **Abstract**

Disclosed herein are deuterium-enriched N Aryl pyridinone compounds, optionally in combination with one or more additional therapeutic agents, pharmaceutical compositions comprising the same, methods of preparation thereof, and methods of use thereof. Such compounds and compositions are useful, for example, in treating diseases, disorders, or conditions such as edema.

## Description

### TECHNICAL FIELD

The present invention is directed to substituted, deuterium-enriched *N*-Aryl pyridinones, pharmaceutically acceptable salts and prodrugs thereof, the chemical synthesis thereof, and use of such compounds for the treatment and/or management of a disease, disorder, or condition.

### CROSS-REFERENCE TO RELATED APPLICATIONS

This application claims the benefit of U.S. Provisional Patent Application Nos. 62/731,570, filed on September 14, 2018; 62/750,377, filed on October 25, 2018; 62/839,256, filed on April 26, 2019; and 62/884,984, filed on August 9, 2019; the entirety of each of which is hereby incorporated by reference.

### BACKGROUND OF THE INVENTION

Fibrosis is a common feature of most chronic diseases, and fibrotic disorders are estimated to contribute to 45% of deaths in the United States (Wynn, Nat Rev Immunol. 2004 Aug; 4(8): 583-594). In organs of epithelial origin, such as lung, liver, skin, and kidney, normal cells and tissues are remodeled with scar tissue composed of collagen and other extracellular matrix molecules, ultimately resulting in loss of organ function and finally organ failure. Pulmonary fibrosis, renal fibrosis, and hepatic cirrhosis are among the more common fibrotic diseases, which together represent a large unmet clinical need.

Idiopathic pulmonary fibrosis (IPF) has received much attention due to the severe course of disease. Drug development efforts in IPF have provided insight translatable to other fibrotic disorders and have led to the recognition of commonalities in fibrotic disease of varying origins. For example, the TGF-β pathway is a known a central mediator of the initiation and maintenance of fibrosis in many fibrotic diseases (Friedman et al., Sci Transl Med. 2013 Jan 9;5(167):167sr1). In vitro mechanistic studies, preclinical animal studies, and solid evidence that this pathway is up-regulated in human disease suggest targeting the TGFβ pathway as treatment of fibrosis. A number of agents, including antibodies directed against TGF-beta or other pathway molecules, such as αvβ6 integrin, are currently being developed and assessed in clinical studies for the treatment of diseases such as advanced focal segmental glomerular sclerosis, scleroderma and IPF. However, even though elevated TGF-beta signaling is a critical component of fibrotic disease, the cytokine also carries out important normal homeostatic activities, including immune regulation and tumor suppression, and as a consequence, design of clinical studies must account for and minimize the potential adverse effects of systemic inhibition of TGFβ activity.

Lymphedema is a chronic debilitating disease of fibrotic and inflammatory origin, that in developed countries such as the United States occurs most often as a complication of cancer treatment. In such cases, lymphedema occurs as a result of iatrogenic injury to the lymphatic system, usually as a result of lymph node dissection or biopsy. Large skin excisions and adjuvant therapy with radiation may also cause lymphedema. See, e.g., Szuba et al., Cancer 95:2260-2267 (2002); Tsai et al., Ann. Surg. Oncol. 16: 1959-72 (2009); Purushotham et al., J. Clin. Oncol. 23: 4312-4321 (2005). According to estimates, as many as 1 in 3 patients who undergo lymph node dissection later develop lymphedema. Conservative estimates suggest that as many as 50,000 new patients are diagnosed annually. See, e.g., DiSipio et al., Lancet Oncol. 14:500-515 (2013); Petrek et al., Cancer 83: 2776-2781 (1998). Because lymphedema is a life-long disease with no cure, the number of affected individuals is increasing annually with current estimates ranging between 5-6 million Americans (Rockson et al., Ann. NY Acad. Sci. 1131: 147-154 (2008)), and over 200 million people worldwide. It is likely that this number will continue to increase in the future since the development of lymphedema is nearly linearly related with cancer survivorship, and because the prevalence of known risk factors for lymphedema, such as obesity and radiation, is rising. See, e.g., Erickson et al., J. Natl. Cancer Inst. 93: 96-111 (2001).

Lymphedema is disfiguring and debilitating; patients have chronic swelling of the affected extremity, a sense of heaviness, pain, discomfort, skin damage, fibrosis, recurrent infections, limited mobility, and decreased quality of life. See, e.g., Hayes et al., Cancer 118:2237-2249 (2012Severe symptoms can limit self care. When lymphedema first develops, the skin displays pitting or dimpling, and as the disease progresses, and skin thickening and fibrosis occurs, the skin can have a leathery texture. This non-pitting edema indicates an irreversible stage of lymphedema, in which the has a mossy or cobblestoned (hyperkeratotic) appearance. Adipose deposition is a defining characteristic of late-stage lymphedema. Skin in chronic lymphedema is highly susceptible to fissures and recurrent cellulitis. Concurrent cutaneous ulcerations, bacterial and fungal infections, and impetigo, a skin condition resulting in red sores, are also common. Lymphorrhea, an oozing of lymphatic fluid, is also frequently observed. Over time, elephantiasis nostras verrucosa can develop, leading to severe disfiguration of body parts. Cosmetic deformities resulting from lymphedema are difficult to conceal, and psychosocial stigmatization and low self-esteem, depression, anxiety, and negative body image are common among lymphedema patients because of impaired mobility, difficulty fitting into clothing, and deformity of limbs and genitalia.

Additionally, in patients with chronic lymphedema lasting greater than 10 years there is a 10% risk of developing angiosarcoma, a highly aggressive malignant tumor with a poor prognosis and a 5-year survival rate. Other cancers have been associated with lymphedema as well.

Once lymphedema develops, it is usually progressive. Despite the fact that lymphedema is common and highly morbid, there is currently no cure, and treatment is palliative with a goal of preventing disease progression rather than restoration of lymphatic function. Beaulac et al., Arch. Surg. 137; 1253-1257 (2002). As a result, patients are required to wear tight, uncomfortable garments for the rest of their lives, in an effort to prevent lymphatic fluid buildup in the affected extremity, and to undergo intense and time consuming physical therapy treatments. Koul et al., Int. J. Radiat. Oncol. Biol. Phys., 67:841-846 (2007). In addition, despite on-going chronic care, some patients still have severe progression of their disease with increasing swelling and frequent infections in the lymphedematous limb.

There are currently no approved drug therapies for the treatment of lymphedema. Furthermore, at present, there is no known pharmacologic therapy that can halt progression or promote resolution of lymphedema. Cormier et al., Ann. Surg. Oncol. 19:642-651 (2012). In addition, there has been little progress toward the development of meaningful treatments for lymphatic diseases. Accordingly, development of targeted treatments for lymphedema is therefore an important goal and is an unmet biomedical need.

### SUMMARY OF THE INVENTION

The compounds described herein are designed to target the underlying fibrosis and inflammation found in fibrotic-mediated and/or collagen-mediated disorders. The compounds described herein are designed to target the underlying fibrosis and inflammation found in the lymphatic system to improve lymphatic function and decrease the symptoms of lymphedema and other lymphatic system disorders, such as those described herein.

In one aspect, the invention relates to a method of treating, preventing, and/or ameliorating a disease, disorder, or condition. The method includes administering to a subject in need thereof an effective amount of deuterium-enriched pirfenidone. The deuterium-enriched pirfenidone has the structure shown in Formula I: 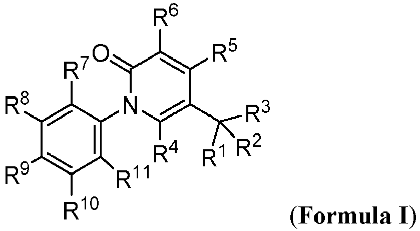 or a pharmaceutically acceptable salt thereof. R¹, R², R³, R⁴, R⁵, R⁶, R⁷, R⁸, R⁹, R¹⁰, and R¹¹ are selected from hydrogen and deuterium. At least one of R¹, R², R³, R⁴, R⁵, R⁶, R⁷, R⁸, R⁹, R¹⁰, and R¹¹ is deuterium. When R⁷, R⁸, R⁹, R¹⁰, and R¹¹ are deuterium, then at least one of R¹, R², R³, R⁴, R⁵, and R⁶ is deuterium.

In some embodiments, at least one of R¹, R², and R³ is deuterium. In some embodiments, at least one of R¹, R², and R³ independently has deuterium enrichment of no less than about 90%. In some embodiments, R¹, R², and R³ are deuterium.

The deuterium-enriched pirfenidone can have the following structure: 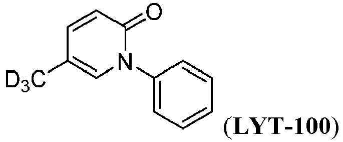 or a pharmaceutically acceptable salt thereof.

In some embodiments, the disease, disorder, or condition is selected from an inflammation-mediated disorder, a fibrotic-meditated disorder, a collagen-mediated disorder, and a fibrotic-mediated and collagen-mediated disorder or a combination of any of these disorders. In some embodiments, the disease, disorder, or condition is selected from idiopathic pulmonary fibrosis, pneumoconiosis, silicosis, chalicosis, asbestosis, anthracosis, lymphedema (primary and/or secondary), systemic sclerosis (scleroderma) or a condition associated with scleroderma, scleroderma interstitial lung disease, focal segmental glomerulosclerosis, juvenile systemic sclerosis (J-SSC), diabetic nephropathy, lupus nephritis, polycystic kidney disease, ANCA vasculitis, membranous nephropathy, minimal change disease, chronic kidney disease, myocardial fibrosis, keloid scar, dermatopolymyositis, fibrotic sarcoidosis, graft-versus-host disease, medical device or implant rejection, fatty liver disease, non-alcoholic steatohepatitis (NASH), and hepatitis-C fibrosis. In some embodiments, the disease, disorder, or condition is selected from neurofibromatosis, Hermansky-Pudlak syndrome, diabetic nephropathy, renal fibrosis, hypertrophic cardiomyopathy (HCM), hypertension-related nephropathy, glomerulosclerosis (FSGS), radiation-induced fibrosis, multiple sclerosis (including secondary progressive multiple sclerosis), uterine leiomyomas (fibroids), alcoholic liver disease selected from hepatic steatosis, hepatic fibrosis, and hepatic cirrhosis, a proliferative disorder selected from an angiogenesis-mediated disorder, a cancer selected from glioma, glioblastoma, breast cancer, colon cancer, melanoma and pancreatic cancer, a fibrotic disorder, an interstitial lung disease, atrial fibrillation (AF), organ transplant rejection, scleroderma and related fibrotic conditions of the skin, endotoxin-induced liver injury after partial hepatectomy or hepatic ischemia, allograft injury after organ transplantation, cystic fibrosis, atrial fibrilation, neutropenia, dermatomyositis, cirrhosis, diffuse parenchymal lung disease, mediastinal fibrosis, tuberculosis, spleen fibrosis caused by sickle-cell anemia, rheumatoid arthritis, systemic sclerosis-related pulmonary fibrosis, sarcoidosis, sarcoidosis-related pulmonary fibrosis, pulmonary fibrosis caused by infection, asbestos-induced pulmonary fibrosis, silica-induced pulmonary fibrosis, environmentally induced pulmonary fibrosis, radiation-induced pulmonary fibrosis, lupus-induced pulmonary fibrosis, drug-induced pulmonary fibrosis, and hypersensitivity pneumonitis, and/or any disorder ameliorated by modulating fibrosis and/or collagen infiltration into tissues.

In some embodiments, the disease, disorder, or condition is selected from idiopathic pulmonary fibrosis, edema (primary and/or secondary), lymphedema (primary and/or secondary), and systemic sclerosis (scleroderma) or a condition associated with scleroderma. In some embodiments, the disease, disorder, or condition is scleroderma and at least one related condition selected from interstitial lung disease, tightening of the skin, joint pain, exaggerated response to cold (Raynaud's disease), and heartburn. In some embodiments, the disease, disorder, or condition is selected from non-alcoholic steatohepatitis (NASH), a fatty liver disease, or Hepatitis-C fibrosis.

The deuterium-enriched pirfenidone can have the structure shown in **Formula I** or a pharmaceutically acceptable salt thereof. In some embodiments, the deuterium-enriched pirfenidone has the structure shown in **Formula I** and is administered orally. In some embodiments, the deuterium-enriched pirfenidone has the structure shown in **Formula I** and is administered locally. In some embodiments, the deuterium-enriched pirfenidone has the structure shown in **Formula I** and is administered orally intravenously.

In some embodiments, the deuterium-enriched pirfenidone has the structure shown as LYT-100: 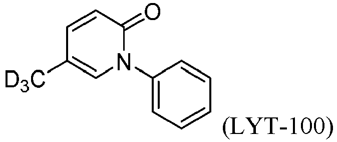 or a pharmaceutically acceptable salt thereof.

In some embodiments, the deuterium-enriched pirfenidone having the following structure: 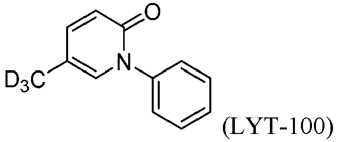 or a pharmaceutically acceptable salt thereof is administered orally.

In some embodiments, the deuterium-enriched pirfenidone having the following structure: 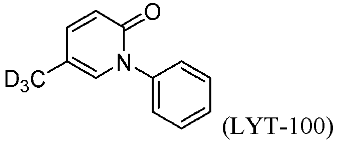 or a pharmaceutically acceptable salt thereof is administered intravenously.

In some embodiments, the deuterium-enriched pirfenidone having the following structure: 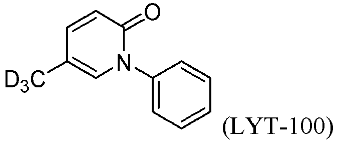 or a pharmaceutically acceptable salt thereof is administered locally.

In some embodiments, the deuterium-enriched pirfenidone having the following structure: 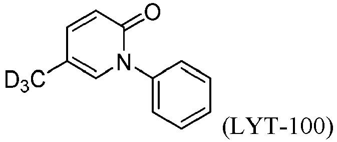 or a pharmaceutically acceptable salt thereof is administered twice daily.

In some embodiments, the deuterium-enriched pirfenidone having the following structure: 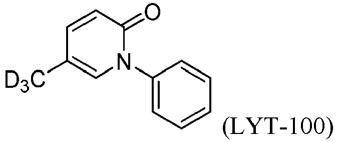 or a pharmaceutically acceptable salt thereof is administered once daily.

In some embodiments, the deuterium-enriched pirfenidone having the following structure: 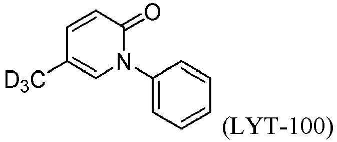 or a pharmaceutically acceptable salt thereof is administered three times daily.

In another aspect, the invention features a method of reducing inflammation and/or fibrosis in a subject having insufficient lymphatic flow. The method includes administering to a subject in need thereof an effective amount of deuterium-enriched pirfenidone having the structure shown in Formula **I:** 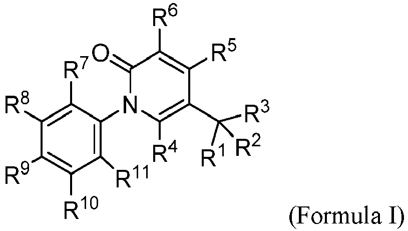 or a pharmaceutically acceptable salt thereof. R¹, R², R³, R⁴, R⁵, R⁶, R⁷, R⁸, R⁹, R¹⁰, and R¹¹ are selected from hydrogen and deuterium. At least one of R¹, R², R³, R⁴, R⁵, R⁶, R⁷, R⁸, R⁹, R¹⁰, and R¹¹ is deuterium. When R⁷, R⁸, R⁹, R¹⁰, and R¹¹ are deuterium, then at least one of R¹, R², R³, R⁴, R⁵, and R⁶ is deuterium.

In some embodiments, at least one of R¹, R², and R³ is deuterium. In some embodiments, at least one of R¹, R², and R³ independently has deuterium enrichment of no less than about 90%. In some embodiments, R¹, R², and R³ are deuterium.

The deuterium-enriched pirfenidone can have the following structure: 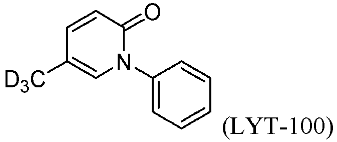 or a pharmaceutically acceptable salt thereof.

In another aspect, the invention features a method of modulating and/or maintaining interstitial fluid balance and/or lymphatic flow in a subject in need thereof. The method includes administering to the subject an effective amount of deuterium-enriched pirfenidone having the structure shown in Formula **I:** 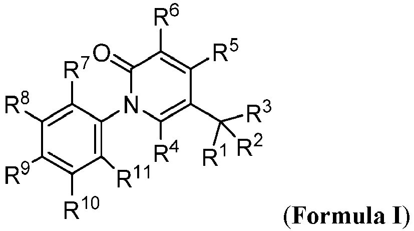 or a pharmaceutically acceptable salt thereof. R¹, R², R³, R⁴, R⁵, R⁶, R⁷, R⁸, R⁹, R¹⁰, and R¹¹ are selected from hydrogen and deuterium. At least one of R¹, R², R³, R⁴, R⁵, R⁶, R⁷, R⁸, R⁹, R¹⁰, and R¹¹ is deuterium. When R⁷, R⁸, R⁹, R¹⁰, and R¹¹ are deuterium, then at least one of R¹, R², R³, R⁴, R⁵, and R⁶ is deuterium.

In some embodiments, at least one of R¹, R², and R³ is deuterium. In some embodiments, at least one of R¹, R², and R³ independently has deuterium enrichment of no less than about 90%. In some embodiments, R¹, R², and R³ are deuterium.

In some embodiments, the deuterium-enriched pirfenidone has the following structure: 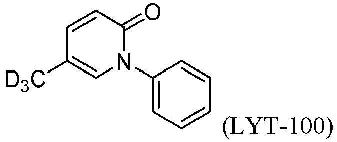 or a pharmaceutically acceptable salt thereof.

In another aspect, the invention relates to a method of treating edema. The method includes administering to a subject in need thereof an effective amount of deuterium-enriched pirfenidone having the structure: 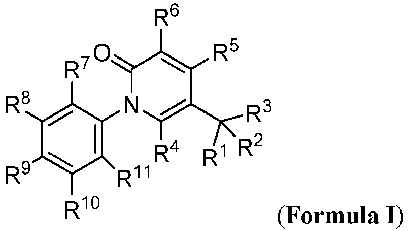 or a pharmaceutically acceptable salt thereof. R¹, R², R³, R⁴, R⁵, R⁶, R⁷, R⁸, R⁹, R¹⁰, and R¹¹ are selected from hydrogen and deuterium. At least one of R¹, R², R³, R⁴, R⁵, R⁶, R⁷, R⁸, R⁹, R¹⁰, and R¹¹ is deuterium. When R⁷, R⁸, R⁹, R¹⁰, and R¹¹ are deuterium, then at least one of R¹, R², R³, R⁴, R⁵, and R⁶ is deuterium. In some embodiments, the deuterium-enriched pirfenidone has the structure: 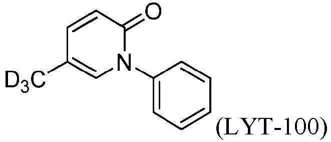 or a pharmaceutically acceptable salt thereof.

In some embodiments, the edema is lymphedema. In some embodiments, the lymphedema is secondary lymphedema. In some embodiments, the lymphedema is primary lymphedema.

In some embodiments, progression of the disease or condition, e.g., edema, is halted in the subject. In some embodiments, there is a stage reduction in the subject. In some embodiments, treating includes decreasing swelling, decreasing inflammation, decreasing fibrosis, decreasing pain, increasing range of motion, decreasing heaviness, decreasing tightness, decreasing skin thickening, and/or improving lymphatic function. In some embodiments, treating includes an improvement in the condition, e.g., edema, as measured by water content, limb volume, tissue firmness, Visual-Analog Scale (VAS) score, Upper Limb Lymphedema score (ULL27), Lymphedema Life Impact Scale (LLIS) score, Functional Assessment of Cancer Therapy breast cancer-specific quality of life tool score (FACT-B +4), lymphedema quality of life score (LYMQOL), Disabilities of the Arm, Shoulder, and Hand score (DASH), and/or Lymphedema Quality of Life Inventory (LQOLI).

The improvement in water content in a subject with edema is a reduction in water content. Thus, in some embodiments, the water content in a limb of a subject with edema is reduced. In some embodiments, the water content in a limb of a subject with edema is reduced by at least 2%, 3%, 4%, 5%, 6%, 7%, 8%, 9%, 10%, 15%, 20%, 25%, 30%, 35%, 40%, 45%, 50%, 60%, 70%, 80%, 90% or more. In some embodiments, the water content in a limb of a subject with edema, for example as measured by bioelectrical impedance spectroscopy (BIS), is reduced by at least 2%, 3%, 4%, 5%, 6%, 7%, 8%, 9%, 10%, 15%, 20%, 25%, 30%, 35%, 40%, 45%, 50%, or more. In some embodiments, the water content in a limb of a subject with edema, for example as measured by Tissue Dielectric Constant (TDC), is reduced by at least 2%, 3%, 4%, 5%, 6%, 7%, 8%, 9%, 10%, 15%, 20%, 25%, 30%, 35%, 40%, 45%, 50%, or more. In some embodiments, limb volume is stabilized or decreased in a subject with edema by at least 2%, 3%, 4%, 5%, 6%, 7%, 8%, 9%, 10%, 15%, 20%, 25%, 30%, 35%, 40%, 45%, 50%, 60%, 70%, 80%, 90%, or more. In some embodiments, limb volume is stabilized or decreased by at least 2%. In some embodiments, tissue firmness in a subject with edema improves by at least 2%, 3%, 4%, 5%, 6%, 7%, 8%, 9%, 10%, 15%, 20%, 30%, 35%, 40%, 45%, 50%, 60%, 70%, 80%, 90% or more. In some embodiments, tissue firmness, as measured by tonometry, improves by 2%, 3%, 4%, 5%, 6%, 7%, 8%, 9%, 10%, 15%, 20%, 30%, 35%, 40%, 45%, 50%, or more. In some embodiments, tissue firmness, for example as measured by tonometry, improves by at least 20%.

In some embodiments, at least one of the positions represented as D independently has deuterium enrichment of no less than about 95%. In some embodiments, at least one of the positions represented as D independently has deuterium enrichment of no less than about 98%. In some embodiments, at least one of the positions represented as D independently has deuterium enrichment of no less than about 99%.

In some embodiments, an effective amount of deuterium-enriched pirfenidone is maintained at a site of lymphedema in the subject.

In some embodiments, the subject has received treatment for cancer, and the lymphedema is associated with the cancer treatment or diagnosis. In some embodiments, the subject has breast cancer-related arm lymphedema. In some embodiments, the subject has mild to moderate breast cancer-related lymphedema. In some embodiments, the subject is receiving or may have received chemotherapy or radiation therapy.

In some embodiments, the deuterium-enriched pirfenidone is administered topically twice a day. In some embodiments, the deuterium-enriched pirfenidone is administered topically once a day. In some embodiments, the deuterium-enriched pirfenidone is administered topically three times a day.

In another aspect, the invention features a method of treating interstitial lung disease (ILD). The method includes administering to a subject in need thereof an effective amount of deuterium-enriched pirfenidone having the structure: 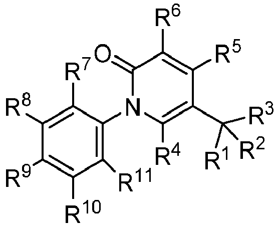 or a pharmaceutically acceptable salt thereof. R¹, R², R³, R⁴, R⁵, R⁶, R⁷, R⁸, R⁹, R¹⁰, and R¹¹ are selected from hydrogen and deuterium. At least one of R¹, R², R³, R⁴, R⁵, R⁶, R⁷, R⁸, R⁹, R¹⁰, and R¹¹ is deuterium. When R⁷, R⁸, R⁹, R¹⁰, and R¹¹ are deuterium, then at least one of R¹, R², R³, R⁴, R⁵, and R⁶ is deuterium. The ILD is treated in the subject.

In some embodiments, the deuterium-enriched pirfenidone has the structure: 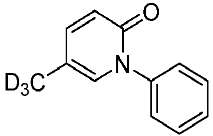 or a pharmaceutically acceptable salt thereof.

In some embodiments, the ILD is idiopathic pulmonary fibrosis (IPF). The deuterium-enriched pirfenidone can be administered orally twice a day, for a total daily dose of 1000 mg. In some embodiments, the initial dosage is titrated from 250 mg to 1000 mg over 2 weeks.

In some embodiments, the subject experiences at least a 5% or 10% reduction in percent predicted forced vital capacity (%FVC).

In another aspect, the invention features a method of treating a fibrotic or collagen infiltration disorder. The method includes administering to a subject in need thereof an effective amount of deuterium-enriched pirfenidone having the structure: 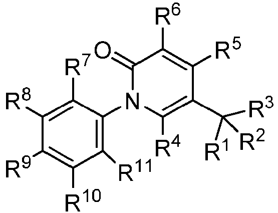 or a pharmaceutically acceptable salt thereof. R¹, R², R³, R⁴, R⁵, R⁶, R⁷, R⁸, R⁹, R¹⁰, and R¹¹ are selected from hydrogen and deuterium. At least one of R¹, R², R³, R⁴, R⁵, R⁶, R⁷, R⁸, R⁹, R¹⁰, and R¹¹ is deuterium. When R⁷, R⁸, R⁹, R¹⁰, and R¹¹ are deuterium, then at least one of R¹, R², R³, R⁴, R⁵, and R⁶ is deuterium. The fibrotic or collagen infiltration disorder is treated in the subject.

In some embodiments, the deuterium-enriched pirfenidone has the structure: 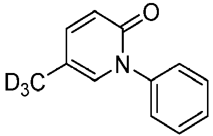 or a pharmaceutically acceptable salt thereof.

In another aspect, the methods include measuring or monitoring a biomarker, e.g., a marker of inflammation, in a subject. In some embodiments, the marker is one or more markers selected from G-CSF, MIG, FGF-2, IL-4, IL-10, lymphotoxin-α/TNF-β, leptin, IL-6, IL-1β, TNF-α, TGF-β1, MMP-9, TIMP-1, and MCP-1. In some embodiments, a biomarker is monitored to monitor the treatment of the subject.

### BRIEF DESCRIPTION OF THE FIGURES

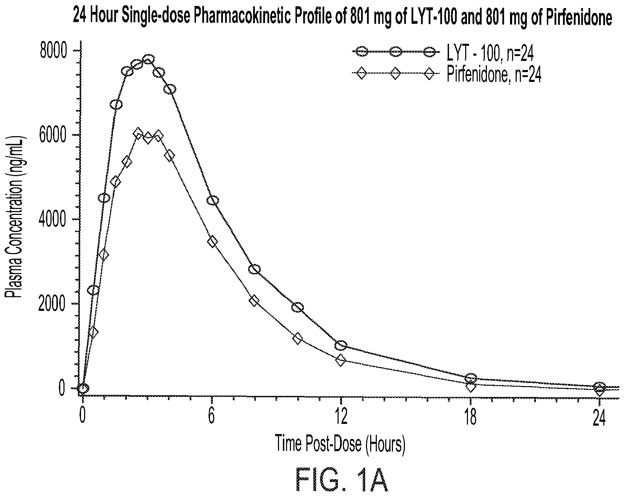

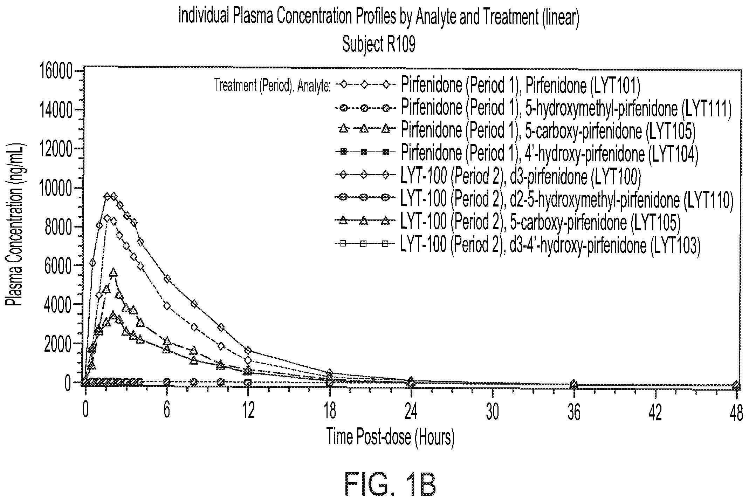

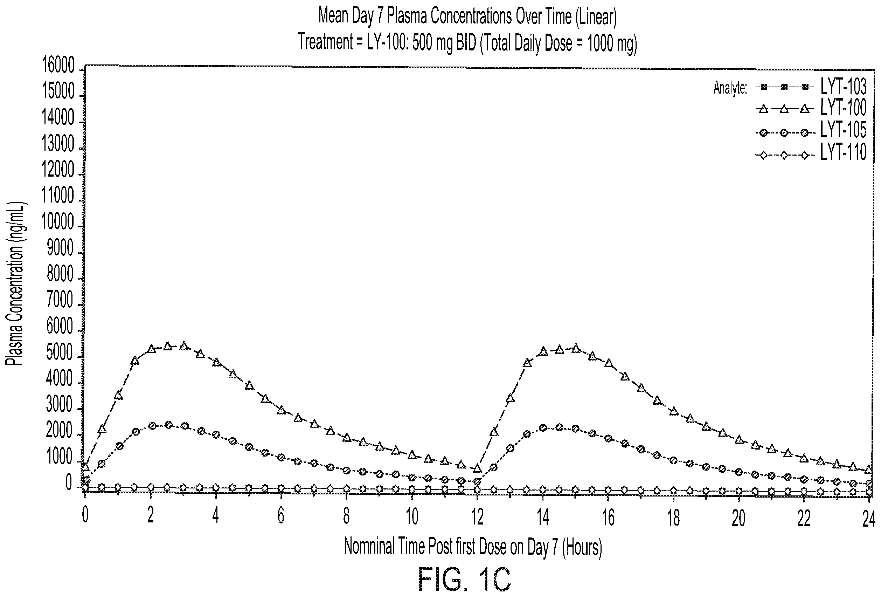

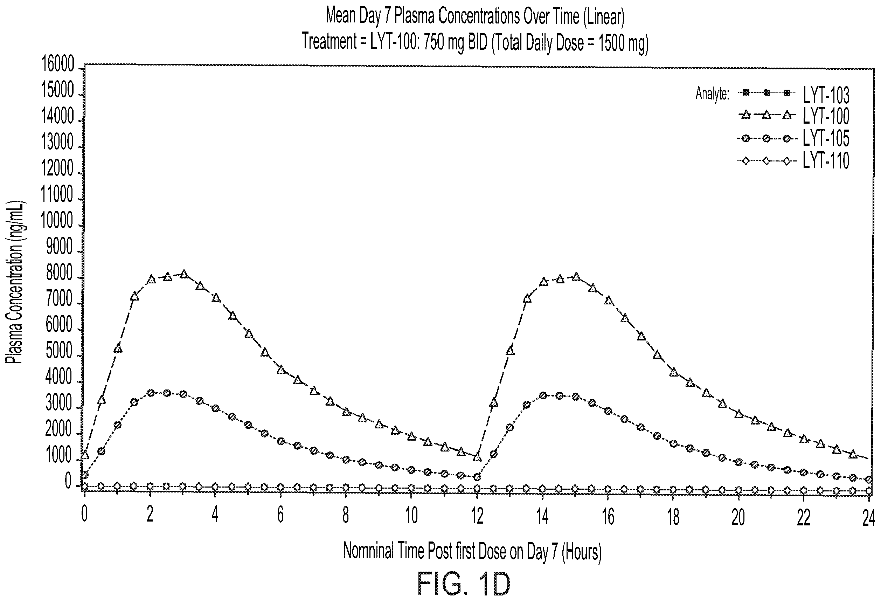

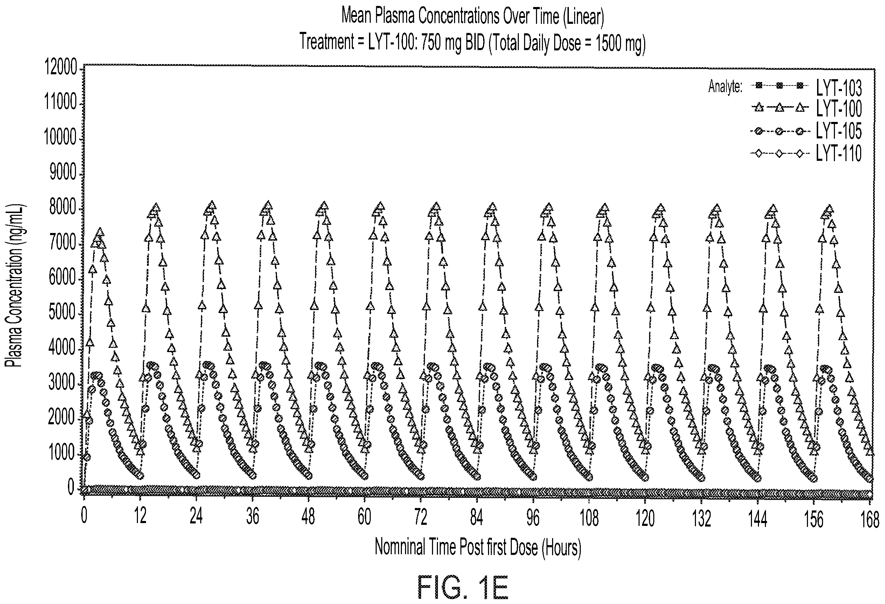

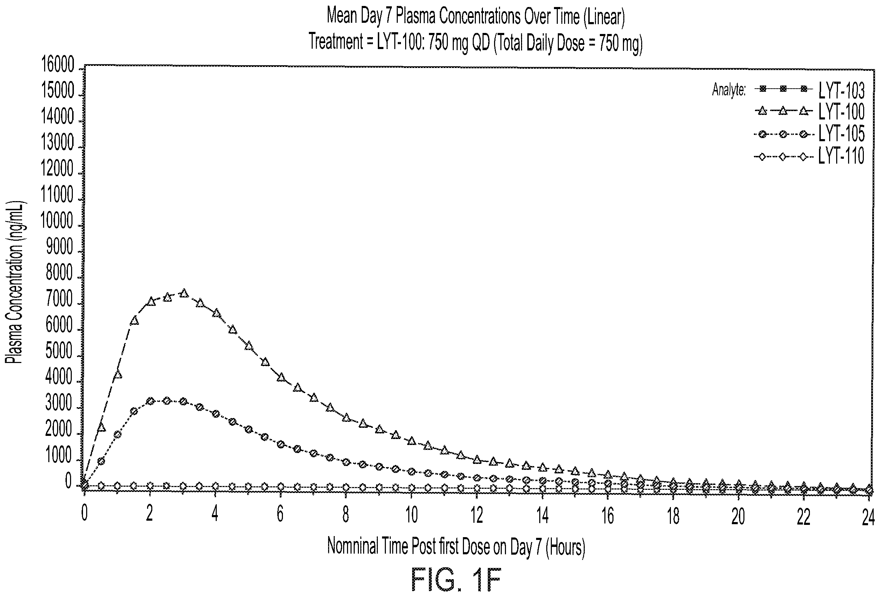

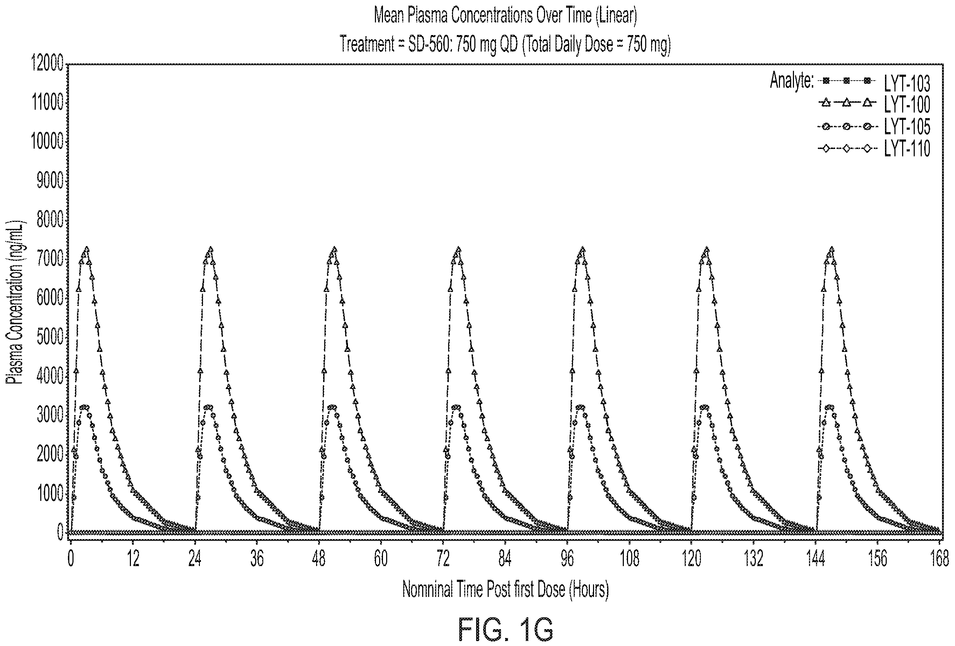

**FIG. 1A** illustrates the single-dose pharmacokinetics of an 801 mg dose of LYT-100 and 801 mg dose of pirfenidone over 24 hours. **FIG. 1B** illustrates an individual's single dose pharmacokinetics of an 801mg dose of LYT-100 and 801 mg dose of pirfenidone over 48 hours. **FIG. 1C** is a model of a 500 mg twice daily dose of LYT-100 (total daily dose of 1000 mg) and its metabolites on day 7. **FIG. 1D** is a model of a 750 mg twice daily dose of LYT-100 (total daily dose of 1500 mg) and its metabolites on day 7. **FIG. 1E** is a model of the first 7 days of the dosing of **FIG. 1D** showing accumulation to steady state. **FIG. 1F** is a model of a 750 mg once daily dose of LYT-100 (total daily dose of 750 mg) and its metabolites on day 7. **FIG. 1G** is a model of the first 7 days of the dosing of **FIG. 1F** showing accumulation to steady state.

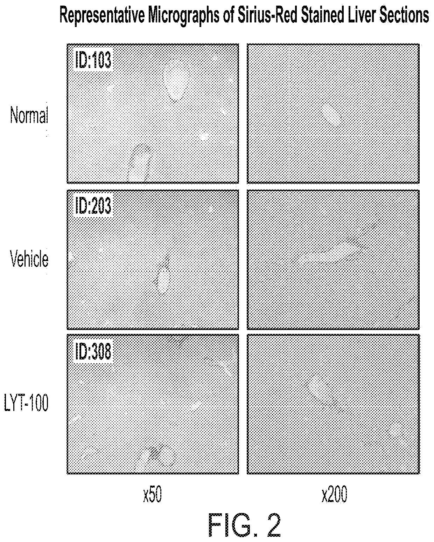

**FIG. 2** depicts representative micrographs of Sirius-red stained liver sections illustrating that LYT-100 significantly reduced the area of fibrosis.

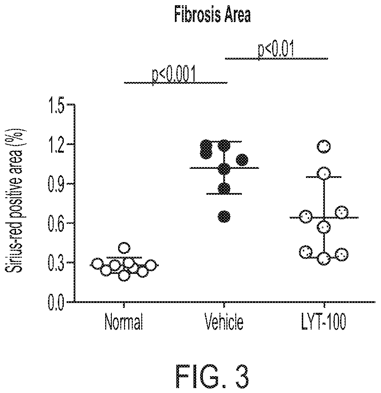

**FIG. 3** illustrates the percent fibrosis area for LYT-100 versus vehicle and control.

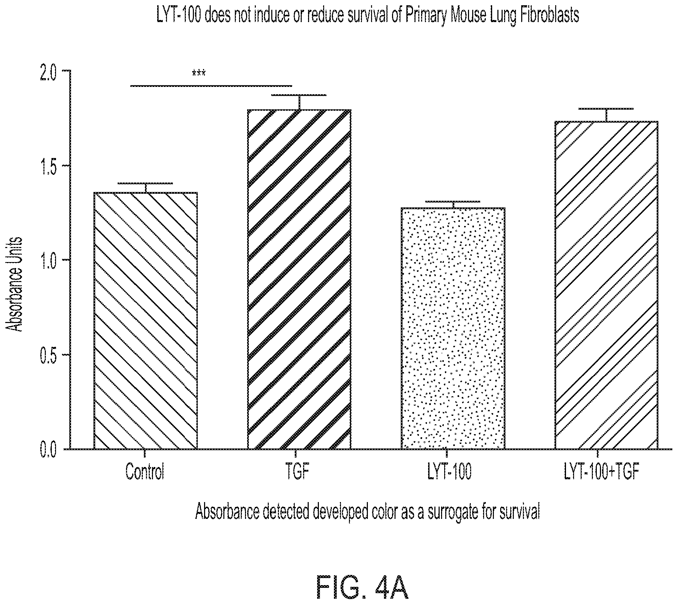

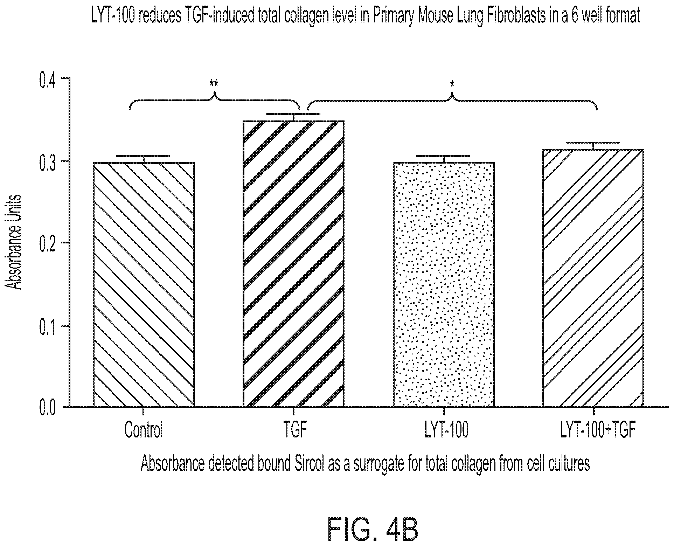

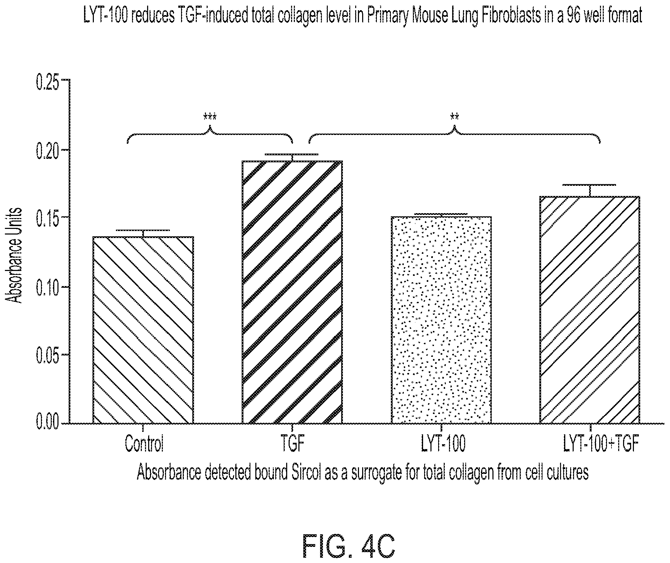

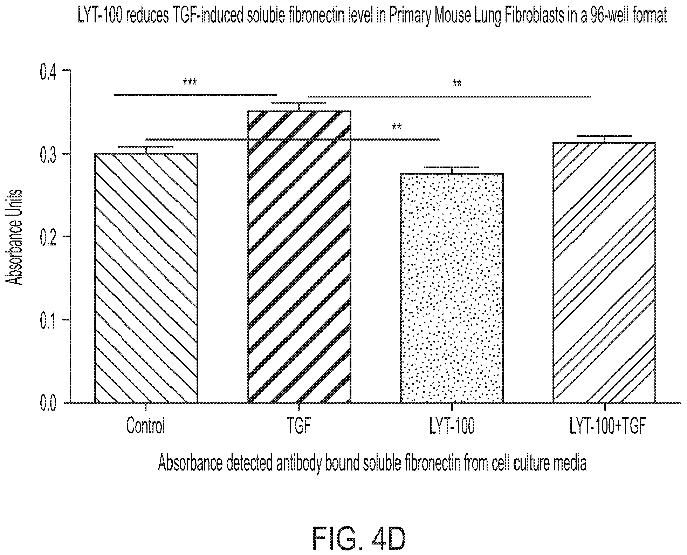

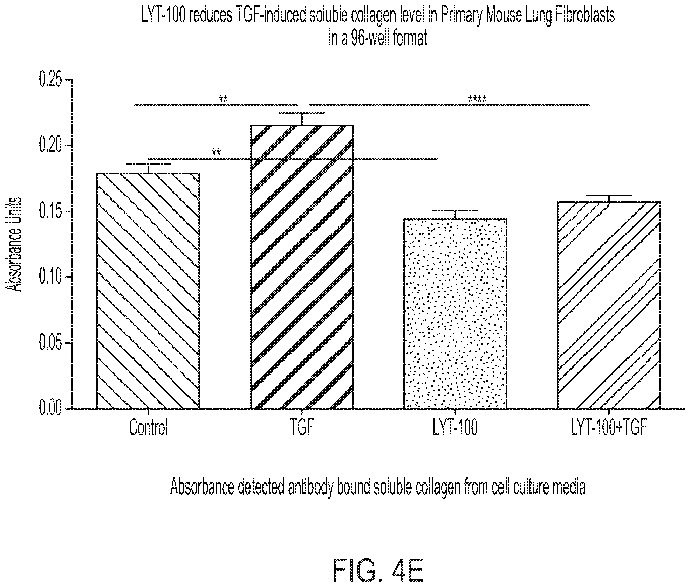

**FIG. 4A** illustrates that LYT-100 does not induce survival of Primary Mouse Lung Fibroblasts (PMFL); **FIG 4B****.** and **FIG. 4C** illustrate LYT-100 reduced TGF-β-induced total collagen level in PMFL a 6 well and 96 well format, respectively; and **FIG. 4D** and **FIG. 4E** illustrate LYT-100 reduced TGF-β-induced soluble fibronectin levels and soluble collagen levels.

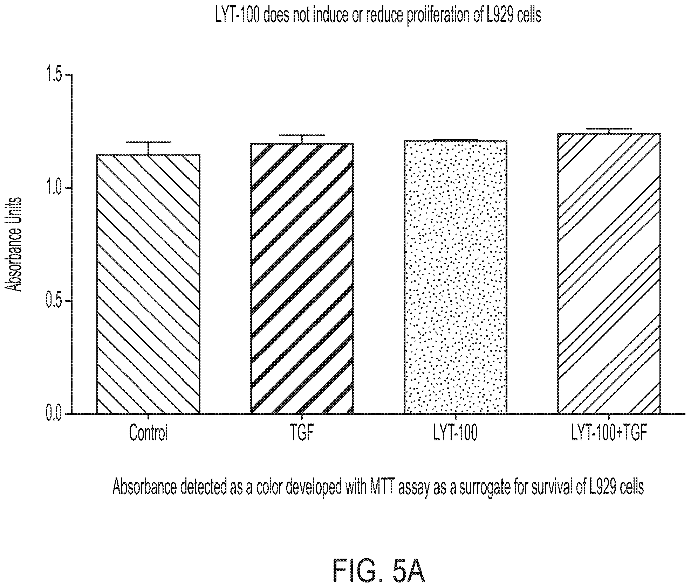

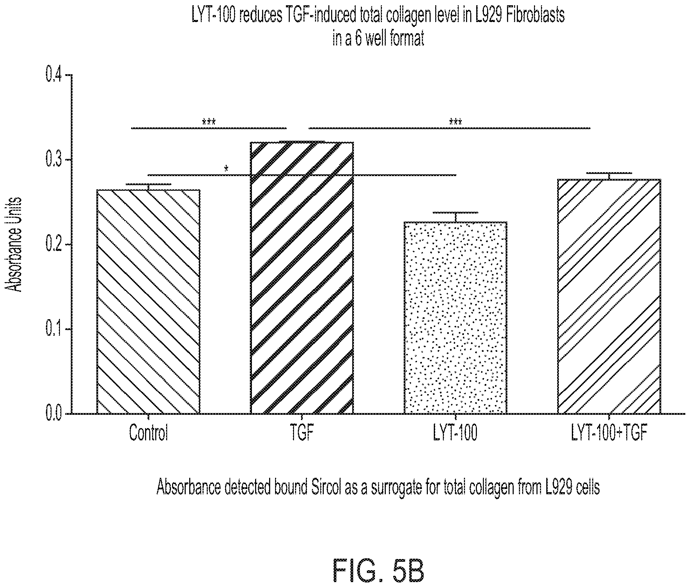

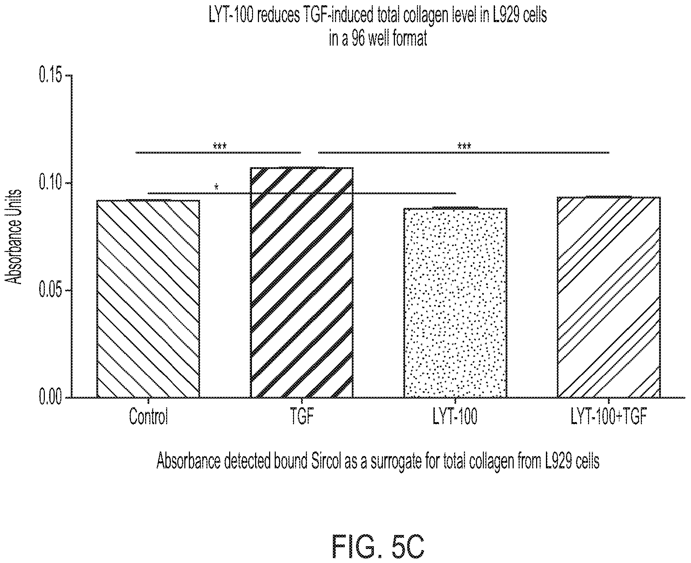

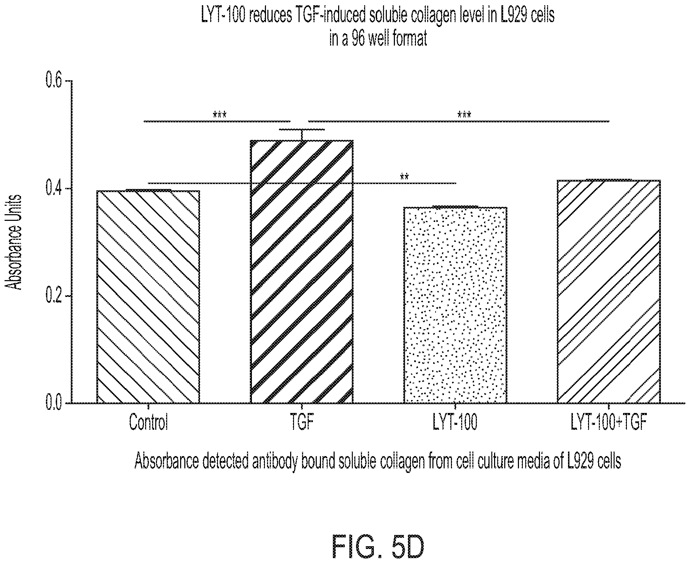

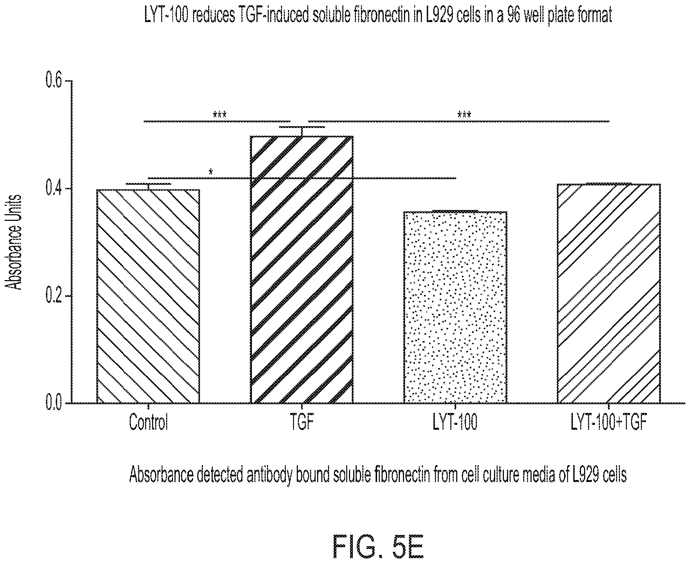

**FIG. 5A** illustrates that LYT-100 does not affect survival of L929 cells. **FIG. 5B****,** illustrates that LYT-100 inhibits TGF-induced collagen synthesis. **FIG. 5C** illustrates that LYT-100 significanly inhibits TGF-β-induced total collagen levels. **FIG. 5D** is a graph illustrating that LYT-100 significantly inhibits TGF-β-induced soluble collagen levels. **FIG. 5E** illustrates that LYT-100 signficantly reduced soluble fibronectin levels, in the absence and presence of TGF-β-induction.

### DETAILED DESCRIPTION OF THE INVENTION

### 1. General Description of Certain Aspects of the Invention

### Deuterium-Enriched Pirfenidone

Certain N-aryl pyridinones of the present invention, including deuterium-enriched pirfenidone compounds, are described in WO 2008/157786, WO 2009/035598, WO 2012/122165, and WO 2015/112701, the entireties of which are hereby incorporated by reference.

Pirfenidone (Deskar^{®}), CAS# 53179-13-8, Pirespa, AMR-69, Pirfenidona, Pirfenidonum, Esbriet, Pirfenex, 5-methyl-1-phenyl-1*H*-pyridin-2-one, 5-Methyl-1-phenyl-2-(1*H*)-pyridone, 5-methyl-1-phenylpyridin-2(1*H*)-one, is an orally administered antifibrotic agent. Pirfenidone is currently approved in the United States and elsewhere for idiopathic pulmonary fibrosis (IPF).

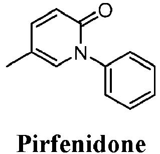

The metabolism of pirfenidone is only partially understood. For example, without wishing to be bound by theory, the methyl group is thought to be susceptible to oxidation, which would lead to a corresponding hydroxymethyl metabolite, "M1." M1 is thought to be further oxidized to a carboxylic acid metabolite, "M2" (Wang et al., Biomedical Chromatography 2006, 20, 1375-1379). A third detected metabolite is believed to be a phase II product possibly originating from M1 or M2. Pirfenidone has a very short half-life in humans.

### Deuterium Kinetic Isotope Effect

In order to eliminate foreign substances from their circulation system, animal tissues express various enzymes, such as the cytochrome P₄₅₀ enzymes or CYPs, esterases, proteases, reductases, dehydrogenases, and monoamine oxidases, to react with and convert these foreign substances to more polar intermediates or metabolites for renal excretion. Some of the most common metabolic reactions of pharmaceutical compounds involve the oxidation of a carbon-hydrogen (C-H) bond to either a carbon-oxygen (C-O) or carbon-carbon (C-C) pi-bond. The resultant metabolites may be stable or unstable under physiological conditions, and can have substantially different pharmacokinetic, pharmacodynamic, and acute and long-term toxicity profiles relative to the parent compounds. For most drugs, such oxidations are generally rapid and ultimately require administration of multiple or high daily doses to maintain therapeutically-effective levels of the drugs in patients.

The relationship between the activation energy and the rate of reaction may be quantified by the Arrhenius equation, k=Ae^{-Eact}/RT, where E_{act} is the activation energy, T is temperature, R is the molar gas constant, k is the rate constant for the reaction, and A (the frequency factor) is a constant specific to each reaction that depends on the probability that the molecules will collide with the correct orientation. The Arrhenius equation states that the fraction of molecules that have enough energy to overcome an energy barrier, that is, those with energy at least equal to the activation energy, depends exponentially on the ratio of the activation energy to thermal energy (RT), the average amount of thermal energy that molecules possess at a certain temperature.

The transition state in a reaction is a short lived state (on the order of 10⁻¹⁴ sec) along the reaction pathway during which the original bonds have stretched to their limit. By definition, the activation energy E_{act} for a reaction is the energy required to reach the transition state of that reaction. Reactions that involve multiple steps will necessarily have a number of transition states, and in these instances, the activation energy for the reaction is equal to the energy difference between the reactants and the most unstable transition state. Once the transition state is reached, the molecules can either revert, thus reforming the original reactants, or the new bonds form giving rise to the products. This dichotomy is possible because both pathways, forward and reverse, result in the release of energy. A catalyst facilitates a reaction process by lowering the activation energy leading to a transition state. Enzymes are examples of biological catalysts that reduce the energy necessary to achieve a particular transition state.

A carbon-hydrogen bond is by nature a covalent chemical bond. Such a bond forms when two atoms of similar electronegativity share some of their valence electrons, thereby creating a force that holds the atoms together. This force or bond strength can be quantified and is expressed in units of energy, and as such, covalent bonds between various atoms can be classified according to how much energy must be applied to the bond in order to break the bond or separate the two atoms.

The bond strength is directly proportional to the absolute value of the ground-state vibrational energy of the bond. This vibrational energy, which is also known as the zero-point vibrational energy, depends on the mass of the atoms that form the bond. The absolute value of the zero-point vibrational energy increases as the mass of one or both of the atoms making the bond increases. Since deuterium (D) is two-fold more massive than hydrogen (H), it follows that a C-D bond is stronger than the corresponding C-H bond. Compounds with C-D bonds are frequently indefinitely stable in H₂O, and have been widely used for isotopic studies. If a C-H bond is broken during a rate-determining step in a chemical reaction (i.e. the step with the highest transition state energy), then substituting a deuterium for that hydrogen will cause a decrease in the reaction rate and the process will slow down. This phenomenon is known as the Deuterium Kinetic Isotope Effect (DKIE) and can range from about 1 (no isotope effect) to very large numbers, such as 50 or more, meaning that the reaction can be fifty, or more, times slower when deuterium is substituted for hydrogen. High DKIE values may be due in part to a phenomenon known as tunneling, which is a consequence of the uncertainty principle. Tunneling is ascribed to the small size of a hydrogen atom, and occurs because transition states involving a proton can sometimes form in the absence of the required activation energy. A deuterium is larger and statistically has a much lower probability of undergoing this phenomenon. Substitution of tritium for hydrogen results in yet a stronger bond than deuterium and gives numerically larger isotope effects.

Discovered in 1932 by Urey, deuterium (D) is a stable and non-radioactive isotope of hydrogen. It was the first isotope to be separated from its element in pure form and is twice as massive as hydrogen, and makes up about 0.02% of the total mass of hydrogen (in this usage meaning all hydrogen isotopes) on earth. When two deuteriums bond with one oxygen, deuterium oxide (D₂O or "heavy water") is formed.

### Deuterated Pyridinone Derivatives

Pirfenidone is a substituted pyridinone-based fibrosis modulator and/or collagen infiltration modulator. The carbon-hydrogen bonds of pirfenidone contain a naturally occurring distribution of hydrogen isotopes, namely ¹H or protium (about 99.9844%), ²H or deuterium (about 0.0156%), and ³H or tritium (in the range between about 0.5 and 67 tritium atoms per 10¹⁸ protium atoms). Increased levels of deuterium incorporation may produce a detectable Kinetic Isotope Effect (KIE) that could affect the pharmacokinetic, pharmacologic and/or toxicologic profiles of such fibrosis modulators and/or collagen-infiltration modulators in comparison with the compound having naturally occurring levels of deuterium.

Pirfenidone is likely metabolized in humans by oxidation of the methyl group. Other sites on the molecule may also undergo transformations leading to metabolites with as-yet-unknown pharmacology/toxicology. Limiting the production of these metabolites has the potential to decrease the danger of the administration of such drugs and may even allow increased dosage and concomitant increased efficacy. All of these transformations can occur through polymorphically-expressed enzymes, thus exacerbating the interpatient variability.

Various deuteration patterns can be used to a) reduce or eliminate unwanted metabolites, b) increase the half-life of the parent drug, c) decrease the number of doses needed to achieve a desired effect, d) decrease the amount of a dose needed to achieve a desired effect, e) increase the formation of active metabolites, if any are formed, and/or f) decrease the production of deleterious metabolites in specific tissues and/or create a more effective drug and/or a safer drug for polypharmacy, whether the polypharmacy be intentional or not. The deuteration approach has strong potential to slow the metabolism via various oxidative and racemization mechanisms.

In one aspect, the present invention provides deuterium-enriched pirfenidone having the structure shown in Formula **I:** 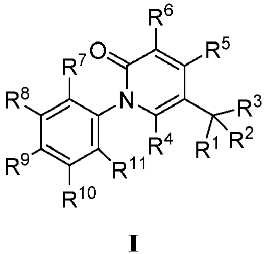 or a pharmaceutically acceptable salt thereof, wherein:

R¹, R², R³, R⁴, R⁵, R⁶, R⁷, R⁸, R⁹, R¹⁰, and R¹¹ are selected from hydrogen and deuterium; and at least one of R¹, R², R³, R⁴, R⁵, R⁶, R⁷, R⁸, R⁹, R¹⁰, and R¹¹ is deuterium. In some embodiments, when R⁷, R⁸, R⁹, R¹⁰, and R¹¹ are deuterium, then at least one of R¹, R², R³, R⁴, R⁵, and R⁶ is deuterium.

In some embodiments, at least one of R¹, R², R³, R⁴, R⁵, R⁶, R⁷, R⁸, R⁹, R¹⁰, and R¹¹ independently has deuterium enrichment of no less than about 1%, no less than about 5%, no less than about 10%, no less than about 20%, no less than about 50%, no less than about 70%, no less than about 80%, no less than about 90%, no less than about 95%, no less than about 96%, no less than about 97 %, no less than about 98%, no less than about 99%, no less than about 99.1%, no less than about 99.2%, no less than about 99.3%, no less than about 99.4%, no less than about 99.5%, no less than about 99.6%, no less than about 99.7%, no less than about 99.8%, or no less than about 99.9%. In some embodiments, including any of the deuterium-enriched pirfenidone compounds described below and elsewhere herein, at least one of R¹, R², R³, R⁴, R⁵, R⁶, R⁷, R⁸, R⁹, R¹⁰, and R¹¹ independently has deuterium enrichment of 90%, 91%, 92%, 93%, 94%, 95%, 96%, 97 %, 98%, 99%, 99.1%, 99.2%, 99.3%, 99.4%, 99.5%, 99.6%, 99.7%, 99.8%, 99.9%, 100% or any incremental numerical fraction within the stated deuterium enrichments.

In yet another embodiment, at least one of R¹, R², and R³ is deuterium.

In yet another embodiment, at least two of R¹, R², and R³ are deuterium.

In yet another embodiment, R¹, R², and R³ are deuterium.

In yet another embodiment, R⁴ is deuterium.

In yet another embodiment, at least one of R⁵ and R⁶ is deuterium.

In yet another embodiment, R⁵ and R⁶ are deuterium.

In yet another embodiment, R⁵ and R⁶ are deuterium; and at least one of R¹, R², R³, R⁴, R⁷, R⁸, R⁹, R¹⁰, and R¹¹ is deuterium.

In yet another embodiment, at least one of R⁷, R⁸, R⁹, R¹⁰, and R¹¹ is deuterium.

In yet another embodiment, R⁷, R⁸, R⁹, R¹⁰, and R¹¹ are deuterium.

In yet another embodiment, R⁷, R⁸, and R⁹, are deuterium, and at least one of R¹, R², R³, R⁴, R⁵, R⁶, R¹⁰, and R¹¹ is deuterium.

In yet another embodiment, at least one of R¹, R², and R³ is deuterium; and R⁴, R⁵, R⁶, R⁷, R⁸, R⁹, R¹⁰, and R¹¹ are hydrogen.

In yet another embodiment, at least two of R¹, R², and R³ is deuterium; and R⁴, R⁵, R⁶, R⁷, R⁸, R⁹, R¹⁰, and R¹¹ are hydrogen.

In yet another embodiment, R¹, R², and R³ are deuterium; and R⁴, R⁵, R⁶, R⁷, R⁸, R⁹, R¹⁰, and R¹¹ are hydrogen.

In yet another embodiment, R⁴ is deuterium; and R¹, R², R³, R⁵, R⁶, R⁷, R⁸, R⁹, R¹⁰, and R¹¹ are hydrogen.

In yet another embodiment, at least one of R⁵ and R⁶ is deuterium; and R¹, R², R³, R⁴, R⁷, R⁸, R⁹, R¹⁰, and R¹¹ are hydrogen.

In yet another embodiment, R⁵ and R⁶ are deuterium; and R¹, R², R³, R⁴, R⁷, R⁸, R⁹, R¹⁰, and R¹¹ are hydrogen.

In yet another embodiment, at least one of R¹, R², R³, R⁴, R⁵, and R⁶ is deuterium; and R⁷, R⁸, R⁹, R¹⁰, and R¹¹ are hydrogen.

In yet another embodiment, R¹, R², R³, R⁴, R⁵, and R⁶ are deuterium; and R⁷, R⁸, R⁹, R¹⁰, and R¹¹ are hydrogen.

In yet another embodiment, at least one of R⁷, R⁸, R⁹, R¹⁰, and R¹¹ is deuterium; and R¹, R², R³, R⁴, R⁵, and R⁶ are hydrogen.

In yet another embodiment, R⁷, R⁸, R⁹, R¹⁰, and R¹¹ are deuterium; and at least one of R¹, R², R³, R⁴, R⁵, and R⁶ is deuterium.

In other embodiments, R¹ is hydrogen. In yet other embodiments, R² is hydrogen. In still other embodiments, R³ is hydrogen. In yet other embodiments, R⁴ is hydrogen. In some embodiments, R⁵ is hydrogen. In yet other embodiments, R⁶ is hydrogen. In still other embodiments, R⁷ is hydrogen. In still other embodiments, R⁸ is hydrogen. In some embodiments, R⁹ is hydrogen. In other embodiments, R¹⁰ is hydrogen. In yet other embodiments, R¹¹ is hydrogen.

In other embodiments, R¹ is deuterium. In yet other embodiments, R² is deuterium. In still other embodiments, R³ is deuterium. In yet other embodiments, R⁴ is deuterium. In some embodiments, R⁵ is deuterium. In yet other embodiments, R⁶ is deuterium. In still other embodiments, R⁷ is deuterium. In still other embodiments, R⁸ is deuterium. In some embodiments, R⁹ is deuterium. In other embodiments, R¹⁰ is deuterium. In yet other embodiments, R¹¹ is deuterium.

In some embodiments, the deuterium-enriched pirfenidone is LYT-100, or a pharmaceutically acceptable salt thereof. LYT-100 has the following structure:

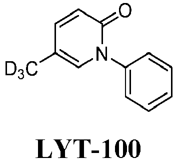

In some embodiments, the deuterium-enriched pirfenidone is a compound or pharmaceutically acceptable salt thereof, or a metabolite thereof, described in WO 2008/157786, WO 2009/035598, WO 2012/122165, or WO 2015/112701, the entireties of which are hereby incorporated by reference.

In one aspect, the present invention provides a deuterium-enriched compound shown in **Table 1,** or a pharmaceutically acceptable salt thereof:

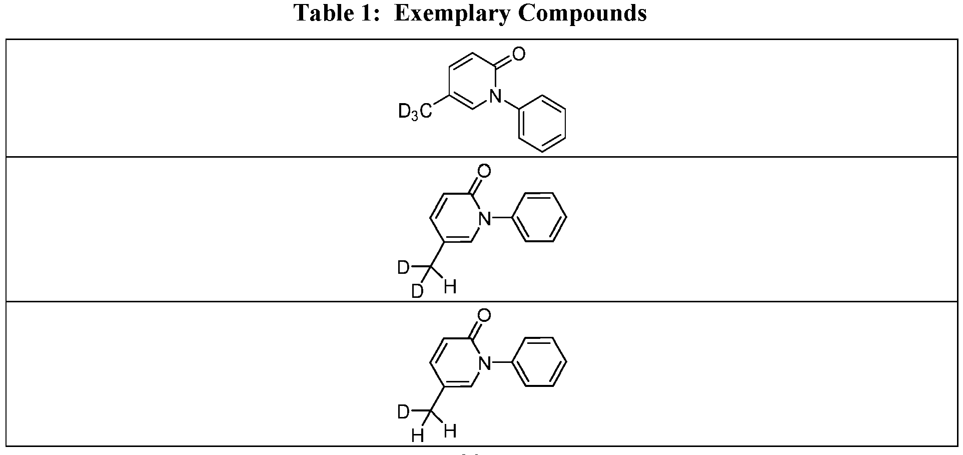

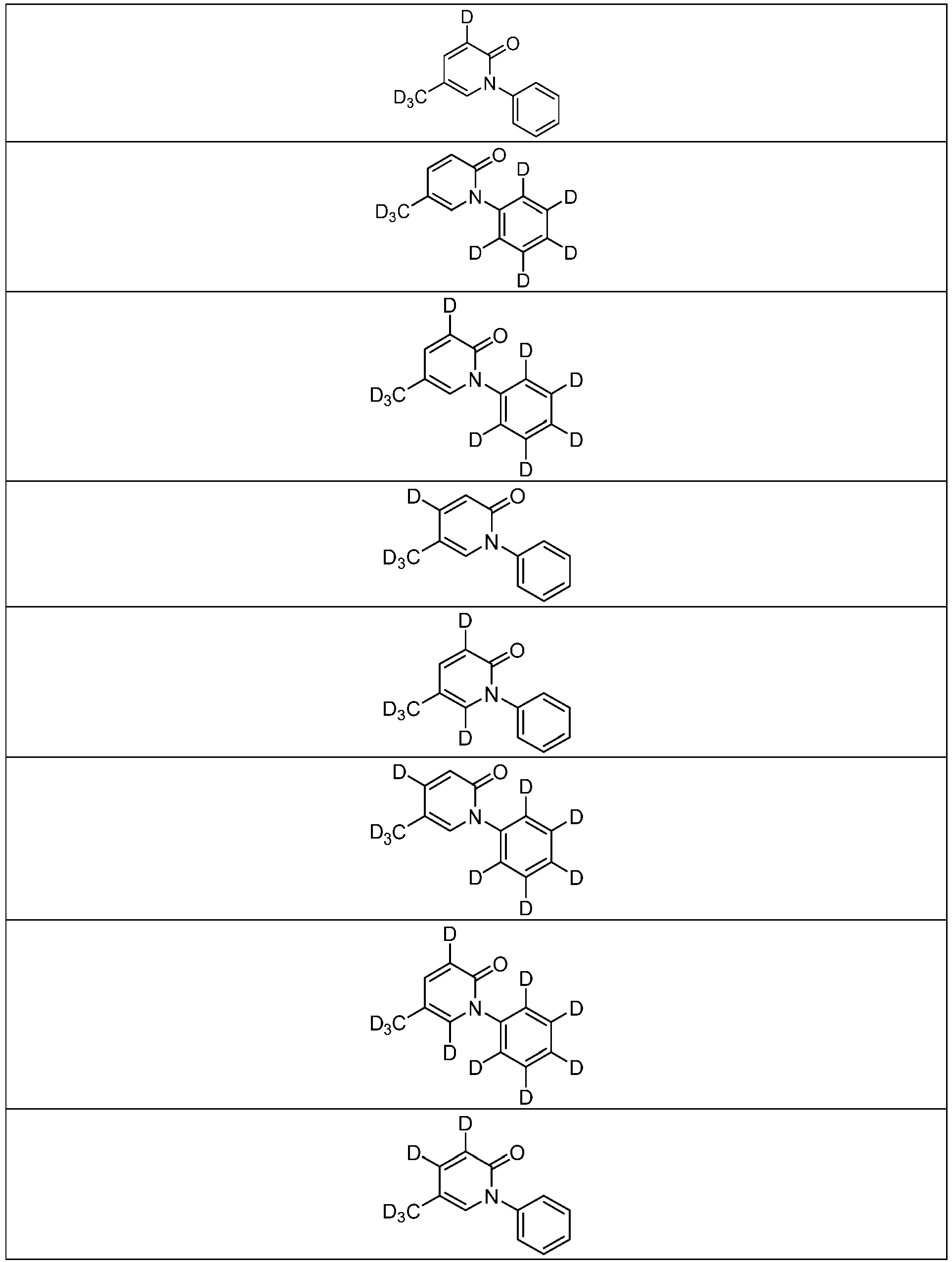

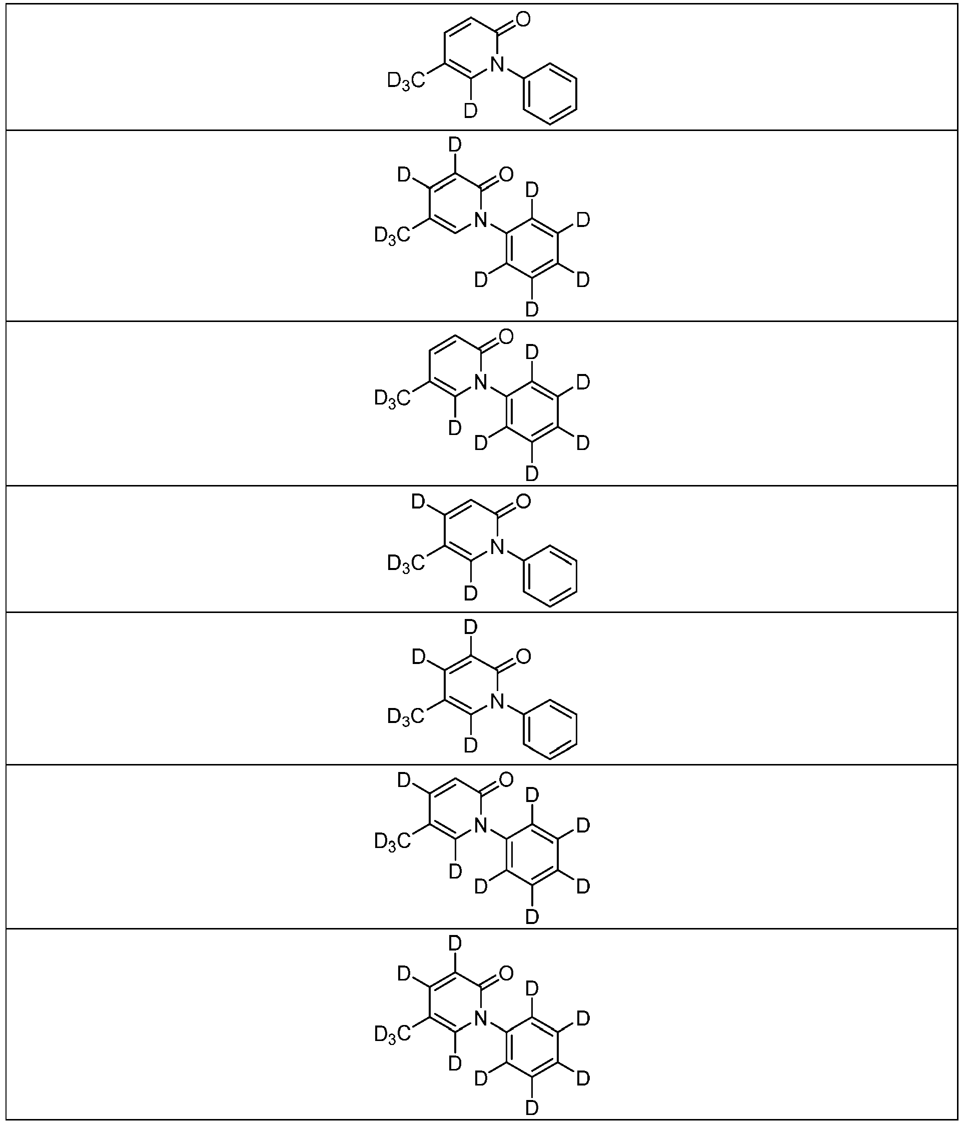

In some embodiments, the present invention provides a compound as depicted in **Table 1,** above, or a pharmaceutically acceptable salt thereof.

In some embodiments, the present invention provides a compound as depicted in **Table 1,** above, or a pharmaceutically acceptable salt thereof, wherein at least one of the positions represented as D independently has deuterium enrichment of no less than about 1%, no less than about 5%, no less than about 10%, no less than about 20%, no less than about 50%, no less than about 70%, no less than about 80%, no less than about 90%, no less than about 95%, no less than about 96%, no less than about 97 %, no less than about 98%, no less than about 99%, no less than about 99.1%, no less than about 99.2%, no less than about 99.3%, no less than about 99.4%, no less than about 99.5%, no less than about 99.6%, no less than about 99.7%, no less than about 99.8%, or no less than about 99.9%. In other embodiments, at least one of the positions represented as D independently has deuterium enrichment of 90%, 91%, 92%, 93%, 94%, 95%, 96%, 97 %, 98%, 99%, 99.1%, 99.2%, 99.3%, 99.4%, 99.5%, 99.6%, 99.7%, 99.8%, 99.9%, 100% or any incremental numerical fraction within the stated deuterium enrichments.

In a further embodiment, said compound is substantially a single enantiomer, a mixture of about 90% or more by weight of the (-)-enantiomer and about 10% or less by weight of the (+)-enantiomer, a mixture of about 90% or more by weight of the (+)-enantiomer and about 10% or less by weight of the (-)-enantiomer, substantially an individual diastereomer, or a mixture of about 90% or more by weight of an individual diastereomer and about 10% or less by weight of any other diastereomer.

In certain embodiments, the compound as disclosed herein contains about 60% or more by weight of the (-)-enantiomer of the compound and about 40% or less by weight of (+)-enantiomer of the compound. In certain embodiments, the compound as disclosed herein contains about 70% or more by weight of the (-)-enantiomer of the compound and about 30% or less by weight of (+)-enantiomer of the compound. In certain embodiments, the compound as disclosed herein contains about 80% or more by weight of the (-)-enantiomer of the compound and about 20% or less by weight of (+)-enantiomer of the compound. In certain embodiments, the compound as disclosed herein contains about 90% or more by weight of the (-)-enantiomer of the compound and about 10% or less by weight of the (+)-enantiomer of the compound. In certain embodiments, the compound as disclosed herein contains about 95% or more by weight of the (-)-enantiomer of the compound and about 5% or less by weight of (+)-enantiomer of the compound. In certain embodiments, the compound as disclosed herein contains about 99% or more by weight of the (-)-enantiomer of the compound and about 1% or less by weight of (+)-enantiomer of the compound.

In certain embodiments, the compound as disclosed herein contains about 60% or more by weight of the (+)-enantiomer of the compound and about 40% or less by weight of (-)-enantiomer of the compound. In certain embodiments, the compound as disclosed herein contains about 70% or more by weight of the (+)-enantiomer of the compound and about 30% or less by weight of (-)-enantiomer of the compound. In certain embodiments, the compound as disclosed herein contains about 80% or more by weight of the (+)-enantiomer of the compound and about 20% or less by weight of (-)-enantiomer of the compound. In certain embodiments, the compound as disclosed herein contains about 90% or more by weight of the (+)-enantiomer of the compound and about 10% or less by weight of the (-)-enantiomer of the compound. In certain embodiments, the compound as disclosed herein contains about 95% or more by weight of the (+)-enantiomer of the compound and about 5% or less by weight of (-)-enantiomer of the compound. In certain embodiments, the compound as disclosed herein contains about 99% or more by weight of the (+)-enantiomer of the compound and about 1% or less by weight of (-)-enantiomer of the compound.

The deuterated compound as disclosed herein may also contain less prevalent isotopes for other elements, including, but not limited to, ¹³C or ¹⁴C for carbon, ¹⁵N for nitrogen, and ¹⁷O or ¹⁸O for oxygen.

Isotopic hydrogen can be introduced into a compound of a compound disclosed herein as disclosed herein by synthetic techniques that employ deuterated reagents, whereby incorporation rates are pre-determined; and/or by exchange techniques, wherein incorporation rates are determined by equilibrium conditions, and may be highly variable depending on the reaction conditions. Synthetic techniques, where tritium or deuterium is directly and specifically inserted by tritiated or deuterated reagents of known isotopic content, may yield high tritium or deuterium abundance, but can be limited by the chemistry required. In addition, the molecule being labeled may be changed, depending upon the severity of the synthetic reaction employed. Exchange techniques, on the other hand, may yield lower tritium or deuterium incorporation, often with the isotope being distributed over many sites on the molecule, but offer the advantage that they do not require separate synthetic steps and are less likely to disrupt the structure of the molecule being labeled.

The compounds as disclosed herein can be prepared by methods known to one of skill in the art and routine modifications thereof, and/or procedures found in Esaki et al., Tetrahedron 2006, 62, 10954-10961, Smith et al., Organic Syntheses 2002, 78, 51-56, U.S. Pat. No. 3,974,281, U.S. Pat. No. 8,680,123, WO2003/014087, WO 2008/157786, WO 2009/035598, WO 2012/122165, or WO 2015/112701; the entirety of each of which is hereby incorporated by reference; and references cited therein and routine modifications thereof.

### Methods, Compositions and Dosing for Treating Fibrotic-mediated Disorders and/or a Collagen-mediated Disorders

Disclosed herein are methods for the treatment, prevention, and/or amelioration of a fibrotic-mediated disorder and/or a collagen-mediated disorder and/or inflammatory disorder comprising administering to a subject in need thereof a deuterium-enriched pirfenidone compound disclosed herein, for example, a compound of Formula I, e.g., a compound listed in Table 1, including e.g., LYT-100. Disclosed herein are methods for the treatment, prevention, and/or amelioration of one or more symptoms of a fibrotic-mediated disorder and/or a collagen-mediated disorder and/or inflammatory disorder comprising administering to a subject in need thereof a deuterium-enriched pirfenidone compound disclosed herein, for example, a compound of Formula I, e.g., a compound listed in Table 1, including e.g., LYT-100. Disclosed herein are methods for the treatment, prevention, and/or amelioration of a fibrotic-mediated disorder comprising administering to a subject in need thereof a deuterium-enriched pirfenidone compound disclosed herein, for example, a compound of Formula I, e.g., a compound listed in Table 1, including e.g., LYT-100. Disclosed herein are methods for the treatment, prevention, and/or amelioration of one or more symptoms of a fibrotic-mediated disorder comprising administering to a subject in need thereof a deuterium-enriched pirfenidone compound disclosed herein, for example, a compound of Formula I, e.g., a compound listed in Table 1, including e.g., LYT-100. Disclosed herein are methods for the treatment, prevention, and/or amelioration of a collagen-mediated disorder comprising administering to a subject in need thereof a deuterium-enriched pirfenidone compound disclosed herein, for example, a compound of Formula I, e.g., a compound listed in Table 1, including e.g., LYT-100. Disclosed herein are methods for the treatment, prevention, and/or amelioration of one or more symptoms of a collagen-mediated disorder comprising administering to a subject in need thereof a deuterium-enriched pirfenidone compound disclosed herein, for example, a compound of Formula I, e.g., a compound listed in Table 1, including e.g., LYT-100. Disclosed herein are methods for the treatment, prevention, and/or amelioration of an inflammatory disorder comprising administering to a subject in need thereof a deuterium-enriched pirfenidone compound disclosed herein, for example, a compound of Formula I, e.g., a compound listed in Table 1, including e.g., LYT-100. Disclosed herein are methods for the treatment, prevention, and/or amelioration of one or more symptoms of an inflammatory disorder comprising administering to a subject in need thereof a deuterium-enriched pirfenidone compound disclosed herein, for example, a compound of Formula I, e.g., a compound listed in Table 1, including e.g., LYT-100.

A fibrotic-mediated disorder and/or a collagen-mediated disorder include, but are not limited to, idiopathic pulmonary fibrosis, uterine fibroids, multiple sclerosis, renal fibrosis, diabetic kidney disease, endotoxin-induced liver injury after partial hepatectomy or hepatic ischemia, allograft injury after organ transplantation, cystic fibrosis, atrial fibrilation, neutropenia, scleroderma, dermatomyositis, cirrhosis, diffuse parenchymal lung disease, mediastinal fibrosis, tuberculosis, spleen fibrosis caused by sickle-cell anemia, rheumatoid arthritis, edema, lymphedema, and/or any disorder ameliorated by modulating fibrosis and/or collagen infiltration into tissues.

In some embodiments, the deuterium-enriched pirfenidone compound used in the disclosed methods has at least one of the following properties: a) decreased inter-individual variation in plasma levels of the compound or a metabolite thereof as compared to the non-isotopically enriched compound; b) increased average plasma levels of the compound per dosage unit thereof as compared to the non-isotopically enriched compound; c) decreased average plasma levels of at least one metabolite of the compound per dosage unit thereof as compared to the non-isotopically enriched compound; d) increased average plasma levels of at least one metabolite of the compound per dosage unit thereof as compared to the non-isotopically enriched compound; and e) an improved clinical effect during the treatment in the subject per dosage unit thereof as compared to the non-isotopically enriched compound. Thus, disclosed herein are methods for treating a subject, including a human, having or suspected of having a fibrotic-mediated disorder and/or a collagen-mediated disorder (e.g., any of the disorders disclosed herein) or for preventing such disorder in a subject prone to the disorder; comprising administering to the subject a therapeutically effective amount of a deuterium-enriched pirfenidone compound as disclosed herein, or a pharmaceutically acceptable salt, solvate, or prodrug thereof; so as to effect one or more of a) - e) above during the treatment of the disorder as compared to the corresponding non-isotopically enriched compound. In some embodiments, the deuterium-enriched pirfenidone compound has at least two of the properties a) through e) above. In some embodiments, the deuterium-enriched pirfenidone compound has three or more of the properties a) through e) above.

In one embodiment is a method for the treatment, prevention, or amelioration of one or more symptoms of a fibrotic-mediated disorder and/or a collagen-mediated disorder. A fibrotic-mediated disorder and/or a collagen-mediated disorder include, but are not limited to, idiopathic pulmonary fibrosis, uterine fibroids, multiple sclerosis, renal fibrosis, diabetic kidney disease, endotoxin-induced liver injury after partial hepatectomy or hepatic ischemia, allograft injury after organ transplantation, cystic fibrosis, atrial fibrilation, neutropenia, scleroderma, dermatomyositis, cirrhosis, diffuse parenchymal lung disease, mediastinal fibrosis, tuberculosis, spleen fibrosis caused by sickle-cell anemia, rheumatoid arthritis, and/or any disorder ameliorated by modulating fibrosis and/or collagen infiltration into tissues.

Disclosed herein are methods for treating a subject, including a human, having or suspected of having a fibrotic-mediated disorder and/or a collagen-mediated disorder (e.g., any of the disorders disclosed herein) or for preventing such disorder in a subject prone to the disorder; comprising administering to the subject a therapeutically effective amount of a deuterium-enriched pirfenidone compound as disclosed herein, or a pharmaceutically acceptable salt, solvate, or prodrug thereof; so as to effect decreased inter-individual variation in plasma levels of the compound or a metabolite thereof, during the treatment of the disorder as compared to the corresponding non-isotopically enriched compound. In certain embodiments, the inter-individual variation in plasma levels of the compounds as disclosed herein, or metabolites thereof, is decreased by greater than about 2%, greater than about 5%, greater than about 10%, greater than about 15%, greater than about 20%, greater than about 25%, greater than about 30%, greater than about 40%, or by greater than about 50% (including any numerical increment between the listed percentages) as compared to the corresponding non-isotopically enriched compound.

Disclosed herein are methods for treating a subject, including a human, having or suspected of having a fibrotic-mediated disorder and/or a collagen-mediated disorder (e.g., any of the disorders disclosed herein) or for preventing such disorder in a subject prone to the disorder; comprising administering to the subject a therapeutically effective amount of a deuterium-enriched pirfenidone compound as disclosed herein, or a pharmaceutically acceptable salt, solvate, or prodrug thereof; so as to affect increased average plasma levels of the compound or decreased average plasma levels of at least one metabolite of the compound per dosage unit as compared to the corresponding non-isotopically enriched compound. In certain embodiments, the average plasma levels of the compound as disclosed herein are increased by greater than about 2%, greater than about 5%, greater than about 10%, greater than about 15%, greater than about 20%, greater than about 25%, greater than about 30%, greater than about 40%, or by greater than about 50% (including any numerical increment between the listed percentages) as compared to the corresponding non-isotopically enriched compounds. In certain embodiments, the average plasma levels of a metabolite of the compound as disclosed herein are decreased by greater than about 2%, greater than about 5%, greater than about 10%, greater than about 15%, greater than about 20%, greater than about 25%, greater than about 30%, greater than about 40%, or by greater than about 50% (including any numerical increment between the listed percentages) as compared to the corresponding non-isotopically enriched compounds.

Plasma levels of the compound as disclosed herein, or metabolites thereof, may be measured using the methods described by Li et al. (Rapid Communications in Mass Spectrometry 2005, 19, 1943-1950).

In some embodiments, the compound has a decreased metabolism by at least one polymorphically-expressed cytochrome P₄₅₀ isoform in the subject per dosage unit thereof as compared to the non-isotopically enriched compound.

In some embodiments, the cytochrome P₄₅₀ isoform is selected from CYP2C8, CYP2C9, CYP2C19, and CYP2D6.

In some embodiments, the compound is characterized by decreased inhibition of at least one cytochrome P₄₅₀ or monoamine oxidase isoform in the subject per dosage unit thereof as compared to the non-isotopically enriched compound.

In certain embodiments, the cytochrome P₄₅₀ or monoamine oxidase isoform is selected from CYP1A1, CYP1A2, CYP1B1, CYP2A6, CYP2A13, CYP2B6, CYP2C8, CYP2C9, CYP2C18, CYP2C19, CYP2D6, CYP2E1, CYP2G1, CYP2J2, CYP2R1, CYP2S1, CYP3A4, CYP3A5, CYP3A5P1, CYP3A5P2, CYP3A7, CYP4A11, CYP4B1, CYP4F2, CYP4F3, CYP4F8, CYP4F11, CYP4F12, CYP4X1, CYP4Z1, CYP5A1, CYP7A1, CYP7B1, CYP8A1, CYP8B1, CYP11A1, CYP11B1, CYP11B2, CYP17, CYP19, CYP21, CYP24, CYP26A1, CYP26B1, CYP27A1, CYP27B1, CYP39, CYP46, CYP51, MAO_{A}, and MAO_{B}.

In some embodiments, the deuterium-enriched pirfenidone compound has at least one of the following properties: a) a half-life greater than 2.5 hours; b) a decreased pill burden; c) increased patient tolerability; d) a lower efficacious dose; e) increased bioavailability; f) increased Cmax; and g) increase in systemic exposure during the treatment in the subject per dosage unit thereof as compared to the non-isotopically enriched compound. Disclosed herein are methods for treating a subject, including a human, having or suspected of having a fibrotic-mediated disorder and/or a collagen-mediated disorder (e.g., any of the disorders disclosed herein) or for preventing such disorder in a subject prone to the disorder; comprising administering to the subject a therapeutically effective amount of a deuterium-enriched pirfenidone compound as disclosed herein, or a pharmaceutically acceptable salt, solvate, or prodrug thereof; so as to effect one or more of a) - g) above during the treatment of the disorder as compared to the corresponding non-isotopically enriched compound.

Disclosed herein are methods for treating a subject, including a human, having or suspected of having a fibrotic-mediated disorder and/or a collagen-mediated disorder (e.g., any of the disorders disclosed herein) or for preventing such disorder in a subject prone to the disorder; comprising administering to the subject a therapeutically effective amount of a deuterium-enriched pirfenidone compound as disclosed herein, or a pharmaceutically acceptable salt, solvate, or prodrug thereof; so as to effect a longer half-life. In some embodiments, the half-life of the deuterium-enriched pirfenidone compounds as disclosed herein, or metabolites thereof, is increased by greater than about 2%, greater than about 5%, greater than about 10%, greater than about 15%, greater than about 20%, greater than about 25%, greater than about 30%, greater than about 40%, by greater than about 50%, by greater than about 60%, by greater than about 70%, by greater than about 80%, by greater than about 90%, or by greater than about 100% (including any numerical increment between the listed percentages) as compared to the corresponding non-isotopically enriched compound. In some embodiments, the half-life of the deuterium-enriched pirfenidone compounds as disclosed herein, or metabolites thereof, is increased by about 1.5-fold, increased by about 2-fold, greater than about 2-fold, greater than about 3-fold, greater than about 4-fold, greater than about greater than about 5-fold, greater than about 10-fold or more (including any numerical increment between the listed percentages) as compared to the corresponding non-isotopically enriched compound.

Disclosed herein are methods for treating a subject, including a human, having or suspected of having a fibrotic-mediated disorder and/or a collagen-mediated disorder (e.g., any of the disorders disclosed herein) or for preventing such disorder in a subject prone to the disorder; comprising administering to the subject a therapeutically effective amount of a deuterium-enriched pirfenidone compound as disclosed herein, or a pharmaceutically acceptable salt, solvate, or prodrug thereof; so as to reduce the pill burden, e.g., effect a pill burden of less than nine (9) capsules per day (TID dosing) of the compound or a metabolite thereof, during the treatment of the disorder as compared to the corresponding non-isotopically enriched compound.

In certain embodiments, the pill burden of the compounds as disclosed herein, is decreased by greater than about 2%, greater than about 5%, greater than about 10%, greater than about 15%, greater than about 20%, greater than about 25%, greater than about 30%, greater than about 40%, or by greater than about 50% (including any numerical increment between the listed percentages) as compared to the corresponding non-isotopically enriched compound.

Disclosed herein are methods for treating a subject, including a human, having or suspected of having a fibrotic-mediated disorder and/or a collagen-mediated disorder (e.g., any of the disorders disclosed herein) or for preventing such disorder in a subject prone to the disorder; comprising administering to the subject a therapeutically effective amount of a deuterium-enriched pirfenidone compound as disclosed herein, or a pharmaceutically acceptable salt, solvate, or prodrug thereof; so as to effect an increased patient tolerability of the compound or a metabolite thereof, during the treatment of the disorder as compared to the corresponding non-isotopically enriched compound. In some embodiments, the patient tolerability is increased by altering the pharmacokinetics, e.g., by increasing the bioavailability (so as to use a lower dose) and/or by extending the half-life of the compound and/or by other means to reduce the side effects of pirfenidone.

In certain embodiments, the patient tolerability of the compounds as disclosed herein, or metabolites thereof, is increased by greater than about 2%, greater than about 5%, greater than about 10%, greater than about 15%, greater than about 20%, greater than about 25%, greater than about 30%, greater than about 40%, by greater than about 50%, by greater than about 60%, by greater than about 70%, by greater than about 80%, by greater than about 90%, or by greater than about 100% (including any numerical increment between the listed percentages) as compared to the corresponding non-isotopically enriched compound. In certain embodiments, the patient tolerability of the compounds as disclosed herein, or metabolites thereof, is increased by about 1.5-fold, increased by about 2-fold, greater than about 2-fold, greater than about 3-fold, greater than about 4-fold, greater than about greater than about 5-fold, greater than about 10-fold or more (including any numerical increment between the listed percentages) as compared to the corresponding non-isotopically enriched compound.

Disclosed herein are methods for treating a subject, including a human, having or suspected of having a fibrotic-mediated disorder and/or a collagen-mediated disorder (e.g., any of the disorders disclosed herein) or for preventing such disorder in a subject prone to the disorder; comprising administering to the subject a therapeutically effective amount of a deuterium-enriched pirfenidone compound as disclosed herein, or a pharmaceutically acceptable salt, solvate, or prodrug thereof; so as to effect a lower efficacious dose per dosage of the compound or a metabolite thereof, during the treatment of the disorder as compared to the corresponding non-isotopically enriched compound.

In certain embodiments, the efficacious dose per dosage of the compounds as disclosed herein, or metabolites thereof, is decreased by greater than about 2%, greater than about 5%, greater than about 10%, greater than about 15%, greater than about 20%, greater than about 25%, greater than about 30%, greater than about 40%, by greater than about 50%, by greater than about 60%, by greater than about 70%, by greater than about 80%, by greater than about 90%, or by greater than about 100% (including any numerical increment between the listed percentages) as compared to the corresponding non-isotopically enriched compound. In certain embodiments, the efficacious dose per dosage of the compounds as disclosed herein, or metabolites thereof, is decreased by about 1.5-fold, decreased by about 2-fold, greater than about 2-fold, greater than about 3-fold, greater than about 4-fold, greater than about greater than about 5-fold, greater than about 10-fold or more (including any numerical increment between the listed percentages) as compared to the corresponding non-isotopically enriched compound.

Disclosed herein are methods for treating a subject, including a human, having or suspected of having a fibrotic-mediated disorder and/or a collagen-mediated disorder (e.g., any of the disorders disclosed herein) or for preventing such disorder in a subject prone to the disorder; comprising administering to the subject a therapeutically effective amount of a deuterium-enriched pirfenidone compound as disclosed herein, or a pharmaceutically acceptable salt, solvate, or prodrug thereof; so as to increase the bioavailability per dosage of the compound or a metabolite thereof, during the treatment of the disorder as compared to the corresponding non-isotopically enriched compound.

In certain embodiments, the bioavailability per dosage of the compounds as disclosed herein, or metabolites thereof, is increased by greater than about 2%, greater than about 5%, greater than about 10%, greater than about 15%, greater than about 20%, greater than about 25%, greater than about 30%, greater than about 40%, by greater than about 50%, by greater than about 60%, by greater than about 70%, by greater than about 80%, by greater than about 90%, or by greater than about 100% (including any numerical increment between the listed percentages) as compared to the corresponding non-isotopically enriched compound. In certain embodiments, the bioavailability per dosage of the compounds as disclosed herein, or metabolites thereof, is increased by about 1.5-fold, decreased by about 2-fold, greater than about 2-fold, greater than about 3-fold, greater than about 4-fold, greater than about greater than about 5-fold, greater than about 10-fold or more (including any numerical increment between the listed percentages) as compared to the corresponding non-isotopically enriched compound.

Disclosed herein are methods for treating a subject, including a human, having or suspected of having or suspected of having a fibrotic-mediated disorder and/or a collagen-mediated disorder (e.g., any of the disorders disclosed herein) or for preventing such disorder in a subject prone to the disorder; comprising administering to the subject a therapeutically effective amount of a deuterium-enriched pirfenidone compound as disclosed herein, or a pharmaceutically acceptable salt, solvate, or prodrug thereof; so as to affect an increase in systemic exposure of the compound per dosage unit as compared to the corresponding non-isotopically enriched compound.

In certain embodiments, the systemic exposure per dosage of the compounds as disclosed herein, or metabolites thereof, is increased by greater than about 10%, greater than about 15%, greater than about 20%, greater than about 25%, greater than about 30%, greater than about 35%, greater than about 40%, greater than about 45%, or by greater than about 50% (including any numerical increment between the listed percentages) as compared to the corresponding non-isotopically enriched compound. In one embodiment, the systemic exposure per dosage of the compounds as disclosed herein is increased by greater than about 35% as compared to the corresponding non-isotopically enriched compound. In one embodiment, the systemic exposure per dosage of the compounds as disclosed herein is increased by about 35% as compared to the corresponding non-isotopically enriched compound.

Disclosed herein are methods for treating a subject, including a human, having or suspected of having or suspected of having a fibrotic-mediated disorder and/or a collagen-mediated disorder (e.g., any of the disorders disclosed herein) or for preventing such disorder in a subject prone to the disorder; comprising administering to the subject a therapeutically effective amount of a deuterium-enriched pirfenidone compound as disclosed herein, or a pharmaceutically acceptable salt, solvate, or prodrug thereof; so as to affect an increase in Cmax of the compound per dosage unit as compared to the corresponding non-isotopically enriched compound.

In certain embodiments, the Cmax per dosage of the compounds as disclosed herein, or metabolites thereof, is increased by greater than about 10%, greater than about 15%, greater than about 20%, greater than about 25%, greater than about 30%, greater than about 35%, greater than about 40%, greater than about 45%, or by greater than about 50% (including any numerical increment between the listed percentages) as compared to the corresponding non-isotopically enriched compound. In one embodiment, the Cmax per dosage of the compounds as disclosed herein is increased by greater than about 25% as compared to the corresponding non-isotopically enriched compound. In one embodiment, the Cmax per dosage of the compounds as disclosed herein is increased by about 25% as compared to the corresponding non-isotopically enriched compound.

In some embodiments, the method treats the disorder while reducing or eliminating a deleterious change in a diagnostic hepatobiliary function endpoint, as compared to the corresponding non-isotopically enriched compound, e.g., pirfenidone. Disclosed herein are methods for treating a subject, including a human, having or suspected of having a fibrotic-mediated disorder and/or a collagen-mediated disorder (e.g., any of the disorders disclosed herein) or for preventing such disorder in a subject prone to the disorder; comprising administering to the subject a therapeutically effective amount of a compound as disclosed herein, or a pharmaceutically acceptable salt, solvate, or prodrug thereof; so as to reduce or eliminate a deleterious change in a diagnostic hepatobiliary function endpoint, as compared to the corresponding non-isotopically enriched compound. In some embodiments, the diagnostic hepatobiliary function endpoint is selected from alanine aminotransferase ("ALT"), serum glutamic-pyruvic transaminase ("SGPT"), aspartate aminotransferase ("AST," "SCOT"), ALT/AST ratios, serum aldolase, alkaline phosphatase ("ALP"), ammonia levels, bilirubin, gamma-glutamyl transpeptidase ("GGTP," "gamma-GTP," "GGT"), leucine aminopeptidase ("LAP"), liver biopsy, liver ultrasonography, liver nuclear scan, 5'-nucleotidase, and blood protein.

Disclosed herein are methods for the treatment, prevention, and/or amelioration of a fibrotic-mediated disorder and/or a collagen-mediated disorder and methods for the treatment, prevention, and/or amelioration of one or more symptoms of a fibrotic-mediated disorder and/or a collagen-mediated disorder comprising administering to a subject in need thereof a deuterium-enriched pirfenidone compound disclosed herein, for example, a compound of Formula I, e.g., a compound listed in Table 1, including e.g., LYT-100, and further comprising administering one or more additional therapeutic agent(s) selected from an anti-T cell agent, an anti-inflammatory agent, an anti-TGF-βI agent, and an anti-angiotensin agent. In some embodiments, the additional therapeutic agent is an anti-inflammatory agent. In some embodiments, the additional therapeutic agent is an anti-T-cell agent. In some embodiments, the additional therapeutic agent is an anti-TGF-βI agent. In some embodiments, the additional therapeutic agent is an anti-angiotensin agent.

In some embodiments, the therapeutic agent is deuterium-enriched pirfenidone, or a pharmaceutically acceptable salt thereof. In some embodiments, the therapeutic agent is LYT-100, or a pharmaceutically acceptable salt thereof. In some embodiments, the therapeutic agent is deuterium-enriched pirfenidone, or a pharmaceutically acceptable salt thereof, in combination with one or more additional therapeutic agents, such as any of the additional therapeutic agents disclosed herein.

In any of the above-described embodiments, the deuterium-enriched pirfenidone compound, e.g., LYT-100, is administered orally twice a day, for a total daily dose of 100-1500 mg. In some embodiments, the daily dose is 100, 200, 250, 300, 400, 500, 600, 700, 750, 800, 900, 1000, or 1500 mg. In some embodiments, the daily dose is 1500 mg. In some embodiments, the daily dose is 1000 mg. In some embodiments, the daily dose is 750 mg. In some embodiments, the daily dose is 500 mg. In some embodiments, the daily dose is 250 mg. In some embodiments, the deuterium-enriched pirfenidone is administered orally 750 mg twice daily. In some embodiments, the deuterium-enriched pirfenidone is administered orally 500 mg twice daily. In some embodiments, the deuterium-enriched pirfenidone is administered orally 250 mg twice daily.

In some embodiments, the deuterium-enriched pirfenidone compound, e.g., LYT-100, is administered orally once a day, for a total daily dose of 100-1500 mg. In some embodiments, the daily dose is 100, 200, 250, 300, 400, 500, 600, 700, 750, 800, 900, 1000, or 1500 mg. In some embodiments, the daily dose is 1500 mg. In some embodiments, the daily dose is 1000 mg. In some embodiments, the daily dose is 750 mg. In some embodiments, the daily dose is 500 mg. In some embodiments, the daily dose is 250 mg. In some embodiments, the deuterium-enriched pirfenidone is administered orally 1500 mg once daily. In some embodiments, the deuterium-enriched pirfenidone is administered orally 1000 mg once daily. In some embodiments, the deuterium-enriched pirfenidone is administered orally 750 mg once daily. In some embodiments, the deuterium-enriched pirfenidone is administered orally 500 mg once daily. In some embodiments, the deuterium-enriched pirfenidone is administered orally 250 mg once daily.

In any of the above-described embodiments, the deuterium-enriched pirfenidone compound, e.g., LYT-100 is administered orally three times a day, for a total daily dose of 100-1500 mg. In some embodiments, the daily dose is 100, 200, 250, 300, 400, 500, 600, 700, 750, 800, 900, 1000, or 1500 mg. In some embodiments, the daily dose is 1500 mg. In some embodiments, the daily dose is 1000 mg. In some embodiments, the daily dose is 750 mg. In some embodiments, the daily dose is 500 mg. In some embodiments, the daily dose is 250 mg. In some embodiments, the deuterium-enriched pirfenidone is administered orally 500 mg three times daily. In some embodiments, the deuterium-enriched pirfenidone is administered orally 333 mg three times daily. In some embodiments, the deuterium-enriched pirfenidone is administered orally 166 mg three times daily.

In some embodiments, the deuterium-enriched pirfenidone is in tablet form. In some embodiments, the deuterium-enriched pirfenidone is taken orally with food.

Thus, provided herein are methods for the treatment, prevention, and/or amelioration of a fibrotic-mediated disorder and/or a collagen-mediated disorder comprising administering to a subject in need thereof a deuterium-enriched pirfenidone compound, for example, a compound of Formula I, e.g., a compound listed in Table 1, including e.g., LYT-100, wherein the deuterium-enriched pirfenidone compound is administered orally twice a day, for a total daily dose of 100-1500 mg. In some embodiments, provided herein are methods for the treatment, prevention, and/or amelioration of a fibrotic-mediated disorder and/or a collagen-mediated disorder comprising administering to a subj ect in need thereof LYT-100, wherein LYT-100 is administered orally twice a day, for a total daily dose of 100-1500 mg. In some embodiments, the daily dose of the deuterium-enriched pirfenidone compound, e.g., LYT-100, is 1500 mg. In some embodiments, the daily dose of the deuterium-enriched pirfenidone compound, e.g., LYT-100, is 1000 mg. In some embodiments, the daily dose of the deuterium-enriched pirfenidone compound, e.g., LYT-100, is 750 mg. In some embodiments, the daily dose of the deuterium-enriched pirfenidone compound, e.g., LYT-100 is 500 mg. In some embodiments, the daily dose of the deuterium-enriched pirfenidone compound, e.g., LYT-100 is 250 mg. In some embodiments, the deuterium-enriched pirfenidone compound, e.g., LYT-100 is administered orally 750 mg twice daily. In some embodiments, the deuterium-enriched pirfenidone compound, e.g., LYT-100, is administered orally 500 mg twice daily. In some embodiments, the deuterium-enriched pirfenidone compound, e.g., LYT-100, is administered orally 250 mg twice daily.

In some embodiments, provided herein are methods for the treatment, prevention, and/or amelioration of a fibrotic-mediated disorder and/or a collagen-mediated disorder comprising administering to a subject in need thereof a deuterirum-enriched pirfenidone compound, for example, a compound of Formula I, e.g., a compound listed in Table 1, including e.g., LYT-100, wherein the deuterirum-enriched pirfenidone compound is administered orally once a day, for a total daily dose of 100-1500 mg. In some embodiments, provided herein are methods for the treatment, prevention, and/or amelioration of a fibrotic-mediated disorder and/or a collagen-mediated disorder comprising administering to a subject in need thereof LYT-100, wherein LYT-100, is administered orally once a day, for a total daily dose of 100-1500 mg. In some embodiments, the daily dose of the deuterium-enriched pirfenidone compound, e.g., LYT-100 is 1500 mg. In some embodiments, the daily dose of the deuterium-enriched pirfenidone compound, e.g., LYT-100, is 1000 mg. In some embodiments, the daily dose of the deuterium-enriched pirfenidone compound, e.g., LYT-100, is 750 mg. In some embodiments, the daily dose of the deuterium-enriched pirfenidone compound, e.g., LYT-100 is 500 mg. In some embodiments, the daily dose of the deuterium-enriched pirfenidone compound, e.g., LYT-100, is 250 mg. In some embodiments, the deuterium-enriched pirfenidone compound, e.g., LYT-100, is administered orally 1500 mg once daily. In some embodiments, the deuterium-enriched pirfenidone compound, e.g., LYT-100, is administered orally 1000 mg once daily. In some embodiments, the deuterium-enriched pirfenidone compound, e.g., LYT-100, is administered orally 750 mg once daily. In some embodiments, the deuterium-enriched pirfenidone compound, e.g., LYT-100, is administered orally 500 mg once daily. In some embodiments, the deuterium-enriched pirfenidone compound, e.g., LYT-100 is administered orally 250 mg once daily.

Thus, provided herein are methods for the treatment, prevention, and/or amelioration of a fibrotic-mediated disorder and/or a collagen-mediated disorder comprising administering to a subject in need thereof a deuterium-enriched pirfenidone compound, for example, a compound of Formula I, e.g., a compound listed in Table 1, including e.g., LYT-100, wherein the deuterium-enriched pirfenidone compound is administered orally three times daily, for a total daily dose of 100-1500 mg. In some embodiments, provided herein are methods for the treatment, prevention, and/or amelioration of a fibrotic-mediated disorder and/or a collagen-mediated disorder comprising administering to a subject in need thereof LYT-100, wherein LYT-100 is administered orally three times a day, for a total daily dose of 100-1500 mg. In some embodiments, the daily dose of the deuterium-enriched pirfenidone compound, e.g., LYT-100, is 1500 mg. In some embodiments, the daily dose of the deuterium-enriched pirfenidone compound, e.g., LYT-100, is 1000 mg. In some embodiments, the daily dose of the deuterium-enriched pirfenidone compound, e.g., LYT-100, is 750 mg. In some embodiments, the daily dose of the deuterium-enriched pirfenidone compound, e.g., LYT-100 is 500 mg. In some embodiments, the daily dose of the deuterium-enriched pirfenidone compound, e.g., LYT-100 is 250 mg. In some embodiments, the deuterium-enriched pirfenidone compound, e.g., LYT-100 is administered orally 500 mg three times daily. In some embodiments, the deuterium-enriched pirfenidone compound, e.g., LYT-100, is administered orally 333 mg three timese daily. In some embodiments, the deuterium-enriched pirfenidone compound, e.g., LYT-100, is administered orally 166 mg twice daily.

In other embodiments, the deuterium-enriched pirfenidone compound is administered orally at a total daily dose of 100-2500 mg. In some embodiments, the deuterium-enriched pirfenidone compound is administered orally at a total daily dose of 100-2000 mg. In some embodiments, the deuterium-enriched pirfenidone compound is administered orally at a total daily dose of 100-1500 mg. In some embodiments, the deuterium-enriched pirfenidone compound is administered orally at a total daily dose of 100-1000 mg. In some embodiments, the deuterium-enriched pirfenidone compound is administered orally at a total daily dose of 100-500 mg. In some embodiments, the daily dose is selected from 100, 200, 250, 300, 400, 500, 600, 700, 750, 800, 900, 1000, 1100, 1200, 1300, 1400, 1500, 1600, 1700,1800, 1900, 2000, 2100, 2200, 2300, 2400, and 2500 mg/day. In some embodiments, the deuterium-enriched pirfenidone compound is administered orally three times/day (TID). In some embodiments, the deuterium-enriched pirfenidone compound is administered orally two times/day (BID). In some embodiments, the deuterium-enriched pirfenidone compound is administered orally once daily (QD). In any of these embodiments, the deuterium-enriched pirfenidone compound has the structire of Formula I, e.g., a compound listed in **Table 1,** including e.g., LYT-100.

### Methods, Compositions and Dosing for Treating Edema

The term "edema" or "oedema," as used herein, is an abnormal accumulation of fluid beneath the skin and in body cavities including, but not limited to, limbs, hands/feet, upper body (breast/chest wall, shoulder, back), lower body (buttocks, abdomen), genital (scrotum, penis, vulva), head, neck, or face. The abnormal accumulation of fluid can occur when capillary filtration exceeds lymphatic drainage. In this way, all edema has a lymphatic component. Edema includes lymphedema, lymphatic dysfunction, lymphatic tissue fibrosis, idiopathic edema, peripheral edema, and eye edema. Edema includes acute edema, chronic edema, post-operative edema, gradual-onset edema, primary edema and secondary edema. Chronic edema is edema that has been present for more than three months and can include lymphedema (primary-failure of the lymphatic development and secondary-following damage to the lymphatics), venous edema, chronic swelling due to immobility, edema related to advanced cancer, chronic swelling associated with lymphedema, chronic swelling related to obesity, and chronic swelling associated with rare vascular malformations such as Klippel-Trenaunay syndrome. Symptoms of edema can include accumulation of fluid beneath the skin and in body cavities, swelling, fullness, or puffiness of tissues, inflammation, fibrosis, heaviness, pain, decreased range of motion, aching, recurring infections, skin thickening, or discomfort. In some embodiments, "edema" does not include pulmonary edema or cerebral edema. In some embodiments, the edema is lymphedema. In some embodiments, the lymphedema is primary lymphedema. In some embodiments, the lymphedema is secondary lymphedema.

Lymphedema is a chronic condition that afflicts millions of people and is characterized by severe swelling in parts of the body, typically the arms or legs, due to the build-up of lymph fluid and inflammation, fibrosis and adipose deposition. Lymph is a clear fluid collected from body tissues that transports fats and proteins from the small intestine, removes bacteria, viruses, toxins, and certain proteins from tissues and supplies white blood cells, specifically lymphocytes, to the bloodstream to help fight infections and other diseases. Lymphedema is a chronic debilitating disease of fibrotic and inflammatory origin, that in developed countries, such as the United States, occurs most often as a complication of cancer treatment. Thus, secondary lymphedema is the most prevalent form of lymphedema, and can develop after surgery, infection or trauma, and is frequently caused by cancer, cancer treatments such as radiation and chemotherapy, trauma or infections resulting in damage to or the removal of lymph nodes. As a complication of cancer treatment, lymphedema occurs as a result of iatrogenic injury to the lymphatic system, usually as a result of lymph node dissection. According to estimates, as many as 1 in 3 patients who undergo lymph node dissection later develop lymphedema. Large skin excisions and adjuvant therapy with radiation may also cause lymphedema. In addition, obesity and radiation are known risk factors for the development of lymphedema.

Lymphedema of the leg and its advanced form, known as elephantiasis, are significant causes of disability and morbidity in areas endemic for lymphatic filariasis, with an estimated 14 million persons affected worldwide (Stocks et al., PLoS Negl Trop Dis. 2015 Oct 23;9(10):e0004171). Over 1.1 billion people worldwide are at risk for lymphatic filariasis (Walsh et al, PLoS Negl Trop Dis. 2016 Aug 22;10(8):e0004917). Lymphatic filariasis is distributed from Latin America, across central Africa, southern Asia and into the Pacific Islands. Filarial infection is mosquito-transmitted, but efforts to control transmission that are based exclusively on mosquito control have had limited success (Lammie et al., Ann N Y Acad Sci. 2002 Dec;979:131-42; discussion 188-96). Wuchereria bancrofti (Wb) is the most widely distributed of the three nematodes known to cause lymphatic filariasis (LF), the other two being Brugia malayi and Brugia timori. Wuchereria bancrofti is the species responsible for 90% of lymphatic filariasis in humans. Filarial infection can cause a variety of clinical manifestations, including lymphoedema of the limbs, genital disease (hydrocele, chylocele, and swelling of the scrotum and penis) and recurrent acute attacks. These acute attacks are caused by secondary infections, to which the lower limbs with lymphatic damage are predisposed, and which are extremely painful and are accompanied by fever. Most infected people do not have symptoms, but virtually all of them have subclinical lymphatic damage and as many as 40% have kidney damage, with proteinuria and hematuria.

Lymphedema is a serious disease with significant health consequences, including disfigurement and debilitation. Patients have chronic swelling of the affected extremity, a sense of heaviness, pain, discomfort, skin damage, fibrosis, recurrent infections, limited mobility, and decreased quality of life.

Dysfunctions of the lymphatic system have remained largely untreated or poorly addressed by current therapeutics. There are currently no approved drug therapies for the treatment of lymphedema. Furthermore, at present, there is no known pharmacologic therapy that can halt progression or promote resolution of lymphedema. The current standard of care for lymphedema is management, primarily with compression and physical therapy to control swelling. These approaches are cumbersome, uncomfortable and non-curative, and they do not address the underlying disease, especially in patients with more severe lymphedema. Even with management, some patients will progress from mild-to-moderate lymphedema to more severe forms. In later stages, patients may also seek ablative surgeries, including liposuction or debulking. These surgeries reduce volume but do not restore lymphatic flow, leading to a dependence on compression. Given that there are currently no drug therapies that treat the underlying causes of lymphedema, the development of targeted treatments for lymphedema is an unmet biomedical need.

There has been little progress toward the development of meaningful treatments for lymphatic diseases. Previous experimental treatments for lymphedema have focused on delivery of lymphangiogenic cytokines. Skobe et al., Nat. Med. 7: 192-198 (2001). For example, some previous studies have focused on repairing damaged lymphatics using lymphangiogenic cytokines such as vascular endothelial growth factor-c (VEGF-C). Tammela et al., Nat. Med. 13: 1458-1466

(2007); Baker et al., Breast Cancer Res. 12:R70 (2010). However, application of this approach, particularly to cancer patients, may be untenable as these same mechanisms regulate tumor growth and metastasis, raising the risk of cancer metastases or recurrence.

In some embodiments, the disclosure provides methods for treating edema comprising administering to a subject in need thereof a deuterium-enriched pirfenidone compound disclosed herein, for example, a compound of Formula I, e.g., a compound listed in Table 1, including e.g., LYT-100. In some embodiments, the disclosure provides methods for treating lymphedema comprising administering to a subject in need thereof a deuterium-enriched pirfenidone compound disclosed herein, for example, a compound of Formula I, e.g., a compound listed in Table 1, including e.g., LYT-100. In some embodiments, the disclosure provides methods for treating secondary lymphedema comprising administering to a subject in need thereof a deuterium-enriched pirfenidone compound disclosed herein, for example, a compound of Formula I, e.g., a compound listed in Table 1, including e.g., LYT-100. In some embodiments, the disclosure provides methods for treating primary lymphedema comprising administering to a subject in need thereof a deuterium-enriched pirfenidone compound disclosed herein, for example, a compound of Formula I, e.g., a compound listed in Table 1, including e.g., LYT-100.

In some embodiments, the lymphedema occurs in one or both arms, such as in the hand, wrist, forearm, elbow, upper arm, shoulder, armit, or combination of arm areas or the entire arm. In some embodiments, the lymphedema occurs in one or both legs, such as in the foot, ankle, leg, knee, upper leg or thigh, groin, hip, or combination of leg areas or the entire leg. In some embodiments, the lymphedema occurs in the head, neck, jaw, chest, breast, thorax, abdomen, pelvis, genitals, or other areas of the body cavity. In some embodiments, the lymphedema occurs in one or more limbs, or in one or more limbs and another area of the body.

In some embodiments the lymphedema results from a vascular defect, including venous insufficiency, venous malformation, arterial malformation, capillary malformation, lymphovascular malformation, or cardiovascular disease.

In some embodiments, the subject has or has had cancer, for example, a cancer comprising a solid tumor. In some embodiments, the subject has or has had breast cancer or a cancer affecting female reproductive organs, cutaneous system, musculoskeletal system, soft tissues of the extremities or trunk, male reproductive system, urinary system, or the head and neck. In some embodiments, the subject has undergone axillary lymph node dissection. In some embodiments, the subject has received treatment for cancer, and the edema, lymphedema, or lymphatic injury is associated with the cancer treatment or diagnosis. For example, the subject may be receiving or may have received chemotherapy or radiation therapy for cancer treatment or other indications, or may have had one or more lymph nodes surgically removed in the course of cancer treatment or diagnosis.

In some embodiments, the subject has sustained a lymphatic injury (for example as the result of removal, ligation or obstruction of lymph nodes or lymph vessels, or fibrosis of lymph tissue), or the subject is obese or has or has had an infection that leads to edema, such as lymphedema. In some embodiments, the infection is a skin infection or a history of skin infection related to lymphedema or lymphatic injury. In some embodiments, the infection is a parasitic infection that obstructs lymphatic flow or injures the lymphatic system. In some embodiments, the subject has sustained lymphatic injury from joint replacement, trauma, burns, radiation, or chemotherapy.

In some embodiments, the disclosure provides methods for preventing edema comprising administering to a subject in need thereof a deuterium-enriched pirfenidone compound disclosed herein, for example, a compound of Formula I, e.g., a compound listed in Table 1, including e.g., LYT-100. In some embodiments, the disclosure provides methods for preventing lymphedema comprising administering to a subject in need thereof a deuterium-enriched pirfenidone compound disclosed herein, for example, a compound of Formula I, e.g., a compound listed in Table 1, including e.g., LYT-100. In some embodiments, the disclosure provides methods for preventing secondary lymphedema comprising administering to a subject in need thereof a deuterium-enriched pirfenidone compound disclosed herein, for example, a compound of Formula I, e.g., a compound listed in Table 1, including e.g., LYT-100. In some embodiments, the disclosure provides methods for preventing primary lymphedema comprising administering to a subject in need thereof a deuterium-enriched pirfenidone compound disclosed herein, for example, a compound of Formula I, e.g., a compound listed in Table 1, including e.g., LYT-100.

In some embodiments, the lymphedema occurs in one or both arms, such as in the hand, wrist, forearm, elbow, upper arm, shoulder, armit, or combination of arm areas or the entire arm. In some embodiments, the lymphedema occurs in one or both legs, such as in the foot, ankle, leg, knee, upper leg or thigh, groin, hip, or combination of leg areas or the entire leg. In some embodiments, the lymphedema occurs in the head, neck, jaw, chest, breast, thorax, abdomen, pelvis, genitals, or other areas of the body cavity. In some embodiments, the lymphedema occurs in one or more limbs, or in one or more limbs and another area of the body.

In some embodiments the lymphedema results from a vascular defect, including venous insufficiency, venous malformation, arterial malformation, capillary malformation, lymphovascular malformation, or cardiovascular disease.

In some embodiments, the disclosure provides methods for amelorating one or more symptoms of edema comprising administering to a subject in need thereof a deuterium-enriched pirfenidone compound disclosed herein, for example, a compound of Formula I, e.g., a compound listed in Table 1, including e.g., LYT-100. In some embodiments, the disclosure provides methods for amelorating one or more symptoms of lymphedema comprising administering to a subject in need thereof a deuterium-enriched pirfenidone compound disclosed herein, for example, a compound of Formula I, e.g., a compound listed in Table 1, including e.g., LYT-100. In some embodiments, the disclosure provides methods for amelorating one or more symptoms of secondary lymphedema comprising administering to a subject in need thereof a deuterium-enriched pirfenidone compound disclosed herein, for example, a compound of Formula I, e.g., a compound listed in Table 1, including e.g., LYT-100. In some embodiments, the disclosure provides methods for amelorating one or more symptoms of primary lymphedema comprising administering to a subject in need thereof a deuterium-enriched pirfenidone compound disclosed herein, for example, a compound of Formula I, e.g., a compound listed in Table 1, including e.g., LYT-100.

In some embodiments, the lymphedema occurs in one or both arms, such as in the hand, wrist, forearm, elbow, upper arm, shoulder, armit, or combination of arm areas or the entire arm. In some embodiments, the lymphedema occurs in one or both legs, such as in the foot, ankle, leg, knee, upper leg or thigh, groin, hip, or combination of leg areas or the entire leg. In some embodiments, the lymphedema occurs in the head, neck, jaw, chest, breast, thorax, abdomen, pelvis, genitals, or other areas of the body cavity. In some embodiments, the lymphedema occurs in one or more limbs, or in one or more limbs and another area of the body.

In some embodiments the lymphedema results from a vascular defect, including venous insufficiency, venous malformation, arterial malformation, capillary malformation, lymphovascular malformation, or cardiovascular disease.

In any of the above-described methods for treating, preventing, or ameliorating one or more symptoms of edema or lymphedema, the deuterium-enriched pirfenidone compound, e.g., LYT-100, is administered orally twice a day, for a total daily dose of 100-1500 mg. In some embodiments, the daily dose is 100, 200, 250, 300, 400, 500, 600, 700, 750, 800, 900, 1000, or 1500 mg. In some embodiments, the daily dose is 1500 mg. In some embodiments, the daily dose is 1000 mg. In some embodiments, the daily dose is 750 mg. In some embodiments, the daily dose is 500 mg. In some embodiments, the daily dose is 250 mg. In some embodiments, the deuterium-enriched pirfenidone is administered orally 750 mg twice daily. In some embodiments, the deuterium-enriched pirfenidone is administered orally 500 mg twice daily. In some embodiments, the deuterium-enriched pirfenidone is administered orally 250 mg twice daily.

In some embodiments, the deuterium-enriched pirfenidone compound, e.g., LYT-100, is administered orally once a day, for a total daily dose of 100-1500 mg. In some embodiments, the daily dose is 100, 200, 250, 300, 400, 500, 600, 700, 750, 800, 900, 1000, or 1500 mg. In some embodiments, the daily dose is 1500 mg. In some embodiments, the daily dose is 1000 mg. In some embodiments, the daily dose is 750 mg. In some embodiments, the daily dose is 500 mg. In some embodiments, the daily dose is 250 mg. In some embodiments, the deuterium-enriched pirfenidone is administered orally 1500 mg once daily. In some embodiments, the deuterium-enriched pirfenidone is administered orally 1000 mg once daily. In some embodiments, the deuterium-enriched pirfenidone is administered orally 750 mg once daily. In some embodiments, the deuterium-enriched pirfenidone is administered orally 500 mg once daily. In some embodiments, the deuterium-enriched pirfenidone is administered orally 250 mg once daily.

In any of the above-described embodiments, the deuterium-enriched pirfenidone compound, e.g., LYT-100 is administered orally three times a day, for a total daily dose of 100-1500 mg. In some embodiments, the daily dose is 100, 200, 250, 300, 400, 500, 600, 700, 750, 800, 900, 1000, or 1500 mg. In some embodiments, the daily dose is 1500 mg. In some embodiments, the daily dose is 1000 mg. In some embodiments, the daily dose is 750 mg. In some embodiments, the daily dose is 500 mg. In some embodiments, the daily dose is 250 mg. In some embodiments, the deuterium-enriched pirfenidone is administered orally 500 mg three times daily. In some embodiments, the deuterium-enriched pirfenidone is administered orally 333 mg three times daily. In some embodiments, the deuterium-enriched pirfenidone is administered orally 166 mg three times daily.

In some embodiments, the deuterium-enriched pirfenidone is in tablet form. In some embodiments, the deuterium-enriched pirfenidone is taken orally with food.

Thus, provided herein are methods for the treatment, prevention, and/or amelioration of edema, e.g.lymphedema comprising administering to a subject in need thereof a deuterium-enriched pirfenidone compound, for example, a compound of Formula I, e.g., a compound listed in Table 1, including e.g., LYT-100, wherein the deuterium-enriched pirfenidone compound is administered orally twice a day, for a total daily dose of 100-1500 mg. In some embodiments, provided herein are methods for the treatment, prevention, and/or amelioration of edeme, e.g., lymphedema comprising administering to a subject in need thereof LYT-100, wherein LYT-100 is administered orally twice a day, for a total daily dose of 100-1500 mg. In some embodiments, the daily dose of the deuterium-enriched pirfenidone compound, e.g., LYT-100, is 1500 mg. In some embodiments, the daily dose of the deuterium-enriched pirfenidone compound, e.g., LYT-100, is 1000 mg. In some embodiments, the daily dose of the deuterium-enriched pirfenidone compound, e.g., LYT-100, is 750 mg. In some embodiments, the daily dose of the deuterium-enriched pirfenidone compound, e.g., LYT-100 is 500 mg. In some embodiments, the daily dose of the deuterium-enriched pirfenidone compound, e.g., LYT-100 is 250 mg. In some embodiments, the deuterium-enriched pirfenidone compound, e.g., LYT-100 is administered orally 750 mg twice daily. In some embodiments, the deuterium-enriched pirfenidone compound, e.g., LYT-100, is administered orally 500 mg twice daily. In some embodiments, the deuterium-enriched pirfenidone compound, e.g., LYT-100, is administered orally 250 mg twice daily.

In some embodiments, provided herein are methods for the treatment, prevention, and/or amelioration of edema, e.g., lymphedema comprising administering to a subject in need thereof a deuterirum-enriched pirfenidone compound, for example, a compound of Formula I, e.g., a compound listed in Table 1, including e.g., LYT-100, wherein the deuterirum-enriched pirfenidone compound is administered orally once a day, for a total daily dose of 100-1500 mg. In some embodiments, provided herein are methods for the treatment, prevention, and/or amelioration of edema, e.g., lymphedema comprising administering to a subject in need thereof LYT-100, wherein LYT-100, is administered orally once a day, for a total daily dose of 100-1500 mg. In some embodiments, the daily dose of the deuterium-enriched pirfenidone compound, e.g., LYT-100 is 1500 mg. In some embodiments, the daily dose of the deuterium-enriched pirfenidone compound, e.g., LYT-100, is 1000 mg. In some embodiments, the daily dose of the deuterium-enriched pirfenidone compound, e.g., LYT-100, is 750 mg. In some embodiments, the daily dose of the deuterium-enriched pirfenidone compound, e.g., LYT-100 is 500 mg. In some embodiments, the daily dose of the deuterium-enriched pirfenidone compound, e.g., LYT-100, is 250 mg. In some embodiments, the deuterium-enriched pirfenidone compound, e.g., LYT-100, is administered orally 1500 mg once daily. In some embodiments, the deuterium-enriched pirfenidone compound, e.g., LYT-100, is administered orally 1000 mg once daily. In some embodiments, the deuterium-enriched pirfenidone compound, e.g., LYT-100, is administered orally 750 mg once daily. In some embodiments, the deuterium-enriched pirfenidone compound, e.g., LYT-100, is administered orally 500 mg once daily. In some embodiments, the deuterium-enriched pirfenidone compound, e.g., LYT-100 is administered orally 250 mg once daily.

Thus, provided herein are methods for the treatment, prevention, and/or amelioration of edema, e.g., lymphedema comprising administering to a subject in need thereof a deuterium-enriched pirfenidone compound, for example, a compound of Formula I, e.g., a compound listed in Table 1, including e.g., LYT-100, wherein the deuterium-enriched pirfenidone compound is administered orally three times daily, for a total daily dose of 100-1500 mg. In some embodiments, provided herein are methods for the treatment, prevention, and/or amelioration of edema, e.g., lymphedema comprising administering to a subject in need thereof LYT-100, wherein LYT-100 is administered orally three times a day, for a total daily dose of 100-1500 mg. In some embodiments, the daily dose of the deuterium-enriched pirfenidone compound, e.g., LYT-100, is 1500 mg. In some embodiments, the daily dose of the deuterium-enriched pirfenidone compound, e.g., LYT-100, is 1000 mg. In some embodiments, the daily dose of the deuterium-enriched pirfenidone compound, e.g., LYT-100, is 750 mg. In some embodiments, the daily dose of the deuterium-enriched pirfenidone compound, e.g., LYT-100 is 500 mg. In some embodiments, the daily dose of the deuterium-enriched pirfenidone compound, e.g., LYT-100 is 250 mg. In some embodiments, the deuterium-enriched pirfenidone compound, e.g., LYT-100 is administered orally 500 mg three times daily. In some embodiments, the deuterium-enriched pirfenidone compound, e.g., LYT-100, is administered orally 333 mg three timese daily. In some embodiments, the deuterium-enriched pirfenidone compound, e.g., LYT-100, is administered orally 166 mg twice daily.

In other embodiments, the deuterium-enriched pirfenidone compound is administered orally at a total daily dose of 100-2500 mg. In some embodiments, the deuterium-enriched pirfenidone compound is administered orally at a total daily dose of 100-2000 mg. In some embodiments, the deuterium-enriched pirfenidone compound is administered orally at a total daily dose of 100-1500 mg. In some embodiments, the deuterium-enriched pirfenidone compound is administered orally at a total daily dose of 100-1000 mg. In some embodiments, the deuterium-enriched pirfenidone compound is administered orally at a total daily dose of 100-500 mg. In some embodiments, the daily dose is selected from 100, 200, 250, 300, 400, 500, 600, 700, 750, 800, 900, 1000, 1100, 1200, 1300, 1400, 1500, 1600, 1700,1800, 1900, 2000, 2100, 2200, 2300, 2400, and 2500 mg/day. In some embodiments, the deuterium-enriched pirfenidone compound is administered orally three times/day (TID). In some embodiments, the deuterium-enriched pirfenidone compound is administered orally two times/day (BID). In some embodiments, the deuterium-enriched pirfenidone compound is administered orally once daily (QD). In any of these embodiments, the deuterium-enriched pirfenidone compound has the structure of Formula I, e.g., a compound listed in Table 1, including e.g., LYT-100.

In some embodiments, the disclosure provides methods for treating edema, e.g., lymphedema, comprising administering to a subject in need thereof a deuterium-enriched pirfenidone compound disclosed herein, for example, a compound of Formula I, e.g., a compound listed in Table 1, including e.g., LYT-100. In some embodiments, the method comprises administering an effective amount of deuterium-enriched pirfenidone, e.g., the deuterated pirfenidone compound having the structure: 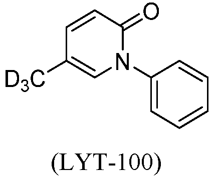 or a pharmaceutically acceptable salt thereof, wherein edema, e.g., lymphedema, is treated in the subj ect.

In some embodiments the lymphedema is secondary lymphedema. Secondary lymphedema can develop after surgery, infection or trauma, and is frequently caused by cancer, cancer treatments such as surgery, biopsy, radiation and chemotherapy, trauma or infections resulting in damage to or the removal of lymph nodes. Accordingly, in some embodiments, the disclosure provides a method for treating edema comprising administering to a subject in need thereof a deuterated pirfenidone compound having the structure:

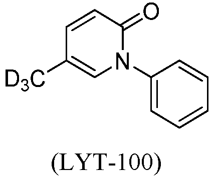

In some embodiments, the disclosure provides a method for treating lymphedema comprising administering to a subject in need thereof a deuterated pirfenidone compound having the structure:

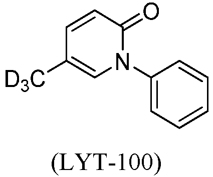

In some embodiments, the disclosure provides a method for treating secondary lymphedema comprising administering to a subject in need thereof a deuterated pirfenidone compound having the structure:

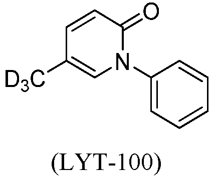

In some embodiments, the disclosure provides a method for treating primary lymphedema comprising administering to a subject in need thereof a deuterated pirfenidone compound having the structure:

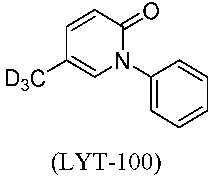

In some embodiments, the disclosure provides methods for preventing edema, e.g., lymphedema, comprising administering to a subject in need thereof a deuterium-enriched pirfenidone compound disclosed herein, for example, a compound of Formula I, e.g., a compound listed in Table 1, including e.g., LYT-100. In some embodiments, the method comprises administering an effective amount of deuterium-enriched pirfenidone, e.g., the deuterated pirfenidone compound having the structure:

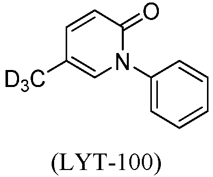

or a pharmaceutically acceptable salt thereof, wherein edema, e.g., lymphedema, is prevented in the subject. In some embodiments the lymphedema is secondary lymphedema. Secondary lymphedema can develop after surgery, infection or trauma, and is frequently caused by cancer, cancer treatments such as surgery, biopsy, radiation and chemotherapy, trauma or infections resulting in damage to or the removal of lymph nodes. Accordingly, in some embodiments, the disclosure provides a method for preventing edema comprising administering to a subject in need thereof a deuterated pirfenidone compound having the structure:

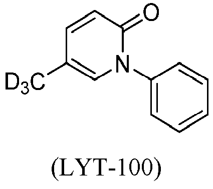

In some embodiments, the disclosure provides a method for preventing lymphedema comprising administering to a subject in need thereof a deuterated pirfenidone compound having the structure:

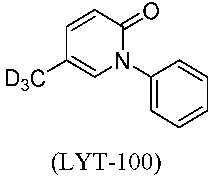

In some embodiments, the disclosure provides a method for preventing secondary lymphedema comprising administering to a subject in need thereof a deuterated pirfenidone compound having the structure:

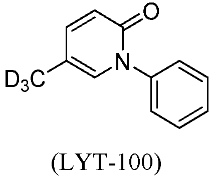

In some embodiments, the disclosure provides a method for preventing primary lymphedema comprising administering to a subject in need thereof a deuterated pirfenidone compound having the structure:

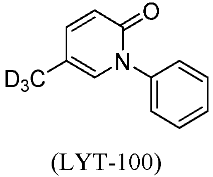

In some embodiments, the disclosure provides methods for ameliorating one or more symptoms of edema, e.g., lymphedema, comprising administering to a subject in need thereof a deuterium-enriched pirfenidone compound disclosed herein, for example, a compound of Formula I, e.g., a compound listed in Table 1, including e.g., LYT-100. In some embodiments, the method comprises administering an effective amount of deuterium-enriched pirfenidone, e.g., the deuterated pirfenidone compound having the structure: 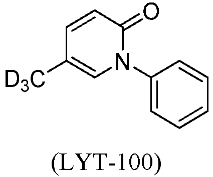 or a pharmaceutically acceptable salt thereof, wherein one or more symptoms of the edema, e.g., lymphedema, is ameliorated in the subject. In some embodiments the lymphedema is secondary lymphedema. Secondary lymphedema can develop after surgery, infection or trauma, and is frequently caused by cancer, cancer treatments such as radiation and chemotherapy, trauma or infections resulting in damage to or the removal of lymph nodes. Accordingly, in some embodiments, the disclosure provides a method for ameliorating one or more symptoms of edema comprising administering to a subject in need thereof a deuterated pirfenidone compound having the structure:

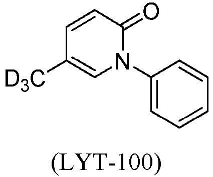

In some embodiments, the disclosure provides a method for ameliorating one or more symptoms of lymphedema comprising administering to a subject in need thereof a deuterated pirfenidone compound having the structure:

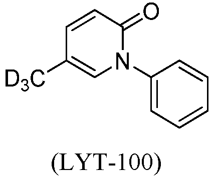

In some embodiments, the disclosure provides a method for ameliorating one or more symptoms of secondary lymphedema comprising administering to a subject in need thereof a deuterated pirfenidone compound having the structure:

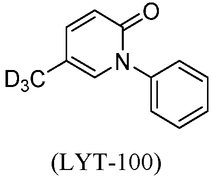

In some embodiments, the disclosure provides a method for ameliorating one or more symptoms of primary lymphedema comprising administering to a subject in need thereof a deuterated pirfenidone compound having the structure:

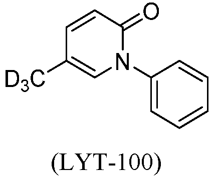

In any of the above embodiments, the one or more symptom(s) ameliorated is selected from: accumulation of fluid beneath the skin and in body cavities, swelling, fullness, swelling or puffiness of tissues, inflammation, fibrosis, heaviness, pain, disfigurement, decreased range of motion, aching, recurring infections, skin thickening, and discomfort.

In any of these embodiments for treating various forms of lymphedema, the lymphedema may occur in one or both arms, such as in the hand, wrist, forearm, elbow, upper arm, shoulder, armit, or combination of arm areas or the entire arm. In some embodiments, the lymphedema occurs in one or both legs, such as in the foot, ankle, leg, knee, upper leg or thigh, groin, hip, or combination of leg areas or the entire leg. In some embodiments, the lymphedema occurs in the head, neck, jaw, chest, breast, thorax, abdomen, pelvis, genitals, or other areas of the body cavity. In some embodiments, the lymphedema occurs in one or more limbs, or in one or more limbs and another area of the body.

In any of these embodiments for treating lymphedema the lymphedema may result from a vascular defect, including venous insufficiency, venous malformation, arterial malformation, capillary malformation, lymphovascular malformation, or cardiovascular disease.

Cellulitis is a serious, potentially life-threatening infection that can affect patients with lymphedema. Cellulitis can increase inflammation and further worsen lymphedema. Patients with lymphedema can have recurrent and progressive episodes of cellulitis requiring intravenous antibiotics. Prophylactic antibiotics are the only available intervention for trying to reduce cellulitis. In some embodiments, provided herein are methods for reducing cellulitis in a subject comprising administering to a subject in need thereof a deuterium-enriched pirfenidone compound disclosed herein, for example, a compound of Formula I, e.g., a compound listed in Table 1, including e.g., LYT-100. In some embodiments, episodes of cellulitis are reduced by at least 5%, 10%, 15%, 20%, 25%, 30%, 35%, 40%, 45%, 50%, or more. In some embodiments, the severity of the infection is decreased from severe to moderate or moderate to mild. In some embodiments, the use of treatment-related or prophylactic antibiotics is reduced.

In any of the above-described methods for treating, preventing, or ameliorating one or more symptoms of edema or lymphedema, the deuterium-enriched pirfenidone compound, e.g., LYT-100, is administered orally twice a day, for a total daily dose of 100-1500 mg. In some embodiments, the daily dose is 100, 200, 250, 300, 400, 500, 600, 700, 750, 800, 900, 1000, or 1500 mg. In some embodiments, the daily dose is 1500 mg. In some embodiments, the daily dose is 1000 mg. In some embodiments, the daily dose is 750 mg. In some embodiments, the daily dose is 500 mg. In some embodiments, the daily dose is 250 mg. In some embodiments, the deuterium-enriched pirfenidone is administered orally 750 mg twice daily. In some embodiments, the deuterium-enriched pirfenidone is administered orally 500 mg twice daily. In some embodiments, the deuterium-enriched pirfenidone is administered orally 250 mg twice daily.

In some embodiments, the deuterium-enriched pirfenidone compound, e.g., LYT-100, is administered orally once a day, for a total daily dose of 100-1500 mg. In some embodiments, the daily dose is 100, 200, 250, 300, 400, 500, 600, 700, 750, 800, 900, 1000, or 1500 mg. In some embodiments, the daily dose is 1500 mg. In some embodiments, the daily dose is 1000 mg. In some embodiments, the daily dose is 750 mg. In some embodiments, the daily dose is 500 mg. In some embodiments, the daily dose is 250 mg. In some embodiments, the deuterium-enriched pirfenidone is administered orally 1500 mg once daily. In some embodiments, the deuterium-enriched pirfenidone is administered orally 1000 mg once daily. In some embodiments, the deuterium-enriched pirfenidone is administered orally 750 mg once daily. In some embodiments, the deuterium-enriched pirfenidone is administered orally 500 mg once daily. In some embodiments, the deuterium-enriched pirfenidone is administered orally 250 mg once daily.

In any of the above-described embodiments, the deuterium-enriched pirfenidone compound, e.g., LYT-100 is administered orally three times a day, for a total daily dose of 100-1500 mg. In some embodiments, the daily dose is 100, 200, 250, 300, 400, 500, 600, 700, 750, 800, 900, 1000, or 1500 mg. In some embodiments, the daily dose is 1500 mg. In some embodiments, the daily dose is 1000 mg. In some embodiments, the daily dose is 750 mg. In some embodiments, the daily dose is 500 mg. In some embodiments, the daily dose is 250 mg. In some embodiments, the deuterium-enriched pirfenidone is administered orally 500 mg three times daily. In some embodiments, the deuterium-enriched pirfenidone is administered orally 333 mg three times daily. In some embodiments, the deuterium-enriched pirfenidone is administered orally 166 mg three times daily.

In some embodiments, the deuterium-enriched pirfenidone is in tablet form. In some embodiments, the deuterium-enriched pirfenidone is taken orally with food.

Thus, provided herein are methods for the treatment, prevention, and/or amelioration of edema, e.g.lymphedema comprising administering to a subject in need thereof a deuterium-enriched pirfenidone compound, for example, a compound of Formula I, e.g., a compound listed in Table 1, including e.g., LYT-100, wherein the deuterium-enriched pirfenidone compound is administered orally twice a day, for a total daily dose of 100-1500 mg. In some embodiments, provided herein are methods for the treatment, prevention, and/or amelioration of edeme, e.g., lymphedema comprising administering to a subject in need thereof LYT-100, wherein LYT-100 is administered orally twice a day, for a total daily dose of 100-1500 mg. In some embodiments, the daily dose of the deuterium-enriched pirfenidone compound, e.g., LYT-100, is 1500 mg. In some embodiments, the daily dose of the deuterium-enriched pirfenidone compound, e.g., LYT-100, is 1000 mg. In some embodiments, the daily dose of the deuterium-enriched pirfenidone compound, e.g., LYT-100, is 750 mg. In some embodiments, the daily dose of the deuterium-enriched pirfenidone compound, e.g., LYT-100 is 500 mg. In some embodiments, the daily dose of the deuterium-enriched pirfenidone compound, e.g., LYT-100 is 250 mg. In some embodiments, the deuterium-enriched pirfenidone compound, e.g., LYT-100 is administered orally 750 mg twice daily. In some embodiments, the deuterium-enriched pirfenidone compound, e.g., LYT-100, is administered orally 500 mg twice daily. In some embodiments, the deuterium-enriched pirfenidone compound, e.g., LYT-100, is administered orally 250 mg twice daily.

In some embodiments, provided herein are methods for the treatment, prevention, and/or amelioration of edema, e.g., lymphedema comprising administering to a subject in need thereof a deuterirum-enriched pirfenidone compound, for example, a compound of Formula I, e.g., a compound listed in Table 1, including e.g., LYT-100, wherein the deuterirum-enriched pirfenidone compound is administered orally once a day, for a total daily dose of 100-1500 mg. In some embodiments, provided herein are methods for the treatment, prevention, and/or amelioration of edema, e.g., lymphedema comprising administering to a subject in need thereof LYT-100, wherein LYT-100, is administered orally once a day, for a total daily dose of 100-1500 mg. In some embodiments, the daily dose of the deuterium-enriched pirfenidone compound, e.g., LYT-100 is 1500 mg. In some embodiments, the daily dose of the deuterium-enriched pirfenidone compound, e.g., LYT-100, is 1000 mg. In some embodiments, the daily dose of the deuterium-enriched pirfenidone compound, e.g., LYT-100, is 750 mg. In some embodiments, the daily dose of the deuterium-enriched pirfenidone compound, e.g., LYT-100 is 500 mg. In some embodiments, the daily dose of the deuterium-enriched pirfenidone compound, e.g., LYT-100, is 250 mg. In some embodiments, the deuterium-enriched pirfenidone compound, e.g., LYT-100, is administered orally 1500 mg once daily. In some embodiments, the deuterium-enriched pirfenidone compound, e.g., LYT-100, is administered orally 1000 mg once daily. In some embodiments, the deuterium-enriched pirfenidone compound, e.g., LYT-100, is administered orally 750 mg once daily. In some embodiments, the deuterium-enriched pirfenidone compound, e.g., LYT-100, is administered orally 500 mg once daily. In some embodiments, the deuterium-enriched pirfenidone compound, e.g., LYT-100 is administered orally 250 mg once daily.

Thus, provided herein are methods for the treatment, prevention, and/or amelioration of edema, e.g., lymphedema comprising administering to a subject in need thereof a deuterium-enriched pirfenidone compound, for example, a compound of Formula I, e.g., a compound listed in Table 1, including e.g., LYT-100, wherein the deuterium-enriched pirfenidone compound is administered orally three times daily, for a total daily dose of 100-1500 mg. In some embodiments, provided herein are methods for the treatment, prevention, and/or amelioration of edema, e.g., lymphedema comprising administering to a subject in need thereof LYT-100, wherein LYT-100 is administered orally three times a day, for a total daily dose of 100-1500 mg. In some embodiments, the daily dose of the deuterium-enriched pirfenidone compound, e.g., LYT-100, is 1500 mg. In some embodiments, the daily dose of the deuterium-enriched pirfenidone compound, e.g., LYT-100, is 1000 mg. In some embodiments, the daily dose of the deuterium-enriched pirfenidone compound, e.g., LYT-100, is 750 mg. In some embodiments, the daily dose of the deuterium-enriched pirfenidone compound, e.g., LYT-100 is 500 mg. In some embodiments, the daily dose of the deuterium-enriched pirfenidone compound, e.g., LYT-100 is 250 mg. In some embodiments, the deuterium-enriched pirfenidone compound, e.g., LYT-100 is administered orally 500 mg three times daily. In some embodiments, the deuterium-enriched pirfenidone compound, e.g., LYT-100, is administered orally 333 mg three timese daily. In some embodiments, the deuterium-enriched pirfenidone compound, e.g., LYT-100, is administered orally 166 mg twice daily.

In other embodiments, the deuterium-enriched pirfenidone compound is administered orally at a total daily dose of 100-2500 mg. In some embodiments, the deuterium-enriched pirfenidone compound is administered orally at a total daily dose of 100-2000 mg. In some embodiments, the deuterium-enriched pirfenidone compound is administered orally at a total daily dose of 100-1500 mg. In some embodiments, the deuterium-enriched pirfenidone compound is administered orally at a total daily dose of 100-1000 mg. In some embodiments, the deuterium-enriched pirfenidone compound is administered orally at a total daily dose of 100-500 mg. In some embodiments, the daily dose is selected from 100, 200, 250, 300, 400, 500, 600, 700, 750, 800, 900, 1000, 1100, 1200, 1300, 1400, 1500, 1600, 1700,1800, 1900, 2000, 2100, 2200, 2300, 2400, and 2500 mg/day. In some embodiments, the deuterium-enriched pirfenidone compound is administered orally three times/day (TID). In some embodiments, the deuterium-enriched pirfenidone compound is administered orally two times/day (BID). In some embodiments, the deuterium-enriched pirfenidone compound is administered orally once daily (QD). In any of these embodiments, the deuterium-enriched pirfenidone compound has the structure of Formula I, e.g., a compound listed in Table 1, including e.g., LYT-100.

Lymphedema typically progresses through multiple stages, with increased fibrosis, limb volume and tissue changes. Of more than 250,000 Americans estimated to be diagnosed with breast cancer each year that undergo surgery, up to one in five will develop secondary lymphedema. Beyond breast cancer, lymphedema can occur in up to 15 percent of cancer survivors with malignancies ranging from melanoma and sarcoma. A subset of lymphedema patients will also experience cellulitis, a bacterial skin infection that can enter through wounds in lymphedematous skin. Cellulitis often requires hospitalization and intravenous antibiotics to treat, and approximately half of patients with cellulitis will have recurrent episodes. In some rare instances, patients with chronic lymphedema may develop lymphangiosarcoma, a malignant tumor. Lymphedema is classified by clinical staging and severity, as shown in the table below.

| **Table 2: Clinical Stages of Lymphedema** | | | |
|---|---|---|---|
| | Stage I | Stage II | Stage III |
| Symptoms | Limb swelling, pitting edema, limb heaviness and discomfort | Limb swelling, skin thickening, dermal fibrosis, fat deposition, non-pitting edema | Disfiguring limb swelling, hyperkeratosis, loss of skin elasticity, skin lesions and overgrowths, massive fibrosis and fat deposition, elephantiasis |
| Additional Clinical Concerns | Lifelong need for compression therapy, chronic progression, repeated infections (cellulitis, lymphangitis), elephantine skin changes, development of lymphangiosarcoma | | |

In some embodiments, the subject or patient has Stage I lymphedema. In some embodiments, the subject or patient has Stage II lymphedema. In some embodiments, the subject or patient has Stage III lymphedema. In some embodiments, the subject or patient is reduced in stage from Stage III to Stage II or Stage I, or from Stage II to Stage I.

The International Society of Lymphology classifies a lymphedematous limb based on staging that describes the condition of the limb. As the disease progresses into later stages, the affected limb can acquire a "woody texture" due to fibrosis. In addition to clinical staging, clinicians use a measurement of limb swelling to capture disease severity. Cancer treatments lead to new lymphedema patients each year, the majority of which will have mild lymphedema: over 70 percent of patients with secondary lymphedema have milder forms of lymphedema, while the remainder have moderate to severe lymphedema. The table below summarizes the percentage of secondary breast cancer-related lymphedema patients who experience various stages of severity of lymphedema.

| **Table 3: Severity of Secondary Lymphedema** | | | |
|---|---|---|---|
| | Mild | Moderate | Severe |
| Relative Limb Volume Change | 5-20% | 20-40% | >40% |
| Percentage Patients | 73% | | 27% |

Accordingly, in some embodiments, patients have mild, moderate or severe secondary lymphedema. In some embodiments, patients have mild to moderate secondary lymphedema. In some embodiments, patient have moderate to severe secondary lymphedema. In some embodiments, patients have mild to severe secondary lymphedema.

The natural history of lymphedema is a chronic and progressive disorder, reflected in the increasing severity of limb swelling. The relative increase of limb volume in the affected limb compared to the unaffected limb worsens over time. In patients with mild lymphedema, approximately 48 percent will progress to more severe stages during the first five years of follow-up. Because of the progressive nature of the disease, many patients will progress to the point where bandaging and compression are incapable of reducing limb volume. The potential loss of limb range of motion and function, the risk of secondary infections and complications and the disfigurement result in physical and emotional suffering in cancer survivors. Secondary lymphedema is a lifelong disease and the affected population is increasing each year due to improved survival of cancer patients, changes in patient and disease factors, including obesity, an aging population and increased use of radiation treatment.

Millions of patients have lymphedema beyond breast cancer-related arm lymphedema. The deuterium-enriched pirfenidone compounds disclosed here, e.g., LYT-100 can be used to treat the underlying mechanisms of other forms of secondary or primary lymphedema, for example, lymphatic filariasis.

The deuterium-enriched pirfenidone compounds disclosed here, e.g., LYT-100 can be used to treat various forms of lymphedema. In some embodiments, the lymphedema to be treated occurs in one or both arms, such as in the hand, wrist, forearm, elbow, upper arm, shoulder, armit, or combination of arm areas or the entire arm. In some embodiments, the lymphedema occurs in one or both legs, such as in the foot, ankle, leg, knee, upper leg or thigh, groin, hip, or combination of leg areas or the entire leg. In some embodiments, the lymphedema occurs in the head, neck, jaw, chest, breast, thorax, abdomen, pelvis, genitals, or other areas of the body cavity. In some embodiments, the lymphedema occurs in one or more limbs, or in one or more limbs and another area of the body.

In any of these embodiments for treating lymphedema the lymphedema may result from a vascular defect, including venous insufficiency, venous malformation, arterial malformation, capillary malformation, lymphovascular malformation, or cardiovascular disease.

The deuterium-enriched pirfenidone compounds disclosed here, e.g., LYT-100 can be used to treat cellulitis, which is a serious, potentially life-threatening infection that can affect patients with lymphedema. In some embodiments, provided herein are methods for reducing cellulitis in a subject comprising administering to a subject in need thereof a deuterium-enriched pirfenidone compound disclosed herein, for example, a compound of Formula I, e.g., a compound listed in Table 1, including e.g., LYT-100.

In some embodiments, the patients have had breast cancer surgery at least 3, 6, 9, or 12 months prior, and who have completed radiation treatment due to breast cancer at least one, two, three, four, five, six, seven, eight, nine, ten, eleven, or twelve months prior. In some embodiment, they are without recurrent cancer more than 6 months after the breast cancer surgery. In some embodiments, patients are those having pitting edema and at least one of the following: increase in relative limb volume of between 10-20% as measured by the truncated cone method of circumferential tape measurement, or a bioimpedance measure of > +6.5 L-Dex. In some embodiments, patients are also on standard of care compression or have a relative limb volume > 10% or L-Dex > 14 as compared to pre-surgery and/or pre-radiation volumes. Thus, in some embodiments, the disclosure provides a method for treating lymphedema comprising administering to a subject in need thereof LYT-100, wherein the subject has an increase in relative limb volume of at least 10% as compared to pre-treatment limb volumes. Thus, in some embodiments, the disclosure provides a method for treating lymphedema comprising administering to a subject in need thereof LYT-100, wherein the subject has an increase in relative limb volume of between 10-20% as compared to pre-treatment limb volumes. In some embodiments, the disclosure provides a method for treating lymphedema comprising administering to a subject in need thereof LYT-100, wherein the subject has an increase in relative limb volume of greater than 20% as compared to pre-treatment limb volumes. In some embodiments, the disclosure provides a method for treating lymphedema comprising administering to a subject in need thereof LYT-100, wherein the subject has an increase in relative limb volume of between 20% - 40% as compared to pre-treatment limb volumes. In some embodiments, the disclosure provides a method for treating lymphedema comprising administering to a subject in need thereof LYT-100, wherein the subject has an increase in relative limb volume of greater than 40% as compared to pre-treatment limb volumes. In some embodiments, the disclosure provides a method for treating lymphedema comprising administering to a subject in need thereof LYT-100, wherein the subject has a bioimpedance measure of at least +6.5 L-Dex as compared with pre-treatment limb volumes. In some embodiments, the disclosure provides a method for treating lymphedema comprising administering to a subject in need thereof LYT-100, wherein the subject has a bioimpedance measure of at least +14 L-Dex as compared with pre-treatment limb volumes.

In some embodiments, the disclosure provides a method for treating edema, e.g., lymphedema comprising administering to a subject in need thereof a deuterium-enriched pirfenidone compound, e.g., a compound of Formula 1, including those compounds listed in Table 1, wherein treatment is demonstrated by the subject having an improvement in one or more of the measurements selected from: a) bioimpedance (as measured, e.g., by BIS), b) limb volume (as measured, e.g., by a perometer or tape measure), c) local tissue water content (as measured, e.g., by the tissue dielectric constant), d) tissue firmness (as measured, e.g., by tonometric device), e) fibrosis (e.g., as measured by tissue firmness), f) pain, g) swelling, h) discomfort, i) function, j) visual-analog pain score, h) Upper Limb Lymphedema Score (ULL27), i) Lymphema Life Impact Scale (LLIS), j) Functional Assessment of Cancer Therapy score (FACT-B+4), k) Lymphedema Quality of Life score (LYMQOL); 1) Disabilities of the Arm, Shoulder, and Hand score (DASH); m) Lymphedema Quality of Life Inventory (LQOLI), n) Granulocyte Colony Stimulating Factor (G-CSF), o) cutaneous histological architecture (CHA); and p) skin thckness (as measured, e.g., by calipers).

In some embodiments, the disclosure provides a method for ameliorating one or more symptoms of edema, e.g., lymphedema comprising administering to a subject in need thereof LYT-100, wherein amelioration of one or more symptoms is demonstrated by the subject having an improvement in one or more of the measurements selected from: a) bioimpedance (as measured, e.g., by BIS), b) limb volume (as measured, e.g., by a perometer or tape measure), c) local tissue water content (as measured, e.g., by the tissue dielectric constant), d) tissue firmness (as measured, e.g., by tonometric device), e) fibrosis (e.g., as measured by tissue firmness), f) pain, g) swelling, h) discomfort, i) function, j) visual-analog pain score, h) Upper Limb Lymphedema Score (ULL27), i) Lymphema Life Impact Scale (LLIS), j) Functional Assessment of Cancer Therapy score (FACT-B+4), k) Lymphedema Quality of Life score (LYMQOL); 1) Disabilities of the Arm, Shoulder, and Hand score (DASH); m) Lymphedema Quality of Life Inventory (LQOLI), n) Granulocyte Colony Stimulating Factor (G-CSF), o) cutaneous histological architecture (CHA); and p) skin thckness (as measured, e.g., by calipers).

Bioimpedance, or water content, can be measured via Bioelectrical impedance spectroscopy (BIS). Multiple frequency bioelectrical impedance spectroscopy (BIS) provides accurate relative measures of protein-rich fluid in the upper limb of patients. BIS is a noninvasive technique that involves passing an extremely small electrical current through the body and measuring the impedance (or resistance) to the flow of this current. The electrical current is primarily conducted by the water containing fluids in the body. BIS quantifies the amount of protein-rich fluid in lymphedema by comparison of the affected and non-affected limbs. In some embodiments, the disclosure provides methods for decreasing bioimpedance in the limb of a subject comprising administering to the subject a deuterium-enriched pirfenidone compound disclosed herein, for example, a compound of Formula I, e.g., a compound listed in Table 1. In some embodiments, the deuterium-enriched pirfenidone compound is LYT-100. Thus, in some embodiments, the disclosure provides methods for decreasing bioimpedance in a subject with edema, e.g.,lymphedema, comprising administering an effective amount of LYT-100. In some embodiments, provided herein are methods for treating lymphedema comprising administering to a subject having lymphedema in at least one limb a deuterium-enriched pirfenidone compound, e.g., LYT-100, wherein the bioimpedance in the subject's limb is decreased as compared to the bioimpedance in the subject's limb prior to the administration of the deuterium-enriched pirfenidone compound, e.g., LYT-100. In some embodiments, bioimpedance is significantly decreased by 3, 4 or 6 months. In some embodiments of the disclosed methods, bioimpedance is decreased by 2%, 3%, 4%, 5%, 6%, 7%, 8%, 9%, 10%, 15%, 20%, 25%, 30%, 35%, 40%, 45%, 50% (and any numerical increment between), or more as compared to bioimpedance in the subject's limb prior to the administration of the deuterium-enriched pirfenidone compound. This decrease is seen in 3, 4, or 6 months in some embodiments. Thus, in some embodiments, the disclosure provides a method for decreasing bioimpedance in the limb of a subject comprising administering to the subject LYT-100, wherein the bioimpedance is decreased. In some embodiments, the disclosure provides a method for decreasing bioimpedance in the limb of a subject comprising administering to the subject LYT-100, wherein the bioimpedance is decreased as compared to bioimpedance in the subject's limb prior to the administration of LYT-100. In some embodiments, the disclosure provides a method for decreasing bioimpedance in the limb of a subject comprising administering to the subject LYT-100, wherein the bioimpedance is decreased by at least 2% as compared to bioimpedance in the subject's limb prior to the administration of LYT-100. In some embodiments, the disclosure provides a method for decreasing bioimpedance in the limb of a subject comprising administering to the subject LYT-100, wherein the bioimpedance is decreased by at least 3%, at least 4%, at least 5%, at least 6%, at least 7%, at least 8%, at least 9%, at least 10%, at least 11%, at least 12%, at least 13%, at least 14% at least 15%, at least 16%, at least 17%, at least 18%, at least 19%, or at least 20% as compared to bioimpedance in the subject's limb prior to the administration of LYT-100. In some embodiments, the disclosure provides a method for decreasing bioimpedance in the limb of a subject comprising administering to the subject LYT-100, wherein the bioimpedance is decreased by greater than 20% as compared to bioimpedance in the subject's limb prior to the administration of LYT-100.

Limb Volume (Perometry). Relative limb volume can be measured by the truncated cone method of circumferential tape measurement. Perometry is a noninvasive technique involving a Perometer (Pero-System), which uses infrared light to scan a limb and obtain measurements of the limb's circumference. In some embodiments, the disclosure provides methods for reducing (decreasing) limb volume in the limb of a subject comprising administering to the subject a deuterium-enriched pirfenidone compound disclosed herein, for example, a compound of Formula I, e.g., a compound listed in Table 1. In some embodiments, the deuterium-enriched pirfenidone compound is LYT-100. Thus, in some embodiments, the disclosure provides methods for decreasing limb volume in a subject with edema, e.g., lymphedema, comprising administering an effective amount of LYT-100. In some embodiments, provided herein are methods for treating lymphedema comprising administering to a subject having lymphedema in at least one limb a deuterium-enriched pirfenidone compound, e.g., LYT-100, wherein the limb volume in the subject's limb is decreased as compared to the limb volume in the subject's limb prior to the administration of the deuterium-enriched pirfenidone compound, e.g., LYT-100. In some embodiments, limb volume is decreased by at least 2%, 3%, 4%, 5%, 6%, 7%, 8%, 9%, 10%, 15%, 20%, 25%, 30%, 35%, 40%, 45%, 50%, or more as compared to limb volume in the subject's limb prior to administration of the deuterium-enriched pirfenidone. In some embodiments, the severity is decreased from severe to moderate or moderate to mild. In some embodiments, this decrease in limb volume is seen in 3, 4, or 6 months. Thus, in some embodiments, the disclosure provides a method for decreasing limb volume in the limb of a subject comprising administering to the subject LYT-100, wherein the limb volume is decreased. In some embodiments, the disclosure provides a method for decreasing limb volume in the limb of a subject comprising administering to the subject LYT-100, wherein the limb volume is decreased as compared to limb volume in the subject's limb prior to the administration of LYT-100. In some embodiments, the disclosure provides a method for decreasing limb volume in the limb of a subject comprising administering to the subject LYT-100, wherein the limb volume is decreased by at least 2% as compared to limb volume in the subject's limb prior to the administration of LYT-100. In some embodiments, the disclosure provides a method for decreasing limb volume in the limb of a subject comprising administering to the subject LYT-100, wherein the limb volume is decreased by at least 3%, at least 4%, at least 5%, at least 6%, at least 7%, at least 8%, at least 9%, at least 10%, at least 11%, at least 12%, at least 13%, at least 14% at least 15%, at least 16%, at least 17%, at least 18%, at least 19%, or at least 20% as compared to limb volume in the subject's limb prior to the administration of LYT-100. In some embodiments, the disclosure provides a method for decreasing limb volume in the limb of a subject comprising administering to the subject LYT-100, wherein the limb volume is decreased by greater than 20% as compared to limb volume in the subject's limb prior to the administration of LYT-100. In some embodiments, the disclosure provides a method for decreasing limb volume in the limb of a subject comprising administering to the subject LYT-100, wherein the limb volume is decreased by 20% - 40% as compared to limb volume in the subject's limb prior to the administration of LYT-100. In some embodiments, the disclosure provides a method for decreasing limb volume in the limb of a subject comprising administering to the subject LYT-100, wherein the limb volume is decreased by greater than 40% as compared to limb volume in the subject's limb prior to the administration of LYT-100.

Tissue Dielectric Constant (MoistureMeterD). The tissue dielectric constant measures the local tissue water content under the skin at various depths ranging from skin to subcutis. The results are converted into a 0-100% scale to reflect subcutaneous fluid deposition that can occur in early stage lymphedema. In some embodiments, the disclosure provides methods for decreasing the tissue dielectric constant in a subject comprising administering to the subject a deuterium-enriched pirfenidone compound disclosed herein, for example, a compound of Formula I, e.g., a compound listed in Table 1. In some embodiments, the deuterium-enriched pirfenidone compound is LYT-100. In some embodiments, provided herein are methods for treating lymphedema comprising administering to a subject having lymphedema in at least one limb a deuterium-enriched pirfenidone compound, e.g., LYT-100, wherein the tissue dielectric constant in the subject's limb is decreased as compared to the tissue dielectric constant in the subject's limb prior to the administration of the deuterium-enriched pirfenidone compound, e.g., LYT-100. Thus, in some embodiments, the disclosure provides methods for decreasing the tisssue dielectric constant in the limb of a subject with edema, e.g., lymphedema, comprising administering an effective amount of LYT-100. In some embodiments, the tissue dielectric constant is decreased by 2%, 3%, 4%, 5%, 6%, 7%, 8%, 9%, 10%, 15%, 20%, 25%, 30%, 35%, 40%, 45%, 50%, or more as compared to the tissue dielectric constant in the subject's limb prior to administration of the deuterium-enriched pirfenidone. In some embodiments, the disclosure provides a method for decreasing the tissue dielectric constant in the limb of a subject comprising administering to the subject LYT-100, wherein the tissue dielectric constant is decreased. In some embodiments, the disclosure provides a method for decreasing the tissue dielectric constant in the limb of a subject comprising administering to the subject LYT-100, wherein the tissue dielectric constant is decreased as compared to the tissue dielectric constant in the subject's limb prior to the administration of LYT-100. In some embodiments, the disclosure provides a method for decreasing the tissue dielectric constant in the limb of a subject comprising administering to the subject LYT-100, wherein the tissue dielectric constant is decreased by at least 2% as compared to the tissue dielectric constant in the subject's limb prior to the administration of LYT-100. In some embodiments, the disclosure provides a method for decreasing the tissue dielectric constant in the limb of a subject comprising administering to the subject LYT-100, wherein the tissue dielectric constant is decreased by at least 3%, at least 4%, at least 5%, at least 6%, at least 7%, at least 8%, at least 9%, at least 10%, at least 11%, at least 12%, at least 13%, at least 14% at least 15%, at least 16%, at least 17%, at least 18%, at least 19%, or at least 20% as compared to the tissue dielectric constant in the subject's limb prior to the administration of LYT-100. In some embodiments, the disclosure provides a method for decreasing the tissue dielectric constant in the limb of a subject comprising administering to the subject LYT-100, wherein the tissue dielectric constant is decreased by greater than 20% as compared to the tissue dielectric constant in the subject's limb prior to the administration of LYT-100.

Tissue Firmness (Tonometry/SkinFibroMeter). A tonometer device is pressed into the skin to measure the amount of force required to make an indent in the tissue. The resulting measurement gauges the degree of firmness or fibrosis (tissue scarring) under the skin to assess the severity of lymphedema. In some embodiments, the disclosure provides methods for decreasing tissue firmness in the limb of a subject comprising administering to the subject a deuterium-enriched pirfenidone compound disclosed herein, for example, a compound of Formula I, e.g., a compound listed in Table 1. In some embodiments, the deuterium-enriched pirfenidone is LYT-100. Thus, in some embodiments, the disclosure provides methods for decreasing tissue firmness in the limb of a subject with edema, e.g., lymphedema, comprising administering an effective amount of LYT-100. In some embodiments, provided herein are methods for treating lymphedema comprising administering to a subject having lymphedema in at least one limb a deuterium-enriched pirfenidone compound, e.g., LYT-100, wherein the tissue firmness in the subject's limb is decreased as compared to the tissue firmness in the subject's limb prior to the administration of deuterium-enriched pirfenidone compound, e.g., LYT-100. In some embodiments, tissue firmness is decreased by 2%, 3%, 4%, 5%, 6%, 7%, 8%, 9%, 10%, 15%, 20%, 25%, 30%, 35%, 40%, 45%, 50%, or more as compared to the tissue firmness in the subject's limb prior to the administration of the deuterium-enriched pirfenidone compound. In some embodiments, the disclosure provides a method for decreasing the tissue firmness in the limb of a subject comprising administering to the subject LYT-100, wherein the tissue firmness is decreased. In some embodiments, the disclosure provides a method for decreasing the tissue firmness in the limb of a subject comprising administering to the subject LYT-100, wherein the tissue firmness is decreased as compared to the tissue firmness in the subject's limb prior to the administration of LYT-100. In some embodiments, the disclosure provides a method for decreasing the tissue firmness in the limb of a subject comprising administering to the subject LYT-100, wherein the tissue firmness is decreased by at least 2% as compared to the tissue firmness in the subject's limb prior to the administration of LYT-100. In some embodiments, the disclosure provides a method for decreasing the tissue firmness in the limb of a subject comprising administering to the subject LYT-100, wherein the tissue firmness is decreased by at least 3%, at least 4%, at least 5%, at least 6%, at least 7%, at least 8%, at least 9%, at least 10%, at least 11%, at least 12%, at least 13%, at least 14% at least 15%, at least 16%, at least 17%, at least 18%, at least 19%, or at least 20% as compared to the tissue firmness in the subject's limb prior to the administration of LYT-100. In some embodiments, the disclosure provides a method for decreasing the tissue firmness in the limb of a subject comprising administering to the subject LYT-100, wherein the tissue firmness is decreased by greater than 20% as compared to the tissue firmness in the subject's limb prior to the administration of LYT-100.

Visual-analogue scales for pain, swelling, discomfort, and function. This graphic scale has a straight line with endpoints from 0 to 10 that is marked by the patient to correlate to their extreme limits of pain, swelling, discomfort and function, ranging from "not at all" to "as bad as it could be." The higher marks on the line indicates the worse condition. In some embodiments, the disclosure provides methods for reducing one or more visual-analog pain scores in a subject comprising administering to the subject a deuterium-enriched pirfenidone compound disclosed herein, for example, a compound of Formula I, e.g., a compound listed in Table 1. In some embodiments, the deuterium-enriched pirfenidone compound is LYT-100. Thus, in some embodiments, the disclosure provides methods for reducing one or more visual-analog pain scores in a subject with edema, e.g., lymphedema, comprising administering an effective amount of LYT-100. In some embodiments, provided herein are methods for treating lymphedema comprising administering to a subject having lymphedema a deuterium-enriched pirfenidone compound, e.g., LYT-100, wherein one or more of the subject's visual-analog pain score(s) is decreased as compared to the subject's visual-analog pain score(s) prior to the administration of the deuterium-enriched pirfenidone compound, e.g., LYT-100. In some embodiments, one or more visual-analog pain score(s) is decreased by 2%, 3%, 4%, 5%, 6%, 7%, 8%, 9%, 10%, 15%, 20%, 25%, 30%, 35%, 40%, 45%, 50%, or more as compared to the corresponding visual-analog pain score(s) in the subject prior to the administration of the deuterium-enriched pirfenidone compound. In some embodiments, the disclosure provides a method for decreasing one or more visual-analog pain score(s) in a subject comprising administering to the subject LYT-100, wherein the one or more visual-analog pain score(s) is decreased. In some embodiments, the disclosure provides a method for decreasing one or more visual-analog pain score(s) in a subject comprising administering to the subject LYT-100, wherein the one or more visual-analog pain score(s) is decreased as compared to the one or more visual-analog pain score(s) in the subject prior to the administration of LYT-100. In some embodiments, the disclosure provides a method for decreasing one or more visual-analog pain score(s) in a subject comprising administering to the subject LYT-100, wherein the one or more visual-analog pain score(s) is decreased by at least 2% as compared to the one or more visual-analog pain score(s) in the subject prior to the administration of LYT-100. In some embodiments, the disclosure provides a method for decreasing one or more visual-analog pain score(s) in a subject comprising administering to the subject LYT-100, wherein the one or more visual-analog pain score(s) is decreased by at least 3%, at least 4%, at least 5%, at least 6%, at least 7%, at least 8%, at least 9%, at least 10%, at least 11%, at least 12%, at least 13%, at least 14% at least 15%, at least 16%, at least 17%, at least 18%, at least 19%, or at least 20% as compared to the one or more visual-analog pain score(s) in the subject prior to the administration of LYT-100. In some embodiments, the disclosure provides a method for decreasing one or more visual-analog pain score(s) in a subject comprising administering to the subject LYT-100, wherein the one or more visual-analog pain score(s) is decreased by greater than 20% as compared to the one or more visual-analog pain score(s) in the subject prior to the administration of LYT-100.

Upper Limb Lymphedema Score 27 (ULL27) is a self-report tool consisting of 27 questions to evaluate arm lymphedema and associated symptoms in breast cancer survivors. Responses are given on a 5-point Likert scale ranging from "never" to "always." At least the following domains are addressed: physical (15 items), psychological (7 items) and social (5 items), with scores ranging from 0 to 100 (100 being the highest score possible). Lower scores indicate a higher quality of life. In some embodiments, the disclosure provides methods for decreasing the ULL27 in a subject comprising administering to the subject a deuterium-enriched pirfenidone compound disclosed herein, for example, a compound of Formula I, e.g., a compound listed in Table 1. In some embodiments, the deuterium-enriched pirfenidone is LYT-100. Thus, in some embodiments, the disclosure provides methods for decreasing the ULL27 in a subject with edema, e.g., lymphedema, comprising administering an effective amount of LYT-100. In some embodiments, provided herein are methods for treating lymphedema comprising administering to a subject having lymphedema in at least one limb a deuterium-enriched pirfenidone compound, e.g., LYT-100, wherein the subject's ULL27 score is decreased as compared to the subject's ULL27 score prior to the administration of the deuterium-enriched pirfenidone compound, e.g., LYT-100. In some embodiments, the ULL27 is decreased by 2%, 3%, 4%, 5%, 6%, 7%, 8%, 9%, 10%, 15%, 20%, 25%, 30%, 35%, 40%, 45%, 50%, or more as compared to the ULL27 in the subject prior to administration of the deuterium-enriched pirfenidone compound. In some embodiments, the disclosure provides a method for decreasing the ULL27 in a subject comprising administering to the subject LYT-100, wherein the ULL27 is decreased. In some embodiments, the disclosure provides a method for decreasing the ULL27 in a subject comprising administering to the subject LYT-100, wherein the ULL27 is decreased as compared to the ULL27 in the subject prior to the administration of LYT-100. In some embodiments, the disclosure provides a method for decreasing the ULL27 in a subject comprising administering to the subject LYT-100, wherein the ULL27 is decreased by at least 2% as compared to the ULL27 in the subject prior to the administration of LYT-100. In some embodiments, the disclosure provides a method for decreasing the ULL27 in the limb of a subject comprising administering to the subject LYT-100, wherein the ULL27 is decreased by at least 3%, at least 4%, at least 5%, at least 6%, at least 7%, at least 8%, at least 9%, at least 10%, at least 11%, at least 12%, at least 13%, at least 14% at least 15%, at least 16%, at least 17%, at least 18%, at least 19%, or at least 20% as compared to the ULL27 in the subject prior to the administration of LYT-100. In some embodiments, the disclosure provides a method for decreasing the ULL27 in the limb of a subject comprising administering to the subject LYT-100, wherein the ULL27 is decreased by greater than 20% as compared to the ULL27 in the subject prior to the administration of LYT-100.

Lymphedema Life Impact Scale (LLIS) is a comprehensive lymphedema-specific instrument to measure impairments, activity limitations, and participation restrictions in patients with any extremity lymphedema. It is an 18-question assessment tool that includes physical, psychosocial, and functional domains. The Life Impact Scale is designed to work in conjunction with an impairment calculator to determine the impairment severity. In some embodiments, the disclosure provides methods for reducing or lowering the LLIS of a subject (e.g., lessening the impairment severity) comprising administering to the subject a deuterium-enriched pirfenidone compound disclosed herein, for example, a compound of Formula I, e.g., a compound listed in Table 1. In some embodiments, the deuterium-enriched pirfenidone is LYT-100. Thus, in some embodiments, the disclosure provides methods for reducing the LLIS in a subject with edema, e.g., lymphedema, comprising administering an effective amount of LYT-100. In some embodiments, provided herein are methods for treating lymphedema comprising administering to a subject having lymphedema in at least one limb a deuterium-enriched pirfenidone compound, e.g., LYT-100, wherein the LLIS in the subject is decreased as compared to the LLIS in the subject prior to the administration of the deuterium-enriched pirfenidone compound, e.g., LYT-100. In some embodiments, LLIS is decreased by 2%, 3%, 4%, 5%, 6%, 7%, 8%, 9%, 10%, 15%, 20%, 25%, 30%, 35%, 40%, 45%, 50%, or more as compared to the LLIS in the subject prior to the administration of the deuterium-enriched pirfenidone compound. In some embodiments, the disclosure provides a method for decreasing the LLIS in a subject comprising administering to the subject LYT-100, wherein the LLIS is decreased. In some embodiments, the disclosure provides a method for decreasing the LLIS in a subject comprising administering to the subject LYT-100, wherein the LLIS is decreased as compared to the LLIS in the subject prior to the administration of LYT-100. In some embodiments, the disclosure provides a method for decreasing the LLIS in a subject comprising administering to the subject LYT-100, wherein the LLIS is decreased by at least 2% as compared to the LLIS in the subject prior to the administration of LYT-100. In some embodiments, the disclosure provides a method for decreasing the LLIS in a subject comprising administering to the subject LYT-100, wherein the LLIS is decreased by at least 3%, at least 4%, at least 5%, at least 6%, at least 7%, at least 8%, at least 9%, at least 10%, at least 11%, at least 12%, at least 13%, at least 14% at least 15%, at least 16%, at least 17%, at least 18%, at least 19%, or at least 20% as compared to the LLIS in the subject prior to the administration of LYT-100. In some embodiments, the disclosure provides a method for decreasing the LLIS in a subject comprising administering to the subject LYT-100, wherein the LLIS is decreased by greater than 20% as compared to the LLIS in the subject prior to the administration of LYT-100.

Functional Assessment of Cancer Therapy breast cancer-specific quality of life tool (FACT-B +4) is a five-point Likert scale where a greater quality of life corresponds to a high score once negatively phrased item scores are reversed. Scores are calculated by summing the subscale scores for physical well-being, social well-being, emotional well-being, functional well-being, and breast cancer additional concerns subscales. In some embodiments, the disclosure provides methods for increasing the FACT-B+4 score of a subject (e.g., improving quality of life) comprising administering to the subject a deuterium-enriched pirfenidone compound disclosed herein, for example, a compound of Formula I, e.g., a compound listed in Table 1. In some embodiments, the deuterium-enriched pirfenidone is LYT-100. Thus, in some embodiments, the disclosure provides methods for increasing the FACT-B+4 score in a subject with edema, e.g., lymphedema, comprising administering an effective amount of LYT-100. In some embodiments, provided herein are methods for treating lymphedema comprising administering to a subject having lymphedema a deuterium-enriched pirfenidone compound, e.g., LYT-100, wherein the FACT-B+4 score is increased in the subject as compared to the FACT-B+4 score in the subject prior to the administration of the deuterium-enriched pirfenidone compound, e.g., LYT-100. In some embodiments, the FACT-B +4 score is increased by 2%, 3%, 4%, 5%, 6%, 7%, 8%, 9%, 10%, 15%, 20%, 25%, 30%, 35%, 40%, 45%, 50%, or more as compared to the FACT-B+4 score in a subject prior to the administration of the deuterium-enriched pirfenidone compound. In some embodiments, the disclosure provides a method for increasing the FACT-B+4 in a subject comprising administering to the subject LYT-100, wherein the FACT-B+4 is increased. In some embodiments, the disclosure provides a method for increasing the FACT-B+4 in a subject comprising administering to the subject LYT-100, wherein the FACT-B+4 is increased as compared to the FACT-B+4 in the subject prior to the administration of LYT-100. In some embodiments, the disclosure provides a method for increasing the FACT-B+4 in a subject comprising administering to the subject LYT-100, wherein the FACT-B+4 is increased by at least 2% as compared to the FACT-B+4 in the subject prior to the administration of LYT-100. In some embodiments, the disclosure provides a method for increasing the FACT-B+4 in a subject comprising administering to the subject LYT-100, wherein the FACT-B+4 is increased by at least 3%, at least 4%, at least 5%, at least 6%, at least 7%, at least 8%, at least 9%, at least 10%, at least 11%, at least 12%, at least 13%, at least 14% at least 15%, at least 16%, at least 17%, at least 18%, at least 19%, or at least 20% as compared to the FACT-B+4 in the subject prior to the administration of LYT-100. In some embodiments, the disclosure provides a method for increasing the FACT-B+4 in a subject comprising administering to the subject LYT-100, wherein the FACT-B+4 is increased by greater than 20% as compared to the FACT-B+4 in the subject prior to the administration of LYT-100.

Lymphedema Quality of Life measure for lymphedema of the limbs (LYMQOL) covers four domains: symptoms, body image/appearance, function, and mood. It also includes an overall quality of life rating. Subjects with more severe limb dysfunction have higher scores correspodning to lower quality of life. In some embodiments, the disclosure provides methods for decreasing the overall LYMQOL of a subject comprising administering to the subject a deuterium-enriched pirfenidone compound disclosed herein, for example, a compound of Formula I, e.g., a compound listed in Table 1. In some embodiments, the deuterium-enriched pirfenidone is LYT-100. Thus, in some embodiments, the disclosure provides methods for decreasing the overall LYMQOL in a subject with edema, e.g., lymphedema, comprising administering an effective amount of LYT-100. In some embodiments, provided herein are methods for treating lymphedema comprising administering to a subject having lymphedema in at least one limb a deuterium-enriched pirfenidone compound, e.g., LYT-100, wherein the LYMQOL is decreased in the subject as compared to the LYMQOL in the subject prior to the administration of the deuterium-enriched pirfenidone compound, e.g., LYT-100. In some embodiment, LYMQOL is decreased by 2%, 3%, 4%, 5%, 6%, 7%, 8%, 9%, 10%, 15%, 20%, 25%, 30%, 35%, 40%, 45%, 50%, or more as compared to the overall LYMQOL in the subject prior to administration of the deuterium-enriched pirfenidone compound. In some embodiments, provided herein are methods for treating lymphedema comprising administering to a subject having lymphedema in at least one limb a deuterium-enriched pirfenidone compound, e.g., LYT-100, wherein the LYMQOL is decreased in the subject as compared to the LYMQOL in the subject prior to the administration of the deuterium-enriched pirfenidone compound, e.g., LYT-100. =In some embodiments, the LYMQOL is decreased by 2%, 3%, 4%, 5%, 6%, 7%, 8%, 9%, 10%, 15%, 20%, 25%, 30%, 35%, 40%, 45%, 50%, or more as compared to the LYMQOL in a subject prior to the administration of the deuterium-enriched pirfenidone compound. In some embodiments, the disclosure provides a method for decreasing the LYMQOL in a subject comprising administering to the subject LYT-100, wherein the LYMQOL is increased. In some embodiments, the disclosure provides a method for decreasing the LYMQOL in a subject comprising administering to the subject LYT-100, wherein the LYMQOL is decreased in the subject as compared to the LYMQOL in the subject prior to the administration of LYT-100. In some embodiments, the disclosure provides a method for decreasing the LYMQOL in a subject comprising administering to the subject LYT-100, wherein the LYMQOL is decreased by at least 2% as compared to the LYMQOL in the subject prior to the administration of LYT-100. In some embodiments, the disclosure provides a method for decreasing the LYMQOL in a subject comprising administering to the subject LYT-100, wherein the LYMQOL is decreased by at least 3%, at least 4%, at least 5%, at least 6%, at least 7%, at least 8%, at least 9%, at least 10%, at least 11%, at least 12%, at least 13%, at least 14% at least 15%, at least 16%, at least 17%, at least 18%, at least 19%, or at least 20% as compared to the LYMQOL in the subject prior to the administration of LYT-100. In some embodiments, the disclosure provides a method for decreasing the LYMQOL in a subject comprising administering to the subject LYT-100, wherein the LYMQOL is decreased by greater than 20% as compared to the LYMQOL in the subject prior to the administration of LYT-100.

Disabilities of the Arm, Shoulder, and Hand score (DASH) is a 30-item disability/symptom scale, scored 0 (no disability) to 100. Each item has five response options. The scores for all items are then used to calculate a scale score ranging from 0 (no disability) to 100 (most severe disability). Items ask about the degree of difficulty in performing different physical activities because of the arm, shoulder, or hand problem (21 items), the severity of each of the symptoms of pain, activity-related pain, tingling, weakness and stiffness (5 items), and the problems impact on social activities, work, sleep, and self-image (4 items). The DASH can detect and differentiate small and large changes of disability over time after surgery in patients with upper-extremity musculoskeletal disorders. A 10-point difference in mean DASH score is considered a significant change indicating therapeutic effect. DASH score can be scored as raw, converted to a 0-100 score, or converted to a logit scale. In some embodiments, the disclosure provides methods for decreasing the DASH score of a subject (e.g., reducing the disability/symptoms) comprising administering to the subject a deuterium-enriched pirfenidone compound disclosed herein, for example, a compound of Formula I, e.g., a compound listed in Table 1. In some embodiments, the deuterium-enriched pirfenidone is LYT-100. Thus, in some embodiments, the disclosure provides methods for decreasing the DASH score in a subject with edema, e.g., lymphedema, comprising administering an effective amount of LYT-100. In some embodiments, provided herein are methods for treating lymphedema comprising administering to a subject having lymphedema in at least one limb a deuterium-enriched pirfenidone compound, e.g., LYT-100, wherein the subject's DASH score is decreased as compared to the subject's DASH score prior to the administatration of the deuterium-enriched pirfenidone compound, e.g., LYT-100. In some embodiments, the DASH score of a subject is decreased by 2%, 3%, 4%, 5%, 6%, 7%, 8%, 9%, 10%, 15%, 20%, 25%, 30%, 35%, 40%, 45%, 50% or more over a three month, six month, nine month, or twelve month period as compared to the DASH score of the subject prior to administration of the deuterium-enriched pirfenidone compound. In some embodiments, the disclosure provides a method for decreasing the DASH score in a subj ect comprising administering to the subject LYT-100, wherein the DASH score is decreased. In some embodiments, the disclosure provides a method for decreasing the DASH score in a subj ect comprising administering to the subject LYT-100, wherein the DASH score in the subject is decreased as compared to the DASH score in the subject prior to the administration of LYT-100. In some embodiments, the disclosure provides a method for decreasing the DASH score in a subj ect comprising administering to the subject LYT-100, wherein the DASH score in the subject is decreased by at least 5 points as compared to the DASH score in the subject prior to the administration of LYT-100. In some embodiments, the disclosure provides a method for decreasing the DASH score in a subject comprising administering to the subject LYT-100, wherein the DASH score in the subject is decreased by at least 10 points as compared to the DASH score in the subject prior to the administration of LYT-100. In some embodiments, the disclosure provides a method for decreasing the DASH score in a subject comprising administering to the subject LYT-100, wherein the DASH score in the subject is decreased by 2%, 3%, 4%, 5%, 6%, 7%, 8%, 9%, 10%, 15%, 20%, 25%, 30%, 35%, 40%, 45%, 50% or more over a three month, six month, nine month, or twelve month period as compared to the DASH score of the subject prior to administration of the deuterium-enriched pirfenidone compound. as compared to the DASH score in the subject prior to the administration of LYT-100. In some embodiments, the disclosure provides a method for decreasing the DASH score in a subject comprising administering to the subject LYT-100, wherein the DASH score in the subject is decreased by greater than 10% as compared to the DASH score in the subject prior to the administration of LYT-100.

Lymphedema Quality of Life Inventory (LQOLI) is the only HRQOL instrument developed and tested in patients with different types of lymphedema. The questionnaire consists of three parts: physical, psychosocial, and practical. In some embodiments, the disclosure provides methods for decreasing the LQOLI of a subject comprising administering to the subject a deuterium-enriched pirfenidone compound disclosed herein, for example, a compound of Formula I, e.g., a compound listed in Table 1. In some embodiments, the deuterium-enriched pirfenidone is LYT-100. Thus, in some embodiments, the disclosure provides methods for decreasing the LQOLI in a subject with edema, e.g., lymphedema, comprising administering an effective amount of LYT-100. In some embodiments, the LQOLI is decreased as compared to the LQOLI of the subject prior to deuterium-enriched pirfenidone administration. Thus, in some embodiments, the disclosure provides methods for decreasing the overall LQOLI in a subject with edema, e.g., lymphedema, comprising administering an effective amount of LYT-100. In some embodiments, provided herein are methods for treating lymphedema comprising administering to a subject having lymphedema a deuterium-enriched pirfenidone compound, e.g., LYT-100, wherein the LQOLI is decreased in the subject as compared to the LQOLI in the subject prior to the administration of the deuterium-enriched pirfenidone compound, e.g., LYT-100. In some embodiments, the disclosure provides a method for decreasing the LQOLI in a subject comprising administering to the subject LYT-100, wherein the LQOLI is decreased. In some embodiments, the disclosure provides a method for decreasing the LQOLI in a subject comprising administering to the subj ect LYT-100, wherein the LQOLI is decreased in the subj ect as compared to the LQOLI in the subject prior to the administration of LYT-100. In some embodiments, the disclosure provides a method for decreasing the LQOLI in a subject comprising administering to the subject LYT-100, wherein the LQOLI is decreased by at least 2% as compared to the LQOLI in the subject prior to the administration of LYT-100. In some embodiments, the disclosure provides a method for decreasing the LQOLI in a subject comprising administering to the subject LYT-100, wherein the LQOLI is decreased by greater than 20% as compared to the LQOLI in the subject prior to the administration of LYT-100.

Systemic Inflammatory Mediator Granulocyte Colony Stimulating Factor (G-CSF) is an inflammatory cytokine, and can be employed as a measure of the systemic inflammatory response of the patient. It can be assessed with Luminex-bead inflammasome analysis of pre- and post-treatment plasma samples. In some embodiments, the disclosure provides methods for decreasing the G-CSF in a subject comprising administering to the subject a deuterium-enriched pirfenidone compound disclosed herein, for example, a compound of Formula I, e.g., a compound listed in Table 1. In some embodiments, the deuterium-enriched pirfenidone is LYT-100. Thus, in some embodiments, the disclosure provides methods for decreasing G-CSF in a subject with edema, e.g., lymphedema, comprising administering an effective amount of LYT-100. In some embodiments, provided herein are methods for treating lymphedema comprising administering to a subject having lymphedema a deuterium-enriched pirfenidone compound, e.g., LYT-100, wherein G-CSF in the subject is decreased as compared to G-CSF in the subject prior to the administration of the deuterium-enriched pirfenidone compound, e.g., LYT-100. In some embodiments, there is a significant decrease in Systemic Inflammatory Mediator Granulocyte Colony Stimulating Factor (G-CSF). In some embodiments, there is a decrease of at least 2%, 3%, 4%, 5%, 6%, 7%, 8%, 9%, 10%, 15%, 20%, 25%, 30%, 35%, 40%, 45%, 50%, or more as compared to the G-CSF in a subject prior to the administration of the deuterium-enriched pirfenidone compound. In some embodiments, the disclosure provides a method for decreasing G-CSF in a subject comprising administering to the subject LYT-100, wherein the G-CSF is decreased. In some embodiments, the disclosure provides a method for decreasing the G-CSF in a subject comprising administering to the subject LYT-100, wherein the G-CSF is decreased as compared to the G-CSF in the subject prior to the administration of LYT-100. In some embodiments, the disclosure provides a method for decreasing the G-CSF in a subject comprising administering to the subject LYT-100, wherein the G-CSF is decreased by at least 2% as compared to the G-CSF in the subject prior to the administration of LYT-100. In some embodiments, the disclosure provides a method for decreasing the G-CSF in a subject comprising administering to the subject LYT-100, wherein the G-CSF is decreased by at least 3%, at least 4%, at least 5%, at least 6%, at least 7%, at least 8%, at least 9%, at least 10%, at least 11%, at least 12%, at least 13%, at least 14% at least 15%, at least 16%, at least 17%, at least 18%, at least 19%, or at least 20% as compared to the G-CSF in the subject prior to the administration of LYT-100. In some embodiments, the disclosure provides a method for decreasing the G-CSF in a subject comprising administering to the subject LYT-100, wherein the G-CSF is decreased by greater than 20% as compared to the G-CSF in the subject prior to the administration of LYT-100.

In some embodiments, there is a significant change from baseline in cutaneous histological architecture (CHA) based on histological specimens of lymphedema skin pre- and post-treatment with LYT-100. This score is based on a scoring system evaluating dermal thickness (0-5), internal mucin content (0-5), deep dermal collagen content (0-5), and perivascular infiltrate (0-5), with a total sum score of 0-20. In some embodiments, the disclosure provides methods for decreasing the CHA score of a subject comprising administering to the subject a deuterium-enriched pirfenidone compound disclosed herein, for example, a compound of Formula I, e.g., a compound listed in Table 1. In some embodiments, the deuterium-enriched pirfenidone is LYT-100. Thus, in some embodiments, the disclosure provides methods for decreasing the CHA score in a subject with edema, e.g., lymphedema, comprising administering an effective amount of LYT-100. In some embodiments, provided herein are methods for treating lymphedema comprising administering to a subject having lymphedema a deuterium-enriched pirfenidone compound, e.g., LYT-100, wherein the CHA score in the subject is decreased as compared to the CHA score in the subject prior to the administration of the deuterium-enriched pirfenidone compound, e.g., LYT-100. In some embodiments, there is a decrease of at least 50% in the CHA. In some embodiments, the CHA decreases at least 40%, at least 30%, at least 25%, at least 20%, at least 15%, at least 10%, at least 5%, at least 2% or at least 1% as compared to the CHA in the subject prior to administration of the deuterium-enriched pirfenidone compound, e.g., LYT-100. In some embodiments, this decrease is seen within 3, 4 or 6 months. In some embodiments, the disclosure provides a method for decreasing the CHA score in a subject comprising administering to the subject LYT-100, wherein the CHA score is decreased. In some embodiments, the disclosure provides a method for decreasing the CHA score in a subject comprising administering to the subject LYT-100, wherein the CHA score is decreased as compared to the CHA score in the subject prior to the administration of LYT-100. In some embodiments, the disclosure provides a method for decreasing the CHA score in a subject comprising administering to the subject LYT-100, wherein the CHA score is decreased by at least 2% as compared to the CHA score in the subject prior to the administration of LYT-100. In some embodiments, the disclosure provides a method for decreasing the CHA score in a subject comprising administering to the subject LYT-100, wherein the CHA score is decreased by at least 3%, at least 4%, at least 5%, at least 6%, at least 7%, at least 8%, at least 9%, at least 10%, at least 11%, at least 12%, at least 13%, at least 14% at least 15%, at least 16%, at least 17%, at least 18%, at least 19%, or at least 20% as compared to the CHA score in the subject prior to the administration of LYT-100. In some embodiments, the disclosure provides a method for decreasing the CHA score in a subject comprising administering to the subject LYT-100, wherein the CHA score is decreased by greater than 20% as compared to the CHA score in the subject prior to the administration of LYT-100.

In some embodiments, the disclosure provides methods for reducing the skin thickness of a subject comprising administering to the subject a deuterium-enriched pirfenidone compound disclosed herein, for example, a compound of Formula I, e.g., a compound listed in Table 1. In some embodiments, the deuterium-enriched pirfenidone is LYT-100. Thus, in some embodiments, the disclosure provides methods for reducing the skin thickness in a subject with edema, e.g., lymphedema, comprising administering an effective amount of LYT-100. In some embodiments, provided herein are methods for treating lymphedema comprising administering to a subject having lymphedema a deuterium-enriched pirfenidone compound, e.g., LYT-100, wherein the skin thickness in the subject is decreased as compared to the skin thickness in the subject prior to the administration of the deuterium-enriched pirfenidone compound, e.g., LYT-100. In some embodiments, measurement of skin thickness by caliper is reduced by at least 50% as compared to the skin thickness in the subject prior to administration of the deuterium-enriched pirfenidone compound, e.g., LYT-100. In some embodiments, measurement of skin thickness by caliper is reduced by at least 40%. 35%, 30%, 25%, 20%, 15%, 10%, 9%, 8%, 7%, 6%, or 5% as compared to the skin thickness in the subject prior to administration of the deuterium-enriched pirfenidone compound, e.g., LYT-100. in some embodiments, skin thickness is reduced by 25% as compared to the skin thickness in the subject prior to administration of the deuterium-enriched pirfenidone compound, e.g., LYT-100. In some embodiments, this reduction is seen within 3, 4 or 6 months. In some embodiments, the disclosure provides a method for decreasing the skin thickness in a subject comprising administering to the subject LYT-100, wherein the skin thickness is decreased. In some embodiments, the disclosure provides a method for decreasing the skin thickness in a subject comprising administering to the subject LYT-100, wherein the skin thickness is decreased as compared to the skin thickness in the subject prior to the administration of LYT-100. In some embodiments, the disclosure provides a method for decreasing the skin thickness in a subject comprising administering to the subject LYT-100, wherein the skin thickness is decreased by at least 10% as compared to the skin thickness in the subject prior to the administration of LYT-100. In some embodiments, the disclosure provides a method for decreasing the skin thickness in a subject comprising administering to the subject LYT-100, wherein the skin thickness is decreased by at least 15%, at least 16%, at least 17%, at least 18%, at least 19%, at least 20%, at least 21%, at least 22%, at least 23%, at least 24%, at least 25%, at least 26% at least 27%, at least 28%, at least 29%, at least 30%, at least 35%, or at least 40% as compared to the skin thickness in the subject prior to the administration of LYT-100. In some embodiments, the disclosure provides a method for decreasing the skin thickness in a subject comprising administering to the subject LYT-100, wherein the skin thickness is decreased by at least 20% as compared to the skin thickness in the subject prior to the administration of LYT-100.

In some embodiments, the present disclosure relates to the use of deuterium-enriched pirfenidone to treat, prevent, and/or ameliorate one or more symptoms associated with edema. In some embodiments, the deuterium-enriched pirfenidone is LYT-100 or a pharmaceutically acceptable salt thereof. In some embodiments, the present disclosure provides a method of treating, preventing nad/or ameliorating one or more symptoms of edema, comprising administering to a subject in need thereof an effective amount of a deuterium-enriched pirfenidone, e.g., a compound of Formula I, e.g., a compound in Table 1. In some embodiments, the deuterium-enriched pirfenidone is LYT-100, or a pharmaceutically acceptable salt thereof.

In some embodiments, the present disclosure relates to the use of deuterium-enriched pirfenidone to treat, prevent, and/or ameliorate one or more symptoms of lymphedema. In some embodiments, the deuterium-enriched pirfenidone is LYT-100 or a pharmaceutically acceptable salt thereof. In some embodiments, the present disclosure provides a method of treating, preventing and/or ameliorating one or more symptoms of lymphedema, comprising administering to a subject in need thereof an effective amount of a deuterium-enriched pirfenidone, e.g., a compound of Formula I, e.g., a compound in Table 1. In some embodiments, the deuterium-enriched pirfenidone administered to the subject in need thereof is LYT-100, or a pharmaceutically acceptable salt thereof.

In some embodiments, the present disclosure relates to the use of deuterium-enriched pirfenidone to treat, prevent, and/or ameliorate one or more symptoms of secondary lymphedema. In some embodiments, the deuterium-enriched pirfenidone is LYT-100 or a pharmaceutically acceptable salt thereof. In some embodiments, the present disclosure provides a method of treating, preventing, and/or ameliorating one or more symptoms of secondary lymphedema, comprising administering to a subject in need thereof an effective amount of a deuterium-enriched pirfenidone, e.g., a compound of Formula I, e.g., a compound in Table 1. In some embodiments, the deuterium-enriched pirfenidone administered to the subject in need thereof is LYT-100, or a pharmaceutically acceptable salt thereof.

In some embodiments, the present disclosure relates to the use of deuterium-enriched pirfenidone to treat, prevent, and/or ameliorate one or more symptoms of breast cancer-related arm lymphedema. In some embodiments, the deuterium-enriched pirfenidone is LYT-100 or a pharmacologically acceptable salt thereof. In some embodiments, the present disclosure provides a method of treating, preventing, and/or ameliorating one or more symptoms of breast cancer-related arm lymphedema, comprising administering to a subject in need thereof an effective amount of a deuterium-enriched pirfenidone, e.g., a compound of Formula I, e.g., a compound in Table 1. In some embodiments, the deuterium-enriched pirfenidone administered to the subject in need thereof is LYT-100, or a pharmaceutically acceptable salt thereof.

In some embodiments, the present disclosure relates to the use of deuterium-enriched pirfenidone to treat, prevent, and/or ameliorate one or more symptoms of lymphedema other than breast cancer-related arm lymphedema. In some embodiments, the deuterium-enriched pirfenidone is LYT-100 or a pharmaceutically-acceptablesalt thereof. In some embodiments, the present disclosure provides a method of treating, preventing, and/or ameliorating one or more symptoms of lymphedema other than breast cancer-related arm lymphedema, comprising administering to a subject in need thereof an effective amount of a deuterium-enriched pirfenidone, e.g., a compound of Formula I, e.g., a compound in Table 1. In some embodiments, the deuterium-enriched pirfenidone administered to the subject in need thereof is LYT-100, or a pharmaceutically acceptable salt thereof.

In some embodiments, the present disclosure relates to the use of deuterium-enriched pirfenidone to treat, prevent, and/or ameliorate one or more symptoms of primary lymphedema. In some embodiments, the deuterium-enriched pirfenidone is LYT-100 or a pharmaceutically-acceptable salt thereof. In some embodiments, the present disclosure provides a method of treating, preventing, and/or ameliorating one or more symptoms of primary lymphedema, comprising administering to a subject in need thereof an effective amount of a deuterium-enriched pirfenidone, e.g., a compound of Formula I, e.g., a compound in Table 1. In some embodiments, the deuterium-enriched pirfenidone administered to the subject in need thereof is LYT-100, or a pharmaceutically acceptable salt thereof.

In some embodiments, the present disclosure relates to the use of deuterium-enriched pirfenidone to treat, prevent, and/or ameliorate one or more symptoms of lymphatic filariasis. In some embodiments, the deuterium-enriched pirfenidone is LYT-100 or a pharmaceutically-acceptable salt thereof. In some embodiments, the present disclosure provides a method of treating, preventing, and/or ameliorating one or more symptoms of lymphatic filariasis, comprising administering to a subject in need thereof an effective amount of a deuterium-enriched pirfenidone, e.g., a compound of Formula I, e.g., a compound in Table 1. In some embodiments, the deuterium-enriched pirfenidone administered to the subject in need thereof is LYT-100, or a pharmaceutically acceptable salt thereof.

In some embodiments, the present disclosure relates to the use of deuterium-enriched pirfenidone to treat, prevent, and/or ameliorate one or more symptoms of other lymphatic and/or fibrotic disorders. Inflammation and fibrosis affect lymphatic flow, thus the pirfenidone agents described herein, e.g., deuterium-enriched pirfenidone can be used to treat other lymphatic flow conditions. As discussed herein, patients have lymphedema beyond breast cancer-related arm lymphedema and pirfenidone agents described herein, e.g., deuterium-enriched pirfenidone can be used to treat the underlying mechanisms of other forms of secondary or primary lymphedema. In some embodiments, the pirfenidone agents described herein, e.g., deuterium-enriched pirfenidone have anti-inflammatory and/or anti-fibrotic activity and can be used to treat fibrotic diseases, including IPF and FSGS. In some embodiments, the deuterium-enriched pirfenidone is LYT-100 or a pharmaceutically-acceptablesalt thereof. In some embodiments, the present disclosure provides a method of treating, preventing, and/or ameliorating one or more symptoms of other lymphatic and/or fibrotic disorders, comprising administering to a subject in need thereof an effective amount of deuterium-enriched pirfenidone. In some embodiments, the deuterium-enriched pirfenidone administered to the subject in need thereof is LYT-100, or a pharmaceutically acceptable salt thereof.

Lymphatic vessels are present in most tissues of the body. These vessels consist of an extensive network of thin-walled vessels that drain protein-rich lymph from extracellular spaces. Major functions of the lymphatic system include maintenance of tissue fluid homeostasis, fatty acid absorption, and mediation of immune responses under normal circumstances. The lymphatic system also plays key roles in disease processes such lymphedema, fibrosis and inflammation. In such lymphatic disorders, lymphatic flow is altered and balance of interstitial fluid perturbed. Consequently, maintaining or restoring interstitial fluid balance and/or maintaining restoring lymphatic flow constitutes one approach to the treatment of these disorders. Without wishing to be bound by theory, administration of an agent that modulates, e.g., increases lymphatic flow, to a subject with a lymphatic disorder can alleviate, treat or prevent the disorder.

In some embodiments, methods are provided herein for modulating and/or maintaining interstitial fluid balance and/or lymphatic flow in a subject in need thereof. In some embodiments, the modulation of lymphatic flow in a subject in need thereof comprises increasing lymphatic flow in said subject. In some embodiments, the method comprises administering an effective amount of a compound, e.g., an effective amount of deuterium-enriched pirfenidone having the structure shown in Formula I. In some embodiments, the compound is LYT-100. In some embodiments, methods are provided herein to treat a lymphatic disorder described herein comprising modulating and/or maintaining interstitial fluid balance and/or lymphatic flow. In some embodiments, the methods comprise increasing lymphatic flow in a subject in need thereof. In some embodiments, the methods comprise administering an effective amount of a compound, e.g., an effective amount of deuterium-enriched pirfenidone having the structure shown in Formula I. In some embodiments, the compound is LYT-100.

Further methods are provided herein, comprising reducing inflammation and/or fibrosis in a subject having insufficient lymphatic flow, the method comprising administering to a subject in need thereof an effective amount of deuterium-enriched pirfenidone having the structure shown in Formula I. In some embodiments, the compound is LYT-100. In some embodiments, methods are provided herein to treat a lymphatic disorder described herein comprising reducing inflammation and/or fibrosis in a subject having insufficient lymphatic flow. In some embodiments, the methods comprise administering an effective amount of a compound, e.g., an effective amount of deuterium-enriched pirfenidone having the structure shown in Formula I. In some embodiments, the compound is LYT-100.

In some embodiments, the deuterium-enriched pirfenidone compound disclosed herein has the ability to effect one or more of the following: a) reduce tissue swelling, b) reduce lymphatic fluid stasis or "pooling," c) reduce tissue fibrosis, d) reduce tissue inflammation, e) reduce infiltration of leukocytes, f) reduce infiltration of macrophages, g) reduce infiltration of naive and differentiated T-cells, h) reduce TGF-β1 expression and reduce expression and/or activation of downstream mediators (e.g., pSmad3), i) reduce levels of angiotensins and/or ACE, j) reduce collagen deposition and/or scar formation, k) improve or increase lymphatic function, 1) improve or increase lymph fluid transport (e.g., lymphatic flow), m) improve or increase lymphangiogenesis, and/or n) improve or increase lymph pulsation frequency.

Thus, disclosed herein are methods for treating a subject, including a human subject, having or suspected of having edema, e.g., lyphedema or for preventing such disorder in a subject prone to the disorder; comprising administering to the subject a therapeutically effective amount of a deuterium-enriched pirfenidone compound as disclosed herein (e.g., compound of Formula I, including, e.g., LYT-100), or a pharmaceutically acceptable salt, solvate, or prodrug thereof; so as to effect one or more of a) - n) above during the treatment of the disorder.

Thus, disclosed herein are methods for treating a subject, including a human subject, having or suspected of having edema, e.g., lymphedema or for preventing edema, e.g., lymphedema in a subject prone to edema, e.g., lymphedema; comprising administering to the subject a therapeutically effective amount of a deuterium-enriched pirfenidone compound as disclosed herein (e.g., compound of Formula I, including, e.g., LYT-100), or a pharmaceutically acceptable salt, solvate, or prodrug thereof; so as to effect one or more of a) - n) above during the treatment of edema, e.g., lymphedema.

In some embodiments, the disclosure provides methods for reducing tissue swelling in a subject, including a human subject, having or suspected of having edema, e.g., lymphedema, or for preventing tissue swelling in a subject prone to edema or lymphedema; comprising administering to the subject a therapeutically effective amount of a deuterium-enriched pirfenidone compound as disclosed herein, for example, a compound of Formula I, e.g., a compound listed in Table 1, e.g., LYT-100, or a pharmaceutically acceptable salt, solvate, or prodrug thereof. In some embodiments, the deuterium-enriched pirfenidone is LYT-100.

In some embodiments, the disclosure provides methods for reducing lymphatic fluid stasis or pooling in a subject, including a human subject, having or suspected of having edema, e.g., lymphedema, or for preventing lymphatic fluid stasis or pooling in a subject prone to edema or lymphedema; comprising administering to the subject a therapeutically effective amount of a deuterium-enriched pirfenidone compound as disclosed herein, for example, a compound of Formula I, e.g., a compound listed in Table 1, e.g., LYT-100, or a pharmaceutically acceptable salt, solvate, or prodrug thereof. In some embodiments, the deuterium-enriched pirfenidone is LYT-100.

In some embodiments, the disclosure provides methods for improving or increasing lymph fluid transport (e.g., increasing lymphatic flow) in a subject, including a human subject, having or suspected of having edema, e.g., lymphedema, or for improving or increasing lymph fluid transport (e.g., increasing lymphatic flow) in a subject prone to edema or lymphedema; comprising administering to the subject a therapeutically effective amount of a deuterium-enriched pirfenidone compound as disclosed herein, for example, a compound of Formula I, e.g., a compound listed in Table 1, e.g., LYT-100, or a pharmaceutically acceptable salt, solvate, or prodrug thereof. In some embodiments, the deuterium-enriched pirfenidone is LYT-100.

In some embodiments, the disclosure provides methods for reducing tissue fibrosis in a subject, including a human subject, having or suspected of having edema, e.g., lymphedema, or for preventing tissue fibrosis in a subject prone to edema or lymphedema; comprising administering to the subject a therapeutically effective amount of a deuterium-enriched pirfenidone compound as disclosed herein, for example, a compound of Formula I, e.g., a compound listed in Table 1, e.g., LYT-100, or a pharmaceutically acceptable salt, solvate, or prodrug thereof. In some embodiments, the deuterium-enriched pirfenidone is LYT-100.

In some embodiments, the disclosure provides methods for reducing tissue inflammation in a subject, including a human subject, having or suspected of having edema, e.g., lymphedema, or for preventing tissue inflammation in a subject prone to edema or lymphedema; comprising administering to the subject a therapeutically effective amount of a deuterium-enriched pirfenidone compound as disclosed herein, for example, a compound of Formula I, e.g., a compound listed in Table 1, e.g., LYT-100, or a pharmaceutically acceptable salt, solvate, or prodrug thereof. In some embodiments, the deuterium-enriched pirfenidone is LYT-100.

In some embodiments, the disclosure provides methods for reducing infiltration of leukocytes in a subject, including a human subject, having or suspected of having edema, e.g., lymphedema, or for preventing infiltration of leukocytes in a subject prone to edema or lymphedema; comprising administering to the subject a therapeutically effective amount of a deuterium-enriched pirfenidone compound as disclosed herein, for example, a compound of Formula I, e.g., a compound listed in Table 1, e.g., LYT-100, or a pharmaceutically acceptable salt, solvate, or prodrug thereof. In some embodiments, the deuterium-enriched pirfenidone is LYT-100.

In some embodiments, the disclosure provides methods for reducing infiltration of macrophages in a subject, including a human subject, having or suspected of having edema, e.g., lymphedema, or for preventing infiltration of macrophages in a subject prone to edema or lymphedema; comprising administering to the subject a therapeutically effective amount of a deuterium-enriched pirfenidone compound as disclosed herein, for example, a compound of Formula I, e.g., a compound listed in Table 1, e.g., LYT-100, or a pharmaceutically acceptable salt, solvate, or prodrug thereof. In some embodiments, the deuterium-enriched pirfenidone is LYT-100.

In some embodiments, the disclosure provides methods for reducing infiltration of naive and differentiated T-cells in a subject, including a human subject, having or suspected of having edema, e.g., lymphedema, or for preventing infiltration of naive and differentiated T-cells in a subject prone to edema or lymphedema; comprising administering to the subject a therapeutically effective amount of a deuterium-enriched pirfenidone compound as disclosed herein, for example, a compound of Formula I, e.g., a compound listed in Table 1, e.g., LYT-100, or a pharmaceutically acceptable salt, solvate, or prodrug thereof. In some embodiments, the deuterium-enriched pirfenidone is LYT-100.

In some embodiments, the disclosure provides methods for reducing TGF-β1 expression and reducing expression and/or activation of downstream mediators (e.g., pSmad3) in a subject, including a human subject, having or suspected of having edema, e.g., lymphedema, or for preventing TGF-β1 expression and preventing expression and/or activation of downstream mediators (e.g., pSmad3)in a subject prone to edema or lymphedema; comprising administering to the subject a therapeutically effective amount of a deuterium-enriched pirfenidone compound as disclosed herein, for example, a compound of Formula I, e.g., a compound listed in Table 1, e.g., LYT-100, or a pharmaceutically acceptable salt, solvate, or prodrug thereof. In some embodiments, the deuterium-enriched pirfenidone is LYT-100.

In some embodiments, the disclosure provides methods for reducing levels of angiotensins and/or ACE in a subject, including a human subject, having or suspected of having edema, e.g., lymphedema, or for preventing levels of angiotensins and/or ACE in a subject prone to edema or lymphedema; comprising administering to the subject a therapeutically effective amount of a deuterium-enriched pirfenidone compound as disclosed herein, for example, a compound of Formula I, e.g., a compound listed in Table 1, e.g., LYT-100, or a pharmaceutically acceptable salt, solvate, or prodrug thereof. In some embodiments, the deuterium-enriched pirfenidone is LYT-100.

In some embodiments, the disclosure provides methods for improving or increasing lymphatic function in a subject, including a human subject, having or suspected of having edema, e.g., lymphedema, or for improving or increasing lymphatic function in a subject prone to edema or lymphedema; comprising administering to the subject a therapeutically effective amount of a deuterium-enriched pirfenidone compound as disclosed herein, for example, a compound of Formula I, e.g., a compound listed in Table 1, e.g., LYT-100, or a pharmaceutically acceptable salt, solvate, or prodrug thereof. In some embodiments, the deuterium-enriched pirfenidone is LYT-100.

In some embodiments, the disclosure provides methods for reducing collagen deposition and/or scar formation in a subject, including a human subject, having or suspected of having edema, e.g., lymphedema, or for preventing collagen deposition and/or scar formation in a subject prone to edema or lymphedema; comprising administering to the subject a therapeutically effective amount of a deuterium-enriched pirfenidone compound as disclosed herein, for example, a compound of Formula I, e.g., a compound listed in Table 1, e.g., LYT-100, or a pharmaceutically acceptable salt, solvate, or prodrug thereof. In some embodiments, the deuterium-enriched pirfenidone is LYT-100.

In some embodiments, the disclosure provides methods for improving or increasing lymphangiogenesis in a subject, including a human subject, having or suspected of having edema, e.g., lymphedema, or for improving or increasing lymphangiogenesis in a subject prone to edema or lymphedema; comprising administering to the subject a therapeutically effective amount of a deuterium-enriched pirfenidone compound as disclosed herein, for example, a compound of Formula I, e.g., a compound listed in Table 1, e.g., LYT-100, or a pharmaceutically acceptable salt, solvate, or prodrug thereof. In some embodiments, the deuterium-enriched pirfenidone is LYT-100.

In some embodiments, the disclosure provides methods for improving or increasing lymph pulsation frequency in a subject, including a human subject, having or suspected of having edema, e.g., lymphedema, or for improving or increasing lymph pulsation frequency in a subject prone to edema or lymphedema; comprising administering to the subject a therapeutically effective amount of a deuterium-enriched pirfenidone compound as disclosed herein, for example, a compound of Formula I, e.g., a compound listed in Table 1, e.g., LYT-100, or a pharmaceutically acceptable salt, solvate, or prodrug thereof. In some embodiments, the deuterium-enriched pirfenidone is LYT-100.

Provided herein are methods for the treatment, prevention, and/or amelioration of edema, e.g., lymphedema, wherein cellulitis is reduced, comprising administering to a subject in need thereof a deuterium-enriched pirfenidone compound, for example, a compound of Formula I, e.g., a compound listed in Table 1, including e.g., LYT-100.

Provided herein are methods for the treatment, prevention, and/or amelioration of cellulitis, comprising administering to a subject in need thereof a deuterium-enriched pirfenidone compound, for example, a compound of Formula I, e.g., a compound listed in Table 1, including e.g., LYT-100.

In any of the above-described methods for treating, preventing, or ameliorating one or more symptoms of edema or lymphedema, the deuterium-enriched pirfenidone compound, e.g., LYT-100, is administered orally twice a day, for a total daily dose of 100-1500 mg. In some embodiments, the daily dose is 100, 200, 250, 300, 400, 500, 600, 700, 750, 800, 900, 1000, or 1500 mg. In some embodiments, the daily dose is 1500 mg. In some embodiments, the daily dose is 1000 mg. In some embodiments, the daily dose is 750 mg. In some embodiments, the daily dose is 500 mg. In some embodiments, the daily dose is 250 mg. In some embodiments, the deuterium-enriched pirfenidone is administered orally 750 mg twice daily. In some embodiments, the deuterium-enriched pirfenidone is administered orally 500 mg twice daily. In some embodiments, the deuterium-enriched pirfenidone is administered orally 250 mg twice daily.

In some embodiments, the deuterium-enriched pirfenidone compound, e.g., LYT-100, is administered orally once a day, for a total daily dose of 100-1500 mg. In some embodiments, the daily dose is 100, 200, 250, 300, 400, 500, 600, 700, 750, 800, 900, 1000, or 1500 mg. In some embodiments, the daily dose is 1500 mg. In some embodiments, the daily dose is 1000 mg. In some embodiments, the daily dose is 750 mg. In some embodiments, the daily dose is 500 mg. In some embodiments, the daily dose is 250 mg. In some embodiments, the deuterium-enriched pirfenidone is administered orally 1500 mg once daily. In some embodiments, the deuterium-enriched pirfenidone is administered orally 1000 mg once daily. In some embodiments, the deuterium-enriched pirfenidone is administered orally 750 mg once daily. In some embodiments, the deuterium-enriched pirfenidone is administered orally 500 mg once daily. In some embodiments, the deuterium-enriched pirfenidone is administered orally 250 mg once daily.

In any of the above-described embodiments, the deuterium-enriched pirfenidone compound, e.g., LYT-100 is administered orally three times a day, for a total daily dose of 100-1500 mg. In some embodiments, the daily dose is 100, 200, 250, 300, 400, 500, 600, 700, 750, 800, 900, 1000, or 1500 mg. In some embodiments, the daily dose is 1500 mg. In some embodiments, the daily dose is 1000 mg. In some embodiments, the daily dose is 750 mg. In some embodiments, the daily dose is 500 mg. In some embodiments, the daily dose is 250 mg. In some embodiments, the deuterium-enriched pirfenidone is administered orally 500 mg three times daily. In some embodiments, the deuterium-enriched pirfenidone is administered orally 333 mg three times daily. In some embodiments, the deuterium-enriched pirfenidone is administered orally 166 mg three times daily.

In some embodiments, the deuterium-enriched pirfenidone is in tablet form. In some embodiments, the deuterium-enriched pirfenidone is taken orally with food.

Thus, provided herein are methods for the treatment, prevention, and/or amelioration of edema, e.g.lymphedema comprising administering to a subject in need thereof a deuterium-enriched pirfenidone compound, for example, a compound of Formula I, e.g., a compound listed in Table 1, including e.g., LYT-100, wherein the deuterium-enriched pirfenidone compound is administered orally twice a day, for a total daily dose of 100-1500 mg. In some embodiments, provided herein are methods for the treatment, prevention, and/or amelioration of edeme, e.g., lymphedema comprising administering to a subject in need thereof LYT-100, wherein LYT-100 is administered orally twice a day, for a total daily dose of 100-1500 mg. In some embodiments, the daily dose of the deuterium-enriched pirfenidone compound, e.g., LYT-100, is 1500 mg. In some embodiments, the daily dose of the deuterium-enriched pirfenidone compound, e.g., LYT-100, is 1000 mg. In some embodiments, the daily dose of the deuterium-enriched pirfenidone compound, e.g., LYT-100, is 750 mg. In some embodiments, the daily dose of the deuterium-enriched pirfenidone compound, e.g., LYT-100 is 500 mg. In some embodiments, the daily dose of the deuterium-enriched pirfenidone compound, e.g., LYT-100 is 250 mg. In some embodiments, the deuterium-enriched pirfenidone compound, e.g., LYT-100 is administered orally 750 mg twice daily. In some embodiments, the deuterium-enriched pirfenidone compound, e.g., LYT-100, is administered orally 500 mg twice daily. In some embodiments, the deuterium-enriched pirfenidone compound, e.g., LYT-100, is administered orally 250 mg twice daily.

In some embodiments, provided herein are methods for the treatment, prevention, and/or amelioration of edema, e.g., lymphedema comprising administering to a subject in need thereof a deuterirum-enriched pirfenidone compound, for example, a compound of Formula I, e.g., a compound listed in Table 1, including e.g., LYT-100, wherein the deuterirum-enriched pirfenidone compound is administered orally once a day, for a total daily dose of 100-1500 mg. In some embodiments, provided herein are methods for the treatment, prevention, and/or amelioration of edema, e.g., lymphedema comprising administering to a subject in need thereof LYT-100, wherein LYT-100, is administered orally once a day, for a total daily dose of 100-1500 mg. In some embodiments, the daily dose of the deuterium-enriched pirfenidone compound, e.g., LYT-100 is 1500 mg. In some embodiments, the daily dose of the deuterium-enriched pirfenidone compound, e.g., LYT-100, is 1000 mg. In some embodiments, the daily dose of the deuterium-enriched pirfenidone compound, e.g., LYT-100, is 750 mg. In some embodiments, the daily dose of the deuterium-enriched pirfenidone compound, e.g., LYT-100 is 500 mg. In some embodiments, the daily dose of the deuterium-enriched pirfenidone compound, e.g., LYT-100, is 250 mg. In some embodiments, the deuterium-enriched pirfenidone compound, e.g., LYT-100, is administered orally 1500 mg once daily. In some embodiments, the deuterium-enriched pirfenidone compound, e.g., LYT-100, is administered orally 1000 mg once daily. In some embodiments, the deuterium-enriched pirfenidone compound, e.g., LYT-100, is administered orally 750 mg once daily. In some embodiments, the deuterium-enriched pirfenidone compound, e.g., LYT-100, is administered orally 500 mg once daily. In some embodiments, the deuterium-enriched pirfenidone compound, e.g., LYT-100 is administered orally 250 mg once daily.

Thus, provided herein are methods for the treatment, prevention, and/or amelioration of edema, e.g., lymphedema comprising administering to a subject in need thereof a deuterium-enriched pirfenidone compound, for example, a compound of Formula I, e.g., a compound listed in Table 1, including e.g., LYT-100, wherein the deuterium-enriched pirfenidone compound is administered orally three times daily, for a total daily dose of 100-1500 mg. In some embodiments, provided herein are methods for the treatment, prevention, and/or amelioration of edema, e.g., lymphedema comprising administering to a subject in need thereof LYT-100, wherein LYT-100 is administered orally three times a day, for a total daily dose of 100-1500 mg. In some embodiments, the daily dose of the deuterium-enriched pirfenidone compound, e.g., LYT-100, is 1500 mg. In some embodiments, the daily dose of the deuterium-enriched pirfenidone compound, e.g., LYT-100, is 1000 mg. In some embodiments, the daily dose of the deuterium-enriched pirfenidone compound, e.g., LYT-100, is 750 mg. In some embodiments, the daily dose of the deuterium-enriched pirfenidone compound, e.g., LYT-100 is 500 mg. In some embodiments, the daily dose of the deuterium-enriched pirfenidone compound, e.g., LYT-100 is 250 mg. In some embodiments, the deuterium-enriched pirfenidone compound, e.g., LYT-100 is administered orally 500 mg three times daily. In some embodiments, the deuterium-enriched pirfenidone compound, e.g., LYT-100, is administered orally 333 mg three timese daily. In some embodiments, the deuterium-enriched pirfenidone compound, e.g., LYT-100, is administered orally 166 mg twice daily.

In other embodiments, the deuterium-enriched pirfenidone compound is administered orally at a total daily dose of 100-2500 mg. In some embodiments, the deuterium-enriched pirfenidone compound is administered orally at a total daily dose of 100-2000 mg. In some embodiments, the deuterium-enriched pirfenidone compound is administered orally at a total daily dose of 100-1500 mg. In some embodiments, the deuterium-enriched pirfenidone compound is administered orally at a total daily dose of 100-1000 mg. In some embodiments, the deuterium-enriched pirfenidone compound is administered orally at a total daily dose of 100-500 mg. In some embodiments, the daily dose is selected from 100, 200, 250, 300, 400, 500, 600, 700, 750, 800, 900, 1000, 1100, 1200, 1300, 1400, 1500, 1600, 1700,1800, 1900, 2000, 2100, 2200, 2300, 2400, and 2500 mg/day. In some embodiments, the deuterium-enriched pirfenidone compound is administered orally three times/day (TID). In some embodiments, the deuterium-enriched pirfenidone compound is administered orally two times/day (BID). In some embodiments, the deuterium-enriched pirfenidone compound is administered orally once daily (QD). In any of these embodiments, the deuterium-enriched pirfenidone compound has the structure of Formula I, e.g., a compound listed in Table 1, including e.g., LYT-100.

In some embodiments, methods described herein include escalation of doses of deuterium-enriched pirfenidone over a certain period until the full maintenance dose is reached. In some embodiments, the escalation period is 7 days. In some embodiments, the escalation period is 14 days. In some embodiments, the escalation period is 21 days. In some embodiments, the methods described herein include reducing a dose. In any of these embodiments, the daily dose is administered in one dose, or split into two or three doses, i.e., administration is once, twice or three times daily.

In some embodiments, the daily dose is escalated from 250 mg to 500 mg. In some embodiments, the daily dose is escalated from 250 mg to 750 mg, wherein a 500 mg step is optionally included. In some embodiments, the daily dose is escalated from 250 mg to 1000 mg, wherein a 500 mg step and/or a 750 mg step is optionally included. In some embodiments, the daily dose is escalated from 250 mg to 1500 mg, wherein a 500 mg step and/or a 750 mg step and/or a 100 mg step is optionally included. In some embodiments, the daily dose is escalated from 500 mg to 750 mg. In some embodiments, the daily dose is escalated from 500 mg to 1000 mg, wherein a 750 mg step is optionally included. In some embodiments, the daily dose is escalated from 500 mg to 1500 mg, wherein a 750 mg step and/or 100 mg step is optionally included. In any of these embodiments, the daily dose is administered in one dose, or split into two or three doses, i.e., administration is once, twice or three times daily.

In some embodiments, the daily dose is escalated from 250 mg to 500 mg over a period of 5 days. In some embodiments, the daily dose is escalated from 250 mg to 750 mg over a period of 5 days, wherein a 500 mg step is optionally included. In some embodiments, the daily dose is escalated from 250 mg to 1000 mg over a period of 5 days, wherein a 500 mg step and/or a 750 mg step is optionally included. In some embodiments, the daily dose is escalated from 250 mg to 1500 mg over a period of 5 days, wherein a 500 mg step and/or a 750 mg step and/or a 100 mg step is optionally included. In some embodiments, the daily dose is escalated from 500 mg to 750 mg over a period of 5 days. In some embodiments, the daily dose is escalated from 500 mg to 1000 mg over a period of 5 days, wherein a 750 mg step is optionally included. In some embodiments, the daily dose is escalated from 500 mg to 1500 mg over a period of 5 days, wherein a 750 mg step and/or 100 mg step is optionally included. In any of these embodiments, the daily dose is administered in one dose, or split into two or three doses, i.e., administration is once, twice or three times daily.

In some embodiments, the daily dose is escalated from 500 mg to 250 mg over a period of 5 days. In some embodiments, the daily dose is reduced from 750 mg to 250 mg over a period of 5 days, wherein a 500 mg step. In some embodiments, the daily dose is reduced from 1000 mg to 250 mg over a period of 5 days, wherein a 750 mg step and/or a 500 mg step is optionally included. In some embodiments, the daily dose is reduced from 1500 mg to 250 over a period of 5 days wherein a 1000 mg step and/or a 750 mg step and/or a 500 mg step is optionally included. In some embodiments, the daily dose is reduced from 750 mg to 500 mg over a period of 5 days. In some embodiments, the daily dose is reduced from 1000 mg to 500 mg over a period of 5 days, wherein 750 mg step is optionally included. In some embodiments, the daily dose is reduced from 1500 mg to 500 mg over a period of 5 days, wherein a 100 mg step and/or a 750 mg step is optionally included. In any of these embodiments, the daily dose is administered in one dose, or split into two or three doses, i.e., administration is once, twice or three times daily.

In some embodiments, the daily dose is escalated from 250 mg to 500 mg over a period of 14 days. In some embodiments, the daily dose is escalated from 250 mg to 750 mg over a period of 14 days, wherein a 500 mg step is optionally included. In some embodiments, the daily dose is escalated from 250 mg to 1000 mg over a period of 14 days, wherein a 500 mg step and/or a 750 mg step is optionally included. In some embodiments, the daily dose is escalated from 250 mg to 1500 mg over a period of 14 days, wherein a 500 mg step and/or a 750 mg step and/or a 100 mg step is optionally included. In some embodiments, the daily dose is escalated from 500 mg to 750 mg over a period of 14 days. In some embodiments, the daily dose is escalated from 500 mg to 1000 mg over a period of 14 days, wherein a 750 mg step is optionally included. In some embodiments, the daily dose is escalated from 500 mg to 1500 mg over a period of 14 days, wherein a 750 mg step and/or 100 mg step is optionally included. In any of these embodiments, the daily dose is administered in one dose, or split into two or three doses, i.e., administration is once, twice or three times daily.

In some embodiments, the daily dose is escalated from 250 mg to 500 mg from day 1 to day7 and then escalated from 500 mg to 1000mg from day 7 to day 14. In some embodiments, the escalation from 500 mg to 1000 mg includes a 750 mg step. In some embodiments, the daily dose is escalated from 500 mg to 750 mg from day 1 to day7 and then escalated from 750 mg to 1000mg from day 7 to day 14. In any of these embodiments, the daily dose is administered in one dose, or split into two or three doses, i.e., administration is once, twice or three times daily.

In some embodiments, the daily dose is escalated from 250 mg to 500 mg over a period of 21 days. In some embodiments, the daily dose is escalated from 250 mg to 750 mg over a period of 21 days, wherein a 500 mg step is optionally included. In some embodiments, the daily dose is escalated from 250 mg to 1000 mg over a period of 21 days, wherein a 500 mg step and/or a 750 mg step is optionally included. In some embodiments, the daily dose is escalated from 250 mg to 1500 mg over a period of 21 days, wherein a 500 mg step and/or a 750 mg step and/or a 100 mg step is optionally included. In some embodiments, the daily dose is escalated from 500 mg to 750 mg over a period of 21 days. In some embodiments, the daily dose is escalated from 500 mg to 1000 mg over a period of 21 days, wherein a 750 mg step is optionally included. In some embodiments, the daily dose is escalated from 500 mg to 1500 mg over a period of 21 days, wherein a 750 mg step and/or 100 mg step is optionally included. In any of these embodiments, the daily dose is administered in one dose, or split into two or three doses, i.e., administration is once, twice or three times daily.

In some embodiments, the daily dose is escalated from 250 mg to 500 mg from day 1 to day 7, then escalated from 500 mg to 750 mg from day 7 to day 14, and then escalated from 750 mg to 1000 mg from day 14 to day 21. In some embodiments, the daily dose is escalated from 500 mg to 750 mg from day 1 to day7, then escalated from 750 mg to 1000mg from day 7 to day 14, then escalated from 1000 mg to 1500 mg. In any of these embodiments, the daily dose is administered in one dose, or split into two or three doses, i.e., administration is once, twice or three times daily.

In a prophylactic context, the pharmaceutical composition of the invention can be administered at any time before or after an event, for example, radiation therapy, chemotherapy, or surgical lymph node dissection, which places a subject at risk of or susceptible to lymphatic injury and/or developing edema. In some embodiments, the pharmaceutical composition is administered prophylactically up to about one week before the event, such as 1, 2, 3, 4, 5, 6, or 7 days before the event. In some instances, the pharmaceutical composition is administered prophylactically on the same day as the event. In some embodiments, the pharmaceutical composition is administered prophylactically within six weeks of the event, for example, within about 1, 2, 3, 4, 5, or 6 days, or within about 1, 2, 3, 4, 5 or 6 weeks of the event. In some embodiments, the pharmaceutical composition is administered prophylactically for about 2-4 weeks or for about 1, 2, 3, 4, 5, or 6 weeks.

In one embodiment, methods of treating lymphedema in a subject comprising administering LYT-100 are provided herein, wherein the treatment duration is selected from 1 week, 2 weeks, 3 weeks, 4 weeks, 5 weeks, 6 weeks, 7 weeks, 8 weeks, 9 weeks, 10 weeks, 11 weeks, 12 weeks, 13 weeks, 14 weeks, 15 weeks, 16 weeks, 17 weeks, 18 weeks, 19 weeks, 20 weeks, 21 weeks, 22 weeks, 23 weeks, 24 weeks, and 25 weeks, and any increment therein.

In one embodiment, methods of treating lymphedema in a subject comprising administering LYT-100 are provided herein, wherein the treatment duration is selected from 1 month, 2 months, 3 months, 4 months, 5 months, 6 months, 7 months, 8 months, 9 months, 10 months, 11 months, or 12 months, and any increment therein. In one embodiment, methods of treating lymphedema in a subject comprising administering LYT-100 are provided herein, wherein the treatment duration is one year, 2 years, 3 years, 4 years, 5 years or greater.

In some embodiments, LYT-100 may be administered with or without food. In some embodiments, LYT-100 is administered with food. In some embodiments, LYT-100 is administered without food.

In some embodiments, the pharmaceutical composition is administered topically once a day or at least once a day. In another embodiment, the pharmaceutical composition is administered topically twice a day or at least twice a day. Where the pharmaceutical composition or method involves prevention of edema, particularly prevention of lymphedema, the composition can be administered within about six weeks of a lymphatic injury, for example within about two weeks of a lymphatic injury.

In some embodiments, the pharmaceutical composition is administered orally once a day or at least once a day. In another embodiment, the pharmaceutical composition is administered orally twice a day or at least twice a day. Where the pharmaceutical composition or method involves prevention of edema, particularly prevention of lymphedema, the composition can be administered within about six weeks of a lymphatic injury, for example within about two weeks of a lymphatic injury.

### Methods, Compositions and Dosing for Treating Interstitial Lung Disease

Idiopathic Pulmonary Fibrosis (IPF) afflicts approximately 100,000 people in the United States, with the drugs nintedanib and pirfenidone as the only available treatments. In preclinical studies, LYT-100 demonstrated favorable anti-fibrotic and anti-inflammatory activity compared to pirfenidone.

Accordingly, provided herein is a method of treating an interstitial lung disease (ILD), comprising administering to a subject in need thereof an effective amount of deuterium-enriched pirfenidone having the structure: 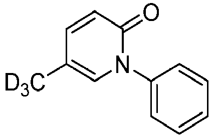 or a pharmaceutically acceptable salt thereof, wherein ILD is treated in the subject.

In certain embodiments, the ILD is idiopathic pulmonary fibrosis (IPF). In some embodiments, the ILD is chILD.

### Clinical Advantages of Deuterium-Enriched Pirfenidone

Pirfenidone is a small molecule that has anti-fibrotic and anti-inflammatory effects. Recent studies have suggested that this activity is due, at least in part, to inhibition of production and activity of TGF-β. Iyer et al., J. Pharmacol. Exp. Ther. 291: 367-373 (1999); Tada et al., Clin. Exper. Pharmacol. Physiol. 28:522-527 (2001); Oku et al., Eur. J. Pharmacol. 590:400-408 (2008). It is currently approved in the United States and elsewhere for oral administration in the treatment of idiopathic pulmonary fibrosis (IPF). Taniguchi et al., Eur. Respir. J. 35:821-829 (2010); Noble et al., Lancet 377: 1760-1769 (2011); King et al., N. Engl. J. Med. 370:2083-2092 (2014). Idiopathic pulmonary fibrosis (IPF) is a debilitating, progressive and fatal fibrotic lung disease, with an approximate median survival of 2-5 years from the time of diagnosis. IPF is one of the most commonly encountered interstitial lung diseases (II,Ds), with increasing incidence and prevalence worldwide. Pirfenidone is one of two approved therapies for the treatment of idiopathic pulmonary fibrosis (IPF). Randomised controlled clinical trials and subsequent post hoc analyses have demonstrated that pirfenidone reduces lung function decline, decreases mortality and improves progression-free survival.

However, Pirfenidone has a very short half-life in humans and consequently relatively frequent dosing is required. The recommended daily maintenance dose of pirfenidone is 801 mg three times per day (2403 mg·day-1) (a total of nine (9) pills per day at full dose) with a 14-day titration period upon treatment initiation.

In addition, for patients with IPF to obtain the maximum benefits of pirfenidone treatment, the adverse events (AEs) associated with pirfenidone need to be managed. The most common AEs are gastrointestinal (GI) and skin-related adverse events, for example, nausea, rash, diarrhea, fatigue, dyspepsia, anorexia, dizziness, gastroesophegeal reflux disease, decreased appetite, decreased weight, photosensitivity, and cough. In addition, several treatment-emergent adverse events have been reported, including upper respiratory infection and bronchitis. A recent study in patients treated with pirfenidone under a compassionate use program demonstrated that 44% of the patients had an adverse event with pirfenidone, with only half of them continuing on pirfenidone after a dose-reduction. Raghu & Thickett. Thorax; 68: 605-608 (2013). Adverse events common with pirfenidone at 2403 mg/day include nausea, rash, fatigue, diarrhea, vomiting, dyspepsia, photosensitivity, and anorexia. Noble et al. Lancet; 377: 1760-69 (2011).

The results of several expanded cinical trials are summarized in Lancaster et al., Eur Resp Rev 2017:26:170057 which reports treatment-emergent adverse events (TEAEs) as rates per 100 PEY (equivalent to the frequency at which a physician might expect these TEAEs to occur if 100 patients with IPF were followed for 1 year). Herein, it is noted that the most common reported AEs leading to discontinuation are nausea, fatigue, diarrhea, and/or rash with frequencies as high as 62.1 per 100 PEY (nausea), 27.6 per 100PEY (diarrhoea), 52.4 per 100PEY( fatigue). In a single-centre, retrospective, observational study of 351 patients who were receiving pirfenidone, 75% of reported AEs were GI-related, with loss of appetite (17%) and nausea/vomiting (15%) being most frequent, similar to what was observed in the phase III trials. The highest number of treatment discontinuations occurred with appetite loss and nausea/vomiting. The incidience of AEs and discontinuation increases with age. The proportion of patients with ADRs leading to dose modification/interruption or discontinuation increased with increasing age: an ADR leading to dose modification/interruption occurred in 32.7% of patients aged ≥80 years and in 18.0% of patients aged <65 years, while an ADR leading to discontinuation occurred in 20.9% of patients aged ≥80 years and in 7.5% of patients aged <65 years.

Several methods for managing AEs associated with pirfenidone have been proposed, including varying the dose titration schedule by using a slower titration, employing dose modifications, including reductions or interruptions (in phase III trials, dose reductions and interruptions occurred in 46% and 41% of patients receiving pirfenidone, respectively, with a median duration of 28 days and 14 days, respectively). Overall, 30% of pirfenidone patients had dose modifications and 29% discontinued permanently due to AEs in phase III trials. In addition, modification of eating habits of the patient is required when adjusting the pirfenidone dose. Taking pirfenidone with a substantial amount of food, specifically the full dose at the end of a substantial meal or spreading out the three capsules during the meal, may reduce the rate of pirfenidone absorption and mitigate the onset of GI-related AEs.

Although slower titration and dose modification may assist in addressing patient AEs, employing such measures has significant therapeutic impact, notably patients who received pirfenidone 1197 mg/day were reported to experience greater lung function decline than patients who were receiving the full dose of 2403 mg/day.

In addition, pirfenidone treatment has liver function Aesad therefore, monitoring liver function is also important during pirfenidone treatment. Elevations of aspartate transaminase (AST) and alanine transaminase (ALT) levels to >3× the upper limit of normal (ULN) occurred in the phase III trials (3.2%), which were managed by dose modifications or discontinuation. If AST and ALT elevations (>3× to ≤5× ULN) occur without symptoms or hyperbilirubinaemia, the dose may be reduced or interrupted until values return to normal. However, in cases in which the AST and ALT elevations (>3× to≤5× ULN) are accompanied by hyperbilirubinaemia or if patients exhibit >5× ULN, pirfenidone must be permanently discontinued.

In addition, patients must be monitored for drug-drug interactions, because the patients taking other oral medications at the same time, may significantly affect pirfenidone metabolism by inhibiting or inducing hepatic enzyme systems (cytochrome P450 1A2 (CYP1A2), CYP3A4, P-glycoprotein). For example, for strong CYP1A2 inhibitors such as fluvoxamine and enoxacin, pirfenidone should be reduced to 267 mg three times daily (801 mg·day-1). For moderate CYP1A2 inhibitors, such as ciprofloxacin at a dosage of 750 mg twice daily, pirfenidone should be reduced to 534 mg three times daily (1602 mg·day-1). Patients should also be assessed for GI intolerance, skin reactions and liver enzyme elevations.

Therefore, pirfenidone treatment requires various AE management strategies, including a slower dose titration for initiating treatment, taking pirfenidone with substantial meals, spacing capsules throughout the meal, diet modification, weight-based dosing regimens and dose reductions and interruptions, as well as continual liver function monitoring.

Accordingly, limitations of pirfenidone include: a short half-life of only about 2.5 hours; a high pill burden (of 9 capsules per day (TID dosing); poor tolerability including nausea, diarrhea and photosensitivity; a high dose required for efficacy that induces side effects; and significant interpatient variability.

In contrast, deuterium-enriched pirfenidone compounds address the deficiencies associated with pirfenidone. The metabolism of pirfenidone is only partially understood. For example, without wishing to be bound by theory, the methyl group is thought to be susceptible to oxidation, which would lead to a corresponding hydroxymethyl metabolite, "M1." M1 is thought to be further oxidized to a carboxylic acid metabolite, "M2" (Wang et al., Biomedical Chromatography 2006, 20, 1375-1379). A third detected metabolite is believed to be a phase II product possibly originating from M1 or M2.

Pirfenidone is a substituted pyridinone-based fibrosis modulator and/or collagen infiltration modulator. The carbon-hydrogen bonds of pirfenidone contain a naturally occurring distribution of hydrogen isotopes, namely 1H or protium (about 99.9844%), 2H or deuterium (about 0.0156%), and 3H or tritium (in the range between about 0.5 and 67 tritium atoms per 1018 protium atoms). Increased levels of deuterium incorporation may produce a detectable Kinetic Isotope Effect (KIE) that could affect the pharmacokinetic, pharmacologic and/or toxicologic profiles of such fibrosis modulators and/or collagen-infiltration modulators in comparison with the compound having naturally occurring levels of deuterium.

Pirfenidone is likely metabolized in humans by oxidation of the methyl group. Other sites on the molecule may also undergo transformations leading to metabolites with as-yet-unknown pharmacology/toxicology. Limiting the production of these metabolites has the potential to decrease the danger of the administration of such drugs and may even allow increased dosage and concomitant increased efficacy. All of these transformations can occur through polymorphically-expressed enzymes, thus exacerbating the interpatient variability.

Accordingly, various deuteration patterns can be used to a) reduce or eliminate unwanted metabolites, b) increase the half-life of the parent drug, c) decrease the number of doses needed to achieve a desired effect, d) decrease the amount of a dose needed to achieve a desired effect, e) increase the formation of active metabolites, if any are formed, and/or f) decrease the production of deleterious metabolites in specific tissues and/or create a more effective drug and/or a safer drug for polypharmacy, whether the polypharmacy be intentional or not. The deuteration approach has strong potential to slow the metabolism via various oxidative and racemization mechanisms.

In one embodiment, the deuterated compounds disclosed herein, e.g., LYT-100 maintain the beneficial aspects of the corresponding non-isotopically enriched molecules while substantially increasing the maximum tolerated dose, decreasing toxicity, increasing the half-life (T1/2), lowering the maximum plasma concentration (Cmax) of the minimum efficacious dose (MED), lowering the efficacious dose and thus decreasing the non-mechanism-related toxicity, and/or lowering the probability of drug-drug interactions.

. In some embodiments, the deuterium-enriched pirfenidone compound used in the disclosed methods has at least one of the following properties: a) decreased inter-individual variation in plasma levels of the compound or a metabolite thereof as compared to the non-isotopically enriched compound; b) increased average plasma levels of the compound per dosage unit thereof as compared to the non-isotopically enriched compound; c) decreased average plasma levels of at least one metabolite of the compound per dosage unit thereof as compared to the non-isotopically enriched compound; d) increased average plasma levels of at least one metabolite of the compound per dosage unit thereof as compared to the non-isotopically enriched compound; and e) an improved clinical effect during the treatment in the subject per dosage unit thereof as compared to the non-isotopically enriched compound. Thus, disclosed herein are methods for treating a subject, including a human, having or suspected of having edema, e.g., lymphedema, or for preventing such disorder in a subject prone to the disorder; comprising administering to the subject a therapeutically effective amount of a deuterium-enriched pirfenidone compound as disclosed herein, or a pharmaceutically acceptable salt, solvate, or prodrug thereof; so as to effect one or more of a) - e) above during the treatment of the disorder as compared to the corresponding non-isotopically enriched compound. In some embodiments, the deuterium-enriched pirfenidone compound has at least two of the properties a) through e) above. In some embodiments, the deuterium-enriched pirfenidone compound has three or more of the properties a) through e) above.

In one embodiment is a method for the treatment, prevention, or amelioration of one or more symptoms of edema, e.g., lymphedema.

Disclosed herein are methods for treating a subject, including a human, having or suspected of having edema, e.g., lymphedema, or for preventing such disorder in a subject prone to the disorder; comprising administering to the subject a therapeutically effective amount of a deuterium-enriched pirfenidone compound as disclosed herein, or a pharmaceutically acceptable salt, solvate, or prodrug thereof; so as to effect decreased inter-individual variation in plasma levels of the compound or a metabolite thereof, during the treatment of the disorder as compared to the corresponding non-isotopically enriched compound. In certain embodiments, the inter-individual variation in plasma levels of the compounds as disclosed herein, or metabolites thereof, is decreased by greater than about 2%, greater than about 5%, greater than about 10%, greater than about 15%, greater than about 20%, greater than about 25%, greater than about 30%, greater than about 40%, or by greater than about 50% (including any numerical increment between the listed percentages) as compared to the corresponding non-isotopically enriched compound.

Disclosed herein are methods for treating a subject, including a human, having or suspected of having edema, e.g., lymphedema, or for preventing such disorder in a subject prone to the disorder; comprising administering to the subject a therapeutically effective amount of a deuterium-enriched pirfenidone compound as disclosed herein, or a pharmaceutically acceptable salt, solvate, or prodrug thereof; so as to affect increased average plasma levels of the compound or decreased average plasma levels of at least one metabolite of the compound per dosage unit as compared to the corresponding non-isotopically enriched compound. In certain embodiments, the average plasma levels of the compound as disclosed herein are increased by greater than about 2%, greater than about 5%, greater than about 10%, greater than about 15%, greater than about 20%, greater than about 25%, greater than about 30%, greater than about 40%, or by greater than about 50% (including any numerical increment between the listed percentages) as compared to the corresponding non-isotopically enriched compounds. In certain embodiments, the average plasma levels of a metabolite of the compound as disclosed herein are decreased by greater than about 2%, greater than about 5%, greater than about 10%, greater than about 15%, greater than about 20%, greater than about 25%, greater than about 30%, greater than about 40%, or by greater than about 50% (including any numerical increment between the listed percentages) as compared to the corresponding non-isotopically enriched compounds.

Plasma levels of the compound as disclosed herein, or metabolites thereof, may be measured using the methods described by Li et al. (Rapid Communications in Mass Spectrometry 2005, 19, 1943-1950).

In some embodiments, the compound has a decreased metabolism by at least one polymorphically-expressed cytochrome P₄₅₀ isoform in the subject per dosage unit thereof as compared to the non-isotopically enriched compound.

In some embodiments, the cytochrome P₄₅₀ isoform is selected from CYP2C8, CYP2C9, CYP2C19, and CYP2D6.

In some embodiments, the compound is characterized by decreased inhibition of at least one cytochrome P₄₅₀ or monoamine oxidase isoform in the subject per dosage unit thereof as compared to the non-isotopically enriched compound.

In certain embodiments, the cytochrome P₄₅₀ or monoamine oxidase isoform is selected from CYP1A1, CYP1A2, CYP1B1, CYP2A6, CYP2A13, CYP2B6, CYP2C8, CYP2C9, CYP2C18, CYP2C19, CYP2D6, CYP2E1, CYP2G1, CYP2J2, CYP2R1, CYP2S1, CYP3A4, CYP3A5, CYP3ASP1, CYP3A5P2, CYP3A7, CYP4A11, CYP4B1, CYP4F2, CYP4F3, CYP4F8, CYP4F11, CYP4F12, CYP4X1, CYP4Z1, CYP5A1, CYP7A1, CYP7B1, CYP8A1, CYP8B1, CYP11A1, CYP11B1, CYP11B2, CYP17, CYP19, CYP21, CYP24, CYP26A1, CYP26B1, CYP27A1, CYP27B1, CYP39, CYP46, CYP51, MAO_{A}, and MAO_{B}.

In some embodiments, the deuterium-enriched pirfenidone compound has at least one of the following properties: a) a half-life greater than 2.5 hours; b) a decreased pill burden; c) increased patient tolerability; d) a lower efficacious dose; e) increased bioavailability; f) increased Cmax; and g) increase in systemic exposure during the treatment in the subject per dosage unit thereof as compared to the non-isotopically enriched compound. Disclosed herein are methods for treating a subject, including a human, having or suspected of having edema, e.g., lymphedema, or for preventing such disorder in a subject prone to the disorder; comprising administering to the subject a therapeutically effective amount of a deuterium-enriched pirfenidone compound as disclosed herein, or a pharmaceutically acceptable salt, solvate, or prodrug thereof; so as to effect one or more of a) - g) above during the treatment of the disorder as compared to the corresponding non-isotopically enriched compound.

Disclosed herein are methods for treating a subject, including a human, having or suspected of having edema, e.g., lymphedema, or for preventing such disorder in a subject prone to the disorder; comprising administering to the subject a therapeutically effective amount of a deuterium-enriched pirfenidone compound as disclosed herein, or a pharmaceutically acceptable salt, solvate, or prodrug thereof; so as to effect a longer half-life. In some embodiments, the half-life of the deuterium-enriched pirfenidone compounds as disclosed herein, or metabolites thereof, is increased by greater than about 2%, greater than about 5%, greater than about 10%, greater than about 15%, greater than about 20%, greater than about 25%, greater than about 30%, greater than about 40%, by greater than about 50%, by greater than about 60%, by greater than about 70%, by greater than about 80%, by greater than about 90%, or by greater than about 100% (including any numerical increment between the listed percentages) as compared to the corresponding non-isotopically enriched compound. In some embodiments, the half-life of the deuterium-enriched pirfenidone compounds as disclosed herein, or metabolites thereof, is increased by about 1.5-fold, increased by about 2-fold, greater than about 2-fold, greater than about 3-fold, greater than about 4-fold, greater than about greater than about 5-fold, greater than about 10-fold or more (including any numerical increment between the listed percentages) as compared to the corresponding non-isotopically enriched compound.

Disclosed herein are methods for treating a subject, including a human, having or suspected of having edema, e.g., lymphedema, or for preventing such disorder in a subject prone to the disorder; comprising administering to the subject a therapeutically effective amount of a deuterium-enriched pirfenidone compound as disclosed herein, or a pharmaceutically acceptable salt, solvate, or prodrug thereof; so as to reduce the pill burden, e.g., effect a pill burden of less than nine (9) capsules per day (TID dosing) of the compound or a metabolite thereof, during the treatment of the disorder as compared to the corresponding non-isotopically enriched compound.

In certain embodiments, the pill burden of the compounds as disclosed herein, is decreased by greater than about 2%, greater than about 5%, greater than about 10%, greater than about 15%, greater than about 20%, greater than about 25%, greater than about 30%, greater than about 40%, or by greater than about 50% (including any numerical increment between the listed percentages) as compared to the corresponding non-isotopically enriched compound.

Disclosed herein are methods for treating a subject, including a human, having or suspected of having edema, e.g., lymphedema, or for preventing such disorder in a subject prone to the disorder; comprising administering to the subject a therapeutically effective amount of a deuterium-enriched pirfenidone compound as disclosed herein, or a pharmaceutically acceptable salt, solvate, or prodrug thereof; so as to effect an increased patient tolerability of the compound or a metabolite thereof, during the treatment of the disorder as compared to the corresponding non-isotopically enriched compound. In some embodiments, the patient tolerability is increased by altering the pharmacokinetics, e.g., by increasing the bioavailability (so as to use a lower dose) and/or by extending the half-life of the compound and/or by other means to reduce the side effects of pirfenidone.

In certain embodiments, the patient tolerability of the compounds as disclosed herein, or metabolites thereof, is increased by greater than about 2%, greater than about 5%, greater than about 10%, greater than about 15%, greater than about 20%, greater than about 25%, greater than about 30%, greater than about 40%, by greater than about 50%, by greater than about 60%, by greater than about 70%, by greater than about 80%, by greater than about 90%, or by greater than about 100% (including any numerical increment between the listed percentages) as compared to the corresponding non-isotopically enriched compound. In certain embodiments, the patient tolerability of the compounds as disclosed herein, or metabolites thereof, is increased by about 1.5-fold, increased by about 2-fold, greater than about 2-fold, greater than about 3-fold, greater than about 4-fold, greater than about greater than about 5-fold, greater than about 10-fold or more (including any numerical increment between the listed percentages) as compared to the corresponding non-isotopically enriched compound.

Disclosed herein are methods for treating a subject, including a human, having or suspected of having edema, e.g., lymphedema, or for preventing such disorder in a subject prone to the disorder; comprising administering to the subject a therapeutically effective amount of a deuterium-enriched pirfenidone compound as disclosed herein, or a pharmaceutically acceptable salt, solvate, or prodrug thereof; so as to effect a lower efficacious dose per dosage of the compound or a metabolite thereof, during the treatment of the disorder as compared to the corresponding non-isotopically enriched compound.

In certain embodiments, the efficacious dose per dosage of the compounds as disclosed herein, or metabolites thereof, is decreased by greater than about 2%, greater than about 5%, greater than about 10%, greater than about 15%, greater than about 20%, greater than about 25%, greater than about 30%, greater than about 40%, by greater than about 50%, by greater than about 60%, by greater than about 70%, by greater than about 80%, by greater than about 90%, or by greater than about 100% (including any numerical increment between the listed percentages) as compared to the corresponding non-isotopically enriched compound. In certain embodiments, the efficacious dose per dosage of the compounds as disclosed herein, or metabolites thereof, is decreased by about 1.5-fold, decreased by about 2-fold, greater than about 2-fold, greater than about 3-fold, greater than about 4-fold, greater than about greater than about 5-fold, greater than about 10-fold or more (including any numerical increment between the listed percentages) as compared to the corresponding non-isotopically enriched compound.

Disclosed herein are methods for treating a subject, including a human, having or suspected of having edema, e.g., lymphedema, or for preventing such disorder in a subject prone to the disorder; comprising administering to the subject a therapeutically effective amount of a deuterium-enriched pirfenidone compound as disclosed herein, or a pharmaceutically acceptable salt, solvate, or prodrug thereof; so as to increase the bioavailability per dosage of the compound or a metabolite thereof, during the treatment of the disorder as compared to the corresponding non-isotopically enriched compound.

In certain embodiments, the bioavailability per dosage of the compounds as disclosed herein, or metabolites thereof, is increased by greater than about 2%, greater than about 5%, greater than about 10%, greater than about 15%, greater than about 20%, greater than about 25%, greater than about 30%, greater than about 40%, by greater than about 50%, by greater than about 60%, by greater than about 70%, by greater than about 80%, by greater than about 90%, or by greater than about 100% (including any numerical increment between the listed percentages) as compared to the corresponding non-isotopically enriched compound. In certain embodiments, the bioavailability per dosage of the compounds as disclosed herein, or metabolites thereof, is increased by about 1.5-fold, decreased by about 2-fold, greater than about 2-fold, greater than about 3-fold, greater than about 4-fold, greater than about greater than about 5-fold, greater than about 10-fold or more (including any numerical increment between the listed percentages) as compared to the corresponding non-isotopically enriched compound.

Disclosed herein are methods for treating a subject, including a human, having or suspected of having or suspected of having edema, e.g., lymphedema, or for preventing such disorder in a subject prone to the disorder; comprising administering to the subject a therapeutically effective amount of a deuterium-enriched pirfenidone compound as disclosed herein, or a pharmaceutically acceptable salt, solvate, or prodrug thereof; so as to affect an increase in systemic exposure of the compound per dosage unit as compared to the corresponding non-isotopically enriched compound.

In certain embodiments, the systemic exposure per dosage of the compounds as disclosed herein, or metabolites thereof, is increased by greater than about 10%, greater than about 15%, greater than about 20%, greater than about 25%, greater than about 30%, greater than about 35%, greater than about 40%, greater than about 45%, or by greater than about 50% (including any numerical increment between the listed percentages) as compared to the corresponding non-isotopically enriched compound. In one embodiment, the systemic exposure per dosage of the compounds as disclosed herein is increased by greater than about 35% as compared to the corresponding non-isotopically enriched compound. In one embodiment, the systemic exposure per dosage of the compounds as disclosed herein is increased by about 35% as compared to the corresponding non-isotopically enriched compound.

Disclosed herein are methods for treating a subject, including a human, having or suspected of having or suspected of having edema, e.g., lymphedema, or for preventing such disorder in a subject prone to the disorder; comprising administering to the subject a therapeutically effective amount of a deuterium-enriched pirfenidone compound as disclosed herein, or a pharmaceutically acceptable salt, solvate, or prodrug thereof; so as to affect an increase in Cmax of the compound per dosage unit as compared to the corresponding non-isotopically enriched compound.

In certain embodiments, the Cmax per dosage of the compounds as disclosed herein, or metabolites thereof, is increased by greater than about 10%, greater than about 15%, greater than about 20%, greater than about 25%, greater than about 30%, greater than about 35%, greater than about 40%, greater than about 45%, or by greater than about 50% (including any numerical increment between the listed percentages) as compared to the corresponding non-isotopically enriched compound. In one embodiment, the Cmax per dosage of the compounds as disclosed herein is increased by greater than about 25% as compared to the corresponding non-isotopically enriched compound. In one embodiment, the Cmax per dosage of the compounds as disclosed herein is increased by about 25% as compared to the corresponding non-isotopically enriched compound.

In some embodiments, the method treats the disorder while reducing or eliminating a deleterious change in a diagnostic hepatobiliary function endpoint, as compared to the corresponding non-isotopically enriched compound, e.g., pirfenidone. Disclosed herein are methods for treating a subject, including a human, having or suspected of having edema, e.g., lymphedema, or for preventing such disorder in a subject prone to the disorder; comprising administering to the subject a therapeutically effective amount of a compound as disclosed herein, or a pharmaceutically acceptable salt, solvate, or prodrug thereof; so as to reduce or eliminate a deleterious change in a diagnostic hepatobiliary function endpoint, as compared to the corresponding non-isotopically enriched compound. In some embodiments, the diagnostic hepatobiliary function endpoint is selected from alanine aminotransferase ("ALT"), serum glutamic-pyruvic transaminase ("SGPT"), aspartate aminotransferase ("AST," "SCOT"), ALT/AST ratios, serum aldolase, alkaline phosphatase ("ALP"), ammonia levels, bilirubin, gamma-glutamyl transpeptidase ("GGTP," "gamma-GTP," "GGT"), leucine aminopeptidase ("LAP"), liver biopsy, liver ultrasonography, liver nuclear scan, 5'-nucleotidase, and blood protein.In some embodiments, the disease, disorder, or condition is selected from idiopathic pulmonary fibrosis, pneumoconiosis, silicosis, chalicosis, asbestosis, anthracosis, lymphedema (primary and/or secondary), systemic sclerosis (scleroderma) and/or a condition associated with scleroderma, juvenile systemic sclerosis (J-SSC), interstitial lung disease, scleroderma interstitial lung disease, focal segmental glomerulosclerosis (FSGS), diffuse lung disease such as diffuse parenchymal lung disease, diabetic nephropathy, lupus nephritis, polycystic kidney disease, ANCA vasculitis, membranous nephropathy, minimal change disease, chronic kidney disease, myocardial fibrosis, keloid scar, dermatopolymyositis, fibrotic sarcoidosis, medical device or implant rejection (such as breast capsular contracture), a fatty liver disease such as non-alcoholic steatohepatitis (NASH), and hepatitis-C fibrosis.

In some embodiments, the disease, disorder, or condition is selected from idiopathic pulmonary fibrosis, lymphedema (primary and/or secondary), systemic sclerosis (scleroderma), juvenile systemic sclerosis (J-SSC), scleroderma interstitial lung disease, or a condition associated with scleroderma. In some embodiments, the disease, disorder, or condition is myocardial fibrosis. In some embodiments, the disease, disorder, or condition is a keloid scar.

In some embodiments, the disease, disorder, or condition is dermatopolymyositis. Dermatopolymyositis (also called PM/DM) is a family of myositis disorders that includes polymyositis and dermatomyositis. In some embodiments, the disease, disorder, or condition is selected from dermatomyositis, juvenile dermatomyositis polymyositis, and inclusion body myositis.

In some embodiments, the disease, disorder, or condition is scleroderma, progressive systemic sclerosis, mixed connective tissue disease, or CREST syndrome.

In some embodiments, the disease, disorder, or condition is fibrotic sarcoidosis.

In some embodiments, the disease, disorder, or condition is surgical implant rejection such as an immune reaction to an implanted medical device or capsular contracture such as breast capsular contracture.

Compounds and combinations of the present invention may be used to treat a variety of diseases, disorders, and conditions. In some embodiments, the disease, disorder, or condition is selected from idiopathic pulmonary fibrosis, neurofibromatosis, Hermansky-Pudlak syndrome, diabetic nephropathy, renal fibrosis, hypertrophic cardiomyopathy (HCM), hypertension-related nephropathy, glomerulosclerosis (FSGS), radiation-induced fibrosis, multiple sclerosis (including secondary progressive multiple sclerosis), uterine leiomyomas (fibroids), alcoholic liver disease (including hepatic steatosis, hepatic fibrosis and hepatic cirrhosis), keloid scarring, hepatitis C virus (HCV) infection, proliferative disorders (including angiogenesis-mediated disorders), cancer (including glioma, glioblastoma, breast cancer, colon cancer, melanoma and pancreatic cancer), fibrotic disorders, interstitial lung diseases, atrial fibrillation (AF), organ transplant rejection, and scleroderma and related fibrotic conditions of the skin.

In some embodiments, the disease, disorder, or condition is diabetic nephropathy, Kimmelstiel-Wilson disease or syndrome, diabetic kidney disease, diabetic nephritis, or intercapillary or intracapillary glomerulosclerosis.

In some embodiments, the method of this invention is used to treat a disease or condition selected from idiopathic pulmonary fibrosis, neurofibromatosis, Hermansky-Pudlak syndrome, diabetic nephropathy, renal failure, hypertrophic cardiomyopathy (HCM), glomerulosclerosis (FSGS), radiation-induced fibrosis, multiple sclerosis, and uterine leiomyomas (fibroids) in a patient in need thereof.

In another particular embodiment, the method of the invention is used to treat renal fibrosis, hepatic fibrosis, uterine leiomyomas, keloid scarring, multiple sclerosis, radiation-associated fibrosis, organ transplant rejection, or cancer in a patient in need thereof.

In still another particular embodiment, the method is used to treat idiopathic pulmonary fibrosis in a patient in need thereof. In another particular embodiment, the method of this invention is used to treat secondary progressive multiple sclerosis in a patient in need thereof. In another particular embodiment, the method of this invention is used to treat pancreatic cancer in a patient in need thereof. In another more particular embodiment, the method of this invention is used to treat renal fibrosis in a patient in need thereof. More particularly the method is used to treat renal fibrosis as the result of diabetic nephropathy, glomerulopathy/FSGS or hypertension-related nephropathy. In still another embodiment, the amount of the compound of this invention administered to treat hepatic fibrosis in a patient in need thereof. Additional diseases, disorders, and conditions that may be treated in accordance with the present invention include those described herein and below.

In some embodiments, the present invention provides a method of treating, preventing, or ameliorating a disease, disorder, or condition selected from a fibrotic-meditated disorder, a collagen-mediated disorder, or a fibrotic-mediated and collagen-mediated disorder, comprising administering to a subject in need thereof an effective amount of deuterium-enriched pirfenidone or a pharmaceutically acceptable salt thereof. In some embodiments, the method further comprises administering an effective amount of an additional therapeutic agent, such as those described herein.

In some embodiments, the deuterium-enriched pirfenidone is a compound of Formula I or a pharmaceutically acceptable salt thereof.

In some embodiments, deuterium-enriched pirfenidone is LYT-100 or a pharmaceutically acceptable salt thereof.

In some embodiments, the deuterium-enriched pirfenidone is co-administered with one or more additional therapeutic agents, such as those described herein.

In some embodiments, the disease, disorder, or condition is selected from systemic sclerosis, systemic sclerosis-related pulmonary fibrosis, sarcoidosis, sarcoidosis-related pulmonary fibrosis, pulmonary fibrosis caused by infection, asbestos-induced pulmonary fibrosis, silica-induced pulmonary fibrosis, environmentally induced pulmonary fibrosis, radiation-induced pulmonary fibrosis, lupus-induced pulmonary fibrosis, drug-induced pulmonary fibrosis, and hypersensitivity pneumonitis, and/or any disorder ameliorated by modulating fibrosis and/or collagen infiltration into tissues.

In some embodiments, the disease, disorder, or condition is selected from idiopathic pulmonary fibrosis, uterine fibroids, multiple sclerosis, renal fibrosis, diabetic kidney disease, endotoxin-induced liver injury after partial hepatectomy or hepatic ischemia, allograft injury after organ transplantation, cystic fibrosis, atrial fibrilation, neutropenia, scleroderma, dermatomyositis, cirrhosis, diffuse parenchymal lung disease, mediastinal fibrosis, tuberculosis, spleen fibrosis caused by sickle-cell anemia, rheumatoid arthritis, and/or any disorder ameliorated by modulating fibrosis and/or collagen infiltration into tissues.

In some embodiments, the disease, disorder, or condition is selected from idiopathic pulmonary fibrosis, uterine fibroids, multiple sclerosis, renal fibrosis, diabetic kidney disease, endotoxin-induced liver injury after partial hepatectomy or hepatic ischemia, allograft injury after organ transplantation, cystic fibrosis, atrial fibrilation, neutropenia, scleroderma, dermatomyositis, cirrhosis, diffuse parenchymal lung disease, mediastinal fibrosis, tuberculosis, spleen fibrosis caused by sickle-cell anemia, and rheumatoid arthritis.

In some embodiments, the present disclosure relates to the use of deuterium-enriched pirfenidone to treat or prevent an inflammatory disease, disorder, or condition. In some embodiments, the deuterium-enriched pirfenidone is LYT-100. In some embodiments, the present disclosure provides a method of treating or preventing an inflammatory disease, disorder, or condition, comprising administering to a subject in need thereof an effective amount of deuterium-enriched pirfenidone. In some embodiments, the deuterium-enriched pirfenidone is LYT-100, or a pharmaceutically acceptable salt thereof.

In some embodiments, the present disclosure relates to the use of deuterium-enriched pirfenidone to treat or prevent a fibrotic disease, disorder, or condition. In some embodiments, the deuterium-enriched pirfenidone is LYT-100. In some embodiments, the present disclosure provides a method of treating or preventing a fibrotic disease, disorder, or condition, comprising administering to a subject in need thereof an effective amount of deuterium-enriched pirfenidone. In some embodiments, the deuterium-enriched pirfenidone is LYT-100, or a pharmaceutically acceptable salt thereof.

In some embodiments, the present disclosure relates to the use of deuterium-enriched pirfenidone to treat or prevent idiopathic pulmonary fibrosis. In some embodiments, the deuterium-enriched pirfenidone is LYT-100.

In some embodiments, the present disclosure provides a method of treating or preventing idiopathic pulmonary fibrosis, comprising administering to a subject in need thereof an effective amount of deuterium-enriched pirfenidone. In some embodiments, the deuterium-enriched pirfenidone is LYT-100, or a pharmaceutically acceptable salt thereof.

In some embodiments, the disease, disorder, or condition is idiopathic pulmonary fibrosis (IPF). In some embodiments, the disease, disorder, or condition is chronic fibrosing alveolitis, fibrosing alveolitis, fibrosing alveolitis lung, fibrosing lung disease, Hamman-Rich syndrome, or alveolar fibrosis.

In some embodiments, the disease, disorder, or condition is systemic sclerosis (scleroderma) and/or a related interstitial lung disease.

In some embodiments, the disease, disorder, or condition is a childhood interstitial lung disease (CHILD). In some embodiments, the childhood interstitial lung disease is selected from a surfactant dysfunction mutation, a childhood lung developmental disorder such as alveolar capillary dysplasia, a lung growth abnormality, neuroendocrine cell hyperplasia of infancy (NEHI), pulmonary interstitial glycogenosis (PIG), idiopathic interstitial pneumonia (such as nonspecific interstitial pneumonia, cryptogenic organizing pneumonia, acute interstitial pneumonia, desquamative interstitial pneumonia, lymphocytic interstitial pneumonia), an alveolar hemorrhage syndrome, an aspiration syndrome, a hypersensitivity pneumonitis, an infectious or postinfectious disease (bronchiolitis obliterans), eosinophilic pneumonia, pulmonary alveolar proteinosis, pulmonary infiltrates with eosinophilia, pulmonary lymphatic disorders (lymphangiomatosis, lymphangiectasis), pulmonary vascular disorders (haemangiomatosis), an interstitial lung disease associated with systemic disease process (such as connective tissue diseases, histiocytosis, malignancy-related lung disease, sarcoidosis, storage diseases), or a disorder of the compromised immune system (such as opportunistic infection, disorders related to therapeutic intervention, lung and bone marrow transplant-associated lung diseases, diffuse alveolar damage of unknown cause).

The various types of childhood interstitial lung diseases (CHILD) can affect many parts of the lungs, including the alveoli (air sacs), bronchial tubes (airways), and capillaries.

In some embodiments, the disease, disorder, or condition is scleroderma and at least one related condition selected from interstitial lung disease, tightening of the skin, joint pain, exaggerated response to cold (Raynaud's disease), and heartburn.

In some embodiments, the disease, disorder, or condition is selected from abnormal wound healing, a skin ulcer or scar, pulmonary fibrosis, fibrosis of lung, liver, kidney, or skin, or Dupuytren's contracture.

In some embodiments, the inflammatory disease is selected from an inflammatory disease of the liver or one that affects liver function. In some embodiments, the inflammatory disease is selected from non-alcoholic steatohepatitis (NASH), a fatty liver disease, or Hepatitis-C fibrosis.

In some embodiments, a method for the treatment, prevention, or amelioration of one or more symptoms of a fibrotic-mediated disorder and/or a collagen-mediated disorder in a subject comprises administering a therapeutically effective amount of a compound as disclosed herein.

In some embodiments, the fibrotic-mediated disorder and/or collagen-mediated disorder is selected from idiopathic pulmonary fibrosis, uterine fibroids, multiple sclerosis, renal fibrosis, diabetic kidney disease, endotoxin-induced liver injury after partial hepatectomy or hepatic ischemia, allograft injury after organ transplantation, cystic fibrosis, atrial fibrilation, neutropenia, scleroderma, dermatomyositis, cirrhosis, diffuse parenchymal lung disease, mediastinal fibrosis, tuberculosis, spleen fibrosis caused by sickle-cell anemia, and rheumatoid arthritis.

In some embodiments, the fibrotic-mediated disorder and/or said collagen-mediated disorder can be lessened, alleviated, or prevented by modulating fibrosis. In some embodiments, the fibrotic-mediated disorder and/or said collagen-mediated disorder can be lessened, alleviated, or prevented by modulating collagen infiltration.

In some embodiments, the treatment and/or prevention methods described herein may be performed in combination with one or more additional edema or lymphedema treatment and/or prevention methods known in the art, for example, treatment methods involving the administration of other therapeutic agents and/or treatment methods involving surgery, massage, compression therapy, fluid drainage therapy, acupuncture, laser, or any other suitable treatment methods.

### Definitions

While the terms used herein are believed to be well understood by one of ordinary skill in the art, definitions are set forth herein to facilitate explanation of the presently-disclosed subject matter.

The term "pharmaceutical composition" refers to a preparation that is in such form as to permit the biological activity of the active ingredient to be effective, and which contains no additional components that are unacceptably toxic to a subject to which the composition would be administered. Pharmaceutical compositions can be in numerous dosage forms, for example, tablet, capsule, liquid, solution, softgel, suspension, emulsion, syrup, elixir, tincture, film, powder, hydrogel, ointment, paste, cream, lotion, gel, mousse, foam, lacquer, spray, aerosol, inhaler, nebulizer, ophthalmic drops, patch, suppository, and/or enema. Pharmaceutical compositions typically comprise a pharmaceutically acceptable carrier, and can comprise one or more of a buffer (e.g. acetate, phosphate or citrate buffer), a surfactant (e.g. polysorbate), a stabilizing agent (e.g. human albumin), a preservative (e.g. benzyl alcohol), a penetration enhancer, an absorption promoter to enhance bioavailability and/or other conventional solubilizing or dispersing agents. Choice of dosage form and excipients depends upon the active agent to be delivered and the disease or disorder to be treated or prevented, and is routine to one of ordinary skill in the art.

The term "deuterium enrichment" refers to the percentage of incorporation of deuterium at a given position in a molecule in the place of hydrogen. For example, deuterium enrichment of 1% at a given position means that 1% of molecules in a given sample contain deuterium at the specified position. Because the naturally occurring distribution of deuterium is about 0.0156%, deuterium enrichment at any position in a compound synthesized using non-enriched starting materials is about 0.0156%. The deuterium enrichment can be determined using conventional analytical methods, such as mass spectrometry and nuclear magnetic resonance spectroscopy.

The term "is/are deuterium," when used to describe a given variable position in a molecule or formula, or the symbol "D," when used to represent a given position in a drawing of a molecular structure, means that the specified position is enriched with deuterium above the naturally occurring distribution of deuterium. In some embodiments, deuterium enrichment is of no less than about 1%, no less than about 5%, no less than about 10%, no less than about 20%, no less than about 50%, no less than about 70%, no less than about 80%, no less than about 90%, no less than about 98%, or in some embodiments no less than about 99% of deuterium at the specified position. In some embodiments, the deuterium enrichment is above 90% at each specified position. In some embodiments, the deuterium enrichment is above 95% at each specified position. In some embodiments, the deuterium enrichment is about 99% at each specified position.

The term "isotopic enrichment" refers to the percentage of incorporation of a less prevalent isotope of an element at a given position in a molecule in the place of the more prevalent isotope of the element.

The term "non-isotopically enriched" refers to a molecule in which the percentages of the various isotopes are substantially the same as the naturally occurring percentages.

The term "fibrosis" refers to the development of excessive fibrous connective tissue within an organ or tissue.

The term "collagen infiltration" refers to the entry of the connective tissue collagen into cells or into the extracellular matrix around cells. This occurs in organs and tissues naturally and under normal circumstances but can occur excessively and accompany or cause disease.

The term "collagen-mediated disorder" refers to a disorder that is characterized by abnormal or undesired collagenic infiltration, that when collagen infiltration activity is modified, leads to the desired responses depending on the route of administration and desired end result. A collagen-mediated disorder may be completely or partially mediated through the modulation of collagen infiltration. In particular, a collagen-mediated disorder is one in which modulation of collagen infiltration activity results in some effect on the underlying disorder, e.g., administering a collagen-infiltration modulator results in some improvement in at least some of the patients being treated.

The term "fibrotic-mediated disorder" refers to a disorder that is characterized by abnormal or undesired fibrotic activity, that when fibrosis activity is modified, leads to the desired responses depending on the route of administration and desired end result. A fibrosis-mediated disorder may be completely or partially mediated through the modulation of fibrosis. In particular, a fibrosis-mediated disorder is one in which modulation of fibrosis activity results in some effect on the underlying disorder, e.g., administering a fibrosis modulator results in some improvement in at least some of the patients being treated.

The terms "fibrosis modulator" or "modulating fibrosis" are meant to be interchangeable and refer to the ability of a compound disclosed herein to alter the occurrence and/or amount of fibrosis. A fibrosis modulator may increase the occurrence or level of fibrosis, may increase or decrease the occurrence and/or amount of fibrosis depending on the concentration of the compound exposed to the adrenergic receptor, or may decrease the occurrence and/or amount of fibrosis. Such activation or inhibition may be contingent on the occurrence of a specific event, such as activation of a signal transduction pathway, and/or may be manifest only in particular cell types.

The terms "collagen-infiltration modulator" or "modulating collagen infiltration" are meant to be interchangeable and refer to the ability of a compound disclosed herein to alter the occurrence and/or amount of collagen infiltration. A fibrosis modulator may increase the occurrence or level of collagen infiltration, may increase or decrease the occurrence and/or amount of collagen infiltration depending on the concentration of the compound exposed to the adrenergic receptor, or may decrease the occurrence and/or amount of collagen infiltration. Such activation or inhibition may be contingent on the occurrence of a specific event, such as activation of a signal transduction pathway, and/or may be manifest only in particular cell types.

An "effective amount" of a composition as disclosed herein is an amount sufficient to carry out a specifically stated purpose. An "effective amount" can be determined empirically and in a routine manner, in relation to the stated purpose, route of administration, and dosage form.

Terms such as "treating" or "treatment" or "to treat" or "alleviating" or "to alleviate" refer to therapeutic measures that cure, slow down, ameliorate or lessen one or more symptoms of, halt progression of, and/or ameliorate or lessen a diagnosed pathologic condition or disorder. Thus, those in need of treatment include those already with the disorder. In some embodiments, treatment may be administered after one or more symptoms have developed. In other embodiments, treatment may be administered in the absence of symptoms. For example, treatment may be administered to a susceptible individual prior to the onset of symptoms (e.g., in light of a history of symptoms and/or in light of genetic or other susceptibility factors). Treatment may also be continued after symptoms have resolved, for example to prevent or delay their recurrence. In some embodiments, a subject is successfully "treated" for a disease or disorder according to the methods provided herein if the patient shows, e.g., total, partial, or transient alleviation or elimination of symptoms associated with the disease or disorder. For example, "treating edema" can include, but is not limited to, decreasing swelling, decreasing inflammation, decreasing fibrosis, decreasing pain, increasing range of motion, decreasing heaviness, decreasing tightness, decreasing skin thickening, and/or improving lymphatic function.

"Prevent" or "prevention" refers to prophylactic or preventative measures that obstruct, delay and/or slow the development of a targeted pathologic condition or disorder or one or more symptoms of a a targeted pathologic condition or disorder. Thus, those in need of prevention include those at risk of or susceptible to developing the disorder. Subjects that are at risk of or susceptible to developing lymphedema include, but are not limited to, cancer patients undergoing radiation therapy, chemotherapy, and/or surgical lymph node dissection. In some embodiments, a disease or disorder is successfully prevented according to the methods provided herein if the patient develops, transiently or permanently, e.g., fewer or less severe symptoms associated with the disease or disorder, or a later onset of symptoms associated with the disease or disorder, than a patient who has not been subject to the methods of the invention.

An "anti-T cell agent" is a molecule that reduces T cell-mediated inflammation, T cell activation, T cell differentiation, and/or T cell proliferation. Classes of anti-T cell agents include calcineurin inhibitors and IL-2 inhibitors. Examples of small molecule anti-T cell agents include tacrolimus, teriflunomide, leflunomide, cyclosporine, and pimecrolimus. Examples of macromolecule anti-T cells agents include denileukin diftitox and Basiliximab.

An "anti-TGF-β1 agent" is a molecule that inhibits the expression, secretion, activation, signaling, or activity of transforming growth factor beta 1. Pirfenidone and deuterium-enriched pirfenidone are examples of small molecule anti-TGF-β1 agents.

An "anti-angiotensin agent" is a molecule that inhibits the activity of AngI or AngII, or a molecule that inhibits AngI to AngII conversion (e.g., ACE inhibitor or ACE agonist). Examples of anti-angiotensin agents include captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099.

Compounds of the present invention include those described generally herein, and are further illustrated by the classes, subclasses, and species disclosed herein. As used herein, the following definitions shall apply unless otherwise indicated. For purposes of this invention, the chemical elements are identified in accordance with the Periodic Table of the Elements, Handbook of Chemistry and Physics, 98^{th} Ed. Additionally, general principles of organic chemistry are described in "Organic Chemistry," Thomas Sorrell, University Science Books, Sausalito: 1999, and March's Advanced Organic Chemistry: Reactions, Mechanisms, and Structure, M. B. Smith and J. March, 7th Edition, John Wiley & Sons, 2013, the entire contents of which are hereby incorporated by reference.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge et al., describe pharmaceutically acceptable salts in detail in J. Pharmaceutical Sciences, 1977, 66, 1-19, incorporated herein by reference. Pharmaceutically acceptable salts of the compounds of this invention include those derived from suitable inorganic and organic acids and bases. Examples of pharmaceutically acceptable, nontoxic acid addition salts include salts of an amino group (or other basic group) formed with inorganic acids such as hydrochloric acid, hydrobromic acid, phosphoric acid, sulfuric acid, and perchloric acid or with organic acids such as acetic acid, oxalic acid, maleic acid, tartaric acid, citric acid, succinic acid, or malonic acid, or by using other methods used in the art such as ion exchange. Other pharmaceutically acceptable salts include adipate, alginate, ascorbate, aspartate, benzenesulfonate, benzoate, besylate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, pivalate, propionate, stearate, succinate, sulfate, tartrate, thiocyanate, p-toluenesulfonate, undecanoate, valerate salts, and the like.

Salts derived from appropriate bases include alkali metal, alkaline earth metal, ammonium and N⁺(C₁₋₄alkyl)₄ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

Unless otherwise stated, structures depicted herein are also meant to include all isomeric (e.g., enantiomeric, diastereomeric, and geometric (or conformational)) forms of the structure; for example, the R and S configurations for each asymmetric center, Z and E double bond isomers, and Z and E conformational isomers. Therefore, single stereochemical isomers as well as enantiomeric, diastereomeric, and geometric (or conformational) mixtures of the present compounds are within the scope of the invention. Unless otherwise stated, all tautomeric forms of the compounds of the invention are within the scope of the invention. Additionally, unless otherwise stated, structures depicted herein are also meant to include compounds that differ only in the presence of one or more isotopically enriched atoms. For example, compounds having the present structures including the replacement of hydrogen by deuterium or tritium, or the replacement of a carbon by a ¹³C- or ¹⁴C-enriched carbon are within the scope of this invention. Such compounds are useful, for example, as analytical tools, as probes in biological assays, or as therapeutic agents in accordance with the present invention.

Disclosed compounds, as well as pharmaceutically acceptable compositions comprising a disclosed compound and a pharmaceutically acceptable excipient, adjuvant, diluent, or carrier, are useful for treating a variety of diseases, disorders, and conditions. Such diseases, disorders, and conditions include those described herein.

One of ordinary skill in the art will recognize that each of the therapeutic agents described herein are known to be associated with treatment of one or more diseases, disorders, or conditions. Accordingly, it will be understood that, in certain embodiments, the present invention provides a method of treating a disease, disorder, or condition in a patient in need thereof, comprising administering to the patient an effective amount of a disclosed compound, combination of compounds, or pharmaceutical composition thereof.

### Other Active Agents

In some embodiments, the present invention provides systemic (e.g., IV or oral) or local (e.g., topical or transdermal) administration of an anti-T cell, anti-TGF-β1, and/or anti-angiotensin agent, such as tacrolimus, deuterium-enriched pirfenidone, teriflunomide, leflunomide, or captopril; or a pharmaceutically acceptable salt thereof. In some embodiments, administration of such a combination improves lymphedema and lymphatic function, and has a variety of other beneficial biological effects, including stimulating lymphangiogenesis, when administered to mammalian subj ects. Moreover, because these agents act at different steps of the fibrosis pathway, in some embodiments, combinations of anti-T cell, anti-TGF-β1, and/or anti-angiotensin agents are more effective than administration of a single agent. In some embodiments, the combination exhibits synergistic effects. In some embodiments, the present invention provides systemic or local administration of an anti-TGF-β and/or anti-TNF-alpha agent, such as deuterium-enriched pirfenidone. In some embodiments, disclosed herein are methods of treating a disease, e.g., edema, that includes administering LYT-100 and pirfenidone, wherein together LYT-100 and pirfenidone provide an effective amount of active(s) to treat the disease.

Tacrolimus is an anti-T cell agent that is FDA approved as a topical formulation and used to treat cutaneous inflammatory/fibrotic diseases including atopic dermatitis (Ruzicka et al., N. Engl. J. Med. 337:816-821 (1997)), psoriasis (Wang et al., J. Cutan. Med. Surg. 18:8-14 (2014)), and localized scleroderma (Mancuso et al., Br. J. Dermatol. 152: 180- 182 (2005)). Tacrolimus is a macrolide produced by the soil bacterium *Streptomyces tsukubaensis* that is well-tolerated when used for prevention of transplant rejection and treatment of a variety of autoimmune diseases. It exerts its anti-T cell properties by binding to FK-506 binding protein 12 (FKBP-12), thus inhibiting calcineurin, and ultimately decreasing IL-2 expression. Clipstone et al., Nature 357:695-697 (1992). Because IL-2 is essential for T cell activation and differentiation of CD4⁺ T cells, calcineurin inhibitors have profound CD4⁺ cell immunosuppressive effects. Liao et al., Immunity 38: 13-25 (2013); Rautajoki et al., Ann. Med. 40:322-335 (2008).

Teriflunomide is an immunosuppressive agent that decreases T cell inflammatory responses. Oral administration of teriflunomide is FDA-approved for the treatment of multiple sclerosis. Williamson et al., J. Biol. Chem. 270:22467-22472 (1995); Davis et al., Biochem. 35: 1270-1273 (1996); Iglesias-Bregna et al., J. Pharmacol. Exp. Ther. 347:203- 211 (2013). Teriflunomide is the active metabolite of leflunomide, and inhibits de novo pyrimidine synthesis by blocking the enzyme dilivdroorotate dehydrogenase. Teriflunomide has also been shown to inhibit activation of Signal transducer and activator of STAT-6, a key regulator of Th2 differentiation. Olsan et al., Proc. Natl. Acad. Sci. USA 108: 18067-18072 (2011). As a result of these mechanisms, teriflunomide inhibits actively dividing Th2 cells and decreases inflammatory responses.

Captopril is an angiotensin-converting enzyme (ACE) inhibitor, approved by the FDA for oral administration in the treatment of hypertension and certain types of heart failure and diabetic nephropathy. ACE converts angiotensin I (AngI) to angiotensin II (AngII) and causes blood vessel constriction, inhibits vasodilatation, and indirectly regulates intravascular fluid volumes by effects on the renin-angiotensin-system (RAS). Therefore, inhibition of ACE has been a mainstay therapy for hypertension. More recent studies have shown that AngII is also a key regulator of fibrosis in a variety of organ systems, including the kidney, liver, and lung. Langham et al., Diabetes Care 29:2670-2675 (2006); Alves de Albuquerque et al., Kidney Intl. 65:846-859 (2004); Osterreicher et al., Hepatology. 50:929- 938 (2009); Mak et al., Mol. Ther. 23: 1434-1443 (2015); Wang et al., Cell Physiol. Biochem. 36:697-711 (2015). The pro-fibrotic effects of AngII are mediated by a number of mechanisms, including production of reactive oxygen species, production of chemokines and cytokines, increased expression of adhesion molecules, and regulation of TGF-β expression/activity. In contrast, AngI has anti-proliferative and anti-fibrotic activities by activating its cell surface receptor. Mas. Clarke et al., Int. J. Hypertens. 2012:307315 (2011). As a result, inhibitors of ACE and/or AngII, such as captopril, losartan, and other similar medications, have been proposed as a potential therapeutic option for fibrotic disorders of the lung, kidney, and liver.

In one aspect, the present invention provides a pharmaceutical composition comprising deuterium enriched pirfenidone in combination with one or more anti-T cell, anti-TGF-β1, and/or anti-angiotensin agents and/or anti-inflammatory agents.

In some embodiments, the anti-T cell agent is selected from tacrolimus, teriflunomide, leflunomide, cyclosporine, pimecrolimus, denileukin diftitox, and basiliximab. In some embodiments, the anti-TGF-β1 agent or anti-angiotensin agent is selected from pirfenidone, deuterium-enriched pirfenidone, captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, and fimasartan. In some embodiments, the anti-angiotensin agent is an ACE agonist, for example, an ACE-2 agonist. The composition can be formulated for systemic administration or for local administration. In some embodiments, the composition is formulated for topical administration.

The pharmaceutical composition of the invention can comprise any combination of anti-T cell, anti-TGF-β1, and/or anti-angiotensin agents.

In some embodiments, the composition comprises deuterium-enriched pirfenidone, for example, a compound of Formula I, e.g., a compound listed in Table 1, including e.g., LYT-100, and one or more anti-T cell agent(s). In some embodiments, the composition comprises deuterium-enriched pirfenidone, for example, a compound of Formula I, e.g., a compound listed in Table 1, including e.g., LYT-100, and one or more anti-T cell agent(s) selected from tacrolimus, teriflunomide, leflunomide, cyclosporine, pimecrolimus, denileukin diftitox, and basiliximab. For instance, in some embodiments, the composition comprises deuterium-enriched pirfenidone, for example a compound of Formula I, e.g., a compound listed in Table 1, including e.g., LYT-100, and tacrolimus. In another example, in some embodiments, the composition comprises deuterium-enriched pirfenidone, for example, a compound of Formula I, e.g., a compound listed in Table 1, including e.g., LYT-100, and teriflunomide.

In some embodiments, the composition comprises deuterium-enriched pirfenidone, for example, a compound of Formula I, e.g., a compound listed in Table 1, including e.g., LYT-100, and one or more anti-TGF-β1 agent(s). Non-limiting examples of anti-TGF-β1 agent(s) include LY550410 and LY580276, SB-505124, or galunisertib (LY2157299 Monohydrate), or deuterium-enriched pirfenidone, e.g., as described herein, e.g., other than LYT-100. In some embodiments, the composition comprises deuterium-enriched pirfenidone, for example, a compound of Formula I, e.g., a compound listed in Table 1, including, e.g., LYT-100, and one or more anti-TGF-β1 agent(s) selected from LY550410 and LY580276, SB-505124, or galunisertib (LY2157299 Monohydrate) or deuterium-enriched pirfenidone, e.g., as described herein, e.g., other than LYT-100. In some embodiments, the composition comprises deuterium-enriched pirfenidone, for example, a compound of Formula I, e.g., a compound listed in Table 1, including, e.g., LYT-100, and a second type of deuterium-enriched pirfenidone, e.g., for example, a compound of Formula I, e.g., a compound listed in Table 1, e.g., other than LYT-100.

In some embodiments, the composition comprises deuterium-enriched pirfenidone, for example, a compound of Formula I, e.g., a compound listed in Table 1, including, e.g., LYT-100, and one or more anti-angiotensin agent(s). In some embodiments, the composition comprises deuterium-enriched pirfenidone, for example, a compound of Formula I, e.g., a compound listed in Table 1, including, e.g., LYT-100, and one or more anti-angiotensin agent(s) selected from captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099. For instance, in some embodiments, the composition comprises deuterium-enriched pirfenidone, for example, a compound of Formula I, e.g., a compound listed in Table 1, including, e.g., LYT-100, and captopril.

In some embodiments, the composition comprises deuterium-enriched pirfenidone, for example, a compound of Formula I, e.g., a compound listed in Table 1, including, e.g., LYT-100, and one or more anti-inflammatory agent(s). Non-limiting examples of anti-inflammatory agents include etodolac, famotidine, fenoprofen, fenoprofen, flurbiprofen, ibuprofen, indomethacin, ketoprofen, ketorolac, lansoprazole, mefenamic acid, meloxicam, misoprostol, nabumetone, naproxen, oxaprozin, piroxicam, sulindac, and tolmetin. Anti-inflammatory agents also include Cox-2 inhibitors, including but not limited to, celecoxib, apricoxib, robenacoxib, valdecoxib, anitrazafen, tilmacoxib, flumizole, cimicoxib, rofecoxib, mavacoxib, and firocoxib. In some embodiments, the composition comprises deuterium-enriched pirfenidone, for example, a compound of Formula I, e.g., a compound listed in Table 1, including, e.g., LYT-100, and one or more anti-inflammatory agent(s) selected from etodolac, famotidine, fenoprofen, fenoprofen, flurbiprofen, ibuprofen, indomethacin, ketoprofen, ketorolac, lansoprazole, mefenamic acid, meloxicam, misoprostol, nabumetone, naproxen, oxaprozin, piroxicam, sulindac, tolmetin, celecoxib, apricoxib, robenacoxib, valdecoxib, anitrazafen, tilmacoxib, flumizole, cimicoxib, rofecoxib, mavacoxib, and firocoxib. For instance, in some embodiments, the composition comprises deuterium-enriched pirfenidone, for example, a compound of Formula I, e.g., a compound listed in Table 1, including, e.g., LYT-100, and ibuprofen.

In some embodiments, the composition comprises deuterium-enriched pirfenidone, for example, a compound of Formula I, e.g., a compound listed in Table 1, including, e.g., LYT-100, one or more anti-T cell agent(s) and optionally one or more anti-angiotensin agent(s). In some embodiments, the composition comprises deuterium-enriched pirfenidone, for example, a compound of Formula I, e.g., a compound listed in Table 1, including, e.g., LYT-100, and one or more anti-T cell agent(s) selected from tacrolimus, teriflunomide, leflunomide, cyclosporine, pimecrolimus, denileukin diftitox, and basiliximab and optionally one or more anti-angiotensin agent(s). In some embodiments, the one or more anti-angiotensin agent(s) is selected from captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099. For instance, in some embodiments, the composition comprises deuterium-enriched pirfenidone, for example, a compound of Formula I, e.g., a compound listed in Table 1, including, e.g., LYT-100, and tacrolimus and one or more anti-angiotensin agent(s), e.g., captopril. In another example, in some embodiments, the composition comprises deuterium-enriched pirfenidone, for example, a compound of Formula I, e.g., a compound listed in Table 1, including, e.g., LYT-100, teriflunomide and one or more anti-angiotensin agent(s), e.g., captopril.

In some embodiments, the composition comprises deuterium-enriched pirfenidone, for example, a compound of Formula I, e.g., a compound listed in Table 1, including, e.g., LYT-100, one or more anti-T cell agent(s) and optionally one or more anti-inflammatory agent(s). In some embodiments, the composition comprises deuterium-enriched pirfenidone, for example, a compound of Formula I, e.g., a compound listed in Table 1, including, e.g., LYT-100, and one or more anti-T cell agent(s) selected from tacrolimus, teriflunomide, leflunomide, cyclosporine, pimecrolimus, denileukin diftitox, and basiliximab and one or more anti-inflammatory agent(s). In some embodiments, the one or more anti-inflammatory agent(s) is selected from etodolac, famotidine, fenoprofen, fenoprofen, flurbiprofen, ibuprofen, indomethacin, ketoprofen, ketorolac, lansoprazole, mefenamic acid, meloxicam, misoprostol, nabumetone, naproxen, oxaprozin, piroxicam, sulindac, tolmetin, celecoxib, apricoxib, robenacoxib, valdecoxib, anitrazafen, tilmacoxib, flumizole, cimicoxib, rofecoxib, mavacoxib, and firocoxib. For instance, in some embodiments, the composition comprises deuterium-enriched pirfenidone, for example, a compound of Formula I, e.g., a compound listed in Table 1, including, e.g., LYT-100, tacrolimus, and ibuprofen. In another example, in some embodiments, the composition comprises deuterium-enriched pirfenidone , for example, a compound of Formula I, e.g., a compound listed in Table 1, including, e.g., LYT-100, and teriflunomide, and ibuprofen.

In some embodiments, the composition comprises deuterium-enriched pirfenidone, for example, a compound of Formula I, e.g., a compound listed in Table 1, including, e.g., LYT-100, one or more anti-TGF-β1 agent(s) and optionally one or more anti-angiotensin agent(s). In some embodiments, the composition comprises deuterium-enriched pirfenidone, for example, a compound of Formula I, e.g., a compound listed in Table 1, including, e.g., LYT-100, and one or more anti-TGF-β1 agent(s) selected from LY550410, LY580276, SB-505124, galunisertib (LY2157299 Monohydrate) , and deuterium-enriched pirfenidone, e.g., as described herein, e.g., other than LYT-100, and optionally one or more anti-angiotensin agent(s). In some embodiments, the anti-angiotensin agent is selected from captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099. For instance, in some embodiments, the composition comprises deuterium-enriched pirfenidone, for example, a compound of Formula I, e.g., a compound listed in Table 1, including, e.g., LYT-100, and one or more anti-TGF-β1 agent(s), e.g., a second form of deuterated pirfenidone, e.g., other than LYT-100, and one or more anti-angiotensin agent(s), e.g., captopril.

In some embodiments, the composition comprises deuterium-enriched pirfenidone, for example, a compound of Formula I, e.g., a compound listed in Table 1, including, e.g., LYT-100, one or more anti-TGF-β1 agent(s) and optionally one or more anti-inflammatory agent(s). In some embodiments, the composition comprises deuterium-enriched pirfenidone, for example, a compound of Formula I, e.g., a compound listed in Table 1, including, e.g., LYT-100, and one or more anti-TGF-β1 agent(s) selected from LY550410, LY580276, SB-505124, (LY2157299 Monohydrate) , and deuterium-enriched pirfenidone, e.g., as described herein, e.g., other than LYT-100, and one or more anti-inflammatory agent(s). In some embodiments, the one or more anti-inflammatory agent(s) are selected from etodolac, famotidine, fenoprofen, fenoprofen, flurbiprofen, ibuprofen, indomethacin, ketoprofen, ketorolac, lansoprazole, mefenamic acid, meloxicam, misoprostol, nabumetone, naproxen, oxaprozin, piroxicam, sulindac, tolmetin, celecoxib, apricoxib, robenacoxib, valdecoxib, anitrazafen, tilmacoxib, flumizole, cimicoxib, rofecoxib, mavacoxib, and firocoxib. For instance, in some embodiments, the composition comprises deuterium-enriched pirfenidone, for example, a compound of Formula I, e.g., a compound listed in Table 1, including, for example, a compound of Formula I, e.g., a compound listed in Table 1, including, e.g., LYT-100, one or more anti-TGF-β1 agent(s), e.g., a deuterium enricher pirfendone described herein, e.g., other than LYT-100, and ibuprofen.

In some embodiments, the composition comprises deuterium-enriched pirfenidone, for example, a compound of Formula I, e.g., a compound listed in Table 1, including e.g., LYT-100, one or more anti-T cell agent(s) and optionally an anti-angiotensin agent. In some embodiments, the composition comprises deuterium-enriched pirfenidone, for example, a compound of Formula I, e.g., a compound listed in Table 1, including e.g., LYT-100, and one or more anti-T cell agent(s) selected from tacrolimus, teriflunomide, leflunomide, cyclosporine, pimecrolimus, denileukin diftitox, and basiliximab. In some embodiments, the anti-angiotensin agent is selected from captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099. For instance, in some embodiments, the composition comprises deuterium-enriched pirfenidone , for example,e.g., a compound of Formula I, e.g., a compound listed in Table 1, including e.g., LYT-100, tacrolimus and optionally captopril. In some embodiments, the composition comprises deuterium-enriched pirfenidone, for example, a compound of Formula I, e.g., a compound listed in Table 1, including e.g., LYT-100, teriflunomide and optionally captopril.

The aforementioned pharmaceutical compositions or combinations of two or more therapeutic agents may be used in treating a disease, disorder, or condition, such as those described below.

### Treatment of Diseases, Disorders, and Conditions with Compounds and Combinations of the Present Invention

In one aspect, the present invention relates to the use of a deuterium-enriched pirfenidone, for example, a compound of Formula I, e.g., a compound listed in Table 1, including for example, e.g., a compound of Formula I, e.g., a compound listed in Table 1, including, e.g., LYT-100, in combination with one or more agents selected from anti-TGF-β1 agent, an anti-T cell agent and/or anti-angiotensin agent to treat a disease, disorder, or condition such as edema, fibrotic disease, or an inflammatory disorder. In one aspect, the present disclosure relates to the use of deuterated pirfenidone ,e.g., a compound of Formula I, e.g., a compound listed in Table 1, including, e.g., LYT-100, in combination with an anti-inflammatory and/or anti-fibrotic therapeutic agent to treat a disease, disorder, or condition such as edema, fibrotic disease, or an inflammatory disorder.

I Provided herein are methods for the treatment, prevention, and/or amelioration of a fibrotic-mediated disorder and/or a collagen-mediated disorder comprising administering to a subject in need thereof a deuterium-enriched pirfenidone compound, for example, a compound of Formula I, e.g., a compound listed in Table 1, including e.g., LYT-100, in combination with one or more anti-T cell agent(s), anti-TGF-β1, anti-angiotensin agents, and/or anti-inflammatory agents. In one aspect, the present invention relates to methods for the treatment, prevention, and/or amelioration of edema, e.g., lymphedema comprising administering to a subject in need thereof a deuterium-enriched pirfenidone compound, for example, a compound of Formula I, e.g., a compound listed in Table 1, including e.g., LYT-100 in combination with one or more anti-T cell agent(s), anti-TGF-β1, anti-angiotensin agents, and/or anti-inflammatory agents. In one aspect, the present invention relates to methods for treatment, prevention, and/or amelioration of one or more symptoms of edema, e.g., lymphedema comprising administering to a subject in need thereof a deuterium-enriched pirfenidone compound, for example, a compound of Formula I, e.g., a compound listed in Table 1, including e.g., LYT-100 in combination with one or more anti-T cell agent(s), anti-TGF-β1, anti-angiotensin agents, and/or anti-inflammatory agents. In one aspect, the present invention relates to methods for treatment, prevention, and/or amelioration of Idiopathic Pulmonary Fibrosis (IPF) and/or one or more symptoms of IPF, comprising administering to a subject in need thereof a deuterium-enriched pirfenidone compound, for example, a compound of Formula I, e.g., a compound listed in Table 1, including e.g., LYT-100 in combination with one or more anti-T cell agent(s), anti-TGF-β1, and/or anti-angiotensin agents.

Provided herein are methods for the treatment, prevention, and/or amelioration of a fibrotic-mediated disorder and/or a collagen-mediated disorder comprising administering to a subject in need thereof a deuterium-enriched pirfenidone compound, for example, a compound of Formula I, e.g., a compound listed in Table 1, including e.g., LYT-100, in combination with one or more anti-T cell agent(s). In some embodiments, the methods for the treatment, prevention, and/or amelioration of a fibrotic-mediated disorder and/or a collagen-mediated disorder comprise administering to a subject in need thereof a deuterium-enriched pirfenidone, for example, a compound of Formula I, e.g., a compound listed in Table 1, including e.g., LYT-100, and one or more anti-T cell agent(s) selected from tacrolimus, teriflunomide, leflunomide, cyclosporine, pimecrolimus, denileukin diftitox, and basiliximab. For instance, in some embodiments, the methods for the treatment, prevention, and/or amelioration of a fibrotic-mediated disorder and/or a collagen-mediated disorder comprise administering to a subject in need thereof a deuterium-enriched pirfenidone, for example a compound of Formula I, e.g., a compound listed in Table 1, including e.g., LYT-100, and tacrolimus. In another example, in some embodiments, the methods for the treatment, prevention, and/or amelioration of a fibrotic-mediated disorder and/or a collagen-mediated disorder comprise administering to a subject in need thereof a deuterium-enriched pirfenidone, for example, a compound of Formula I, e.g., a compound listed in Table 1, including e.g., LYT-100, and teriflunomide..

Provided herein are methods for the treatment, prevention, and/or amelioration of edema, e.g., lymphedema comprising administering to a subject in need thereof a deuterium-enriched pirfenidone compound, for example, a compound of Formula I, e.g., a compound listed in Table 1, including e.g., LYT-100 in combination with one or more anti-T cell agent(s). In some embodiments, the methods for the treatment, prevention, and/or amelioration of edema, e.g., lymphedema comprise administering to a subject in need thereof deuterium-enriched pirfenidone, for example, a compound of Formula I, e.g., a compound listed in Table 1, including e.g., LYT-100, and one or more anti-T cell agent(s) selected from tacrolimus, teriflunomide, leflunomide, cyclosporine, pimecrolimus, denileukin diftitox, and basiliximab. For instance, in some embodiments, the methods for the treatment, prevention, and/or amelioration of edema, e.g., lymphedema comprise administering to a subject in need thereof deuterium-enriched pirfenidone, for example a compound of Formula I, e.g., a compound listed in Table 1, including e.g., LYT-100, and tacrolimus. In another example, in some embodiments, the methods for the treatment, prevention, and/or amelioration of edema, e.g., lymphedema comprise administering to a subject in need thereof deuterium-enriched pirfenidone, for example, a compound of Formula I, e.g., a compound listed in Table 1, including e.g., LYT-100, and teriflunomide.

Provided herein are methods for treatment, prevention, and/or amelioration of one or more symptoms of edema, e.g., lymphedema comprising administering to a subject in need thereof a deuterium-enriched pirfenidone compound, for example, a compound of Formula I, e.g., a compound listed in Table 1, including e.g., LYT-100 in combination with one or more anti-T cell agent(s). In some embodiments, the methods for treatment, prevention, and/or amelioration of one or more symptoms of edema, e.g., lymphedema comprise administering to a subject in need thereof a deuterium-enriched pirfenidone, for example, a compound of Formula I, e.g., a compound listed in Table 1, including e.g., LYT-100, and one or more anti-T cell agent(s) selected from tacrolimus, teriflunomide, leflunomide, cyclosporine, pimecrolimus, denileukin diftitox, and basiliximab. For instance, in some embodiments, the methods for treatment, prevention, and/or amelioration of one or more symptoms of edema, e.g., lymphedema comprise administering to a subject in need thereof a deuterium-enriched pirfenidone, for example a compound of Formula I, e.g., a compound listed in Table 1, including e.g., LYT-100, and tacrolimus. In another example, in some embodiments, the methods for treatment, prevention, and/or amelioration of one or more symptoms of edema, e.g., lymphedema comprise administering to a subject in need thereof a deuterium-enriched pirfenidone, for example, a compound of Formula I, e.g., a compound listed in Table 1, including e.g., LYT-100, and teriflunomide.

Provided herein are methods for treatment, prevention, and/or amelioration of Idiopathic Pulmonary Fibrosis (IPF) and/or one or more symptoms of IPF, comprising administering to a subject in need thereof a deuterium-enriched pirfenidone compound, for example, a compound of Formula I, e.g., a compound listed in Table 1, including e.g., LYT-100 in combination with one or more anti-T cell agent(s). In some embodiments, the methods for treatment, prevention, and/or amelioration of Idiopathic IPF and/or one or more symptoms of IPF, comprise administering to a subject in need thereof a deuterium-enriched pirfenidone, for example, a compound of Formula I, e.g., a compound listed in Table 1, including e.g., LYT-100, and one or more anti-T cell agent(s) selected from tacrolimus, teriflunomide, leflunomide, cyclosporine, pimecrolimus, denileukin diftitox, and basiliximab. For instance, in some embodiments, the methods for treatment, prevention, and/or amelioration of IPF and/or one or more symptoms of IPF comprise administering to a subject in need thereof a deuterium-enriched pirfenidone, for example a compound of Formula I, e.g., a compound listed in Table 1, including e.g., LYT-100, and tacrolimus. In another example, in some embodiments, the methods for treatment, prevention, and/or amelioration of IPF and/or one or more symptoms of IPF comprise administering to a subject in need thereof a deuterium-enriched pirfenidone, for example, a compound of Formula I, e.g., a compound listed in Table 1, including e.g., LYT-100, and teriflunomide.

Provided herein are methods for the treatment, prevention, and/or amelioration of a fibrotic-mediated disorder and/or a collagen-mediated disorder comprising administering to a subject in need thereof a deuterium-enriched pirfenidone compound, for example, a compound of Formula I, e.g., a compound listed in Table 1, including e.g., LYT-100 in combination with one or more anti-TGF-β1 agent(s). In some embodiments, the methods for the treatment, prevention, and/or amelioration of a fibrotic-mediated disorder and/or a collagen-mediated disorder comprise administering to a subject in need thereof a deuterium-enriched pirfenidone, for example, a compound of Formula I, e.g., a compound listed in Table 1, including e.g., LYT-100, and one or more anti-TGF-β1 agent(s) selected from LY550410 and LY580276, SB-505124, or galunisertib (LY2157299 Monohydrate) or deuterium-enriched pirfenidone, e.g., as described herein, for example, a compound of Formula I, e.g., a compound listed in Table 1, e.g., other than LYT-100. In some embodiments, the methods for the treatment, prevention, and/or amelioration of a fibrotic-mediated disorder and/or a collagen-mediated disorder comprise administering to a subject in need thereof a deuterium-enriched pirfenidone, for example, a compound of Formula I, e.g., a compound listed in Table 1, including e.g., LYT-100, and a second type of deuterium-enriched pirfenidone, e.g., for example, a compound of Formula I, e.g., a compound listed in Table 1, e.g., other than LYT-100.

Provided herein are methods for the treatment, prevention, and/or amelioration of edema, e.g., lymphedema comprising administering to a subject in need thereof a deuterium-enriched pirfenidone compound, for example, a compound of Formula I, e.g., a compound listed in Table 1, including e.g., LYT-100, in combination with one or more anti-TGF-β1 agent(s). In some embodiments, the methods for the treatment, prevention, and/or amelioration of edema, e.g., lymphedema comprise administering to a subject in need thereof deuterium-enriched pirfenidone, for example, a compound of Formula I, e.g., a compound listed in Table 1, including e.g., LYT-100, and one or more anti-TGF-β1 agent(s) selected from LY550410 and LY580276, SB-505124, galunisertib (LY2157299 Monohydrate), and deuterium-enriched pirfenidone, e.g., as described herein, e.g., other than LYT-100. In some embodiments, the methods for the treatment, prevention, and/or amelioration of edema, e.g., lymphedema comprise administering to a subject in need thereof a deuterium-enriched pirfenidone, for example, a compound of Formula I, e.g., a compound listed in Table 1, including e.g., LYT-100, and a second type of deuterium-enriched pirfenidone, e.g., for example, a compound of Formula I, e.g., a compound listed in Table 1, e.g., other than LYT-100.

Provided herein are methods for treatment, prevention, and/or amelioration of one or more symptoms of edema, e.g., lymphedema comprising administering to a subject in need thereof a deuterium-enriched pirfenidone compound, for example, a compound of Formula I, e.g., a compound listed in Table 1, including e.g., LYT-100 in combination with one or more anti-TGF-β1 agent(s). In some embodiments, the methods for treatment, prevention, and/or amelioration of one or more symptoms of edema, e.g., lymphedema comprise administering to a subject in need thereof a deuterium-enriched pirfenidone, for example, a compound of Formula I, e.g., a compound listed in Table 1, including e.g., LYT-100, and one or more anti-TGF-β1 agent(s) selected from LY550410 and LY580276, SB-505124, or galunisertib (LY2157299 Monohydrate) or deuterium-enriched pirfenidone, e.g., as described herein, other than LYT-100. In some embodiments, the methods for treatment, prevention, and/or amelioration of one or more symptoms of edema, e.g., lymphedema comprise administering to a subject in need thereof a deuterium-enriched pirfenidone, for example, a compound of Formula I, e.g., a compound listed in Table 1, including e.g., LYT-100, and a second type of deuterium-enriched pirfenidone, e.g., for example, a compound of Formula I, e.g., a compound listed in Table 1, e.g., other than LYT-100.

Provided herein are methods for treatment, prevention, and/or amelioration of IPF and/or one or more symptoms of IPF, comprising administering to a subject in need thereof a deuterium-enriched pirfenidone compound, for example, a compound of Formula I, e.g., a compound listed in Table 1, including e.g., LYT-100 in combination with one or more anti-TGF-β1 agent(s). In some embodiments, the methods for treatment, prevention, and/or amelioration of IPF and/or one or more symptoms of IPF, comprise administering to a subject in need thereof a deuterium-enriched pirfenidone, for example, a compound of Formula I, e.g., a compound listed in Table 1, including e.g., LYT-100, and one or more anti-TGF-β1 agent(s) selected from LY550410 and LY580276, SB-505124, or galunisertib (LY2157299 Monohydrate) or deuterium-enriched pirfenidone, e.g., as described herein, e.g., other than LYT-100. In some embodiments, the methods for treatment, prevention, and/or amelioration of IPF and/or one or more symptoms of IPF, comprise administering to a subject in need thereof a deuterium-enriched pirfenidone, for example, a compound of Formula I, e.g., a compound listed in Table 1, including e.g., LYT-100, and a second type of deuterium-enriched pirfenidone, e.g., for example, a compound of Formula I, e.g., a compound listed in Table 1, e.g., other than LYT-100.

Provided herein are methods for the treatment, prevention, and/or amelioration of a fibrotic-mediated disorder and/or a collagen-mediated disorder comprising administering to a subject in need thereof a deuterium-enriched pirfenidone compound, for example, a compound of Formula I, e.g., a compound listed in Table 1, including e.g., LYT-100 in combination with an anti-angiotensin agent. In some embodiments, the methods for the treatment, prevention, and/or amelioration of a fibrotic-mediated disorder and/or a collagen-mediated disorder comprise administering to a subject in need thereof a deuterium-enriched pirfenidone, for example, a compound of Formula I, e.g., a compound listed in Table 1, including e.g., LYT-100, and one or more anti-angiotensin agent(s) selected from captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099. For instance, in some embodiments, the methods for the treatment, prevention, and/or amelioration of a fibrotic-mediated disorder and/or a collagen-mediated disorder comprise administering to a subject in need thereof a deuterium-enriched pirfenidone, for example, a compound of Formula I, e.g., a compound listed in Table 1, including, e.g., LYT-100, and captopril.

Provided herein are methods for the treatment, prevention, and/or amelioration of edema, e.g., lymphedema comprising administering to a subject in need thereof a deuterium-enriched pirfenidone compound, for example, a compound of Formula I, e.g., a compound listed in Table 1, including, e.g., LYT-100 in combination with an anti-angiotensin agent. In some embodiments, the methods for the treatment, prevention, and/or amelioration of edema, e.g., lymphedema comprise administering to a subject in need thereof a deuterium-enriched pirfenidone, for example, a compound of Formula I, e.g., a compound listed in Table 1, including, e.g., LYT-100, and one or more anti-angiotensin agent(s) selected from captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099. For instance, in some embodiments, the methods for the treatment, prevention, and/or amelioration of edema, e.g., lymphedema comprise administering to a subject in need thereof a deuterium-enriched pirfenidone, for example, a compound of Formula I, e.g., a compound listed in Table 1, including, e.g., LYT-100, and captopril.

Provided herein are methods for treatment, prevention, and/or amelioration of one or more symptoms of edema, e.g., lymphedema comprising administering to a subject in need thereof a deuterium-enriched pirfenidone compound, for example, a compound of Formula I, e.g., a compound listed in Table 1, including, e.g., LYT-100, in combination with an anti-angiotensin agent. In some embodiments, the methods for treatment, prevention, and/or amelioration of one or more symptoms of edema, e.g., lymphedema comprise administering to a subject in need thereof a deuterium-enriched pirfenidone, for example, a compound of Formula I, e.g., a compound listed in Table 1, including e.g., LYT-100, and one or more anti-angiotensin agent(s) selected from captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099. For instance, in some embodiments, the methods for treatment, prevention, and/or amelioration of one or more symptoms of edema, e.g., lymphedema comprise administering to a subject in need thereof a deuterium-enriched pirfenidone, for example, a compound of Formula I, e.g., a compound listed in Table 1, including e.g., LYT-100, and captopril.

Provided herein are methods for treatment, prevention, and/or amelioration of IPF and/or one or more symptoms of IPF, comprising administering to a subject in need thereof a deuterium-enriched pirfenidone compound, for example, a compound of Formula I, e.g., a compound listed in Table 1, including, e.g., LYT-100, in combination with an anti-angiotensin agent. In some embodiments, the methods for treatment, prevention, and/or amelioration of IPF and/or one or more symptoms of IPF, comprise administering to a subject in need thereof a deuterium-enriched pirfenidone, for example, a compound of Formula I, e.g., a compound listed in Table 1, including e.g., LYT-100, and one or more anti-angiotensin agent(s) selected from captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099. For instance, in some embodiments, the methods for treatment, prevention, and/or amelioration of IPF and/or one or more symptoms of IPF, comprise administering to a subject in need thereof a deuterium-enriched pirfenidone, for example, a compound of Formula I, e.g., a compound listed in Table 1, including e.g., LYT-100, and captopril.

Provided herein are methods for the treatment, prevention, and/or amelioration of a fibrotic-mediated disorder and/or a collagen-mediated disorder comprising administering to a subject in need thereof a deuterium-enriched pirfenidone compound, for example, a compound of Formula I, e.g., a compound listed in Table 1, including, e.g., LYT-100, in combination with one or more anti-inflammatory agent(s). In some embodiments, the methods for the treatment, prevention, and/or amelioration of a fibrotic-mediated disorder and/or a collagen-mediated disorder comprise administering to a subject in need thereof a deuterium-enriched pirfenidone, for example, a compound of Formula I, e.g., a compound listed in Table 1, including e.g., LYT-100, and one or more anti-inflammatory agent(s) selected from etodolac, famotidine, fenoprofen, fenoprofen, flurbiprofen, ibuprofen, indomethacin, ketoprofen, ketorolac, lansoprazole, mefenamic acid, meloxicam, misoprostol, nabumetone, naproxen, oxaprozin, piroxicam, sulindac, tolmetin, celecoxib, apricoxib, robenacoxib, valdecoxib, anitrazafen, tilmacoxib, flumizole, cimicoxib, rofecoxib, mavacoxib, and firocoxib. For instance, in some embodiments, the methods for the treatment, prevention, and/or amelioration of a fibrotic-mediated disorder and/or a collagen-mediated disorder comprise administering to a subject in need thereof a deuterium-enriched pirfenidone, for example, a compound of Formula I, e.g., a compound listed in Table 1, including e.g., LYT-100, and ibuprofen.

Provided herein are methods for the treatment, prevention, and/or amelioration of edema, e.g., lymphedema comprising administering to a subject in need thereof a deuterium-enriched pirfenidone compound, for example, a compound of Formula I, e.g., a compound listed in Table 1, including, e.g., LYT-100, in combination with one or more anti-inflammatory agent(s). In some embodiments, the methods for the treatment, prevention, and/or amelioration of edema, e.g., lymphedema comprise administering to a subject in need thereof a deuterium-enriched pirfenidone, for example, a compound of Formula I, e.g., a compound listed in Table 1, including e.g., LYT-100, and one or more anti-inflammatory agent(s) selected from selected from etodolac, famotidine, fenoprofen, fenoprofen, flurbiprofen, ibuprofen, indomethacin, ketoprofen, ketorolac, lansoprazole, mefenamic acid, meloxicam, misoprostol, nabumetone, naproxen, oxaprozin, piroxicam, sulindac, tolmetin, celecoxib, apricoxib, robenacoxib, valdecoxib, anitrazafen, tilmacoxib, flumizole, cimicoxib, rofecoxib, mavacoxib, and firocoxib. For instance, in some embodiments, the methods for the treatment, prevention, and/or amelioration of edema, e.g., lymphedema comprise administering to a subject in need thereof a deuterium-enriched pirfenidone, for example, a compound of Formula I, e.g., a compound listed in Table 1, including e.g., LYT-100, and ibuprofen.

Provided herein are methods for treatment, prevention, and/or amelioration of one or more symptoms of edema, e.g., lymphedema comprising administering to a subject in need thereof a deuterium-enriched pirfenidone compound, for example, a compound of Formula I, e.g., a compound listed in Table 1, including, e.g., LYT-100, in combination with one or more anti-inflammatory agent(s). In some embodiments, the methods for treatment, prevention, and/or amelioration of one or more symptoms of edema, e.g., lymphedema comprise administering to a subject in need thereof a deuterium-enriched pirfenidone, for example, a compound of Formula I, e.g., a compound listed in Table 1, including e.g., LYT-100, and one or more anti-inflammatory agent(s) selected from selected from etodolac, famotidine, fenoprofen, fenoprofen, flurbiprofen, ibuprofen, indomethacin, ketoprofen, ketorolac, lansoprazole, mefenamic acid, meloxicam, misoprostol, nabumetone, naproxen, oxaprozin, piroxicam, sulindac, tolmetin, celecoxib, apricoxib, robenacoxib, valdecoxib, anitrazafen, tilmacoxib, flumizole, cimicoxib, rofecoxib, mavacoxib, and firocoxib. For instance, in some embodiments, the methods for treatment, prevention, and/or amelioration of one or more symptoms of edema, e.g., lymphedema comprise administering to a subject in need thereof a deuterium-enriched pirfenidone, for example, a compound of Formula I, e.g., a compound listed in Table 1, including e.g., LYT-100, and ibuprofen.

Provided herein are methods for treatment, prevention, and/or amelioration of IPF and/or one or more symptoms of IPF, comprising administering to a subject in need thereof a deuterium-enriched pirfenidone compound, for example, a compound of Formula I, e.g., a compound listed in Table 1, including, e.g., LYT-100, in combination with one or more anti-inflammatory agent(s). In some embodiments, the methods for treatment, prevention, and/or amelioration of IPF and/or one or more symptoms of IPF, comprise administering to a subject in need thereof a deuterium-enriched pirfenidone, for example, a compound of Formula I, e.g., a compound listed in Table 1, including e.g., LYT-100, and one or more anti-inflammatory agent(s) selected from selected from etodolac, famotidine, fenoprofen, fenoprofen, flurbiprofen, ibuprofen, indomethacin, ketoprofen, ketorolac, lansoprazole, mefenamic acid, meloxicam, misoprostol, nabumetone, naproxen, oxaprozin, piroxicam, sulindac, tolmetin, celecoxib, apricoxib, robenacoxib, valdecoxib, anitrazafen, tilmacoxib, flumizole, cimicoxib, rofecoxib, mavacoxib, and firocoxib. For instance, in some embodiments, the methods for treatment, prevention, and/or amelioration of IPF and/or one or more symptoms of IPF, comprise administering to a subject in need thereof a deuterium-enriched pirfenidone, for example, a compound of Formula I, e.g., a compound listed in Table 1, including e.g., LYT-100, and ibuprofen.

Provided herein are methods for the treatment, prevention, and/or amelioration of a fibrotic-mediated disorder and/or a collagen-mediated disorder comprising administering to a subject in need thereof a deuterium-enriched pirfenidone compound, for example, a compound of Formula I, e.g., a compound listed in Table 1, including, e.g., LYT-100 in combination with one or more anti-T cell agent(s) and optionally one or more anti-angiotensin agent(s). In some embodiments, the methods for the treatment, prevention, and/or amelioration of a fibrotic-mediated disorder and/or a collagen-mediated disorder comprise administering to a subject in need thereof a deuterium-enriched pirfenidone, for example, a compound of Formula I, e.g., a compound listed in Table 1, including e.g., LYT-100, and one or more anti-T cell agent(s) selected from tacrolimus, teriflunomide, leflunomide, cyclosporine, pimecrolimus, denileukin diftitox, and basiliximab and optionally an anti-angiotensin agent. In some embodiments, the one or more anti-angiotensin agent(s) is selected from captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099. For instance, in some embodiments, the methods for the treatment, prevention, and/or amelioration of a fibrotic-mediated disorder and/or a collagen-mediated disorder comprise administering to a subject in need thereof a deuterium-enriched pirfenidone, for example, a compound of Formula I, e.g., a compound listed in Table 1, including e.g., LYT-100, and tacrolimus and an anti-angiotensin agent, e.g., captopril. In another example, in some embodiments, the methods for the treatment, prevention, and/or amelioration of a fibrotic-mediated disorder and/or a collagen-mediated disorder comprise administering to a subject in need thereof a deuterium-enriched pirfenidone, for example, a compound of Formula I, e.g., a compound listed in Table 1, including e.g., LYT-100, teriflunomide and an anti-angiotensin agent, e.g., captopril.

Provided herein are methods for the treatment, prevention, and/or amelioration of edema, e.g., lymphedema comprising administering to a subject in need thereof a deuterium-enriched pirfenidone compound, for example, a compound of Formula I, e.g., a compound listed in Table 1, including, e.g., LYT-100 in combination with one or more anti-T cell agent(s) and optionally one or more anti-angiotensin agent(s). In some embodiments, the methods for the treatment, prevention, and/or amelioration of edema, e.g., lymphedema comprise administering to a subject in need thereof a deuterium-enriched pirfenidone, for example, a compound of Formula I, e.g., a compound listed in Table 1, including e.g., LYT-100, and one or more anti-T cell agent(s) selected from tacrolimus, teriflunomide, leflunomide, cyclosporine, pimecrolimus, denileukin diftitox, and basiliximab and optionally an anti-angiotensin agent. In some embodiments, the one or more anti-angiotensin agent(s) is selected from captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099. For instance, in some embodiments, the methods for the treatment, prevention, and/or amelioration of edema, e.g., lymphedema comprise administering to a subject in need thereof a deuterium-enriched pirfenidone, for example, a compound of Formula I, e.g., a compound listed in Table 1, including e.g., LYT-100, and tacrolimus and an anti-angiotensin agent, e.g., captopril. In another example, in some embodiments, the methods for the treatment, prevention, and/or amelioration of edema, e.g., lymphedema comprise administering to a subject in need thereof a deuterium-enriched pirfenidone, for example, a compound of Formula I, e.g., a compound listed in Table 1, including e.g., LYT-100, teriflunomide and an anti-angiotensin agent, e.g., captopril.

Provided herein are methods for treatment, prevention, and/or amelioration of one or more symptoms of edema, e.g., lymphedema comprising administering to a subject in need thereof a deuterium-enriched pirfenidone compound, for example, a compound of Formula I, e.g., a compound listed in Table 1, including, e.g., LYT-100 in combination with one or more anti-T cell agent(s) and optionally one or more anti-angiotensin agent(s). In some embodiments, the methods for treatment, prevention, and/or amelioration of one or more symptoms of edema, e.g., lymphedema comprise administering to a subject in need thereof a deuterium-enriched pirfenidone, for example, a compound of Formula I, e.g., a compound listed in Table 1, including e.g., LYT-100, and one or more anti-T cell agent(s) selected from tacrolimus, teriflunomide, leflunomide, cyclosporine, pimecrolimus, denileukin diftitox, and basiliximab and optionally an anti-angiotensin agent. In some embodiments, the one or more anti-angiotensin agent(s) is selected from captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099. For instance, in some embodiments, the methods for treatment, prevention, and/or amelioration of one or more symptoms of edema, e.g., lymphedema comprise administering to a subject in need thereof a deuterium-enriched pirfenidone, for example, a compound of Formula I, e.g., a compound listed in Table 1, including e.g., LYT-100, and tacrolimus and an anti-angiotensin agent, e.g., captopril. In another example, in some embodiments, the methods for treatment, prevention, and/or amelioration of one or more symptoms of edema, e.g., lymphedema comprise administering to a subject in need thereof a deuterium-enriched pirfenidone, for example, a compound of Formula I, e.g., a compound listed in Table 1, including e.g., LYT-100, teriflunomide and an anti-angiotensin agent, e.g., captopril.

Provided herein are methods for treatment, prevention, and/or amelioration of IPF and/or one or more symptoms of IPF comprising administering to a subject in need thereof a deuterium-enriched pirfenidone compound, for example, a compound of Formula I, e.g., a compound listed in Table 1, including, e.g., LYT-100 in combination with one or more anti-T cell agent(s) and optionally one or more anti-angiotensin agent(s). In some embodiments, the methods for treatment, prevention, and/or amelioration of IPF and/or one or more symptoms of IPF comprise administering to a subject in need thereof a deuterium-enriched pirfenidone, for example, a compound of Formula I, e.g., a compound listed in Table 1, including e.g., LYT-100, and one or more anti-T cell agent(s) selected from tacrolimus, teriflunomide, leflunomide, cyclosporine, pimecrolimus, denileukin diftitox, and basiliximab and optionally an anti-angiotensin agent. In some embodiments, the one or more anti-angiotensin agent(s) is selected from captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099. For instance, in some embodiments, the methods for treatment, prevention, and/or amelioration of IPF and/or one or more symptoms of IPF comprise administering to a subject in need thereof a deuterium-enriched pirfenidone, for example, a compound of Formula I, e.g., a compound listed in Table 1, including e.g., LYT-100, and tacrolimus and an anti-angiotensin agent, e.g., captopril. In another example, in some embodiments, the methods for treatment, prevention, and/or amelioration of IPF and/or one or more symptoms of IPF comprise administering to a subject in need thereof a deuterium-enriched pirfenidone, for example, a compound of Formula I, e.g., a compound listed in Table 1, including e.g., LYT-100, teriflunomide and an anti-angiotensin agent, e.g., captopril.

Provided herein are methods for the treatment, prevention, and/or amelioration of a fibrotic-mediated disorder and/or a collagen-mediated disorder comprising administering to a subject in need thereof a deuterium-enriched pirfenidone compound, for example, a compound of Formula I, e.g., a compound listed in Table 1, including, e.g., LYT-100, in combination with one or more anti-T cell agent(s) and optionally one or more anti-inflammatory agent(s). In some embodiments, the methods for the treatment, prevention, and/or amelioration of a fibrotic-mediated disorder and/or a collagen-mediated disorder comprise administering to a subject in need thereof a deuterium-enriched pirfenidone, for example, a compound of Formula I, e.g., a compound listed in Table 1, including e.g., LYT-100, and one or more anti-T cell agent(s) selected from tacrolimus, teriflunomide, leflunomide, cyclosporine, pimecrolimus, denileukin diftitox, and basiliximab and one or more anti-inflammatory agent(s). In some embodiments, the one or more anti-inflammatory agent(s) is selected from etodolac, famotidine, fenoprofen, fenoprofen, flurbiprofen, ibuprofen, indomethacin, ketoprofen, ketorolac, lansoprazole, mefenamic acid, meloxicam, misoprostol, nabumetone, naproxen, oxaprozin, piroxicam, sulindac, tolmetin, celecoxib, apricoxib, robenacoxib, valdecoxib, anitrazafen, tilmacoxib, flumizole, cimicoxib, rofecoxib, mavacoxib, and firocoxib. For instance, in some embodiments, the methods for the treatment, prevention, and/or amelioration of a fibrotic-mediated disorder and/or a collagen-mediated disorder comprise administering to a subject in need thereof a deuterium-enriched pirfenidone , for example, a compound of Formula I, e.g., a compound listed in Table 1, including e.g., LYT-100, tacrolimus, and ibuprofen. In another example, in some embodiments, the methods for the treatment, prevention, and/or amelioration of a fibrotic-mediated disorder and/or a collagen-mediated disorder comprise administering to a subject in need thereof a deuterium-enriched pirfenidone , for example, a compound of Formula I, e.g., a compound listed in Table 1, including e.g., LYT-100, and teriflunomide, and ibuprofen.

Provided herein are methods for the treatment, prevention, and/or amelioration of edema, e.g., lymphedema comprising administering to a subject in need thereof a deuterium-enriched pirfenidone compound, for example, a compound of Formula I, e.g., a compound listed in Table 1, including, e.g., LYT-100, in combination with one or more anti-T cell agent(s) and optionally one or more anti-inflammatory agent(s). In some embodiments, the methods for the treatment, prevention, and/or amelioration of edema, e.g., lymphedema comprise administering to a subject in need thereof a deuterium-enriched pirfenidone, for example, a compound of Formula I, e.g., a compound listed in Table 1, including e.g., LYT-100, and one or more anti-T cell agent(s) selected from tacrolimus, teriflunomide, leflunomide, cyclosporine, pimecrolimus, denileukin diftitox, and basiliximab and one or more anti-inflammatory agent(s). In some embodiments, the one or more anti-inflammatory agent(s) is selected from etodolac, famotidine, fenoprofen, fenoprofen, flurbiprofen, ibuprofen, indomethacin, ketoprofen, ketorolac, lansoprazole, mefenamic acid, meloxicam, misoprostol, nabumetone, naproxen, oxaprozin, piroxicam, sulindac, tolmetin, celecoxib, apricoxib, robenacoxib, valdecoxib, anitrazafen, tilmacoxib, flumizole, cimicoxib, rofecoxib, mavacoxib, and firocoxib. For instance, in some embodiments, the methods for the treatment, prevention, and/or amelioration of edema, e.g., lymphedema comprise administering to a subject in need thereof a deuterium-enriched pirfenidone , for example, a compound of Formula I, e.g., a compound listed in Table 1, including e.g., LYT-100, tacrolimus, and ibuprofen. In another example, in some embodiments, the methods for the treatment, prevention, and/or amelioration of edema, e.g., lymphedema comprise administering to a subject in need thereof a deuterium-enriched pirfenidone , for example, a compound of Formula I, e.g., a compound listed in Table 1, including e.g., LYT-100, and teriflunomide, and ibuprofen.

Provided herein are methods for treatment, prevention, and/or amelioration of one or more symptoms of edema, e.g., lymphedema comprising administering to a subject in need thereof a deuterium-enriched pirfenidone compound, for example, a compound of Formula I, e.g., a compound listed in Table 1, including, e.g., LYT-100, in combination with one or more anti-T cell agent(s) and optionally one or more anti-inflammatory agent(s). In some embodiments, the methods for treatment, prevention, and/or amelioration of one or more symptoms of edema, e.g., lymphedema comprise administering to a subject in need thereof a deuterium-enriched pirfenidone, for example, a compound of Formula I, e.g., a compound listed in Table 1, including e.g., LYT-100, and one or more anti-T cell agent(s) selected from tacrolimus, teriflunomide, leflunomide, cyclosporine, pimecrolimus, denileukin diftitox, and basiliximab and one or more anti-inflammatory agent(s). In some embodiments, the one or more anti-inflammatory agent(s) is selected from etodolac, famotidine, fenoprofen, fenoprofen, flurbiprofen, ibuprofen, indomethacin, ketoprofen, ketorolac, lansoprazole, mefenamic acid, meloxicam, misoprostol, nabumetone, naproxen, oxaprozin, piroxicam, sulindac, tolmetin, celecoxib, apricoxib, robenacoxib, valdecoxib, anitrazafen, tilmacoxib, flumizole, cimicoxib, rofecoxib, mavacoxib, and firocoxib. For instance, in some embodiments, the methods for treatment, prevention, and/or amelioration of one or more symptoms of edema, e.g., lymphedema compriss administering to a subject in need thereof a deuterium-enriched pirfenidone , for example, a compound of Formula I, e.g., a compound listed in Table 1, including e.g., LYT-100, tacrolimus, and ibuprofen. In another example, in some embodiments, the methods for treatment, prevention, and/or amelioration of one or more symptoms of edema, e.g., lymphedema comprise administering to a subject in need thereof a deuterium-enriched pirfenidone, for example, a compound of Formula I, e.g., a compound listed in Table 1, including e.g., LYT-100, and teriflunomide, and ibuprofen.

Provided herein are methods for treatment, prevention, and/or amelioration of IPF and/or one or more symptoms of IPF comprising administering to a subject in need thereof a deuterium-enriched pirfenidone compound, for example, a compound of Formula I, e.g., a compound listed in Table 1, including, e.g., LYT-100, in combination with one or more anti-T cell agent(s) and optionally one or more anti-inflammatory agent(s). In some embodiments, the methods for treatment, prevention, and/or amelioration of IPF and/or one or more symptoms of IPF comprise administering to a subject in need thereof a deuterium-enriched pirfenidone, for example, a compound of Formula I, e.g., a compound listed in Table 1, including e.g., LYT-100, and one or more anti-T cell agent(s) selected from tacrolimus, teriflunomide, leflunomide, cyclosporine, pimecrolimus, denileukin diftitox, and basiliximab and one or more anti-inflammatory agent(s). In some embodiments, the one or more anti-inflammatory agent(s) is selected from etodolac, famotidine, fenoprofen, fenoprofen, flurbiprofen, ibuprofen, indomethacin, ketoprofen, ketorolac, lansoprazole, mefenamic acid, meloxicam, misoprostol, nabumetone, naproxen, oxaprozin, piroxicam, sulindac, tolmetin, celecoxib, apricoxib, robenacoxib, valdecoxib, anitrazafen, tilmacoxib, flumizole, cimicoxib, rofecoxib, mavacoxib, and firocoxib. For instance, in some embodiments, the methods for treatment, prevention, and/or amelioration of IPF and/or one or more symptoms of IPF comprise administering to a subject in need thereof a deuterium-enriched pirfenidone , for example, a compound of Formula I, e.g., a compound listed in Table 1, including e.g., LYT-100, tacrolimus, and ibuprofen. In another example, in some embodiments, the methods for treatment, prevention, and/or amelioration of IPF and/or one or more symptoms of IPF comprise administering to a subject in need thereof a deuterium-enriched pirfenidone , for example, a compound of Formula I, e.g., a compound listed in Table 1, including e.g., LYT-100, and teriflunomide, and ibuprofen.

Provided herein are methods for the treatment, prevention, and/or amelioration of a fibrotic-mediated disorder and/or a collagen-mediated disorder comprising administering to a subject in need thereof a deuterium-enriched pirfenidone compound, for example, a compound of Formula I, e.g., a compound listed in Table 1, including, e.g., LYT-100, in combination with one or more anti-TGF-β1 agent(s) and optionally an anti-angiotensin agent. In some embodiments, the methods for the treatment, prevention, and/or amelioration of a fibrotic-mediated disorder and/or a collagen-mediated disorder comprise administering to a subject in need thereof a deuterium-enriched pirfenidone, for example, a compound of Formula I, e.g., a compound listed in Table 1, including e.g., LYT-100, and one or more anti-TGF-β1 agent(s) selected from LY550410, LY580276, SB-505124, galunisertib (LY2157299 Monohydrate) , and deuterium-enriched pirfenidone, e.g., as described herein, other than LYT-100, and optionally an anti-angiotensin agent. In some embodiments, the anti-angiotensin agent is selected from captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099. For instance, in some embodiments, the methods for the treatment, prevention, and/or amelioration of a fibrotic-mediated disorder and/or a collagen-mediated disorder comprise administering to a subject in need thereof a deuterium-enriched pirfenidone, for example,e.g., a compound of Formula I, e.g., a compound listed in Table 1, including e.g., LYT-100, and one or more anti-TGF-β1 agent(s), e.g., a second form of deuterated pirfenidone, e.g., other than LYT-100, and an anti-angiotensin agent, e.g., captopril.

Provided herein are methods for the treatment, prevention, and/or amelioration of edema, e.g., lymphedema comprising administering to a subject in need thereof a deuterium-enriched pirfenidone compound, for example, a compound of Formula I, e.g., a compound listed in Table 1, including, e.g., LYT-100, in combination with one or more anti-TGF-β1 agent(s) and optionally an anti-angiotensin agent. In some embodiments, the methods for the treatment, prevention, and/or amelioration of edema, e.g., lymphedema comprise administering to a subject in need thereof a deuterium-enriched pirfenidone, for example, a compound of Formula I, e.g., a compound listed in Table 1, including e.g., LYT-100, and one or more anti-TGF-β1 agent(s) selected from LY550410, LY580276, SB-505124, galunisertib (LY2157299 Monohydrate) , and deuterium-enriched pirfenidone, e.g., as described herein, other than LYT-100, and optionally an anti-angiotensin agent. In some embodiments, the anti-angiotensin agent is selected from captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099. For instance, in some embodiments, the methods for the treatment, prevention, and/or amelioration of edema, e.g., lymphedema comprise administering to a subject in need thereof a deuterium-enriched pirfenidone , for example,e.g., a compound of Formula I, e.g., a compound listed in Table 1, including e.g., LYT-100, and one or more anti-TGF-β1 agent(s), e.g., a second form of deuterated pirfenidone, e.g., other than LYT-100, and an anti-angiotensin agent, e.g., captopril.

Provided herein are methods for treatment, prevention, and/or amelioration of one or more symptoms of edema, e.g., lymphedema comprising administering to a subject in need thereof a deuterium-enriched pirfenidone compound, for example, a compound of Formula I, e.g., a compound listed in Table 1, including, e.g., LYT-100, in combination with one or more anti-TGF-β1 agent(s) and optionally an anti-angiotensin agent. In some embodiments, the methods for treatment, prevention, and/or amelioration of one or more symptoms of edema, e.g., lymphedema comprise administering to a subject in need thereof a deuterium-enriched pirfenidone, for example, a compound of Formula I, e.g., a compound listed in Table 1, including e.g., LYT-100, and one or more anti-TGF-β1 agent(s) selected from LY550410, LY580276, SB-505124, galunisertib (LY2157299 Monohydrate) , and deuterium-enriched pirfenidone, e.g., as described herein, other than LYT-100, and optionally an anti-angiotensin agent. In some embodiments, the anti-angiotensin agent is selected from captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099. For instance, in some embodiments, the methods for treatment, prevention, and/or amelioration of one or more symptoms of edema, e.g., lymphedema comprise administering to a subject in need thereof a deuterium-enriched pirfenidone , for example,e.g., a compound of Formula I, e.g., a compound listed in Table 1, including e.g., LYT-100, and one or more anti-TGF-β1 agent(s), e.g., a second form of deuterated pirfenidone, e.g., other than LYT-100, and an anti-angiotensin agent, e.g., captopril.

Provided herein are methods for treatment, prevention, and/or amelioration of IPF and/or one or more symptoms of IPF comprising administering to a subject in need thereof a deuterium-enriched pirfenidone compound, for example, a compound of Formula I, e.g., a compound listed in Table 1, including, e.g., LYT-100, in combination with one or more anti-TGF-β1 agent(s) and optionally an anti-angiotensin agent. In some embodiments, the methods for treatment, prevention, and/or amelioration of IPF and/or one or more symptoms of IPF comprise administering to a subject in need thereof a deuterium-enriched pirfenidone, for example, a compound of Formula I, e.g., a compound listed in Table 1, including e.g., LYT-100, and one or more anti-TGF-β1 agent(s) selected from LY550410, LY580276, SB-505124, galunisertib (LY2157299 Monohydrate) , and deuterium-enriched pirfenidone, e.g., as described herein, other than LYT-100, and optionally an anti-angiotensin agent. In some embodiments, the anti-angiotensin agent is selected from captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099. For instance, in some embodiments, the methods for treatment, prevention, and/or amelioration of IPF and/or one or more symptoms of IPF comprise administering to a subject in need thereof a deuterium-enriched pirfenidone , for example,e.g., a compound of Formula I, e.g., a compound listed in Table 1, including e.g., LYT-100, and one or more anti-TGF-β1 agent(s), e.g., a second form of deuterated pirfenidone, e.g., other than LYT-100, and an anti-angiotensin agent, e.g., captopril.

Provided herein are methods for the treatment, prevention, and/or amelioration of a fibrotic-mediated disorder and/or a collagen-mediated disorder comprising administering to a subject in need thereof a deuterium-enriched pirfenidone compound, for example, a compound of Formula I, e.g., a compound listed in Table 1, including, e.g., LYT-100, in combination with one or more anti-TGF-β1 agent(s) and optionally one or more anti-inflammatory agent(s). In some embodiments, the methods for the treatment, prevention, and/or amelioration of a fibrotic-mediated disorder and/or a collagen-mediated disorder comprise administering to a subject in need thereof a deuterium-enriched pirfenidone, for example, a compound of Formula I, e.g., a compound listed in Table 1, including e.g., LYT-100, and one or more anti-TGF-β1 agent(s) selected from LY550410, LY580276, SB-505124, (LY2157299 Monohydrate) , and deuterium-enriched pirfenidone, e.g., as described herein, other than LYT-100, and one or more anti-inflammatory agent(s). In some embodiments, the one or more anti-inflammatory agent(s) are selected from selected from etodolac, famotidine, fenoprofen, fenoprofen, flurbiprofen, ibuprofen, indomethacin, ketoprofen, ketorolac, lansoprazole, mefenamic acid, meloxicam, misoprostol, nabumetone, naproxen, oxaprozin, piroxicam, sulindac, tolmetin, celecoxib, apricoxib, robenacoxib, valdecoxib, anitrazafen, tilmacoxib, flumizole, cimicoxib, rofecoxib, mavacoxib, and firocoxib. For instance, in some embodiments, the methods for the treatment, prevention, and/or amelioration of a fibrotic-mediated disorder and/or a collagen-mediated disorder comprise administering to a subject in need thereof a deuterium-enriched pirfenidone, for example, a compound of Formula I, e.g., a compound listed in Table 1, including , for example,e.g., a compound of Formula I, e.g., a compound listed in Table 1, including e.g., LYT-100, one or more anti-TGF-β1 agent(s), e.g., a deuterium enricher pirfendone described herein other than LYT-100, and ibuprofen.

Provided herein are methods for the treatment, prevention, and/or amelioration of edema, e.g., lymphedema comprising administering to a subject in need thereof a deuterium-enriched pirfenidone compound, for example, a compound of Formula I, e.g., a compound listed in Table 1, including, e.g., LYT-100, in combination with one or more anti-TGF-β1 agent(s) and optionally one or more anti-inflammatory agent(s). In some embodiments, the methods for the treatment, prevention, and/or amelioration of edema, e.g., lymphedema comprise administering to a subject in need thereof a deuterium-enriched pirfenidone, for example, a compound of Formula I, e.g., a compound listed in Table 1, including e.g., LYT-100, and one or more anti-TGF-β1 agent(s) selected from LY550410, LY580276, SB-505124, (LY2157299 Monohydrate) , and deuterium-enriched pirfenidone, e.g., as described herein, other than LYT-100, and one or more anti-inflammatory agent(s). In some embodiments, the one or more anti-inflammatory agent(s) are selected from selected from etodolac, famotidine, fenoprofen, fenoprofen, flurbiprofen, ibuprofen, indomethacin, ketoprofen, ketorolac, lansoprazole, mefenamic acid, meloxicam, misoprostol, nabumetone, naproxen, oxaprozin, piroxicam, sulindac, tolmetin, celecoxib, apricoxib, robenacoxib, valdecoxib, anitrazafen, tilmacoxib, flumizole, cimicoxib, rofecoxib, mavacoxib, and firocoxib. For instance, in some embodiments, the methods for the treatment, prevention, and/or amelioration of edema, e.g., lymphedema comprise administering to a subject in need thereof a deuterium-enriched pirfenidone, for example, a compound of Formula I, e.g., a compound listed in Table 1, including , for example,e.g., a compound of Formula I, e.g., a compound listed in Table 1, including e.g., LYT-100, one or more anti-TGF-β1 agent(s), e.g., a deuterium enricher pirfendone described herein other than LYT-100, and ibuprofen.

Provided herein are methods for treatment, prevention, and/or amelioration of one or more symptoms of edema, e.g., lymphedema comprising administering to a subject in need thereof a deuterium-enriched pirfenidone compound, for example, a compound of Formula I, e.g., a compound listed in Table 1, including, e.g., LYT-100, in combination with one or more anti-TGF-β1 agent(s) and optionally one or more anti-inflammatory agent(s). In some embodiments, the methods for treatment, prevention, and/or amelioration of one or more symptoms of edema, e.g., lymphedema comprise administering to a subject in need thereof a deuterium-enriched pirfenidone, for example, a compound of Formula I, e.g., a compound listed in Table 1, including e.g., LYT-100, and one or more anti-TGF-β1 agent(s) selected from LY550410, LY580276, SB-505124, (LY2157299 Monohydrate) , and deuterium-enriched pirfenidone, e.g., as described herein, other than LYT-100, and one or more anti-inflammatory agent(s). In some embodiments, the one or more anti-inflammatory agent(s) are selected from selected from etodolac, famotidine, fenoprofen, fenoprofen, flurbiprofen, ibuprofen, indomethacin, ketoprofen, ketorolac, lansoprazole, mefenamic acid, meloxicam, misoprostol, nabumetone, naproxen, oxaprozin, piroxicam, sulindac, tolmetin, celecoxib, apricoxib, robenacoxib, valdecoxib, anitrazafen, tilmacoxib, flumizole, cimicoxib, rofecoxib, mavacoxib, and firocoxib. For instance, in some embodiments, the methods for treatment, prevention, and/or amelioration of one or more symptoms of edema, e.g., lymphedema comprise administering to a subject in need thereof a deuterium-enriched pirfenidone, for example, a compound of Formula I, e.g., a compound listed in Table 1, including, for example,e.g., a compound of Formula I, e.g., a compound listed in Table 1, including e.g., LYT-100, one or more anti-TGF-β1 agent(s), e.g., a deuterium enricher pirfendone described herein other than LYT-100, and ibuprofen.

Provided herein are methods for treatment, prevention, and/or amelioration of IPF and/or one or more symptoms of IPF comprising administering to a subject in need thereof a deuterium-enriched pirfenidone compound, for example, a compound of Formula I, e.g., a compound listed in Table 1, including, e.g., LYT-100, in combination with one or more anti-TGF-β1 agent(s) and optionally one or more anti-inflammatory agent(s). In some embodiments, the methods for treatment, prevention, and/or amelioration of IPF and/or one or more symptoms of IPF comprise administering to a subject in need thereof a deuterium-enriched pirfenidone, for example, a compound of Formula I, e.g., a compound listed in Table 1, including e.g., LYT-100, and one or more anti-TGF-β1 agent(s) selected from LY550410, LY580276, SB-505124, (LY2157299 Monohydrate) , and deuterium-enriched pirfenidone, e.g., as described herein, other than LYT-100, and one or more anti-inflammatory agent(s). In some embodiments, the one or more anti-inflammatory agent(s) are selected from selected from etodolac, famotidine, fenoprofen, fenoprofen, flurbiprofen, ibuprofen, indomethacin, ketoprofen, ketorolac, lansoprazole, mefenamic acid, meloxicam, misoprostol, nabumetone, naproxen, oxaprozin, piroxicam, sulindac, tolmetin, celecoxib, apricoxib, robenacoxib, valdecoxib, anitrazafen, tilmacoxib, flumizole, cimicoxib, rofecoxib, mavacoxib, and firocoxib. For instance, in some embodiments, the methods for treatment, prevention, and/or amelioration of IPF and/or one or more symptoms of IPF comprise administering to a subject in need thereof a deuterium-enriched pirfenidone, for example, a compound of Formula I, e.g., a compound listed in Table 1, including , for example,e.g., a compound of Formula I, e.g., a compound listed in Table 1, including e.g., LYT-100, one or more anti-TGF-β1 agent(s), e.g., a deuterium enricher pirfendone described herein other than LYT-100, and ibuprofen.

Provided herein are methods for the treatment, prevention, and/or amelioration of a fibrotic-mediated disorder and/or a collagen-mediated disorder comprising administering to a subject in need thereof a deuterium-enriched pirfenidone compound, for example, a compound of Formula I, e.g., a compound listed in Table 1, including, e.g., LYT-100, in combination with one or more anti-T cell agent(s) and optionally an anti-angiotensin agent. In some embodiments, the methods for the treatment, prevention, and/or amelioration of a fibrotic-mediated disorder and/or a collagen-mediated disorder comprise administering to a subject in need thereof a deuterium-enriched pirfenidone, for example, a compound of Formula I, e.g., a compound listed in Table 1, including e.g., LYT-100, and one or more anti-T cell agent(s) selected from tacrolimus, teriflunomide, leflunomide, cyclosporine, pimecrolimus, denileukin diftitox, and basiliximab. In some embodiments, the anti-angiotensin agent is selected from captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099. For instance, in some embodiments, the methods for the treatment, prevention, and/or amelioration of a fibrotic-mediated disorder and/or a collagen-mediated disorder comprise administering to a subject in need thereof a deuterium-enriched pirfenidone , for example, a compound of Formula I, e.g., a compound listed in Table 1, including e.g., LYT-100, tacrolimus and optionally captopril. In some embodiments, the methods for the treatment, prevention, and/or amelioration of a fibrotic-mediated disorder and/or a collagen-mediated disorder comprise administering to a subject in need thereof a deuterium-enriched pirfenidone, for example, a compound of Formula I, e.g., a compound listed in Table 1, including e.g., LYT-100, teriflunomide and optionally captopril.

Provided herein are methods for the treatment, prevention, and/or amelioration of edema, e.g., lymphedema comprising administering to a subject in need thereof a deuterium-enriched pirfenidone compound, for example, a compound of Formula I, e.g., a compound listed in Table 1, including, e.g., LYT-100, in combination with one or more anti-T cell agent(s) and optionally an anti-angiotensin agent. In some embodiments, the methods for the treatment, prevention, and/or amelioration of edema, e.g., lymphedema comprise administering to a subject in need thereof a deuterium-enriched pirfenidone, for example, a compound of Formula I, e.g., a compound listed in Table 1, including e.g., LYT-100, and one or more anti-T cell agent(s) selected from tacrolimus, teriflunomide, leflunomide, cyclosporine, pimecrolimus, denileukin diftitox, and basiliximab. In some embodiments, the anti-angiotensin agent is selected from captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099. For instance, in some embodiments, the methods for the treatment, prevention, and/or amelioration of edema, e.g., lymphedema comprise administering to a subject in need thereof a deuterium-enriched pirfenidone , for example, a compound of Formula I, e.g., a compound listed in Table 1, including e.g., LYT-100, tacrolimus and optionally captopril. In some embodiments, the methods for the treatment, prevention, and/or amelioration of edema, e.g., lymphedema comprise administering to a subject in need thereof a deuterium-enriched pirfenidone, for example, a compound of Formula I, e.g., a compound listed in Table 1, including e.g., LYT-100, teriflunomide and optionally captopril.

Provided herein are methods for treatment, prevention, and/or amelioration of one or more symptoms of edema, e.g., lymphedema comprising administering to a subject in need thereof a deuterium-enriched pirfenidone compound, for example, a compound of Formula I, e.g., a compound listed in Table 1, including, e.g., LYT-100, in combination with one or more anti-T cell agent(s) and optionally an anti-angiotensin agent. In some embodiments, the methods for treatment, prevention, and/or amelioration of one or more symptoms of edema, e.g., lymphedema comprise administering to a subject in need thereof a deuterium-enriched pirfenidone, for example, a compound of Formula I, e.g., a compound listed in Table 1, including e.g., LYT-100, and one or more anti-T cell agent(s) selected from tacrolimus, teriflunomide, leflunomide, cyclosporine, pimecrolimus, denileukin diftitox, and basiliximab. In some embodiments, the anti-angiotensin agent is selected from captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099. For instance, in some embodiments, the methods for treatment, prevention, and/or amelioration of one or more symptoms of edema, e.g., lymphedema comprise administering to a subject in need thereof a deuterium-enriched pirfenidone , for example, a compound of Formula I, e.g., a compound listed in Table 1, including e.g., LYT-100, tacrolimus and optionally captopril. In some embodiments, the methods for treatment, prevention, and/or amelioration of one or more symptoms of edema, e.g., lymphedema comprise administering to a subject in need thereof a deuterium-enriched pirfenidone, for example, a compound of Formula I, e.g., a compound listed in Table 1, including e.g., LYT-100, teriflunomide and optionally captopril.

Provided herein are methods for treatment, prevention, and/or amelioration of IPF and/or one or more symptoms of IPF comprising administering to a subject in need thereof a deuterium-enriched pirfenidone compound, for example, a compound of Formula I, e.g., a compound listed in Table 1, including, e.g., LYT-100, in combination with one or more anti-T cell agent(s) and optionally an anti-angiotensin agent. In some embodiments, the methods for treatment, prevention, and/or amelioration of IPF and/or one or more symptoms of IPF comprise administering to a subject in need thereof a deuterium-enriched pirfenidone, for example, a compound of Formula I, e.g., a compound listed in Table 1, including e.g., LYT-100, and one or more anti-T cell agent(s) selected from tacrolimus, teriflunomide, leflunomide, cyclosporine, pimecrolimus, denileukin diftitox, and basiliximab. In some embodiments, the anti-angiotensin agent is selected from captopril, zofenopril, enalapril, lisinopril, ramipril, quinapril, perindopril, benazepril, imidapril, trandolapril, cilazapril, fosinopril, losartan, irbesartan, olmesartan, candesartan, telmisartan, valsartan, fimasartan, diminazene aceturate, xanthenone, and AVE 099. For instance, in some embodiments, the methods for treatment, prevention, and/or amelioration of IPF and/or one or more symptoms of IPF comprise administering to a subject in need thereof a deuterium-enriched pirfenidone , for example, a compound of Formula I, e.g., a compound listed in Table 1, including e.g., LYT-100, tacrolimus and optionally captopril. In some embodiments, the methods for treatment, prevention, and/or amelioration of IPF and/or one or more symptoms of IPF comprise administering to a subject in need thereof a deuterium-enriched pirfenidone, for example, a compound of Formula I, e.g., a compound listed in Table 1, including e.g., LYT-100, teriflunomide and optionally captopril.

### Pharmaceutical Compositions, Formulations, Dosage Forms, and Administration

According to another embodiment, the present invention provides a composition comprising a disclosed compound, or a pharmaceutically acceptable salt thereof, and a pharmaceutically acceptable excipient, carrier, adjuvant, diluent, or vehicle. The amount of compound in the composition is an amount effective to treat the relevant disease, disorder, or condition in a patient in need thereof (an "effective amount"). For example, in some embodiments, the "effective amount" of deuterium-enriched pirfenidone is a dosage of deuterium-enriched pirfenidone, e.g., LYT-100, provided herein for the treatment, prevention, and/or amelioration of one or symptoms of a fibrotic-mediated disorder and/or a collagen-mediated disorder, such as any of the fibrotic-mediated disorders and/or a collagen-mediated disorders disclosed herein, including, for example, edema and lymphedema. In some embodiments, a composition of the present disclosure is formulated for oral administration to a patient.

The term "pharmaceutically acceptable excipient, carrier, adjuvant, diluent, or vehicle" refers to a non-toxic excipient, carrier, adjuvant, diluent, or vehicle that does not destroy the pharmacological activity of the agent with which it is formulated. Pharmaceutically acceptable excipients, carriers, adjuvants, diluents, or vehicles that may be used in the disclosed compositions include, but are not limited to, ion exchangers, alumina, stearates such as aluminum stearate, lecithin, serum proteins such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. In some embodiments, the composition is formulated as a lipophilic mixture, such as a lipid-based composition.

Compositions of the present invention may be administered orally, parenterally, enterally, intracisternally, intraperitoneally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. In some embodiments, the composition is administered orally, intraperitoneally, or intravenously. In some embodiments, the composition is a transmucosal formulation. In some embodiments, the composition is injected directly into the lymphatic system. Sterile injectable forms of the compositions of this invention may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

To aid in delivery of the composition, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

Pharmaceutically acceptable compositions may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, may also be added. For oral administration in a capsule form, useful diluents include lactose and dried corn starch. When aqueous suspensions are required for oral use, the active ingredient is combined with emulsifying and suspending agents. If desired, certain sweetening, flavoring or coloring agents may also be added.

Alternatively, pharmaceutically acceptable compositions may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols.

In some embodiments, the pharmaceutically acceptable composition is formulated for oral administration. Such formulations may be administered with or without food. In some embodiments, the pharmaceutically acceptable composition is administered without food. In other embodiments, the pharmaceutically acceptable composition is administered with food.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated.

Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of inj ectables.

Injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a compound of the present invention, it is often desirable to slow the absorption of the compound from subcutaneous or intramuscular injection. This may be accomplished by the use of a liquid suspension of crystalline or amorphous material with poor water solubility. The rate of absorption of the compound then depends upon its rate of dissolution that, in turn, may depend upon crystal size and crystalline form. Alternatively, delayed absorption of a parenterally administered compound form is accomplished by dissolving or suspending the compound in an oil vehicle. Injectable depot forms are made by forming microencapsule matrices of the compound in biodegradable polymers such as polylactide-polyglycolide. Depending upon the ratio of compound to polymer and the nature of the particular polymer employed, the rate of compound release can be controlled. Examples of other biodegradable polymers include poly(orthoesters) and poly(anhydrides). Depot injectable formulations are also prepared by entrapping the compound in liposomes or microemulsions that are compatible with body tissues.

Compositions for rectal or vaginal administration are preferably suppositories which can be prepared by mixing the compounds of this invention with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polethylene glycols and the like.

Therapeutic agents can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, and eye drops are also contemplated as being within the scope of this invention. Additionally, the present invention contemplates the use of transdermal patches, which have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

In some embodiments, the compound is formulated as an orally administerable, lipid-based formulation. Lipid-based formulations for oral delivery are known in the art and may include, for example, substantially non-aqueous vehicles which typically contain one or more lipid components. The lipid vehicles and resulting lipid formulations may be usefully classified as described below according to their shared common features according to the lipid formulation classification system (LFCS) (Pouton, C. W., Eur. J. Pharm. Sci. 11 (Supp 2), S93-S98, 2000; Pouton, C. W., Eur. J. Pharm. Sci. 29 278-287, 2006).

Lipid vehicles, and the resulting lipid formulations, may contain oil/lipids and/or surfactants, optionally with co-solvents. In the LFCS terminology, Type I formulations include oils or lipids which require digestion, such as mono, di and tri-glycerides and combinations thereof. Type II formulations are water-insoluble self emulsifying drug delivery systems (SEDDS) which contain lipids and oils used in Type I formulations, with additional water insoluble surfactants. Type III formulations are SEDDS or self-microemulsifying drug delivery systems (SMEDDS) which contain lipids and oils used in Type I formulations, with additional water-soluble surfactants and/or co-solvents (Type IIIa) or a greater proportion of water-soluble components (Type IIIb). Type IV formulations contain predominantly hydrophilic surfactants and co-solvents (e.g. PEG, propylene glycol and diethylene glycol monoethyl ether) and are useful for drugs which are poorly water soluble but not lipophilic. Any such lipid formulation (Type I-IV) is contemplated herein for use with a disclosed compound or pharmaceutical composition thereof.

In some embodiments, the lipid vehicle contains one or more oils or lipids, without additional surfactants, co-surfactants or co-emulsifiers, or co-solvents, i.e. it consists essentially of one or more oils or lipids. In some further embodiments, the lipid vehicle contains one or more oils or lipids together with one or more water-insoluble surfactants, optionally together with one or more co-solvents. In some embodiments, the lipid vehicle contains one or more oils or lipids together with one or more water-soluble surfactants, optionally together with one or more co-solvents. In some embodiments, the lipid vehicle contains a mixture of oil/lipid, surfactant and co-solvent. In some embodiments, the lipid vehicle consists essentially of one or more surfactants/co-surfactants/co-emulsifiers, and/or solvents/co-solvents.

Examples of oils or lipids which may be used in the present invention include almond oil, babassu oil, blackcurrant seed oil, borage oil, canola oil, castor oil, coconut oil, cod liver oil, corn oil, cottonseed oil, evening primrose oil, fish oil, grape seed oil, mustard seed oil, olive oil, palm kernel oil, palm oil, peanut oil, rapeseed oil, safflower oil, sesame oil, shark liver oil, soybean oil, sunflower oil, walnut oil, wheat germ oil, avocado oil, bran oil, hydrogenated castor oil, hydrogenated coconut oil, hydrogenated cottonseed oil, hydrogenated palm oil, hydrogenated soybean oil, partially hydrogenated soybean oil, hydrogenated vegetable oil, caprylic/capric glycerides, fractionated triglycerides, glyceryl tricaprate, glyceryl tricaproate, glyceryl tricaprylate, glyceryl tricaprylate/caprate, glyceryl tricaprylate/caprate, glyceryl tricaprylate/caprate/laurate, glyceryl tricaprylate/caprate/linoleate, glyceryl tricaprylate/caprate/stearate, glyceryl trilaurate, glyceryl monolaurate, glyceryl behenate, glyceryl monolinoleate, glyceryl trilinolenate, glyceryl trioleate, glyceryl triundecanoate, glyceryl tristearate linoleic glycerides, saturated polyglycolized glycerides, synthetic medium chain triglycerides containing primarily C₈₋₁₂ fatty acid chains, medium chain triglycerides containing primarily C₈₋₁₂ fatty acid chains, long chain triglycerides containing primarily >C₁₂ fatty acid chains, modified triglycerides, fractionated triglycerides, and mixtures thereof.

Examples of mono and diglycerides which may be used in such formulations include glycerol mono- and diesters having fatty acid chains from 8 to 40 carbon atoms, including hydrolysed coconut oils (e.g. Capmul^{®} MCM), hydrolysed corn oil (e.g. Maisine^{™}35-l). In some embodiments, the monoglycerides and diglycerides are mono-or di- saturated fatty acid esters of glycerol having fatty acid chains of 8 to 18 carbon chain length (e.g. glyceryl monostearate, glyceryl distearate, glyceryl monocaprylate, glyceryl dicaprylate, glyceryl monocaprate and glyceryl dicaprate). Mixtures of fatty acids ("structured glycerides") adapted for enhancing the absorption and transport of lipid soluble compounds are disclosed in, e.g., U.S. Patent No. 6,013,665, which is hereby incorporated by reference.

In some embodiments, a disclosed compound is formulated with an exosome, e.g., encapsulated in an exosome. In some embodiments, the exosome is derived from milk of a mammal (milk exosome). In some embodiments, a deuterium-enriched pirfenidone is formulated with an exosome, e.g., encapsulated in an exosome. In some embodiments, deuterium-enriched pirfenidone is formulated with a milk exosome. In some embodiments, a LYT-100is formulated with an exosome, e.g., encapsulated in an exosome. In some embodiments, LYT-100is formulated with a milk exosome.

Exemplary suitable milk exosomes capable of loading (e.g., encapsulating) therapeutic agents and methods of producing such milk exosomes are described in International Patent Application PCT/US2014/018601, filed February 26, 2014, published as WO2014/134132, and International Patent Application PCT/US2017/063681, filed November 29, 2017, published as WO/2018/102397, the contents of each of which are herein incorporated by reference it their entireties.

Suitable surfactants for use in the lipid formulations include propylene glycol mono- and di-esters of C₈₋₂₂ fatty acids, such as, but not limited to, propylene glycol monocaprylate, propylene glycol dicaprylate, propylene glycol monolaurate, sold under trade names such as Capryol^{®} 90, Labrafac^{®} PG, Lauroglycol^{®} FCC, sugar fatty acid esters, such as, but not limited to, sucrose palmitate, sucrose laurate, and sucrose stearate; sorbitan fatty acid esters such as, but not limited to, sorbitan laurate, sorbitan palmitate, and sorbitan oleate; polyoxyethylene sorbitan fatty acid esters such as, but not limited to, polysorbate 20, polysorbate 40, polysorbate 60, polysorbate 80, and polysorbate 85; polyoxyethylene mono- and di-fatty acid esters including, but not limited to, polyoxyl 40 stearate and polyoxyl 40 oleate; a mixture of polyoxyethylene mono- and di-esters of C₈₋₂₂ fatty acids and glyceryl mono-, di-, and tri-esters of C₈₋₂₂ fatty acids as sold under tradenames such as Labrasol^{®}, Gelucire^{®} 44/14, Gelucire^{®} 50/13, and Labrafil^{®}; polyoxyethylene castor oils compound such as, but not limited to, polyoxyl 35 castor oil, polyoxyl 40 hydrogenated castor oil, and polyoxyl 60 hydrogenated castor oil, as are sold under tradenames such as Cremophor^{®}/Kolliphor EL, Cremophor^{®}/Kolliphor^{®} RH40, and Cremophor^{®}/Kolliphor^{®} RH60; polyoxyethylene alkyl ethers including, but not limited to, polyoxyl 20 cetostearyl ether and polyoxyl 10 oleyl ether; DL-α-tocopheryl polyethylene glycol succinate; glyceryl mono-, di-, and tri-esters; glyceryl mono-, di-, and tri-esters of C₈₋₂₂ fatty acids; sucrose mono-, di-, and tri-esters; sodium dioctylsulfosuccinate; polyoxyethylene-polyoxypropylene copolymers such as, but not limited to poloxamer 124, poloxamer 188, and poloxamer 407; polyoxyethylene ethers of C₈₋₂₂ fatty alcohols including, but not limited to, polyoxyethylenelauryl alcohol, polyoxyethylenecetyl alcohol, polyoxyethylene stearyl alcohol, polyoxyethyleneoleyl alcohol, as sold under tradenames such as Brij^{®} 35, Brij^{®} 58, Brij^{®} 78, Brij^{®} 98, or a mixture of any two or more thereof.

A co-emulsifier, or co-surfactant, may be used in the formulation. A suitable co-emulsifier or co-surfactant may be a phosphoglyceride; a phospholipid, for example lecithin, or a free fatty acid that is liquid at room temperature, for example, iso-stearic acid, oleic acid, linoelic acid, linolenic acid, palmitic acid, stearic acid, lauric acid, capric acid, caprylic acid, and caproic acid.

Suitable solvents/co-solvents include ethanol, propylene glycol, polyethylene glycol, diethylene glycol monoethyl ether, and glycerol.

A polymer may also be used in the formulation to inhibit drug precipitation or to alter the rate of drug release. A range of polymers have been shown to impart these properties and are well known to those skilled in the art. Suitable polymers include hydroxypropylmethylcellulose, hydroxypropylmethylcellulose acetyl succinate, other cellulose-derived polymers such as methylcellulose; poly(meth)acrylates, such as the Eudragit series of polymers, including Eudragit E100, polyvinylpyrrolidone, or others as described in, e.g. Warren et al., Mol. Pharmaceutics 2013, 10, 2823-2848.

Formulations may be chosen specifically to provide for sustained release of the active in the gastrointestinal (GI) tract in order to control the rate of absorption. Many different approaches may be used to achieve these ends including the use of high melting point lipids that disperse/erode slowly in the GI tract, or polymers that form a matrix that slowly erodes. These formulations may take the form of large monolithic dose forms or may be present as micro or nano-particulate matrices as described in, for example, in Mishra, Handbook of Encapsulation and Controlled Release, CRC Press, Boca Raton, (2016) ISBN 978-1-4822-3234-9, Wilson and Crowley, Controlled Release in Oral Drug Delivery, Springer, NY, ISBN 978-1-4614-1004-1 (2011) or Wise, Handbook of Pharmaceutical Controlled Release Technology, Marcel Dekker, NY, ISBN 0-82467-0369-3 (2000).

Formulations may also contain materials commonly known to those skilled in the art to be included in lipid based formulations, including antioxidants, for example, butylated hydroxyanisole (BHA) or butylated hydroxytoluene (BHT) and solidifying agents such as microporous silica, for example magnesium alumino-metasilicate (Neusilin).

### Combination Therapies

A disclosed compound or a pharmaceutically acceptable salt thereof, or pharmaceutical composition thereof, may be administered to a patient in need thereof in combination with one or more additional therapeutic agents and/or therapeutic processes.

The compound or a pharmaceutically acceptable salt or pharmaceutical composition thereof can be administered alone or in combination with one or more other therapeutic compounds, possible combination therapy taking the form of fixed combinations or the administration of the compound or composition and one or more other therapeutic compounds being staggered or given independently of one another, or the combined administration of fixed combinations and one or more other therapeutic compounds. A compound or a pharmaceutically acceptable salt or pharmaceutical composition thereof can besides or in addition be administered for tumor therapy in combination with chemotherapy, radiotherapy, immunotherapy, phototherapy, surgical intervention, or a combination of these. Long-term therapy is equally possible as is adjuvant therapy in the context of other treatment strategies, as described above. Other possible treatments are therapy to maintain the patient's status after tumor regression, or even chemopreventive therapy, for example in patients at risk.

Such additional agents may be administered separately from a compound or a pharmaceutically acceptable salt or pharmaceutical composition thereof, as part of a multiple dosage regimen. Alternatively, those agents may be part of a single dosage form, mixed together with a disclosed compound in a single composition. If administered as part of a multiple dosage regime, the two active agents may be submitted simultaneously, sequentially or within a period of time from one another.

As used herein, the term "combination," "combined," and related terms refers to the simultaneous or sequential administration of therapeutic agents in accordance with the present disclosure. For example, a compound or a pharmaceutically acceptable salt or pharmaceutical composition thereof may be administered with another therapeutic agent simultaneously or sequentially in separate unit dosage forms or together in a single unit dosage form. Accordingly, the present disclosure provides a single unit dosage form comprising a disclosed compound, an additional therapeutic agent, and a pharmaceutically acceptable excipient, carrier, adjuvant, diluent, or vehicle. In some embodiments, the additional agent is formulated in a separate composition from the disclosed compound.

The amount of both a disclosed compound or a pharmaceutically acceptable salt or pharmaceutical composition thereof and additional therapeutic agent (in those compositions which comprise an additional therapeutic agent as described above) that may be combined with the carrier materials to produce a single dosage form will vary depending upon the patient treated and the particular mode of administration. In certain embodiments, compositions of this invention are formulated so that a dosage of between 0.01-100 mg/kg body weight/day of a disclosed compound can be administered.

In those compositions which comprise an additional therapeutic agent, that additional therapeutic agent and the disclosed compound may act synergistically. Therefore, the amount of additional therapeutic agent in such compositions will be less than that required in a monotherapy utilizing only that therapeutic agent. In such compositions, a dosage of between 0.01-100 mg/kg body weight/day of the additional therapeutic agent can be administered.

The amount of additional therapeutic agent present in the compositions of this invention will be no more than the amount that would normally be administered in a composition comprising that therapeutic agent as the only active agent. In some embodiments, the amount of additional therapeutic agent in the presently disclosed compositions will range from about 50% to 100% of the amount normally present in a composition comprising that agent as the only therapeutically active agent.

The structure of the active compounds identified by code numbers, generic or trade names may be taken from the actual edition of the standard compendium "The Merck Index" or from databases, e.g. Patents International (e.g. IMS World Publications).

The exact amount of therapeutic agent required to be effective in any treatment regimen will vary from subject to subject, depending on the species, age, and general condition of the subject, the severity of the infection, the particular agent, its mode of administration, and the like. Disclosed compounds are preferably formulated in unit dosage form for ease of administration and uniformity of dosage. The expression "unit dosage form" as used herein refers to a physically discrete unit of agent appropriate for the patient to be treated. It will be understood, however, that the total daily usage of a disclosed compound or a pharmaceutically acceptable salt or pharmaceutical composition thereof and any co-administered additional therapeutic agents will be decided by the attending physician within the scope of sound medical judgment. The specific effective dose level for any particular patient or organism will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound or composition; the duration of the treatment; drugs used in combination or coincidental with the specific compound or composition employed, and like factors well known in the medical arts.

The term "subject" or "patient," as used herein, means any subject, such as an animal, for example a mammalian subject, for whom diagnosis, prognosis, or therapy is desired. Mammalian subjects include humans, domestic animals, farm animals, sports animals, and zoo animals including, e.g., humans, non-human primates, dogs, cats, guinea pigs, rabbits, rats, mice, horses, cattle, and so on. In some embodiments, the term "subject" is meant to include a "patient". In some embodiments, the term "patient" is meant to refer to a mammalian subject, including a human subject. In some embodiments, the patient is human subject.

In some embodiments, pirfenidone or deuterium-enriched pirfenidone is co-administered with one or more additional therapeutic agents selected from sepsis agents, antibacterials, anti-fungals, anti-coagulants, thrombolytics, steroidal drugs, non-steroidal anti-inflammatory drugs (NSAIDs), opioids, anesthetics, calcium channel blockers, Beta-blockers, nitrates or nitrites, ACE inhibitors, statins, platelet aggregation inhibitors, adenosine, digitoxin, anti-arrhythmic agents, sympathomimetic drugs, endothelin converting enzyme (ECE) inhibitors, thromboxane enzyme antagonists, potassium channel openers, thrombin inhibitors, growth factor inhibitors, platelet activating factor (PAF) antagonists, anti-platelet agents, Factor VIIa Inhibitors, Factor Xa Inhibitors, renin inhibitors, neutral endopeptidase (NEP) inhibitors, vasopepsidase inhibitors, HMG CoA reductase inhibitors, squalene synthetase inhibitors, fibrates, bile acid sequestrants, anti-atherosclerotic agents, MTP Inhibitors, potassium channel activators, alpha-PDE5 agents, beta-PDE5 agents, diuretics, anti-diabetic agents, PPAR-gamma agonists, mineralocorticoid enzyme antagonists, aP2 inhibitors, protein tyrosine kinase inhibitors, antiinflammatories, antiproliferatives, chemotherapeutic agents, immunosuppressants, anticancer agents, cytotoxic agents, antimetabolites, farnesyl-protein transferase inhibitors, hormonal agents, microtubule-disruptor agents, microtubule-stablizing agents, topoisomerase inhibitors, prenyl-protein transferase inhibitors, cyclosporins, TNF-alpha inhibitors, cyclooxygenase-2 (COX-2) inhibitors, gold compounds, antalarmin, Z-338 and platinum coordination complexes.

In some embodiments, the additional therapeutic agent is a steroidal drug.

In some embodiments, the steroidal drug is selected from the group consisting of aldosterone, beclometasone, betamethasone, deoxycorticosterone acetate, fludrocortisone acetate, hydrocortisone (cortisol), prednisolone, prednisone, methylprenisolone, dexamethasone, and triamcinolone.

In some embodiments, said therapeutic agent is a non-steroidal anti-inflammatory agent.

In some embodiments, the non-steroidal anti-inflammatory agent is selected from the group consisting of aceclofenac, acemetacin, amoxiprin, aspirin, azapropazone, benorilate, bromfenac, carprofen, celecoxib, choline magnesium salicylate, diclofenac, diflunisal, etodolac, etoracoxib, faislamine, fenbuten, fenoprofen, flurbiprofen, ibuprofen, indometacin, ketoprofen, ketorolac, lornoxicam, loxoprofen, lumiracoxib, meclofenamic acid, mefenamic acid, meloxicam, metamizole, methyl salicylate, magnesium salicylate, nabumetone, naproxen, nimesulide, oxyphenbutazone, parecoxib, phenylbutazone, piroxicam, salicyl salicylate, sulindac, sulfinprazone, suprofen, tenoxicam, tiaprofenic acid, and tolmetin.

In some embodiments, the compounds provided herein are combined with one or more therapeutic agents for sepsis treatment, including, but not limited to, drotrecogin-α or a biosimilar equivalent of activated protein C.

In certain embodiments, the compounds provided herein can be combined with one or more steroidal drugs, including, but not limited to, aldosterone, beclometasone, betamethasone, deoxycorticosterone acetate, fludrocortisone acetate, hydrocortisone (cortisol), prednisolone, prednisone, methylprenisolone, dexamethasone, and triamcinolone.

In other embodiments, the compounds provided herein can be combined with one or more antibacterial agents, including, but not limited to, amikacin, amoxicillin, ampicillin, arsphenamine, azithromycin, aztreonam, azlocillin, bacitracin, carbenicillin, cefaclor, cefadroxil, cefamandole, cefazolin, cephalexin, cefdinir, cefditorin, cefepime, cefixime, cefoperazone, cefotaxime, cefoxitin, cefpodoxime, cefprozil, ceftazidime, ceftibuten, ceftizoxime, ceftriaxone, cefuroxime, chloramphenicol, cilastin, ciprofloxacin, clarithromycin, clindamycin, cloxacillin, colistin, dalfopristan, demeclocycline, dicloxacillin, dirithromycin, doxycycline, erythromycin, enafloxacin, ertepenem, ethambutol, flucloxacillin, fosfomycin, furazolidone, gatifloxacin, geldanamycin, gentamicin, herbimicin, imipenem, isoniazide, kanamicin, levofloxacin, linezolid, lomefloxacin, loracarbef, mafenide, moxifloxacin, meropenem, metronidazole, mezlocillin, minocycline, mupirozin, nafcillin, neomycin, netilmicin, nitrofurantoin, norfloxacin, ofloxacin, oxytetracycline, penicillin, piperacillin, platensimycin, polymixin B, prontocil, pyrazinamide, quinupristine, rifampin, roxithromycin, spectinomycin, streptomycin, sulfacetamide, sulfamethizole, sulfamethoxazole, teicoplanin, telithromycin, tetracycline, ticarcillin, tobramycin, trimethoprim, troleandomycin, trovafloxacin, and vancomycin.

In some embodiments, the compounds provided herein can be combined with one or more antifungal agents, including, but not limited to, amorolfine, amphotericin B, anidulafungin, bifonazole, butenafine, butoconazole, caspofungin, ciclopirox, clotrimazole, econazole, fenticonazole, filipin, fluconazole, isoconazole, itraconazole, ketoconazole, micafungin, miconazole, naftifine, natamycin, nystatin, oxyconazole, ravuconazole, posaconazole, rimocidin, sertaconazole, sulconazole, terbinafine, terconazole, tioconazole, and voriconazole.

In other embodiments, the compounds provided herein can be combined with one or more anticoagulants, including, but not limited to, acenocoumarol, argatroban, bivalirudin, lepirudin, fondaparinux, heparin, phenindione, warfarin, and ximalagatran.

In certain embodiments, the compounds provided herein can be combined with one or more thrombolytics, but not limited to, anistreplase, reteplase, t-PA (alteplase activase), streptokinase, tenecteplase, and urokinase.

In certain embodiments, the compounds provided herein can be combined with one or more non-steroidal anti-inflammatory agents, including, but not limited to, aceclofenac, acemetacin, amoxiprin, aspirin, azapropazone, benorilate, bromfenac, carprofen, celecoxib, choline magnesium salicylate, diclofenac, diflunisal, etodolac, etoracoxib, faislamine, fenbuten, fenoprofen, flurbiprofen, ibuprofen, indometacin, ketoprofen, ketorolac, lornoxicam, loxoprofen, lumiracoxib, meclofenamic acid, mefenamic acid, meloxicam, metamizole, methyl salicylate, magnesium salicylate, nabumetone, naproxen, nimesulide, oxyphenbutazone, parecoxib, phenylbutazone, piroxicam, salicyl salicylate, sulindac, sulfinprazone, suprofen, tenoxicam, tiaprofenic acid, and tolmetin.

In some embodiments, the compounds provided herein can be combined with one or more antiplatelet agents, including, but not limited to, abciximab, cilostazol, clopidogrel, dipyridamole, ticlopidine, and tirofibin.

The compounds disclosed herein can also be administered in combination with other classes of compounds, including, but not limited to, anti-arrhythmic agents, such as propranolol; sympathomimetic drugs, such as norepinephrine; opioids, such as tramadol; anesthetics, such as ketamine; calcium channel blockers, such as diltiazem; Beta-blockers, such as atenolol; nitrates or nitrites, such as glyceryl trinitrate; endothelin converting enzyme (ECE) inhibitors, such as phosphoramidon; thromboxane receptor antagonists, such as ifetroban; potassium channel openers; thrombin inhibitors, such as hirudin; growth factor inhibitors, such as modulators of PDGF activity; platelet activating factor (PAF) antagonists; anti-platelet agents, such as GPIIb/IIIa blockers (e.g., abdximab, eptifibatide, and tirofiban), P2Y(AC) antagonists (e.g., clopidogrel, ticlopidine and CS-747), and aspirin; anticoagulants, such as warfarin; low molecular weight heparins, such as enoxaparin; Factor VIIa Inhibitors and Factor Xa Inhibitors; renin inhibitors; neutral endopeptidase (NEP) inhibitors; vasopepsidase inhibitors (dual NEP-ACE inhibitors), such as omapatrilat and gemopatrilat; HMG CoA reductase inhibitors, such as pravastatin, lovastatin, atorvastatin, simvastatin, NK-104 (a.k.a. itavastatin, nisvastatin, or nisbastatin), and ZD-4522 (also known as rosuvastatin, or atavastatin or visastatin); squalene synthetase inhibitors; fibrates; bile acid sequestrants, such as questran; niacin; anti-atherosclerotic agents, such as ACAT inhibitors; MTP Inhibitors; calcium channel blockers, such as amlodipine besylate; potassium channel activators; alpha-adrenergic agents; diuretics, such as chlorothlazide, hydrochlorothiazide, flumethiazide, hydroflumethiazide, bendroflumethiazide, methylchlorothiazide, trichloromethiazide, polythiazide, benzothlazide, ethacrynic acid, tricrynafen, chlorthalidone, furosenilde, musolimine, bumetanide, triamterene, amiloride, and spironolactone; thrombolytic agents, such as tissue plasminogen activator (tPA), recombinant tPA, streptokinase, urokinase, prourokinase, and anisoylated plasminogen streptokinase activator complex (APSAC); anti-diabetic agents, such as biguanides (e.g. metformin), glucosidase inhibitors (e.g., acarbose), insulins, meglitinides (e.g., repaglinide), sulfonylureas (e.g., glimepiride, glyburide, and glipizide), thiozolidinediones (e.g. troglitazone, rosiglitazone and pioglitazone), and PPAR-gamma agonists; mineralocorticoid receptor antagonists, such as spironolactone and eplerenone; growth hormone secretagogues; aP2 inhibitors; phosphodiesterase inhibitors, such as PDE III inhibitors (e.g., cilostazol) and PDE V inhibitors (e.g., sildenafil, tadalafil, vardenafil); protein tyrosine kinase inhibitors; antiinflammatories; antiproliferatives, such as methotrexate, FK506 (tacrolimus, Prograf), mycophenolate mofetil; chemotherapeutic agents; immunosuppressants; anticancer agents and cytotoxic agents (e.g., alkylating agents, such as nitrogen mustards, alkyl sulfonates, nitrosoureas, ethylenimines, and triazenes); antimetabolites, such as folate antagonists, purine analogues, and pyrridine analogues; antibiotics, such as anthracyclines, bleomycins, mitomycin, dactinomycin, and plicamycin; enzymes, such as L-asparaginase; farnesyl-protein transferase inhibitors; hormonal agents, such as glucocorticoids (e.g., cortisone), estrogens/antiestrogens, androgens/antiandrogens, progestins, and luteinizing hormone-releasing hormone anatagonists, and octreotide acetate; microtubule-disruptor agents, such as ecteinascidins; microtubule-stablizing agents, such as pacitaxel, docetaxel, and epothilones A-F; plant-derived products, such as vinca alkaloids, epipodophyllotoxins, and taxanes; and topoisomerase inhibitors; prenyl-protein transferase inhibitors; and cyclosporins; steroids, such as prednisone and dexamethasone; cytotoxic drugs, such as azathiprine and cyclophosphamide; TNF-alpha inhibitors, such as tenidap; anti-TNF antibodies or soluble TNF receptor, such as etanercept, rapamycin, and leflunimide; and cyclooxygenase-2 (COX-2) inhibitors, such as celecoxib and rofecoxib; and miscellaneous agents such as, hydroxyurea, procarbazine, mitotane, hexamethylmelamine, gold compounds, platinum coordination complexes, such as cisplatin, satraplatin, and carboplatin.

### Kits/Articles of Manufacture

For use in the therapeutic applications described herein, kits and articles of manufacture are also described herein. Such kits can comprise a carrier, package, or container that is compartmentalized to receive one or more containers such as vials, tubes, and the like, each of the container(s) comprising one of the separate elements to be used in a method described herein. Suitable containers include, for example, bottles, vials, syringes, and test tubes. The containers can be formed from a variety of materials such as glass or plastic.

For example, the container(s) can comprise one or more compounds described herein, optionally in a composition or in combination with another agent as disclosed herein. The container(s) optionally have a sterile access port (for example the container can be an intravenous solution bag or a vial having a stopper pierceable by a hypodermic injection needle). Such kits optionally comprise a compound with an identifying description or label or instructions relating to its use in the methods described herein.

A kit will typically comprise one or more additional containers, each with one or more of various materials (such as reagents, optionally in concentrated form, and/or devices) desirable from a commercial and user standpoint for use of a compound described herein. Non-limiting examples of such materials include, but are not limited to, buffers, diluents, filters, needles, syringes; carrier, package, container, vial and/or tube labels listing contents and/or instructions for use, and package inserts with instructions for use. A set of instructions will also typically be included.

A label can be on or associated with the container. A label can be on a container when letters, numbers or other characters forming the label are attached, molded or etched into the container itself; a label can be associated with a container when it is present within a receptacle or carrier that also holds the container, e.g., as a package insert. A label can be used to indicate that the contents are to be used for a specific therapeutic application. The label can also indicate directions for use of the contents, such as in the methods described herein. These other therapeutic agents may be used, for example, in the amounts indicated in the Physicians' Desk Reference (PDR) or as otherwise determined by one of ordinary skill in the art.

### 4. Methods of Making Deuterium-Enriched Compounds

### General Methods for Making Deuterium-Enriched Compounds

The compounds of this invention may be prepared or isolated in general by synthetic and/or semi-synthetic methods known to those skilled in the art for analogous compounds and by methods described in detail herein. Synthesis of certain A-aryl pyridinones of the present invention, including pirfenidone and deuterium-enriched pirfenidone compounds, are described in WO 2008/157786, WO 2009/035598, WO 2012/122165, and WO 2015/112701, the entireties of which are hereby incorporated by reference.

In some embodiments, protecting groups (as defined below) can be used to manipulate therapeutic agents in preparation of the final compound, for example, to prevent undesired side reactions from taking place.

In the synthesis methods described herein, where a particular protecting group ("PG"), leaving group ("LG"), or transformation condition is depicted, one of ordinary skill in the art will appreciate that other protecting groups, leaving groups, and transformation conditions are also suitable and are contemplated. Such groups and transformations are described in detail inMarch's Advanced Organic Chemistry: Reactions, Mechanisms, and Structure, M. B. Smith and J. March, 7th Edition, John Wiley & Sons, 2013, Comprehensive Organic Transformations, R. C. Larock, 3rd Edition, John Wiley & Sons, 2018, and Protective Groups in Organic Synthesis, P. G. M. Wuts, 5th edition, John Wiley & Sons, 2014, the entirety of each of which is hereby incorporated herein by reference.

As used herein, the phrase "leaving group" (LG) includes, but is not limited to, halogens (*e.g.,* fluoride, chloride, bromide, iodide), sulfonates (*e.g.,* mesylate, tosylate, benzenesulfonate, brosylate, nosylate, triflate), diazonium, and the like.

As used herein, the phrase "oxygen protecting group" includes, for example, carbonyl protecting groups, hydroxyl protecting groups, etc. Hydroxyl protecting groups are well known in the art and include those described in detail in Protective Groups in Organic Synthesis, P. G. M. Wuts, 5th edition, John Wiley & Sons, 2014, and Philip Kocienski, in Protecting Groups, Georg Thieme Verlag Stuttgart, New York, 1994, the entireties of which are incorporated herein by reference. Examples of suitable hydroxyl protecting groups include, but are not limited to, esters, allyl ethers, ethers, silyl ethers, alkyl ethers, arylalkyl ethers, and alkoxyalkyl ethers. Examples of such esters include formates, acetates, carbonates, and sulfonates. Specific examples include formate, benzoyl formate, chloroacetate, trifluoroacetate, methoxyacetate, triphenylmethoxyacetate, p-chlorophenoxyacetate, 3-phenylpropionate, 4-oxopentanoate, 4,4-(ethylenedithio)pentanoate, pivaloate (trimethylacetyl), crotonate, 4-methoxy-crotonate, benzoate, p-benzylbenzoate, 2,4,6-trimethylbenzoate, carbonates such as methyl, 9-fluorenylmethyl, ethyl, 2,2,2-trichloroethyl, 2-(trimethylsilyl)ethyl, 2-(phenylsulfonyl)ethyl, vinyl, allyl, and p-nitrobenzyl. Examples of such silyl ethers include trimethylsilyl, triethylsilyl, t-butyldimethylsilyl, t-butyldiphenylsilyl, triisopropylsilyl, and other trialkylsilyl ethers. Alkyl ethers include methyl, benzyl, p-methoxybenzyl, 3,4-dimethoxybenzyl, trityl, t-butyl, allyl, and allyloxycarbonyl ethers or derivatives. Alkoxyalkyl ethers include acetals such as methoxymethyl, methylthiomethyl, (2-methoxyethoxy)methyl, benzyloxymethyl, beta-(trimethylsilyl)ethoxymethyl, and tetrahydropyranyl ethers. Examples of arylalkyl ethers include benzyl, p-methoxybenzyl (MPM), 3,4-dimethoxybenzyl, O-nitrobenzyl, p-nitrobenzyl, p-halobenzyl, 2,6-dichlorobenzyl, p-cyanobenzyl, and 2- and 4-picolyl.

Amino protecting groups are well known in the art and include those described in detail in Protective Groups in Organic Synthesis, P. G. M. Wuts, 5th edition, John Wiley & Sons, 2014, and Philip Kocienski, in Protecting Groups, Georg Thieme Verlag Stuttgart, New York, 1994, the entireties of which are incorporated herein by reference. Suitable amino protecting groups include, but are not limited to, aralkylamines, carbamates, cyclic imides, allyl amines, amides, and the like. Examples of such groups include t-butyloxycarbonyl (Boc), ethyloxycarbonyl, methyloxycarbonyl, trichloroethyloxycarbonyl, allyloxycarbonyl (Alloc), benzyloxocarbonyl (Cbz), allyl, phthalimide, benzyl (Bn), fluorenylmethylcarbonyl (Fmoc), formyl, acetyl, chloroacetyl, dichloroacetyl, trichloroacetyl, phenylacetyl, trifluoroacetyl, benzoyl, and the like.

One of skill in the art will appreciate that various functional groups present in compounds of the invention such as aliphatic groups, alcohols, carboxylic acids, esters, amides, aldehydes, halogens and nitriles can be interconverted by techniques well known in the art including, but not limited to reduction, oxidation, esterification, hydrolysis, partial oxidation, partial reduction, halogenation, dehydration, partial hydration, and hydration. See, for example, March's Advanced Organic Chemistry: Reactions, Mechanisms, and Structure, M. B. Smith and J. March, 7th Edition, John Wiley & Sons, 2013, Comprehensive Organic Transformations, R. C. Larock, 3rd Edition, John Wiley & Sons, 2018, the entirety of each of which is incorporated herein by reference.

Embodiments of the present disclosure can be further defined by reference to the following non-limiting examples. It will be apparent to those skilled in the art that many modifications, both to materials and methods, can be practiced without departing from the scope of the present disclosure.

### EXEMPLIFICATION

Example 1 illustrates the unexpected pharmacokinetic profile of deuterated pirfenidone which provides a significantly reduced pill burden and efficacy at a significantly lower dose, with a significant potential for reducing dose-related side effects and for reduced interpatient variability as compared to pirfenidone. Example 2 provides a dosing and food effect study for deuterated pirfenidone as well as its efficacy in lymphedema. Examples 3, 4, 5, and 6 illustrate the anti-fibrotic and anti-inflammatory efficacy of deuterated pirfenidone.

### Example 1: LYT-100 Increases Systemic Exposure in Humans

LYT-100 was studied in a single dose, double-blinded, cross-over clinical trial of 24 healthy volunteers to assess safety and pharmacokinetics (PK). Following screening, eligible healthy volunteer subjects were admitted to a single clinical study site and were randomized to 1 of 2 treatment sequences. Subjects received a single 801 mg oral dose of either LYT-001 or pirfenidone in Period 1 and, following washout, crossed over to receive the other treatment in period 2. In each period, a standardized breakfast was provided to subjects prior to administration of study drug (to compare the PK profiles in the clinically relevant fed state) and plasma samples were collected over a 48-hour period after dosing for evaluation of PK. Dosing between the 2 periods was separated by a minimum of 7 days. Subjects completed the study upon completion of the 48-hour post-dose assessments following dosing period 2.

To avoid confounding the analysis of results with any influence of formulations, both study drugs (LYT-100 and pirfenidone) were synthesized using the same manufacturing process and were provided as unformulated powder in capsules: LYT-100 801 mg (267 mg capsules x 3); and pirfenidone 801 mg (267 mg capsules x 3).

All capsules were identical in size, shape, and external color. Both the LYT-100 and the pirfenidone used in this trial were provided in hard-shell gelatin capsules containing 267 mg of either LYT-100 or pirfenidone powder with no excipients. The order of the two treatments was assigned via a randomization schema in a 1:1 ratio such that half of the subjects received LYT-100 first and the other half received pirfenidone first.

The plasma concentrations of LYT-100, pirfenidone, and their respective associated metabolites (e.g., 5-carboxy-pirfenidone, 5-hydroxymethyl-pirfenidone, and 4'-hydroxy- pirfenidone) and sample collection times were used for calculation of the following pharmacokinetic parameters for each subject and treatment:

| | |
|---|---|
| Cₘₐₓ | maximum observed plasma concentration, obtained directly from the Plasma concentration time profile. |
| tₘₐₓ | time of the maximum observed plasma concentration, obtained by inspection. If the maximum Plasma concentration occurs at more than one time point, the first is chosen. |
| AUC₀₋ₜ | the area under the plasma concentration versus time curve, from time 0 to the last quantifiable concentration, calculated by the linear trapezoidal method. |
| λ_{z} | apparent first order terminal elimination rate, obtained from the slope of the line, fitted by linear least squares regression, through the terminal points of the log (base e) concentration-time profiles. |
| t_{1/2} | the apparent first-order terminal elimination half-life, calculated by the equation t_{½} = ln(2)/ λ_{z}. |
| AUC_{(inf)} | the area under the Plasma concentration versus time curve from time 0 extrapolated to infinity, by adding Cₜ/λ_{z} to AUC₀₋ₜ, where Cₜ is the last quantifiable concentration. |
| %AUC_{extra p} | the percentage of AUC_{(inf)} obtained by extrapolation, calculated by Cₜ/λ_{z} expressed as a percentage of the total AUC_{(inf)}. |
| CL/F | Oral clearance, calculated as (Dose / AUC_{0→∞}) |
| V_{z}/F | volume of distribution during the terminal phase after oral administration, calculated as (CL/F / λ_{z}) |

**FIG. 1A** and Tables 4-6 summarize the pharmacokinetics over 24 hours of the active LYT-100 (deupirfenidone) and control pirfenidone (LYT-101), their partially active metabolites, deuterated 5-hydroxymethyl-pirfenidone (LYT-110) and nondeuterated 5-hydroxymethyl-pirfenidone (LYT-111), respectively; and common metabolite, nondeuterated 5-carboxy-pirfenidone (LYT-105). **FIG. 1B** is an individual's single dose pharmacokinetics of an 801mg dose of LYT-100 and 801 mg dose of pirfenidone over 48 hours showing LYT-100, pirfenidone and the metabolites of each. The mean Cmax values in Table 4 are different when compared with the maximum concentration observed in **FIG. 1A. FIG. 1A** is a plot of the mean of the concentration values calculated at each (nominal) time point, producing a mean concentration time curve. The mean Cmax values are computed from each individual subjects Cmax value (which could occur at different Tmax times for each individual), i.e., the mean Cmax value reported in the PK parameter table is not the Cmax for a mean concentration-time curve.

**FIG. 1C** is a model of a 500 mg twice daily dose of LYT-100 (total daily dose of 1000 mg) on day 7 based on the clinical trial pharmacokinetics. **FIG. 1D** is a model of a 750 mg twice daily dose of LYT-100 (total daily dose of 1500 mg) on day 7 based on the clinical trial pharmacokinetics. **FIG. 1E** is a model of the first 7 days of the dosing of **FIG. 1D** showing accumulation to steady state. **FIG. 1F** is a model of a 750 mg once daily dose of LYT-100 (total daily dose of 750 mg) on day 7 based on the clinical trial pharmacokinetics.

**Table 4: Summary of Key Pharmacokinetic Parameters for LYT-100 and Pirfenidone**

| **Pharmacokinetic Parameter** | **Statistics** | **Treatment = LYT-100** | **Treatment = Pirfenidone** |
|---|---|---|---|
| | **(n=24)** | **Analyte = LYT-100** | **Analyte = pirfenidone** |
| Cₘₐₓ (ng/mL) | Mean (CV%) | 8835 (27%) | 7100 (25%) |
| tₘₐₓ (hr) | Median, (Range) | 2.25 (1.00 - 4.00) | 2.50 (1.50 - 4.00) |
| AUC₀₋ₜ (hr*ng/mL) | Mean (CV%) | 56639 (46%) | 41091 (42%) |
| AUC_{(inf)} (hr*ng/mL) | Mean (CV%) | 57032 (46%) | 41316 (42%) |
| %AUC extrap | Mean (CV%) | 0.674 (56%) | 0.602 (54%) |
| Kel (1/hr) | Mean (CV%) | 0.274 (35%) | 0.300 (27%) |
| t_{1/2} (hr) | Mean (CV%) | 2.81 (31%) | 2.48 (29%) |
| CL/P (L/hr) | Mean (CV%) | 17.8 (53%) | 23.5 (48%) |
| V_{z}/F( L) | Mean (CV%) | 63.1 (31%) | 76.5 (31%) |

**Table 5: Summary of Key Pharmacokinetic Parameters for Partially Active Metabolites: deuterated 5-hydroxymethyl-pirfenidone (LYT-110) and nondeuterated 5-hydroxymethyl-pirfenidone (LYT-111)**

| **Pharmacokinetic Parameter** | **Statistics** | **Treatment = LYT-100** | **Treatment = Pirfenidone** |
|---|---|---|---|
| | **(n=24)** | **Analyte = LYT-110** | **Analyte = LYT-111** |
| Cₘₐₓ (ng/mL) | Mean (CV%) | 20.6 (30%) | 17.1 (36%) |
| tₘₐₓ (hr) | Median, (Range) | 2.50 (1.00 - 4.00) | 2.50 (1.50 - 4.00) |
| AUC₀₋ₜ (hr*ng/mL) | Mean (CV%) | 111 (32%) | 75.8 (42%) |
| AUC_{(inf)} (hr*ng/mL) | Mean (CV%) | 124 (32%)^{a} | 89.7 (41%) ^{b} |
| %AUC extrap | Mean (CV%) | 10.1 (35%)^{a} | 10.3 (33%) ^{b} |
| Kel (1/hr) | Mean (CV%) | 0.294 (44%) ^{a} | 0.367 (29%) ^{b} |
| t_{1/2} (hr) | Mean (CV%) | 2.76 (38%)^{a} | 2.04 (29%) ^{b} |

| | | | |
|---|---|---|---|
| Key: a:n = 23, b:n = 19 | | | |

**Table 6: Summary of Key Pharmacokinetic Parameters for 5-carboxy-pirfenidone (LYT-105).**

| **Pharmacokinetic Parameter** | **Statistics** | **Treatment = LYT-100** | **Treatment = Pirfenidone** |
|---|---|---|---|
| | **(n=24)** | **Analyte = LYT-105** | **Analyte = LYT-105** |
| Cₘₐₓ (ng/mL) | Mean (CV%) | 3970 (32%) | 5241 (31%) |
| tₘₐₓ (hr) | Median, (Range) | 2.50 (1.50 - 4.00) | 3.00 (1.50 - 4.00) |
| AUC₀₋ₜ (hr*ng/mL) | Mean (CV%) | 23090 (19%) | 26932 (19%) |
| AUC(inf) (hr*ng/mL) | Mean (CV%) | 23350 (19%) | 27159 (19%) |
| %AUC extrap | Mean (CV%) | 1.13 (63%) | 0.843 (58%) |
| Kel (1/hr) | Mean (CV%) | 0.262 (33%) | 0.288 (24%) |
| t_{1/2} (hr) | Mean (CV%) | 2.91 (30%) | 2.55 (25%) |

It was observed that the systemic exposure of LYT-100 was about 35% greater than for pirfenidone, and about 25% greater for Cmax, with no appreciable difference in the apparent elimination half-life.

The increased systemic exposure to LYT-100 was accompanied by changes in the relative abundance of downstream metabolites. Following LYT-100 and pirfenidone, the most abundant measured circulating metabolite was 5-carboxy-pirfenidone (LYT-105). 5-carboxy-pirfenidone was reduced after LYT-100 relative to pirfenidone by approximately 15% and 25% for AUC and Cmax, respectively. As a percent of the parent analyte AUC_{0-∞}, 5-carboxy-pirfenidone represented 43.8% for LYT-100 as compared to 65.9% for pirfenidone (Table 7). The remaining measured metabolites, 5-hydroxymethyl-pirfenidone (LYT-111) and 4'-hydroxy-pirfenidone (LYT-104), were far less abundant, representing less than 2% of parent in terms of AUC. The formation of the metabolite 5-hydroxymethyl-pirfenidone was approximately 50% greater in terms of overall systemic exposure (AUC) after administration of LYT-100. Similarly, 4'-hydroxy-pirfenidone was detectable more frequently after LYT-100 than after pirfenidone. Given the low plasma concentrations of these metabolites, however, these changes contributed little to the overall pharmacokinetic profile of LYT-100 relative to pirfenidone.

**Table 7: Summary of Metabolite/Parent Ratio, Overall**

| **Pharmacokinetic Parameter** | **Statistic** | **Metabolite/Parent Ratio^{a}** | | | | | |
|---|---|---|---|---|---|---|---|
| | | **Pirfenidone (LYT-101)** | | | **LYT-100** | | |
| | | **LYT-105/ LYT-101** | **LYT-111/ LYT-101** | **LYT-104/ LYT-101** | **LYT-105/ LYT-100** | **LYT-110/ LYT-100** | **LYT-103/ LYT-100** |
| Cmax (ng/mL) | N | 24 | 24 | 24 | 24 | 24 | 24 |
| | Mean | 68.1% | 0.2% | 0.0% | 43.3% | 0.2% | 0.1% |
| | Std Dev | 29.0% | 0.1% | 0.0% | 22.2% | 0.1% | 0.0% |
| | CV(%) | 42.6% | 37.5% | 94.5% | 51.2% | 39.1% | 28.0% |
| | Median | 57.5% | 0.2% | 0.0% | 33.5% | 0.2% | 0.1% |
| | Minimum | 36.7% | 0.1% | 0.0% | 22.5% | 0.1% | 0.1% |
| | Maximum | 128.6% | 0.4% | 0.1% | 103.3% | 0.4% | 0.2% |
| AUC_{0-∞} (hr*ng/mL) | N | 24 | 19 | 0 | 24 | 23 | 10 |
| | Mean | 65.9% | 0.2% | - | 43.8% | 0.2% | 0.1% |
| | Std Dev | 28.1% | 0.1% | - | 22.4% | 0.1% | 0.0% |
| | CV(%) | 42.7% | 35.8% | - | 51.2% | 38.0% | 33.8% |
| | Median | 53.7% | 0.2% | - | 32.3% | 0.2% | 0.1% |
| | Minimum | 35.4% | 0.1% | - | 21.2% | 0.1% | 0.1% |
| | Maximum | 128.9% | 0.4% | - | 101.9% | 0.4% | 0.2% |

| | | | | | | | |
|---|---|---|---|---|---|---|---|
| ^{a} The analytes were pirfenidone (LYT-101), nondeuterated 5-hydroxymethyl-pirfenidone (LYT-111), nondeuterated 5-carboxy-pirfenidone (LYT-105), nondeuterated 4'-hydroxy-pirfenidone (LYT-104), LYT-100 (deupirfenidone), deuterated 5-hydroxymethyl-pirfenidone (LYT-110), and deuterated 4'-hydroxy-pirfenidone (LYT-103). AUC_{0-∞}=area under the plasma concentration versus time curve from zero to infinity; Cmax=maximum observed plasma concentration; CV=coefficient of variation; hr=hour; mL=milliliter; MW=molecular weight; N=number; ng=nanogram. Metabolite/Parent Ratio Formula = Parameter (Metabolite)/Parameter (Parent) * MW (Parent)/MW (Metabolite) Pharmacokinetic parameters determined using Phoenix WinNonlin v6.3 (Certara) | | | | | | | |

On average, after administration of LYT-100, the 5-carboxypirfenidone metabolite (LYT-105) represented 43.8% of the parent in comparison to 65.9% of the parent after administration of pirfenidone. This difference in exposure was not associated with a change in half-life, suggesting formation, and not clearance of this non-deuterated metabolite is affected by the deuterium substitution in the parent molecule.

Administration in the fed state of a single 801 mg dose of LYT-100 resulted in overall greater exposure (AUC, Cmax) than observed with administration of an 801 mg dose of pirfenidone. No appreciable difference in the apparent elimination t_{½} or time to Cmax was observed for the 2 compounds. The higher peak and overall exposure of LYT-100 was associated with a lower systemic exposure of the 5-carboxy-pirfenidone, suggesting the kinetic isotope effect at least partially protects against pre-systemic conversion of pirfenidone into 5-carboxy-pirfenidone.

The deuterium kinetic isotope effect appears independent of phenotype when comparing exposure between deuterated and non-deuterated pirfenidone. CYP1A2 has been reported as the main metabolizing enzyme for pirfenidone and higher enzyme activity in the hyperinduced CYP1A2 phenotype is associated with lower exposure of both deuterated and non-deuterated forms of pirfenidone relative to normal expression levels.

Overall, single doses of LYT-100 and pirfenidone were well tolerated and have a comparable safety profile. No clinically significant differences were observed between the 2 treatments in terms of type, severity, or frequency of treatment emergent adverse events. The most common adverse event following either treatment was headache. Of interest, although administration of the 801 mg dose of LYT-100 resulted in greater drug exposure than with the same pirfenidone dose, the incidence of gastrointestinal and nervous system adverse events was not increased with LYT-100 administration as compared to pirfenidone. No significant changes in laboratory parameters, vital signs, or ECGs were observed following either treatment.

### Example 2: L YT-100 Dosing and Food Effect Study, and Efficacy in Lymphedema

A multiple dose and food effect study of LYT-100 in healthy volunteers, and testing for efficacy in patients with breast cancer-related upper limb secondary lymphedema is being conducted.

The overall double-blind, placebo-controlled study design for Part 1 and Part 2: evaluates the safety and tolerability of multiple doses of immediate-release LYT-100 versus placebo; compares the pharmacokinetic profiles of multiple doses of immediate-release LYT-100 versus placebo over 5-days; compares the pharmacokinetic profiles of a maximally tolerated dose (MTD); and evaluates the maximally tolerated dose and regimen.

The overall open-label design for Part 3 evaluates the effect of the doses of LYT-100 in patients with secondary lymphedema post-axial node dissection over a 24-week treatment period. To further assess the safety, tolerability and pharmacokinetic profile of LYT-100 over a 6-month dosing period, and study biomarkers and endpoints of disease and progression in mild to moderate lymphedema in breast cancer-related lymphedema patients taking LYT-100 are evaluated.

In Part 1, LYT-100 is dosed twice per day for 5 days following a 10 hour fast. The first dose is administered 30 minutes into a standardized breakfast, followed by a standardized lunch 4 hours later. The second dose is given 10 hours following the morning dose and 30 minutes into a standardized dinner. Up to three different doses of Immediate Release (IR) LYT-100 capsules ranging from 250 mg twice per day (BID) up to 750 mg BID are studied. The study will start at a medium dose, e.g., 500 mg BID and adjust to either lower or higher doses, with no dose exceeding 750 mg BID.

In Part 2, the maximally tolerated dose achieved in Part 1 is repeated for one day with LYT-100 administered following a fasted single morning dose and single evening dose administered 10 hours post-morning dose and on an empty stomach in healthy volunteers.

In Part 3, LYT-100 is dosed using the dose determined in Part 1 and 2, and is taken twice per day as instructed for 24-weeks. Part 3 patients are males and females having had breast cancer surgery at least 3 months prior, and who have completed radiation treatment due to breast cancer at least one month prior. They are without recurrent cancer ≥ 6 months after the breast cancer surgery. Patients are those having pitting edema and **at least one** of the following: increase in relative limb volume of between 10-20% as measured by the truncated cone method of circumferential tape measurement, or a bioimpedance measure of > +6.5 L-Dex. Patients are also on standard of care compression or agree to use compression ≥ 4 weeks prior to screening if relative limb volume > 10% or L-Dex > 14.

| **Table 8: Cohorts and Number of Subjects per Treatment Arm** | | | | |
|---|---|---|---|---|
| **Cohort** | **PART 1 ^{A}** (Healthy Volunteers) | **Placebo** | **LYT-100** | **LYT-100 dose** |
| **1** | **Multiple Dose (n=8)** | 2 subjects | 6 subjects | 500 mg, BID with food |
| **2** | **Multiple Dose (n=8)** | 2 subjects | 6 subjects | ≤ 750 mg, BID* with food |
| **3** | **Multiple Dose (n=8)** | 2 subjects ^{A} | 6 subjects ^{A} | ≤ 750 mg, BID* with food |
| | | | | |

| **Cohort** | **PART 2** ^{A} (Healthy Volunteers) | **Placebo** | **LYT-100** | **LYT-100 dose** |
|---|---|---|---|---|
| **4** | **Food Effect Cohort 4 (n=8)** | 2 subjects ^{A} (7-day washout minimum between Cohort 3 and 4) | 6 subjects ^{A} (7-day washout minimum between Cohort 3 and 4) | ≤ 750 mg, BID**on an empty stomach |
| | | | | |

| **Cohort** | **PART 3** ^{A} (Patients with Secondary Lymphedema) | **Placebo** | **LYT-100** | **LYT-100 dose** |
|---|---|---|---|---|
| **5** | **Patient Cohort (n=32)** | 0 subjects (will use historical data as reference) | 32 subjects | ≤ 750 mg, BID** |

| | | | | |
|---|---|---|---|---|
| ^{A} Subjects in the last cohort achieving the maximally tolerated dose of the multiple dose study (e.g., Cohort 3) will return following a 7-day washout minimum from Part 1 dosing with meals and will return for Part 2 dosing fasting or on an empty stomach (Cohort 4). * Adjustment of dosing after prior cohort will be to either a lower or higher dose(s), with no dose exceeding 750 mg BID (e.g., 250 mg, BID or 750 mg BID, respectively) ** A optimally tolerated dose of (IR) LYT-100 capsules, will be taken twice per day, and not to exceed 750 mg BID. | | | | |

### Part 3 Criteria for Evaluation

Fibrotic and inflammatory biomarkers. Serum biomarkers G-CSF, MIG, FGF-2, IL-4, IL-10, lymphotoxin-α/TNF-β, leptin, IL-6, IL-1β, TNF-α, TGF-β1, MMP-9, TIMP-1, and MCP-1 are evaluated.

Bioimpedance, or water content measured via Bioelectrical impedance spectroscopy (BIS). Multiple frequency bioelectrical impedance spectroscopy (BIS) provides accurate relative measures of protein-rich fluid in the upper limb of patients. BIS is a noninvasive technique that involves passing an extremely small electrical current through the body and measuring the impedance (or resistance) to the flow of this current. The electrical current is primarily conducted by the water containing fluids in the body. BIS quantifies the amount of protein-rich fluid in lymphedema by comparison of the affected and non-affected limbs.

Limb Volume (Perometry). Relative limb volume is measured by the truncated cone method of circumferential tape measurement. Perometry is a noninvasive technique involving a Perometer (Pero-System), which uses infrared light to scan a limb and obtain measurements of the limb's circumference.

Tissue Dielectric Constant (MoistureMeterD). The tissue dielectric constant measures the local tissue water content under the skin at various depths ranging from skin to subcutis. The results are converted into a 0-100% scale to reflect subcutaneous fluid deposition that can occur in early stage lymphedema.

Tissue Firmness (Tonometry/SkinFibroMeter). A tonometer device is pressed into the skin to measure the amount of force required to make an indent in the tissue. The resulting measurement gauges the degree of firmness or fibrosis (tissue scarring) under the skin to assess the severity of lymphedema. (r) at dorsal surface of arm 10cm below the elbow.

Visual-analogue scales for pain, swelling, discomfort, and function. This graphic scale has a straight line with endpoints from 0 to 10 that is marked by the patient to correlate to their extreme limits of pain, swelling, discomfort and function, ranging from "not at all" to "as bad as it could be." The higher marks on the line indicates the worse condition.

Upper Limb Lymphedema Score 27 (ULL27) is a self-report tool consisting of 27 questions to evaluate arm lymphedema and associated symptoms in breast cancer survivors. Responses are given on a 5-pount Likert scale ranging from "never" to "always." Five domains are addressed: physical (15 items), psychological (7 items) and social (5 items), with scores ranging from 0 to 100 (100 being the best score possible). Lower scores indicate a higher quality of life

Lymphedema Life Impact Scale (LLIS) is a comprehensive lymphedema-specific instrument to measure impairments, activity limitations, and participation restrictions in patients with any extremity lymphedema. It is an 18-question assessment tool that includes physical, psychosocial, and functional domains. The Scale is designed to work in conjunction with an impairment calculator to determine the impairment severity.

The protein-rich interstitial fluid accumulation in lymphedema leads to inflammation and an accumulation of fibroblasts, adipocytes, and keratinocytes that transform the initially soft swollen tissue into a hard fibrotic tissue with stiff, thickened skin. Fibrosis is a scarring process, which is characterized by excess deposition of collageneous and non-collagenous extracellular matrix (ECM) due to the accumulation, proliferation, and activation of fibroblasts and myofibroblasts.

Fibroblasts are the main cells that produce, maintain, and reabsorb extracellular matrix (ECM) (reviewed in Kendall and Feghali-Bostwick, Front. Pharmacol., 27 May 2014). Fibroblasts produce the structural proteins of the ECM, expressing different ECMs in different tissues requiring differing degrees of rigidity and flexibility; e.g., fibril rigidity is provided by collagen type I, while expansive stretching ability is provided by elastin proteins. As the major producers of ECM, fibroblasts are also the central mediators of the pathological fibrotic accumulation of ECM and of the cellular proliferation and differentiation that occurs in response to prolonged tissue injury and chronic inflammation in multiple fibrotic diseases including lymphedema.

During initiation and progression of fibrotic disease, such as lymphedema, fibroblasts become activated by inflammatory cytokines and differentiate into myofibroblasts that are characterized by up-regulated cellular migration and a contractile apparatus. Myofibroblasts also display exaggerated ECM production, with increase in the relative production of collagen type I, which stimulates increased chemical signaling secretion and signaling responsiveness. The response is amplified, i.e., cytokines, such as TGFβ1, provide further myofibroblast activation, promoting further collagen deposition, and so forth.

Fibroblasts and myofibroblasts also produce adhesive proteins such as fibronectin and laminin, which form the connection between cells and the ECM and are essential for collagen assembly into ECM. During fibrosis, aberrant fibronectin-matrix assembly is a major contributing factor to the switch from normal tissue repair to dysregulated fibrosis. Although collagen is the most predominant ECM component of fibrotic tissue, excessive deposition of fibronectin also occurs, and precedes the collagen deposition (To and Midwood, Fibrogenesis Tissue Repair. 2011; 4: 21, and references therein). For example, in glomerular and interstitial fibrosis, a significantly elevated expression of total fibronectin is observed, with increased levels of EIIIA+, EIIIB+ and oncofetal (IIICS+) isoforms detected in specific areas of the kidney and in areas of fibrosis.

In addition to ECM deposition, fibroblasts also serve as key players of the immune system with active roles in inflammation and immune cell recruitment (reviewed in Linthout et al., Cardiovascular Research, Volume 102, Issue 2, 1 May 2014, Pages 258-269). On the one hand, fibroblasts drive homing of circulating leucocytes via the release of chemokines, promote the recruitment of circulating leucocytes, and aid retention and survival of immune cells in fibrotic tissue. On the other hand, fibroblasts are activated by components of the innate and adaptive immunity; i.e., they are stimulated chemically by inflammatory agents to differentiate into myofibroblasts with up-regulated rates of matrix production. In other words, fibroblasts can contribute to chronic inflammation, and reciprocally, inflammatory cytokines can promote fibroblast to myofibroblast transition, facilitating fibrosis.

### Example 3: LYT-100 Significantly Reduced Area of Fibrosis in Mouse Model

Non-alcoholic steatohepatitis (NASH) is characterized by lobular inflammation, hepatocyte ballooning and degeneration progressing to liver fibrosis. LYT-100 was orally administered at 0 mL/kg (Vehicle only: 0.5% CMC) or 10 mL/kg twice daily from 6-9 weeks of age in 18 male mice in which NASH mice was induced by a single subcutaneous injection of 200 µg streptozotocin solution 2 days after birth and feet with a high fat diet after 4 weeks of age. LYT-100 was administered at an oral dose of 30 mg/kg twice daily (60 mg/kg/day). In addition, nine non-NASH mice were fed with a normal diet and monitored.

**FIG. 2** depicts representative micrographs of Sirius-red stained liver sections illustrating that LYT-100 significantly reduced the area of fibrosis. Specifically, liver sections from the vehicle group exhibited collagen deposition in the pericentral region of the liver lobule. And the LYT-100 group showed a significant reduction in the fibrosis area compared to the vehicle group. These results demonstrate that LYT-100 has a potential to inhibit the progression of fibrosis. **FIG. 3** illustrates the percent fibrosis area for LYT-100 versus vehicle and control. The results are also summarized **Table 9** below.

**Table 9: Fibrosis Area**

| | | | |
|---|---|---|---|
| Parameter (mean ± SD) Fibrosis Area (%) | Normal (n=9) 0.27 ± 0.06 | Vehicle (n=7) 1.02 ± 0.20 | LYT-100 (n=8) 0.64 ± 0.31* |

| | | | |
|---|---|---|---|
| *p<0.01, Vehicle vs LYT-100 | | | |

Liver sections from the Vehicle group exhibited severe micro- and macro vesicular fat deposition, hepatocellular ballooning and inflammatory cell infiltration. While LYT-100 hepatocyte ballooning was similar to Vehicle, scores were lower for lobular inflammation and steatosis. **(Table 10).**

**Table 10: NAFLD Activity Score**

| Group | n | Score | | | | | | | | | | | NAS (mean ± SD) |
|---|---|---|---|---|---|---|---|---|---|---|---|---|---|
| | | Steatosis | | | | Lobular Inflammation | | | | Hepatocyte ballooning | | | |
| | | 0 | 1 | 2 | 3 | 0 | 1 | 2 | 3 | 0 | 1 | 2 | |
| Normal | 9 | 9 | - | - | - | 9 | - | - | - | 9 | - | - | 0.0 ± 0.0 |
| Vehicle | 7 | 2 | 5 | - | - | - | 2 | 1 | 4 | - | 1 | 6 | 4.9 ± 1.2 |
| LYT-100 | 8 | 4 | 3 | 1 | - | - | 5 | 3 | - | - | - | 8 | 4.0 ± 1.1 |

### Definition of NAS Components

| **Item** | **Score** | **Extent** |
|---|---|---|
| Steatosis | | |
| | 0 | <5% |
| | 1 | 5-33% |
| | 2 | >33%-66% |
| | 3 | >66% |
| Hepatocyte Ballooning | 0 | None |
| | 1 | Few balloon cells |
| | 2 | Many cells/prominent ballooning |
| | 0 | No foci |
| Lobular Inflammation | 1 | <2 foci/200x |
| | 2 | 2-4 foci/200x |
| | 3 | >4 foci/200x |

As evidenced above, LYT-100 significantly reduced the area of fibrosis, reduced inflammation, and reduced accumulation of fat (steatosis), as compared to the untreated NASH mice.

### Example 4: LYT-100 Reduction of TGF-β-induced proliferation and collagen levels in Primary Mouse Lung Fibroblasts

LYT-100 was evaluated for an ability to reduce the TGF-β-induced proliferation of, and collagen levels in, Primary Mouse Lung Fibroblasts (PMLF).

Inhibition of p38 members by LYT-100 is important as p38 members are activated by TGF-β signaling pathway. TGF-β activation, in turn plays a significant role in transcriptional induction of the collagen type IA2. The collagen type IA2 makes up the majority of extracellular matrix, which accumulates during progression of, e.g., IPF. Deposition of collagen is one of the most important components of fibrotic lung tissue, a process primarily induced by TGF-β. Since accumulation of insoluble collagen encroaches on the alveolar space, it plays pivotal role in distortion of lung architecture and progression of IPF. Therefore, inhibition of TGF-β -induced collagen synthesis is an important target for IPF. In addition to insoluble (structural) collagen, fibrotic lungs of IPF patients also show high levels of non-structural (soluble) collagen.

Although this type of collagen may eventually become insoluble collagen, until then, soluble collagen can serve as a ligand for integrin receptors of lung fibroblasts and epithelial cells. Binding of soluble collagen to these receptors induces proliferation and migration of these cells. Fibronectin is another important component of fibrotic lungs as it is induced by TGF-β and functions both as a structural component of extra cellular matrix (ECM), as well as a ligand for integrin receptors. Just like soluble collagen, binding of fibronectin to integrin receptors induces the proliferation of fibroblast and epithelial cells of the lungs and plays significant role in progression of IPF.

### Preparation of Primary Mouse Lung Fibroblast

Primary Mouse lung fibroblast were prepared as follows. One lung was removed from 2 months old male BalbC Mouse, perfused with sterile PBS, minced and incubated in 2 ml of serum free Dulbecco's Modified Eagle's Medium (DMEM) containing 100 µg/ml of collagenase I for one hour at 37°C. Each sample was centrifuged at 1500 r.p.m (revolution per minute) for 5 minutes, washed three times with PBS and the final cellular pellet was resuspended in DMEM supplemented with 10% serum and Pen/Strep, and incubated in 150 mm plates at 37ₒC with 80% humidity and %% CO₂. The growth medium was removed and fresh medium was added every day for 10 days.

### Testing the effect of LYT-100 on Survival of Primary Mouse Lung Fibroblast

LYT-100 was evaluated for an ability to alter TGF-β-induced proliferation of PMLF. At the end of 10-day incubation period above, lung fibroblasts were confluent. Before testing the effect of LYT-100 on survival of these cells, fibroblasts were tripsinized and five thousand cells were plated into 96 well plate in 200 µL complete DMEM, and incubated until cells reached to 95-100% confluency, then the medium was removed and complete DMEM containing Prolin (10 µM) and Ascorbic acid (20 µg/ml) was added. LYT-100, dissolved in pure ethanol, was added to the plates at a final concentration of 500 µM 1 h prior to addition of TGF-β (5ng/ml), and cells were further incubated for 72 hrs. One hundred µL of the growth medium was removed and 20 µL of MTT stock solution (prepared in PBS at 5.5 mg/ml concentration) was added and cells were incubated for 4 hrs, then 100 µl of DMSO was added, and absorbance of developed color was monitored at 540-690 nm.

As shown at **FIG. 4A****,** LYT-100 did not affect the survival of PMLF alone. TGF-β (5 ng/ml) significantly induced the proliferation of PMLF by nearly 45% (p=0.001), and LYT-100 did appear to diminish TGF-β-induced proliferation of PMLF by 10%, but this effect was not statistically significant (p=0.19).

### TGF-β-induced Insoluble Collagen Synthesis using 6-well plate format

The effect of LYT-100 on inhibition of TGF-β-induced collagen synthesis was evaluated in PMLF in a 6-well format. One hundred thousand Primary Mouse Lung Fibroblasts were plated in 6-well plates and incubated in complete DMEM until they reached confluency. The incubation medium was removed and complete DMEM containing Prolin (10 µM) and Ascorbic acid (20 µg/ml) was added. LYT-100 was added to the plates at a final concentration of 500µM 1 h prior addition of TGF-β (5ng/ml), and cells were further incubated for 72 hrs.

Supernatant was removed, cells were washed with cold PBS, 1 ml Sircol reagent was added. The Sircol reagent contains the collagen binding dye Sirius red. The cells were scraped off with Sircol reagent and samples were shaken for 5 h at room temperature (RT), centrifuged at 10,000 rpm for 5 min, supernatant was removed, the pellet was washed in 0.5 M acetic acid to remove unbound dye, and recentrifuged at 10,000 rpm for 5 min, supernatant was removed and the final pellet was dissolved in 1 ml 0.5M NaOH and shaken at RT for 5 h. A sample of 100 µl of resultant solution was placed in 96-well. The color reaction was assessed by optical density at a wave length of 600 nm.

As shown in **FIG. 4B****,** PMLF responded to TGF-β with increased total collagen levels, (increase of 21%; p=0.0087). LYT-100 inhibited this induction by 15% (p=0.026), as compared to the TGF-β alone, without reducing the background level of collagen.

### TGF-β-induced Insoluble Collagen Synthesis using 96-well plate format

The effect of LYT-100 on TGF-β-induced collagen was confirmed in a high throughput collagen assay using 96-well plate format. Approximately 5,000 primary mouse fibroblasts were plated in complete DMEM in 96 well plates and incubated for 3 days at which time the cultures achieved confluency. After cells reached confluency, the medium was removed and fresh DMEM supplemented with ascorbic acid (20 µg /ml) and prolin (10 µMol) was added. LYT-100 was then added to the appropriate cultures at a final incubation concentration of 500 µM. One hour later, TGF-β was added to the appropriate cultures at a final concentration of 5 ng/ml. After 72 hours, the media was replaced with a 0.5% glutaraldehyde solution. After 30 minutes, the adherent cells were washed and subsequently incubated with acetic acid at a final concentration of 0.5M. After a 30 min room temperature incubation, and subsequent washing steps, the wells were incubated with Sircol reagent. After 5 hours, the unbound dye was removed and the plates were washed and allowed to dry. To extract collagen-bound Sircol, 100 µL of alkaline solution (0.5M NaOH) was added and plates were shaken for 1 h on rotary shaker at room temperature. Absorbance at 600 nm was determined to detect bound collagen.

As shown in **FIG. 4C****,** in the 6-well format, TGF-β induced insoluble collagen level by 40% (p=0.0002), LYT-100 diminished this TGF-β-stimulated collagen accumulation by 24% (p=0.0003) without reducing the background level of collagen.

### TGF-β-induced Soluble Fibronectin and Collagen Synthesis

LYT-100 was evaluated for its ability to modify TGF-β-induced soluble fibronectin and soluble collagen synthesis using a selective ELISA. Approximately 5,000 primary mouse lung fibroblasts were plated in complete DMEM in 96 well plates and incubated for 3 days at which time the cultures achieved confluency. After cells reached to confluency, medium was removed and fresh DMEM supplemented with ascorbic acid (20 µg /ml) and prolin (10 µM) was added. LYT-100 was then added to the appropriate cultures at a final incubation concentration of 500 µM. One hour later, TGF-β (5 ng/ml) was added to the appropriate cultures at a final concentration. After 72 hours, 200 µl samples of the supernatant were placed onto an ELISA plate and incubated overnight. After blocking with %1 BSA for 2 h, plates were incubated with either an anti-collagen type I antibody or an anti-fibronectin antibody.

The plates were washed after 1 hour and incubated with secondary horseradish peroxidase-conjugated antibodies (anti-goat for the collagen antibody, anti-rabbit for the fibronectin antibody). After a series of washing steps the color reagent TMB (3,3',5,5'-Tetramethylbenzidine) was added and 15 minutes later the reactions were terminated with equal volumes of 2 N H2SO4. The levels of soluble collagen and fibronectin were determined by evaluating absorbance at 450 nm.

Referring to **FIG. 4D****,** TGF-β induced the level of soluble fibronectin by 16% (p=0.0021). LYT-100 inhibited TGF-β-dependent induction of fibronectin by 11% (p=0.0185). Moreover, LYT-100 also inhibited the background level of soluble fibronectin by 10% (p=0.03).

As shown in **FIG. 4E****,** TGF-β induced the level of soluble collagen by 20% (p=0.0185). LYT-100 inhibited this TGF-β-dependent increase by 36% (p=0.0001). Moreover, it also inhibited background level of soluble collagen by 23% (p=0.0115).

In summary, LYT-100 was found to: (i) reduce TGF-β-induced cell proliferation, (ii) reduce both background and TGF-β-induced levels of insoluble (structural) collagen; (iii) reduce both background and TGF-β-induced levels of soluble collagen; and (iv) reduce both background and TGF-β-induced levels of soluble fibronectin.

During the progression of IPF, an accumulation of extra cellular matrix components such as collagen and an increase in the fibroblast population is observed. Persistent proliferation of fibroblasts is considered an important contributor to the lung architecture in IPF, including the diminished interstitial spaces of the alveoli. Thus, reducing TGF-β-induced proliferation of fibroblasts and structural collagen with LYT-100 has the potential to prolong lung function in IPF. In addition to inhibiting TGF-β-induced insoluble collagen level, LYT-100 also inhibits TGF-β-induced secreted collagen and fibronectin β. Secreted collagen and fibronectin not only increase the rate of formation of fibrotic foci in the lung, these proteins can also act as ligands for integrin receptors. When integrin receptors are activated they induce not only the proliferation of epithelial cells and fibroblasts of the lungs, but they also, along with TGF-β, induce epithelial mesenchymal transition (EMT) of the epithelial cells of the lungs. EMT causes these cells to migrate to different regions of the lungs. This migration is considered to be a very important contributor for the generation of new fibrotic foci in the lungs and progression of IPF.

LYT-100 has the ability to inhibit TGF-β-induced pro-fibrotic processes and to reduce basal factors, which have the potential to exacerbate ongoing fibrosis.

### Example 5: Effect of LYT-100 on L929 Cells

The effect of LYT-100 on survival of L929 Cells was determined. Five thousand L929 cells were plated in completed DMEN and incubated until confluency for 3 days. Medium was removed and complete DMEM containg Prolin (20 µg/ml) and ascorbic acid (10 uM) was added. LYT-100 was given at 500µM 1 h prior addition of TGFb (5ng/ml), and cells were further incubated for 72 hrs. 100µL of medium was removed, 20µL MTT solution was added for 4 hrs, then 100 µl of DMSO was added, and absorbance of developed dark pink color was determined at 54-690 nM. **FIG. 5A** illustrates that LYT-100 does not affect survival of L929 cells.

The effect of LYT-100 on TGF-induced collagen synthesis in 6-wells was determined. 100,000 L929 cells were plated in complete DMEN and incubated until confluency for 3 days. Medium was removed and complete DMEM containg Prolin (20 µg/ml) and ascorbic acid (10 µM) was added. LYT-100 was given at 500µM 1 hour prior addition of TGF-(3 (5ng/ml). Cells were further incubated for 72 hrs. Supernatant was removed, cells were washed with cold PBS, 1 ml SIRCOL reagent was added onto the cells and cells were scraped off, samples were shaken for 5 h.at RT, centrifuged at 10.000 rpm for 5 min, supernatant was removed, pellet was dissolved in 0.5 M acetic acid to remove unbound dye, and re-centrifuged at 10.000 rpm for 5 min, supernatant was removed and final pelet was dissolved in 1 ml 0.5M NaOH, shaken at RT for 5 h, 100 µl of resulted solution was placed in 96-well and O.D was determined at 600. The results are summarized in **FIG. 5B****,** which illustrates that LYT-100 inhibits TGF-induced collagen synthesis. LYT-100 also significantly inhibits collagen synthesis in the absence of added TGF-β.

Next, the effect of LYT-100 onTGF-induced collagen synthesis was confimed using 96-well plate format. Five thousand L929 cells were plated in complete DMEN and incubated until confluency for 3 days. Medium was removed and complete DMEM containg Prolin (20 µg/ml) and ascorbic acid (10 µM) was added. LYT-100 was given at 500µM 1 h prior addition of TGF-β (5ng/ml). Cells were further incubated for 72 hrs. Supernatant was removed, 0.5% gluteraldehyde was added for 30 min at RT, removed, washed 3X with ddwater, 0.5 M acetic acid was added for 30 min at RT, removed, washed with water, air dried and 100 µl SIRCOL dye was added for 5 h at RT. Dye was removed, plate was washed extensively under running water, air dried and 200 µl of 0.5 M NaOH was added, plates were shaken at RT for 1 h, and OD was determined at 600 nm. The results summarized in **FIG. 5C** illustrate that LYT-100 significanly inhibited or reduced TGF-β-induced total collagen levels. LYT-100 also signficantly inhibited or reduced total collagen level in the absence of TGF-β induction.

The effect of LYT-100 on TGF-induced Soluble Collagen Synthesis was determined using a 96-well plate format. Five thousand L929 cells were plated in complete DMEN and incubated until confluency for 3 days. Medium was removed and complete DMEM containing Prolin (20 µg/ml) and ascorbic acid (10 µM) was added. LYT-100 was given at 500µM 1 h prior addition of TGF-β (5ng/ml). Cells were further incubated for 72 hrs. 200 µl supernatant of 96-well SIRCOL plate was placed onto ELISA plate and incubated O/N. Next day, supernatant was removed and 100 ul of 1%BSA in PBST was added and incubated for 2 h at RT, BSA was removed, plate was washed 3x with 200 µl of PBST, and anti-collagen type I a.b was added at 1 :2000 dilution (prepared in %1 BSA in PBST), incubated at RT for 1 h, primary a.b was removed, plate was washed 3x with 200 µl PBST, and secondary anti-goat HRP was added at 1:2000 dilution, incubate at RT for 1 h, removed, plate was washed 3x with 200 µl PBST and 100 µl of TMB solution was added for color development for 15 min, then 100 µl of 2 N H2SO4 was added to stop the reaction and O.D of developed yellow color was determined at 450 nm.

As illustrated in **FIG. 5D****,** LYT-100 significantly inhibits TGF-β-induced soluble collagen levels. LYT-100 also signficantly reduced soluble collagen levels in the absence of TGF-β-induction.

Fibronectin is another important component of fibrotic lungs as it is induced by TGF-β and functions both as a structural component of extra cellular matrix as well as well as a ligand for integrin receptors. Just like soluble collagen, binding of fibronectin to integrin receptors induces the proliferation of fibroblast and epithelial cells of the lungs. The effect of LYT-100 of TGF-induced soluble fibronectin synthesis was determined using a process similar to that described in the above paragraph for soluble collagen synthesis except that a fibronectin ELISA was used. As illustrated in **FIG. 5E****,** LYT-100 signficantly reduced soluble fibronectin levels, in the absence and presence of TGF-β-induction.

### Example 6: LYT-100 Study in Mouse Model of Lymphedema

This experiment tests the effect of LYT-100 and LYT-101 (pirfenidone) on the mouse tail model of lymphedema. LYT-100 and LYT-101 is delivered for 6-12 weeks via b.i.d. oral gavage, in mice with ablated tail lymphatics via circumferential excision and ablation of collecting lymphatic trunks. Tail volume is measured weekly for all animals, starting pre-surgery and continuing through the 12-week interim sacrifice and 18-week study conclusion. At sacrifice, tails are harvested for histology and immunofluorescent imaging to characterize tissue changes with surgery and LYT-100 or LYT-101 treatment. Tail volume and markers of lymphatics, fibrosis, and inflammation are compared between LYT-100, LYT-101, and an active-surgery/control-drug group.

The effect of LYT-100 and LYT-101 is examined on tail volume in an experimental model of lymphedema, as measured weekly by the truncated cone method. And the effect of LYT-100 and LYT-101 on histological markers in an experimental model of lymphedema, including markers of lymphatics, fibrosis, and inflammation will also be evaluated.

**Animals:** 66 adult females (10-14 week old) C57BL/6 J mice, fed *ad libitum* in 12:12 light / temperature-controlled and pathogen-free facility. 10 animals per group, with allowance for 1 spare per/group. Oral gavage can be performed using 1.5-in., curved, 20-gauge, stainless steel feeding needles with a 2.25-mm ball dipped in 1 g/mL sucrose.

**Surgery:** The superficial and deep collecting lymphatics of the mid portion of the tail were excised using a 2-mm full-thickness skin and subcutaneous excision performed at a distance of 15 mm from the base of the tail. Lymphatic trunks (collecting lymphatics) adjacent to the lateral veins were identified and ablated through controlled, limited cautery application under a surgical microscope.

The dosing amounts, route and schedule are provided in **Table 11.**

| **Table 11** | | | | |
|---|---|---|---|---|
| Group | Test article | Test article preparation | Dosing | Dosing route and schedule |
| Group 1 | LYT-100 | Crystals ground into fine powder and suspended in 0.5% carboxymethycellulose (40mg/mL) | 250mg/kg/day | Oral gavage, twice daily |
| Group 2 | LYT-101 | Crystals ground into fine powder and suspended in 0.5% carboxymethycellulose (40mg/mL) | 250mg/kg/day | Oral gavage, twice daily |
| Group 3 | Control | 0.5% carboxymethycellulose | 10 mL/kg/day | Oral gavage, twice daily |
| Group 4 | LYT-100 | Crystals ground into fine powder and suspended in 0.5% carboxymethycellulose (40mg/mL) | 250mg/kg/day | Oral gavage, twice daily |
| Group 5 | LYT-101 | Crystals ground into fine powder and suspended in 0.5% carboxymethycellulose (40mg/mL) | 250mg/kg/day | Oral gavage, twice daily |
| Group 6 | Control | 0.5% carboxymethycellulose | 10 mL/kg/day | Oral gavage, twice daily |

Measurements are provided in **Table 12.**

| **Table 12: Measurements** | |
|---|---|
| Tail volume | Calculated with truncated cone formula (Sitzia 1995) and confirmed using histological measurements of soft tissue thickness of the skin/subcutaneous tissues was measured serially using digital images of histology slides stained with hematoxylin and eosin |
| Histology | Tissues fixed in 4% paraformaldehyde at 4° C., decalcified in 5% sodium EDTA (Santa Cruz Biotechnology, Dallas, Tex.), embedded in paraffin, and sectioned at 5 micrometers. Cut sections rehydrated and heat-mediated antigen unmasking performed using 90° C sodium citrate (Sigma-Aldrich). Non-specific binding blocked with 2% BSA/20% animal serum. Tissues incubated overnight with primary antibody at 4° C. Primary antibodies used for immunohistochemical stains include goat anti-mouse LYVE-1, rat anti-mouse CD45, rabbit anti-mouse CD4, Cy3-conjugated mouse anti-αSMA (from Sigma-Aldrich), rabbit anti-human IFN-γ, rabbit anti-mouse TGF-β1, rabbit anti-mouse p-SMAD3, rabbit anti-mouse collagen I (all from ABCAM, Cambridge, MA) |
| Immunofluorescence imaging | Immunofluorescence staining performed with AlexaFluor fluorophore-conjugated secondary antibodies (Life Technologies, Norwalk, CT). Images scanned using Mirax imaging software (Carl Zeiss). Peri-lymphatic CD45+ and CD4+ cell counts assessed by counting positively stained cells within 50 µm of the most inflamed lymphatic vessel in each quadrant of the leg. Positively stained cells counted by two blinded reviewers in four randomly-selected, 40× high-power fields in a minimum of 4 fields per animal. Collagen I deposition quantified using Metamorph software (Molecular Devices, Sunnyvale, CA) in dermal areas of 5 µm cross-sections. This analysis confirmed using picrosirius red staining (Polysciences, Warrington, PA) using manufacturer's instructions. Scar index quantified with Metamorph software by calculating the ratio of red-orange:green-yellow fibers with higher numbers representing increased scarring. |

Study procedure and timing are provided in **Table 13.**

| **Table 13: Study Details** | | |
|---|---|---|
| Time | Procedure | Notes |
| 0 weeks | Surgery | Lymphatic tail surgery |
| 6 weeks | Begin intervention (daily oral gavage) | |
| 12 weeks | Interim sacrifice groups | |
| 18 weeks | Late sacrifice groups | |
| | | |
| Weekly | Tail volume measurement | From pre-surgery |
| | Statistical Analysis | ANOVA |

Further aspects of the invention are described in the following lettered paragraphs:
A. A method of treating edema, comprising administering to a subject in need thereof an effective amount of deuterium-enriched pirfenidone having the structure: 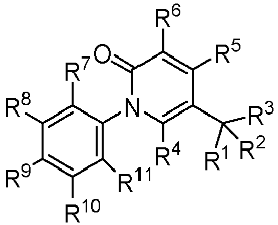 or a pharmaceutically acceptable salt thereof,
   wherein R¹, R², R³, R⁴, R⁵, R⁶, R⁷, R⁸, R⁹, R¹⁰, and R¹¹ are selected from hydrogen and deuterium; and
   at least one of R¹, R², R³, R⁴, R⁵, R⁶, R⁷, R⁸, R⁹ , R¹⁰, and R¹¹ is deuterium; and when R⁷, R⁸, R⁹, R¹⁰, and R¹¹ are deuterium, then at least one of R¹, R², R³, R⁴, R⁵, and R⁶ is deuterium; and
   wherein edema is treated in the subject.
B. The method of lettered paragraph A, wherein the deuterium-enriched pirfenidone has the structure: 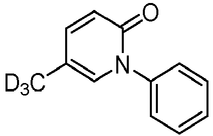 or a pharmaceutically acceptable salt thereof.
C. The method of lettered paragraph A or B, wherein the edema is lymphedema.
D. The method of lettered paragraph C, wherein the lymphedema is secondary lymphedema.
E. The method according to any of the preceding lettered paragraphs, wherein the subject has received treatment for cancer, and the lymphedema is associated with the cancer treatment or diagnosis.
F. The method according to any of the preceding lettered paragraphs, wherein the subject has breast cancer-related arm lymphedema.
G. The method according to any of the preceding lettered paragraphs, wherein the subject has mild to moderate breast cancer-related lymphedema.
H. The method according to any of the preceding lettered paragraphs, wherein the subject is receiving or has received chemotherapy or radiation therapy.
I. The method according to any of the preceding lettered paragraphs, wherein at least one of the positions represented as D independently has deuterium enrichment of no less than about 95%.
J. The method according to any of the preceding lettered paragraphs, wherein at least one of the positions represented as D independently has deuterium enrichment of no less than about 98%.
K. The method according to any of the preceding lettered paragraphs, wherein at least one of the positions represented as D independently has deuterium enrichment of no less than about 99%.
L. The method according to any of the preceding lettered paragraphs wherein the deuterium-enriched pirfenidone is administered orally at a total daily dose of 250 - 2500 mg.
M. The method of lettered paragraph L, wherein the deuterium-enriched pirfenidone is administered orally at a total daily dose of 500 - 1500 mg.
N. The method of lettered paragraph M, wherein the deuterium-enriched pirfenidone is administered orally at a total daily dose of 750 - 1000 mg.
O. The method of lettered paragraph M, wherein the deuterium-enriched pirfenidone is administered orally twice a day at a total daily dose of 1500 mg.
P. The method of lettered paragraph M, wherein the deuterium-enriched pirfenidone is administered orally twice a day at a total daily dose of 1000 mg.
Q. The method of lettered paragraph M, wherein the deuterium-enriched pirfenidone is administered orally twice a day at a total daily dose of 500 mg.
R. The method of lettered paragraph M, wherein the deuterium-enriched pirfenidone is administered orally once a day at a total daily dose of 1500 mg.
S. The method of lettered paragraph M, wherein the deuterium-enriched pirfenidone is administered orally once a day at a total daily dose of 1000 mg.
T. The method of lettered paragraph M, wherein the deuterium-enriched pirfenidone is administered orally once a day at a total daily dose of 750 mg.
U. The method of lettered paragraph M, wherein the deuterium-enriched pirfenidone is administered orally once a day at a total daily dose of 500 mg.
V. The method according to any of the preceding lettered paragraphs wherein the deuterium-enriched pirfenidone is administered with food.
W. The method according to any of the preceding lettered paragraphs wherein the deuterium-enriched pirfenidone is administered without food.
X. The method according to any of the preceding lettered paragraphs, wherein the deuterium-enriched pirfenidone is in tablet form.
Y. A method of treating interstitial lung disease (ILD), comprising administering to a subject in need thereof an effective amount of deuterium-enriched pirfenidone having the structure: 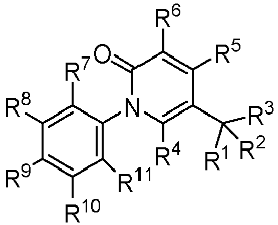 or a pharmaceutically acceptable salt thereof,
   wherein R¹, R², R³, R⁴, R⁵, R⁶, R⁷, R⁸, R⁹, R¹⁰, and R¹¹ are selected from hydrogen and deuterium; and
   at least one of R¹, R², R³, R⁴, R⁵, R⁶, R⁷, R⁸, R⁹ , R¹⁰, and R¹¹ is deuterium; and when R⁷, R⁸, R⁹, R¹⁰, and R¹¹ are deuterium, then at least one of R¹, R², R³, R⁴, R⁵, and R⁶ is deuterium; and
   wherein ILD is treated in the subject.
Z. The method of lettered paragraph R, wherein the deuterium-enriched pirfenidone has the structure: 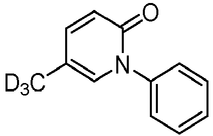 or a pharmaceutically acceptable salt thereof.
AA. The method of lettered paragraph Y or Z, wherein the ILD is idiopathic pulmonary fibrosis (IPF).
AB. The method according to any one of lettered paragraphs Y-AA, wherein at least one of the positions represented as D independently has deuterium enrichment of no less than about 95%.
AC. The method according to any one of lettered paragraphs Y-AB, wherein at least one of the positions represented as D independently has deuterium enrichment of no less than about 98%.
AD. The method according to any one of lettered paragraphs Y-AC, wherein at least one of the positions represented as D independently has deuterium enrichment of no less than about 99%.
AE. The method according to any one of lettered paragraphs Y-AD, wherein the deuterium-enriched pirfenidone is administered orally at a total daily dose of 250 - 2500 mg.
AF. The method according to any one of lettered paragraphs Y-AD, wherein the deuterium-enriched pirfenidone is administered orally at a total daily dose of 500 - 1500 mg.
AG. The method according to any one of lettered paragraphs Y-AD, wherein the deuterium-enriched pirfenidone is administered orally at a total daily dose of 750 - 1000 mg.
AH. The method according to any one of lettered paragraphs Y-AD, wherein the deuterium-enriched pirfenidone is administered orally at a total daily dose of 1500 mg.
AI. The method according to any one of lettered paragraphs Y-AD, wherein the deuterium-enriched pirfenidone is administered orally at a total daily dose of of 1000 mg.
AJ. The method according to any one of lettered paragraphs Y-AD, wherein the deuterium-enriched pirfenidone is administered orally at a total daily dose of 500 mg.
AK. The method according to any one of lettered paragraphs Y-AD, wherein the deuterium-enriched pirfenidone is administered orally at a total daily dose of 1500 mg.
AL. The method according to any one of lettered paragraphs Y-AD, wherein the deuterium-enriched pirfenidone is administered orally at a total daily dose of 1000 mg.
AM. The method according to any one of lettered paragraphs Y-AD, wherein the deuterium-enriched pirfenidone is administered orally at a total daily dose of 750 mg.
AN. The method according to any one of lettered paragraphs Y-AD, wherein the deuterium-enriched pirfenidone is administered orally at a total daily dose of 500 mg.
AO. The method according to any one of lettered paragraphs Y-AN, wherein the deuterium-enriched pirfenidone is administered with food.
AP. The method according to any one of lettered paragraphs Y-AN, wherein the deuterium-enriched pirfenidone is administered without food.
AQ. The method according to any one of lettered paragraphs Y-AP, wherein the deuterium-enriched pirfenidone is in tablet form.
AR. A method of treating a fibrotic or collagen infiltration disorder, comprising administering to a subject in need thereof an effective amount of deuterium-enriched pirfenidone having the structure: 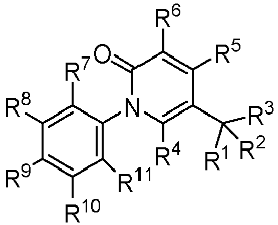 or a pharmaceutically acceptable salt thereof,
   wherein R¹, R², R³, R⁴, R⁵, R⁶, R⁷, R⁸, R⁹, R¹⁰, and R¹¹ are selected from hydrogen and deuterium; and
   at least one of R¹, R², R³, R⁴, R⁵, R⁶, R⁷, R⁸, R⁹ , R¹⁰, and R¹¹ is deuterium; and when R⁷, R⁸, R⁹, R¹⁰, and R¹¹ are deuterium, then at least one of R¹, R², R³, R⁴, R⁵, and R⁶ is deuterium; and
   wherein the fibrotic or collagen infiltration disorder is treated in the subject.
AS. The method of lettered paragraph X, wherein the deuterium-enriched pirfenidone has the structure: 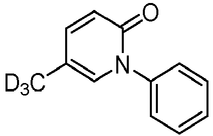 or a pharmaceutically acceptable salt thereof.
AT. The method according to any one of lettered paragraphs AR-AS, wherein at least one of the positions represented as D independently has deuterium enrichment of no less than about 95%.
AU. The method according to lettered paragraph AT, wherein at least one of the positions represented as D independently has deuterium enrichment of no less than about 98%.
AV. The method according to lettered paragraph AU, wherein at least one of the positions represented as D independently has deuterium enrichment of no less than about 99%.
AW. The method according to any one of lettered paragraphs AR-AV, wherein the deuterium-enriched pirfenidone is administered orally at a total daily dose of 250 - 2500 mg.
AX. The method according to any one of lettered paragraphs AR-AV, wherein the deuterium-enriched pirfenidone is administered orally at a total daily dose of 500 - 1500 mg.
AY. The method according to any one of lettered paragraphs AR-AV, wherein the deuterium-enriched pirfenidone is administered orally at a total daily dose of 750 - 1000 mg.
AZ. The method according to any one of lettered paragraphs AR-AV, wherein the deuterium-enriched pirfenidone is administered orally at a total daily dose of 1500 mg.
BA. The method according to any one of lettered paragraphs AR-AV, wherein the deuterium-enriched pirfenidone is administered orally at a total daily dose of 1000 mg.
BB. The method according to any one of lettered paragraphs AR-AV, wherein the deuterium-enriched pirfenidone is administered orally at a total daily dose of 500 mg.
BC. The method according to any one of lettered paragraphs AR-AV, wherein the deuterium-enriched pirfenidone is administered orally at a total daily dose of 1500 mg.
BD. The method according to any one of lettered paragraphs AR-AV, wherein the deuterium-enriched pirfenidone is administered orally at a total daily dose of 1000 mg.
BE. The method according to any one of lettered paragraphs AR-AV, wherein the deuterium-enriched pirfenidone is administered orally at a total daily dose of 750 mg.
BF. The method according to any one of lettered paragraphs AR-AV, wherein the deuterium-enriched pirfenidone is administered orally at a total daily dose of 500 mg.
BG. The method according to any one of lettered paragraphs AR-BF, wherein the deuterium-enriched pirfenidone is administered with food.
BH. The method according to any one of lettered paragraphs AR-BF, wherein the deuterium-enriched pirfenidone is administered without food.
BI. The method according to any one of lettered paragraphs AR-BH, wherein the deuterium-enriched pirfenidone is in tablet form.

## Claims

1. A deuterium-enriched pirfenidone for use in the treatment of an Interstitial Lung Disease (ILD) in a subject, the deuterium-enriched pirfenidone having the structure: 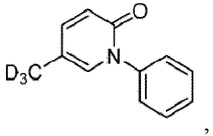 wherein the deuterium-enriched pirfenidone is for administration orally at a total daily dose from 100 mg to 2500 mg.

2. The deuterium-enriched pirfenidone of claim 1, wherein the deuterium-enriched pirfenidone is for administration orally at a total daily dose from 100 mg to 2000 mg.

3. The deuterium-enriched pirfenidone of claim 1, wherein the deuterium-enriched pirfenidone is for administration orally at a total daily dose from 1000 mg to 2000 mg.

4. The deuterium-enriched pirfenidone for use according to any one of claims 1-3, wherein the deuterium-enriched pirfenidone is for administration three times daily.

5. The deuterium-enriched pirfenidone for use according to any one of claims 1-3, wherein the deuterium-enriched pirfenidone is for administration twice daily.

6. The deuterium-enriched pirfenidone for use according to any one of claims 1 to 5, wherein the deuterium-enriched pirfenidone is for administration with food.

7. The deuterium-enriched pirfenidone for use according to any one of claims 1 to 5, wherein the deuterium-enriched pirfenidone is for administration without food.

8. The deuterium-enriched pirfenidone for use according to any one of claims 1 to 7, wherein the deuterium-enriched pirfenidone is in tablet form.

9. The deuterium-enriched pirfenidone for use according to any one of claims 1 to 8, wherein the ILD is childhood ILD (chILD).
